# EUROPEAN PATENT APPLICATION

(11) **EP 3 834 846 A1**
(43) Date of publication of application: **16.06.2021**
(21) Application number: 20209320.9
(22) Date of filing: 12.10.2018
(51) Int. Cl.: A61K 47/68, A61P 35/00, C07K 16/28

(54) **ANTI-CD71 ACTIVATABLE ANTIBODY DRUG CONJUGATES AND METHODS OF USE THEREOF**

(30) Priority: 14.10.2017 US 201762572467 P
(62) Divisional of application: 18797311.0
(71) Applicant: AbbVie Inc., North Chicago, IL 60064 (US)
(72) Inventor: SINGH, Shweta, South San Francisco, CA California 94080 (US); RICHARDSON, Jennifer Hope, South San Francisco, CA California 94080 (US); SERWER, Laura Patterson, South San Francisco, CA California 94080 (US); TERRETT, Jonathan Alexander, South San Francisco, CA California 94080 (US); MORGAN-LAPPE, Susan E., North Chicago, IL Illinois 60064 (US); HENRIQUES, Tracy, North Chicago, IL Illinois 60064 (US); RALSTON, Sherry L., North Chicago, IL Illinois 60064 (US); LEANNA, Marvin Robert, North Chicago, IL Illinois 60064 (US); BADAGNANI, Ilaria, North Chicago, IL Illinois 60064 (US); BOSE, Sahana, North Chicago, IL Illinois 60064 (US)
(74) Representative: Schlich, George

(57) **Abstract**

The invention relates generally to conjugated activatable antibodies that bind CD71 in their active form and methods of making and using these anti-CD71 conjugated activatable antibodies in a variety of therapeutic, diagnostic and prophylactic indications.

## Description

### CROSS-REFERENCE TO RELATED APPLICATIONS

This application claims the benefit of U.S. Provisional Application No. 62/572,467, filed October 14, 2017, the contents of which are incorporated herein by reference in its entirety.

### REFERENCE TO SEQUENCE LISTING

The "Sequence Listing" submitted electronically concurrently herewith pursuant to 37 C.F.R. § 1.821 in computer readable form (CFR) via EFS-Web as file name "CYTM056001US_120CT2018_FINAL_ST25.txt" is incorporated herein by reference. The electronic copy of the Sequence Listing was created on October 12, 2018, and the disk size is 96 kilobytes.

### FIELD OF THE INVENTION

The invention relates generally to activatable antibody drug conjugates (AADC) that bind CD71 in an activated state, and methods of making and using these anti-CD71 conjugated activatable antibodies in a variety of therapeutic, diagnostic and prophylactic indications.

### BACKGROUND OF THE INVENTION

Antibody-based therapies have proven effective treatments for several diseases but in some cases, toxicities due to broad target expression have limited their therapeutic effectiveness. In addition, antibody-based therapeutics have exhibited other limitations such as rapid clearance from the circulation following administration.

In the realm of small molecule therapeutics, strategies have been developed to provide prodrugs of an active chemical entity. Such prodrugs are administered in a relatively inactive (or significantly less active) form. Once administered, the prodrug is metabolized *in vivo* into the active compound. Such prodrug strategies can provide for increased selectivity of the drug for its intended target and for a reduction of adverse effects.

Accordingly, there is a continued need in the field of antibody-based therapeutics for antibodies that mimic the desirable characteristics of the small molecule prodrug.

### SUMMARY OF THE INVENTION

The disclosure provides conjugated activatable antibodies that specifically bind CD71, also known as transferrin receptor protein 1 (TfR1).

In an aspect of the invention, provided herein is a conjugated activatable antibody comprising the structure of Formula (I) or a salt thereof: 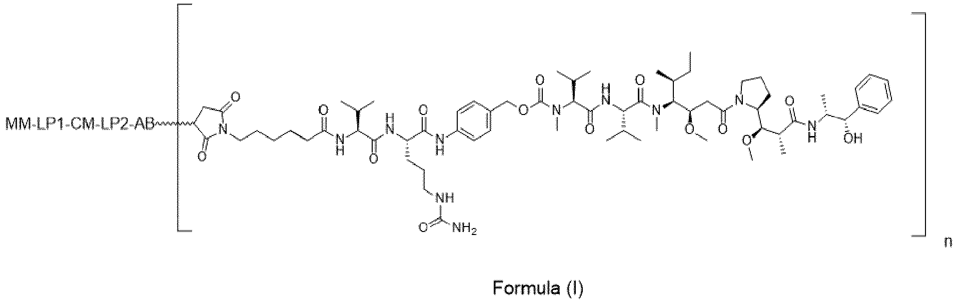 wherein (i) AB is an antibody that specifically binds to human CD71 and comprises a heavy chain variable region comprising a CDRH1 sequence comprising SEQ ID NO: 9, a CDRH2 sequence comprising SEQ ID NO: 10, and a CDRH3 sequence comprising SEQ ID NO: 11; and a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (I) or a salt thereof wherein (i) AB is an antibody that specifically binds to human CD71 and comprises a heavy chain variable region comprising a sequence of SEQ ID NO: 5 and a light chain variable region comprising a sequence of SEQ ID NO: 7; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (I) or a salt thereof wherein (i) AB is an antibody that specifically binds to human CD71 and comprises a heavy chain comprising a sequence of SEQ ID NO: 167 and a light chain comprising a sequence of SEQ ID NO: 19; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (I) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 169, and wherein "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (I) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 170 wherein "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In a related aspect of the invention, provided herein a conjugated activatable antibody comprising the structure of Formula (II) or a salt thereof: 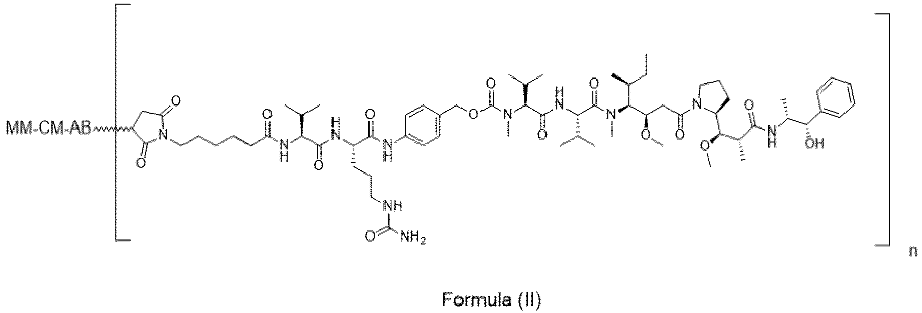 wherein (i) AB is an antibody that specifically binds to human CD71 and comprises a heavy chain variable region comprising a CDRH1 sequence comprising SEQ ID NO: 9, a CDRH2 sequence comprising SEQ ID NO: 10, and a CDRH3 sequence comprising SEQ ID NO: 11; and a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15; (ii) MM is a masking moiety comprising the amino acid sequence of SEQ ID NO: 18, wherein the MM inhibits the binding of the AB to human CD71 when the conjugated activatable antibody is in an uncleaved state; and (iii) CM is a cleavable moiety comprising the sequence of SEQ ID NO: 156, wherein the CM is a polypeptide that functions as a substrate for a protease; and (b) wherein "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (II) or a salt thereof wherein (i) AB is an antibody that specifically binds to human CD71 and comprises a heavy chain variable region comprising a sequence of SEQ ID NO: 5 and a light chain variable region comprising a sequence of SEQ ID NO: 7; (ii) MM is a masking moiety comprising the amino acid sequence of SEQ ID NO: 18, wherein the MM inhibits the binding of the AB to human CD71 when the conjugated activatable antibody is in an uncleaved state; and (iii) CM is a cleavable moiety comprising the sequence of SEQ ID NO: 156, wherein the CM is a polypeptide that functions as a substrate for a protease; and (b) wherein "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (II) or a salt thereof wherein (i) AB is an antibody that specifically binds to human CD71 and comprises a heavy chain comprising a sequence of SEQ ID NO: 167 and a light chain comprising a sequence of SEQ ID NO: 19; (ii) MM is a masking moiety comprising the amino acid sequence of SEQ ID NO: 18, wherein the MM inhibits the binding of the AB to human CD71 when the conjugated activatable antibody is in an uncleaved state; and (iii) CM is a cleavable moiety comprising the sequence of SEQ ID NO: 156, wherein the CM is a polypeptide that functions as a substrate for a protease; and (b) wherein "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (II) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 169, and wherein "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In a related aspect of the invention, provided herein is a conjugated activatable antibody comprising the structure of Formula (II) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 170, and wherein "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (I) or a salt thereof, wherein (i) AB is an antibody that specifically binds to human CD71 and comprises a heavy chain variable region comprising a CDRH1 sequence comprising SEQ ID NO: 9, a CDRH2 sequence comprising SEQ ID NO: 10, and a CDRH3 sequence comprising SEQ ID NO: 11; and a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (I) or a salt thereof, wherein (i) AB is an antibody that specifically binds to human CD71 and comprises a heavy chain variable region comprising a sequence of SEQ ID NO: 5 and a light chain variable region comprising a sequence of SEQ ID NO: 7; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (I) or a salt thereof, wherein (i) AB is an antibody that specifically binds to human CD71 and comprises a heavy chain comprising a sequence of SEQ ID NO: 167 and a light chain comprising a sequence of SEQ ID NO: 19; (ii) MM is a masking moiety comprising the amino acid sequence of SEQ ID NO: 18, wherein the MM inhibits the binding of the AB to human CD71 when the conjugated activatable antibody is in an uncleaved state; (iii) LP1 is a first linking moiety comprising the amino acid sequence of SEQ ID NO: 207; (iv) CM is a cleavable moiety comprising the sequence of SEQ ID NO: 156, wherein the CM is a polypeptide that functions as a substrate for a protease; and (v) LP2 is a second linking moiety comprising the amino acid sequence of SEQ ID NO: 38; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (I) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 169; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (I) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 170; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (II) or a salt thereof, wherein (i) AB is an antibody that specifically binds to human CD71 and comprises a heavy chain variable region comprising a CDRH1 sequence comprising SEQ ID NO: 9, a CDRH2 sequence comprising SEQ ID NO: 10, and a CDRH3 sequence comprising SEQ ID NO: 11; and a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15; (ii) MM is a masking moiety comprising the amino acid sequence of SEQ ID NO: 18, wherein the MM inhibits the binding of the AB to human CD71 when the conjugated activatable antibody is in an uncleaved state; (iii) CM is a cleavable moiety comprising the sequence of SEQ ID NO: 156, wherein the CM is a polypeptide that functions as a substrate for a protease; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (II) or a salt thereof, wherein (i) AB is an antibody that specifically binds to human CD71 and comprises a heavy chain variable region comprising a sequence of SEQ ID NO: 5 and a light chain variable region comprising a sequence of SEQ ID NO: 7; (ii) MM is a masking moiety comprising the amino acid sequence of SEQ ID NO: 18, wherein the MM inhibits the binding of the AB to human CD71 when the conjugated activatable antibody is in an uncleaved state; (iii) CM is a cleavable moiety comprising the sequence of SEQ ID NO: 156, wherein the CM is a polypeptide that functions as a substrate for a protease; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (II) or a salt thereof, wherein (i) AB is an antibody that specifically binds to human CD71 and comprises a heavy chain comprising a sequence of SEQ ID NO: 167 and a light chain comprising a sequence of SEQ ID NO: 19; (ii) MM is a masking moiety comprising the amino acid sequence of SEQ ID NO: 18, wherein the MM inhibits the binding of the AB to human CD71 when the conjugated activatable antibody is in an uncleaved state; (iii) CM is a cleavable moiety comprising the sequence of SEQ ID NO: 156, wherein the CM is a polypeptide that functions as a substrate for a protease; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (II) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 169; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are methods of manufacturing a conjugated activatable antibody comprising the structure of Formula (II) or a salt thereof wherein MM-LP1-CM-LP2-AB is an activatable antibody, wherein the AB is an antibody that specifically binds to human CD71, wherein the activatable antibody comprises a heavy chain comprising a sequence of SEQ ID NO: 167 and a light chain comprising a sequence of SEQ ID NO: 170; and (b) wherein "n" is 2; the method comprising (i) reducing an activatable antibody comprising MM-LP1-CM-LP2-AB with a reducing agent; and (ii) conjugating one or more vcMMAE to the reduced activatable antibody. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In another aspect of the invention, provided herein are pharmaceutical compositions comprising a conjugated activatable antibody of one iteration of Formula (I) or Formula (II). In some embodiments the pharmaceutical compositions may comprise a pharmaceutically acceptable carrier.

In another aspect of the invention, provided herein are methods of treating, alleviating a symptom of, or delaying the progression of a cancer in a subject, the method comprising administering a therapeutically effective amount of the conjugated activatable antibody of Formula (I) or Formula (II), or the pharmaceutical composition comprising the conjugated activatable antibody of one iteration of Formula (I) or Formula (II) and a optionally, a pharmaceutically acceptable carrier, to a subject in need thereof for a cancer selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.

In another aspect of the invention, provided herein is a conjugated activatable antibody comprising (a) an activatable antibody (AA) comprising in an uncleaved state the structural arrangement from N-terminus to C-terminus as follows: MM-CM-AB, wherein (i) AB is an antibody that specifically binds to mammalian CD71 and comprises the heavy chain variable region sequence of SEQ ID NO: 5 and the light chain variable region sequence of SEQ ID NO: 7; (ii) MM is a masking moiety comprising the amino acid sequence of SEQ ID NO: 18, wherein the MM coupled to the AB inhibits the binding of the AB to CD71 when the conjugated activatable antibody is in an uncleaved state; (iii) CM is a cleavable moiety comprising the sequence of SEQ ID NO: 156 coupled to the AB wherein the CM is a polypeptide that functions as a substrate for a protease; and (b) monomethyl auristatin E (MMAE), wherein the activatable antibody is conjugated to two equivalents of MMAE. In some embodiments, the conjugated activatable antibody wherein the AB is an antibody having a heavy chain constant region, and wherein the C-terminal residue of the heavy chain constant region is not a lysine. In some embodiments, the conjugated activatable antibody wherein the N-terminal glutamate on either the heavy chain and/or light chain is optionally either pyroglutamate or post-translationally modified to pyroglutamate.

In another aspect of the invention, provided herein is a conjugated activatable antibody having the formula AA-(AG)p wherein (a) AA is an activatable antibody comprising in an uncleaved state the structural arrangement from N-terminus to C-terminus as follows: MM-CM-AB, wherein (i) AB is an antibody that specifically binds to mammalian CD71 and comprises the heavy chain variable region sequence of SEQ ID NO: 5 and the light chain variable region sequence of SEQ ID NO: 7; (ii) MM is a masking moiety comprising the amino acid sequence of SEQ ID NO: 18, wherein the MM coupled to the AB inhibits the binding of the AB to CD71 when the conjugated activatable antibody is in an uncleaved state; (iii) CM is a cleavable moiety comprising the sequence of SEQ ID NO: 156 coupled to the AB wherein the CM is a polypeptide that functions as a substrate for a protease; and (b) AG is an agent conjugated to the AA, wherein the agent is MMAE and wherein p is 2. In some embodiments, the conjugated activatable antibody wherein the AB is an antibody having a heavy chain constant region, and wherein the C-terminal residue of the heavy chain constant region is not a lysine. In some embodiments, the conjugated activatable antibody wherein the N-terminal glutamate on either the heavy chain and/or light chain is optionally either pyroglutamate or post-translationally modified to pyroglutamate.

In another aspect of the invention, provided herein is a method of manufacturing a conjugated activatable antibody comprising (a) conjugating at least one MMAE to an activatable antibody (AA) thereby producing a composition comprising AA-(MMAE)p, wherein p is 1 to 8; and (b) enriching the composition for the conjugated activatable antibody species in which p is 2, wherein AA comprises in an uncleaved state comprises the structural arrangement from N-terminus to C-terminus as follows: MM-CM-AB wherein AB is an antibody that specifically binds to mammalian CD71 and comprises the heavy chain variable region sequence of SEQ ID NO: 5 and the light chain variable region sequence of SEQ ID NO: 7; (ii) MM is a masking moiety comprising the amino acid sequence of SEQ ID NO: 18, wherein the MM coupled to the AB inhibits the binding of the AB to CD71 when the conjugated activatable antibody is in an uncleaved state; (iii) CM is a cleavable moiety comprising the sequence of SEQ ID NO: 156 coupled to the AB wherein the CM is a polypeptide that functions as a substrate for a protease; and (b) AG is an agent conjugated to the AA, wherein the agent is MMAE and wherein p is 2. In some embodiments, the conjugated activatable antibody wherein the AB is an antibody having a heavy chain constant region, and wherein the C-terminal residue of the heavy chain constant region is not a lysine. In some embodiments, the conjugated activatable antibody wherein the N-terminal glutamate on either the heavy chain and/or light chain is optionally either pyroglutamate or post-translationally modified to pyroglutamate.

In another aspect of the invention, provided herein is any conjugated activatable antibody, or a pharmaceutical composition, as disclosed herein, for use as a medicament.

In another aspect of the invention, provided herein is any conjugated activatable antibody, or a pharmaceutical composition, as disclosed herein, for use in the treatment of cancer, optionally wherein the cancer is selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.

In another aspect of the invention, provided herein is a kit comprising at least one activatable antibody as disclosed herein. The kit may further comprise one or more vcMMAE, and/or a reducing agent.

### BRIEF DESCRIPTION OF THE DRAWINGS

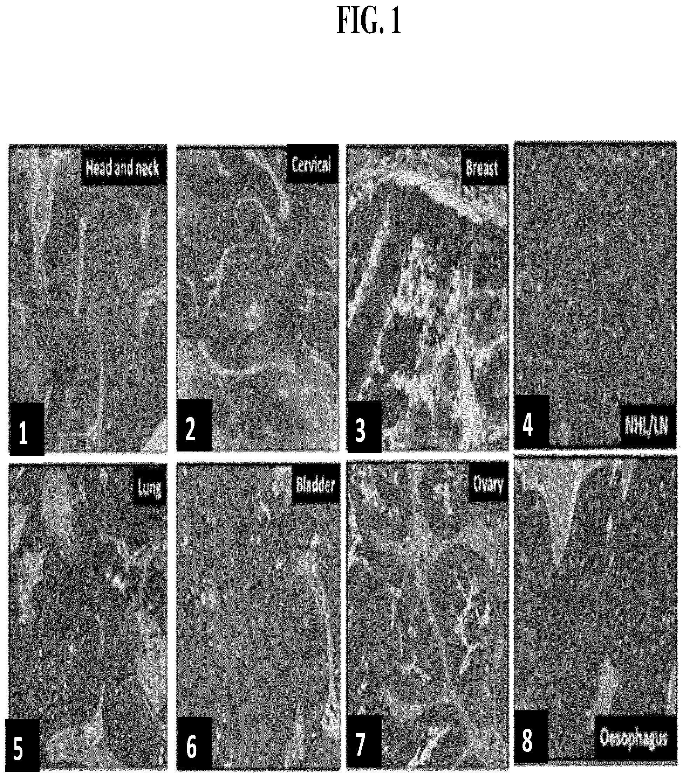

FIG. 1, as discussed in Example 5, depicts exemplary immunohistochemical (IHC) assays to determine levels of CD71 expression in various primary and metastatic cancer tissue types. The exemplary results shown in this figure and example showed that CD71 is expressed at high levels in primary tumors of a variety of human cancers.

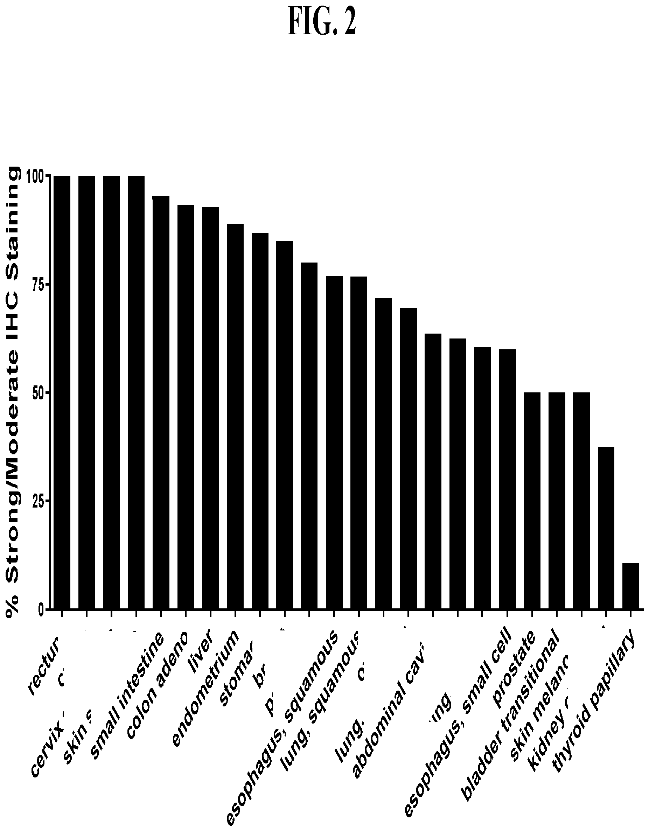

FIG. 2, as discussed in Example 5, depicts exemplary studies of the expression level of CD71 in multiple patient-derived metastatic cancer samples. The exemplary results shown in this figure and example showed that CD71 is expressed at high levels in metastatic tumors in a variety of human cancers.

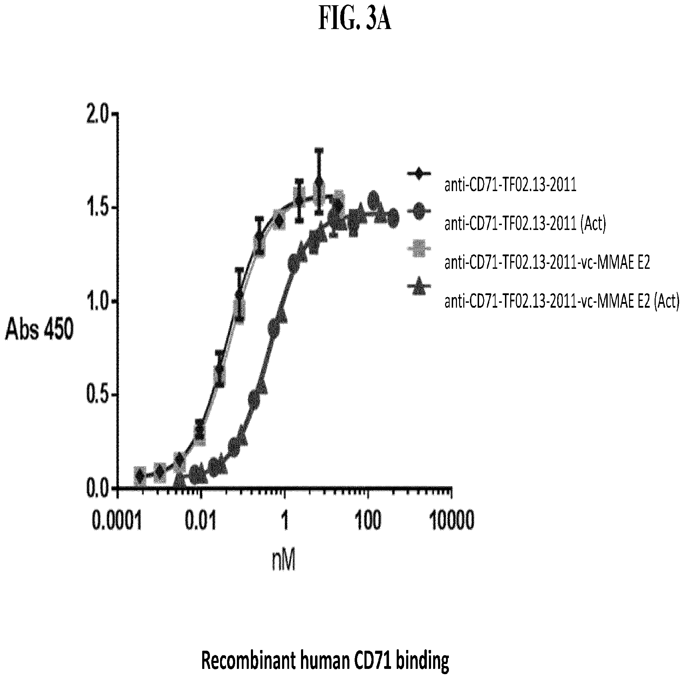

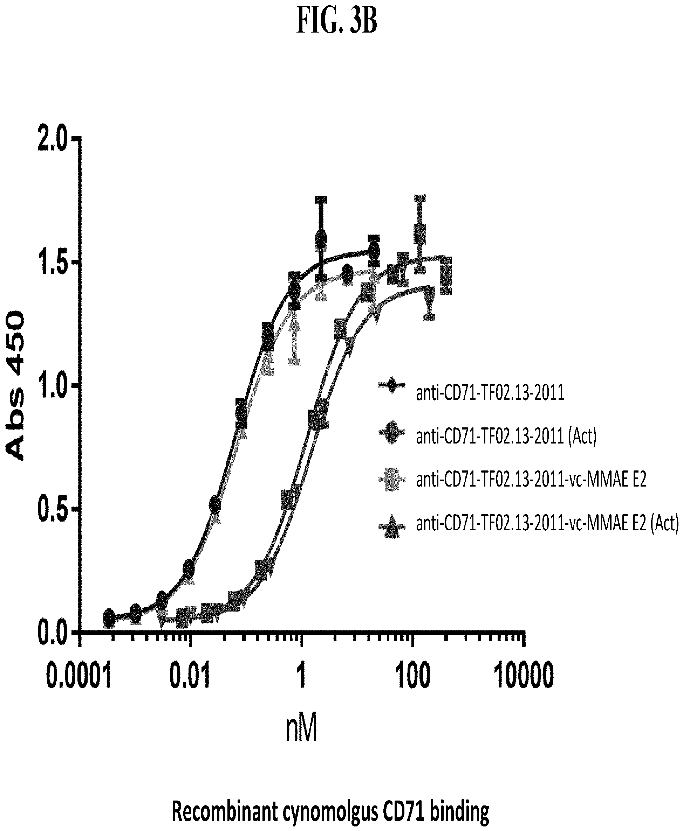

FIGS. 3A and 3B, as discussed in Example 4, depict an exemplary *in vitro* assay of the ability of unconjugated and conjugated anti-CD71 activatable antibodies of the disclosure to bind human or cynomolgus recombinant CD71 when the activatable antibody is intact or proteolytically activated (denoted as "ACT"). The exemplary results shown in these figures and example showed that the anti-CD71 conjugated activatable antibody bound to CD71 at levels comparable to its unconjugated anti-CD71 activatable antibody counterpart, and also both bound CD71 with an equivalent increased affinity upon protease activation.

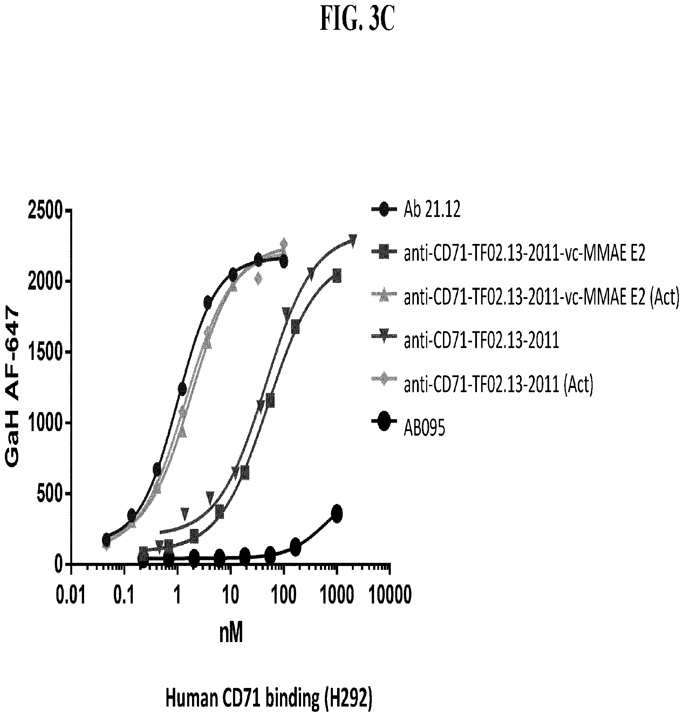

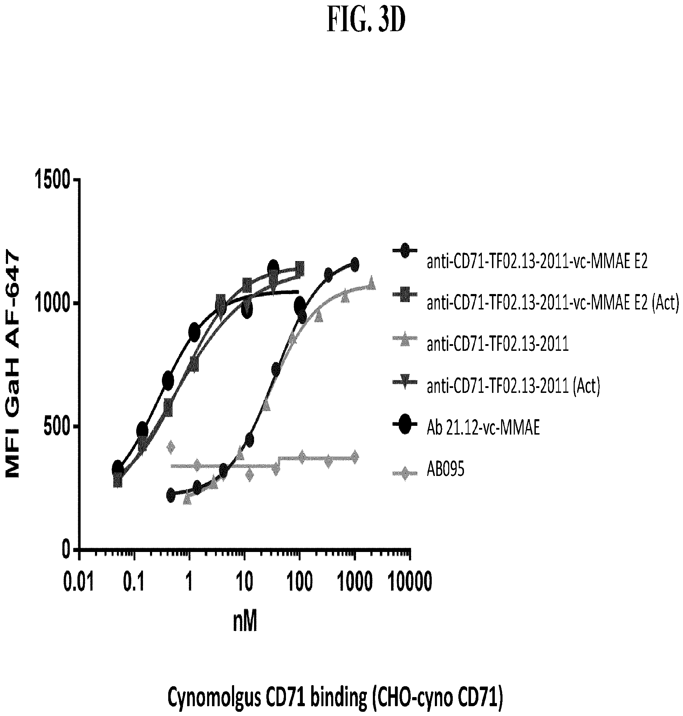

FIGS. 3C and 3D, as discussed in Example 4, depict an exemplary *in vitro* assay of the ability of unconjugated and conjugated anti-CD71 activatable antibodies of the disclosure to bind human or cynomolgus CD71 on a cell surface when the activatable antibody is intact or proteolytically activated (denoted as "ACT"). The exemplary results shown in these figures and example showed that the anti-CD71 conjugated activatable antibody bound to cell-surface CD71 at levels comparable to its unconjugated anti-CD71 activatable antibody counterpart, and also both bound cell-surface CD71 with an equivalent increased affinity upon protease activation.

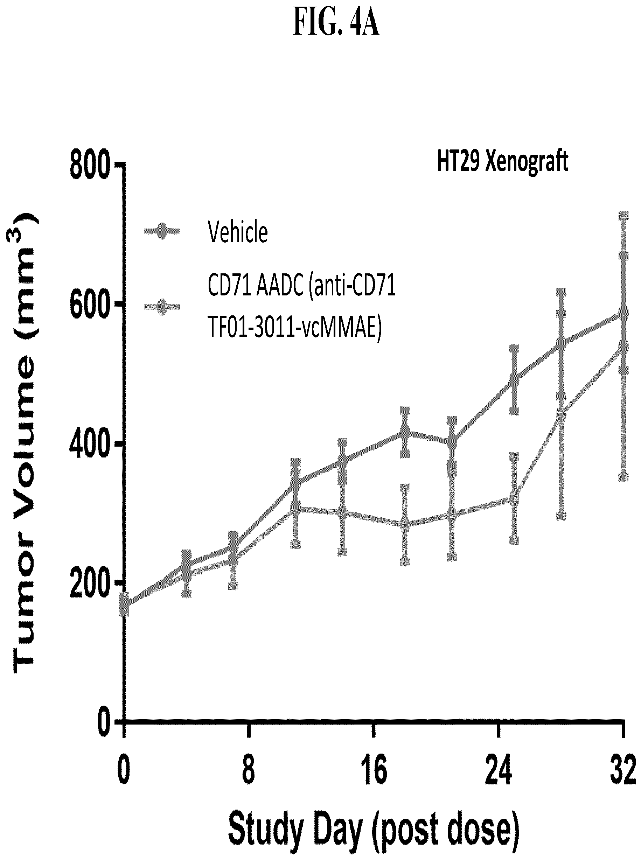

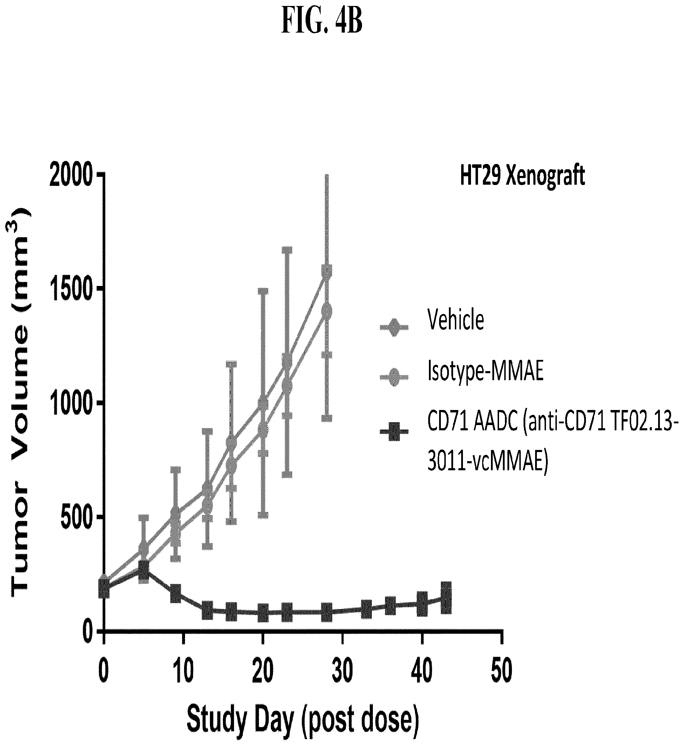

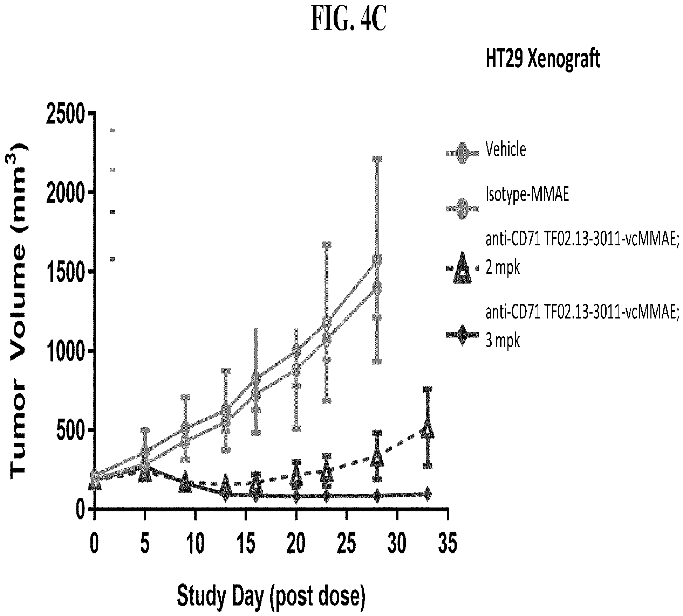

FIGS. 4A, 4B, and 4C, as discussed in Example 6, depict exemplary efficacy studies of anti-CD71 conjugated activatable antibodies (AADCs) of the present disclosure in a mouse xenograft model (anti-CD71 TF01-3011-MMAE vs. anti-CD71 TF02.13-2011-MMAE). These exemplary results shown in these figures and example that an AADC with the lower affinity masking moiety (TF02.13) demonstrated a higher efficacy than an AADC with a higher affinity masking moiety (TF01).

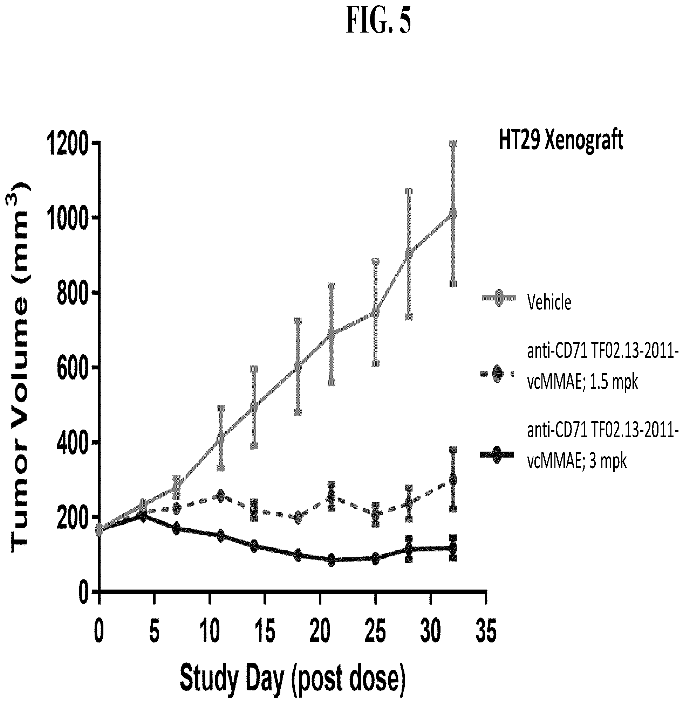

FIG. 5, as discussed in Example 7, depicts an exemplary efficacy study of anti-CD71 conjugated activatable antibodies (AADCs) of the present disclosure in a mouse xenograft model. The exemplary results shown in this figure and example demonstrate that the efficacy of the indicated AADC (anti-CD71 TF02.13-2011-vcMMAE) with a less cleavable substrate is substantially the same as the efficacy of the AADC (anti-CD71 TF02.13-3011-vcMMAE) with a more cleavable substrate shown in FIGS. 4B and 4C.

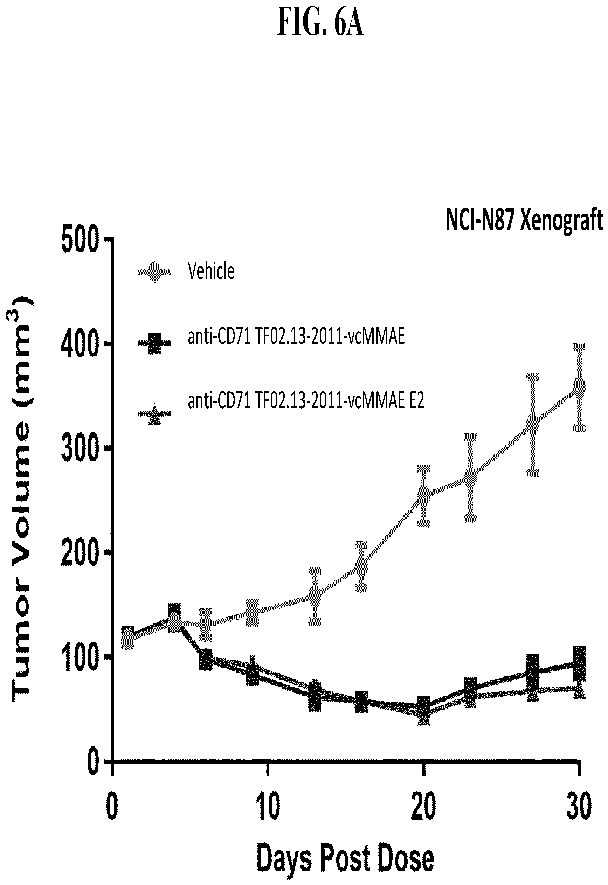

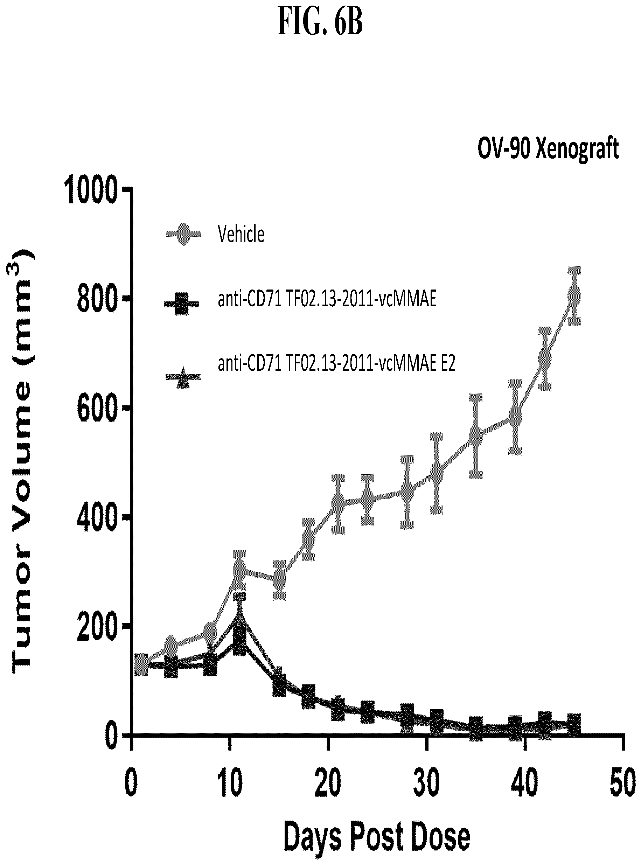

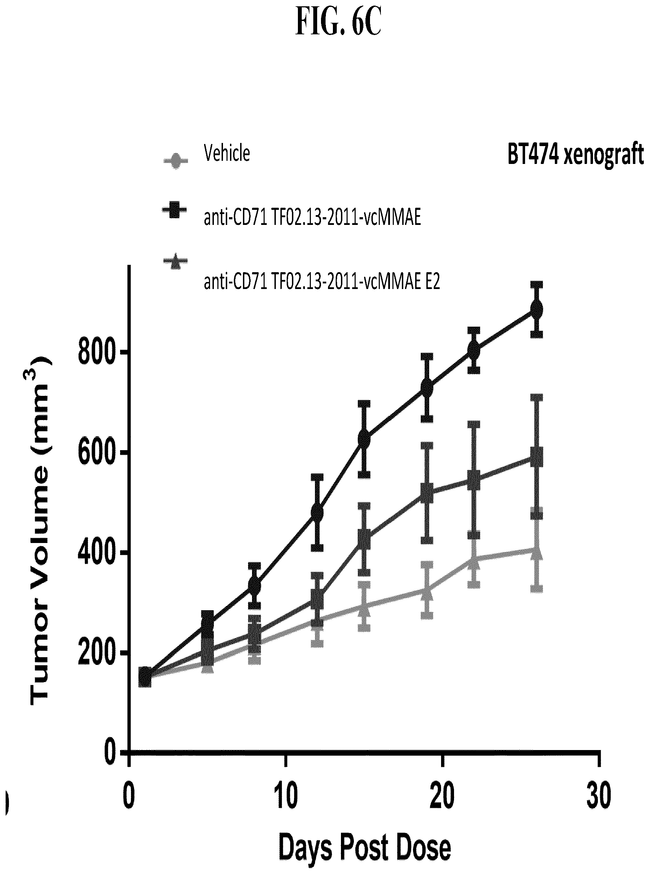

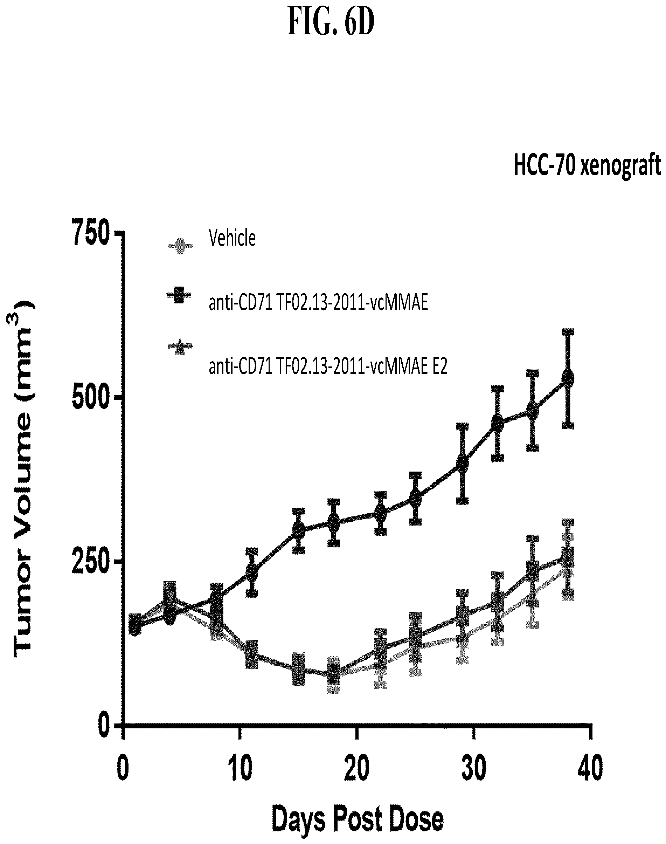

FIGS. 6A, 6B, 6C, and 6D, as discussed in Example 8, depict exemplary efficacy studies of anti-CD71 conjugated activatable antibodies (AADCs) with different DAR (anti-CD71 TF02.13-2011-vcMMAE with a DAR ∼3 vs. anti-CD71 TF02.13-2011-vcMMAE E2 with a DAR ∼2) of the present disclosure in a mouse xenograft model. The exemplary results shown in these figures and example demonstrate that the efficacy of dose-matched AADCs (anti-CD71 TF02.13-2011-vcMMAE) having different DARs showed comparable efficacy.

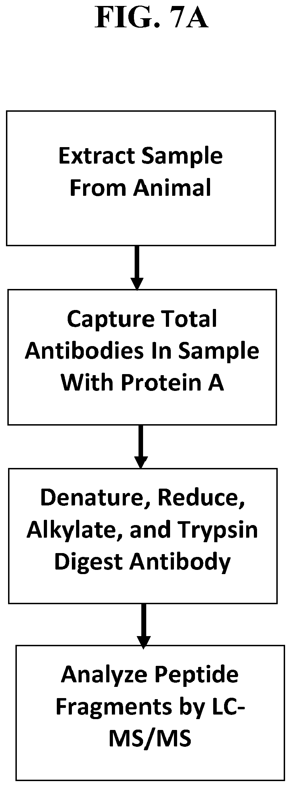

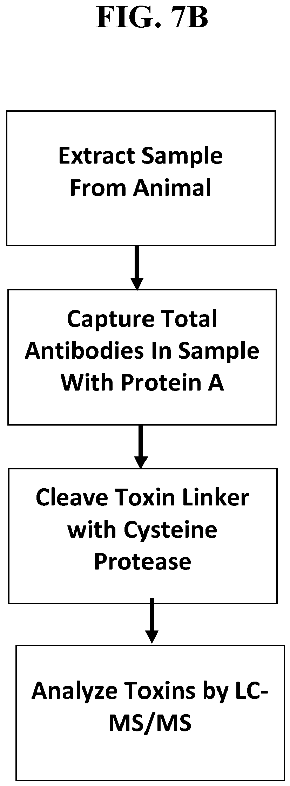

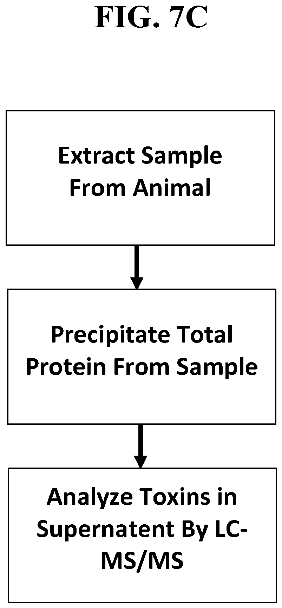

FIGS. 7A, 7B, and 7C, as discussed in Example 9, show exemplary schematic workflows for analyzing metabolic products resulting from administration of anti-CD71 conjugated activatable antibodies (AADCs) of the present disclosure.

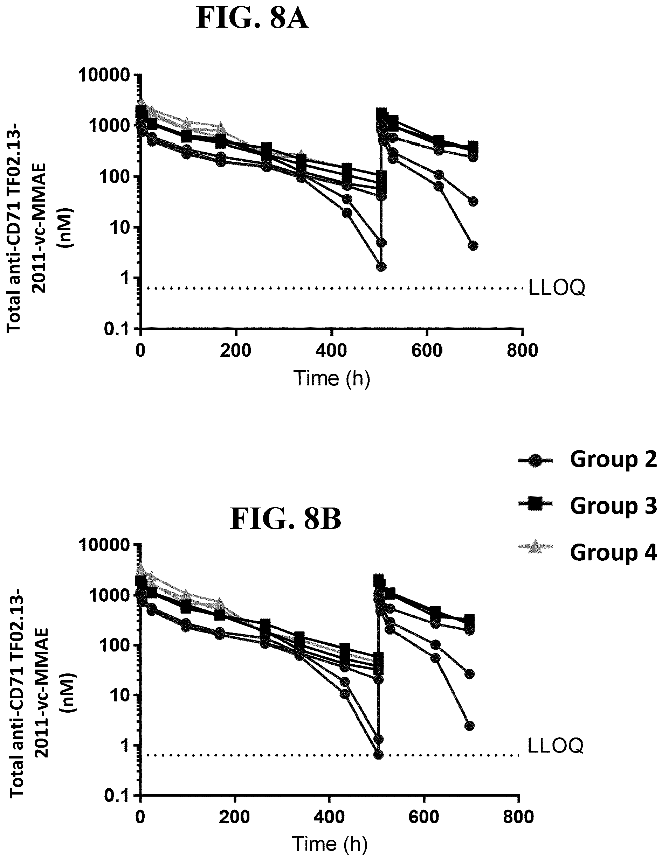

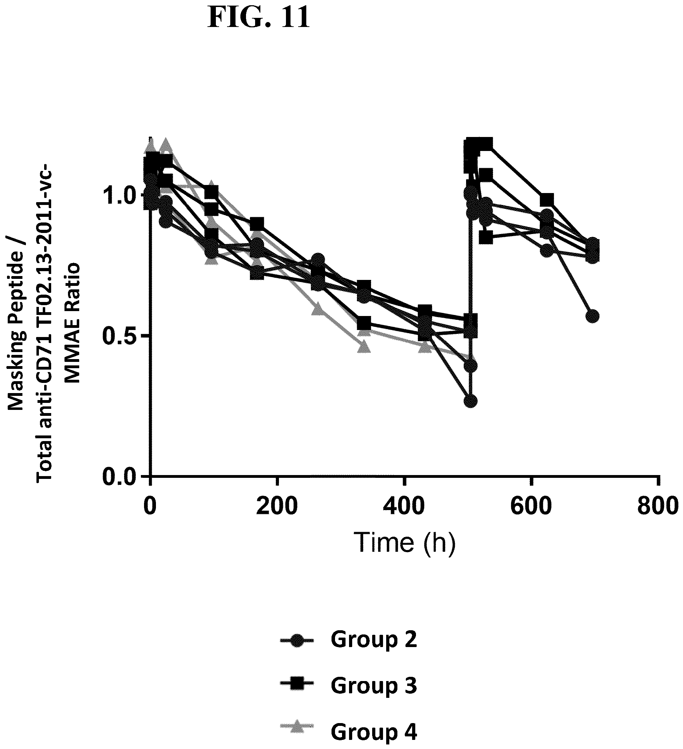

FIGS. 8A, 8B, 9A, 9B, 10, and 11, as discussed in Examples 11-14, depict exemplary time courses of metabolic by-products following administration of anti-CD71 conjugated activatable antibodies (AADCs) of the present disclosure in an animal model. The exemplary results shown in these figures and example demonstrate that the amount of total and intact AADC of the present disclosure (anti-CD71 TF02.13-2011-vcMMAE E2) is maintained in the animals throughout dosing in a dose-proportional amount, and the amount of MMAE that is conjugated to activatable antibody is substantially higher than the amount of unconjugated MMAE throughout dosing and at all dosing levels.

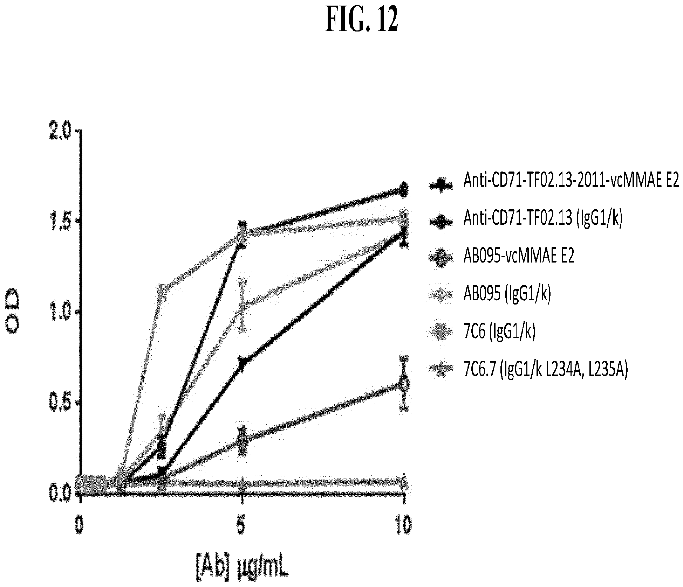

FIG. 12, as discussed in Example 17, depicts exemplary titration of the indicated test articles to iC3b protein fragment, representing their ability to activate the complement cascade. The exemplary results shown in this figure and example demonstrate that the AADC of the present disclosure (anti-CD71 TF02.13-2011-vcMMAE E2) demonstrated a lower ability for complement activation compared to its unconjugated activatable antibody.

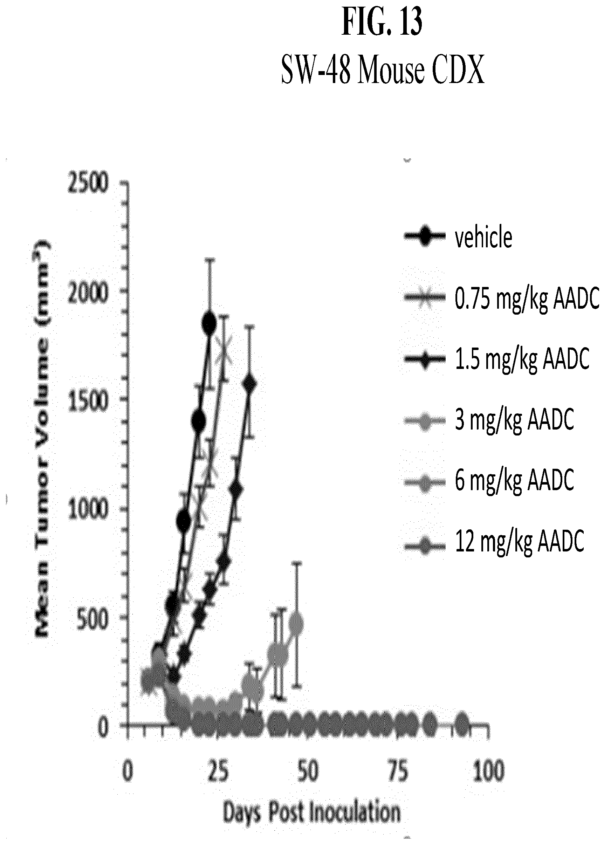

FIG. 13, as discussed in Example 19, depicts exemplary efficacy of the AADC of the present disclosure (anti-CD71 TF02.13-2011-vcMMAE E2) in a mouse xenograft model using human colorectal cell lines. The exemplary results shown in this figure and example demonstrate that the AADC of the present disclosure showed significant tumor growth inhibition at all dosages, with complete regression observed at the highest dosages.

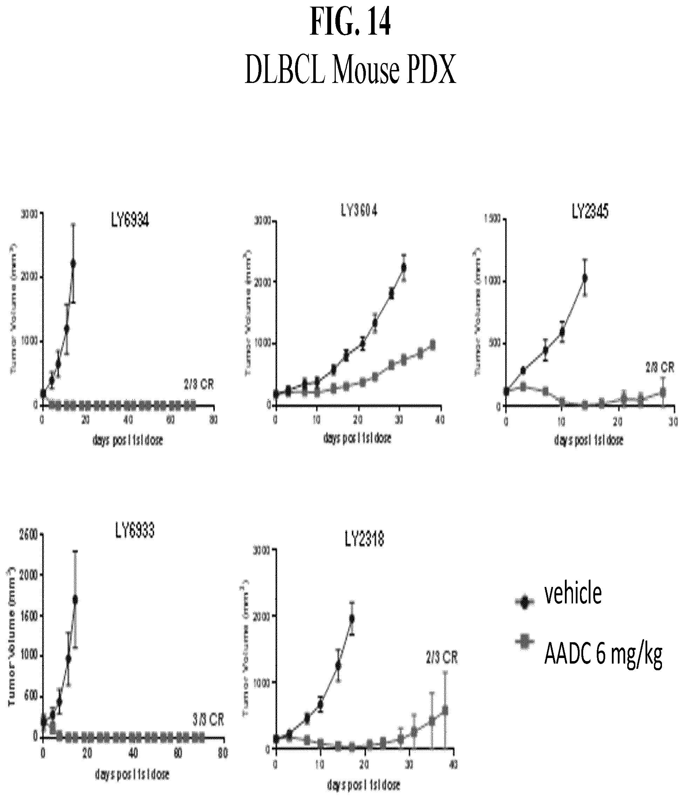

FIG. 14, as discussed in Example 20, depicts exemplary efficacy of the AADC of the present disclosure (anti-CD71 TF02.13-2011-vcMMAE E2) in a mouse xenograft model using human patient-derived tumors (DLBCL). The exemplary results shown in this figure and example demonstrate that the AADC of the present disclosure showed significant tumor growth inhibition after a single administration, with complete responses observed in some cases.

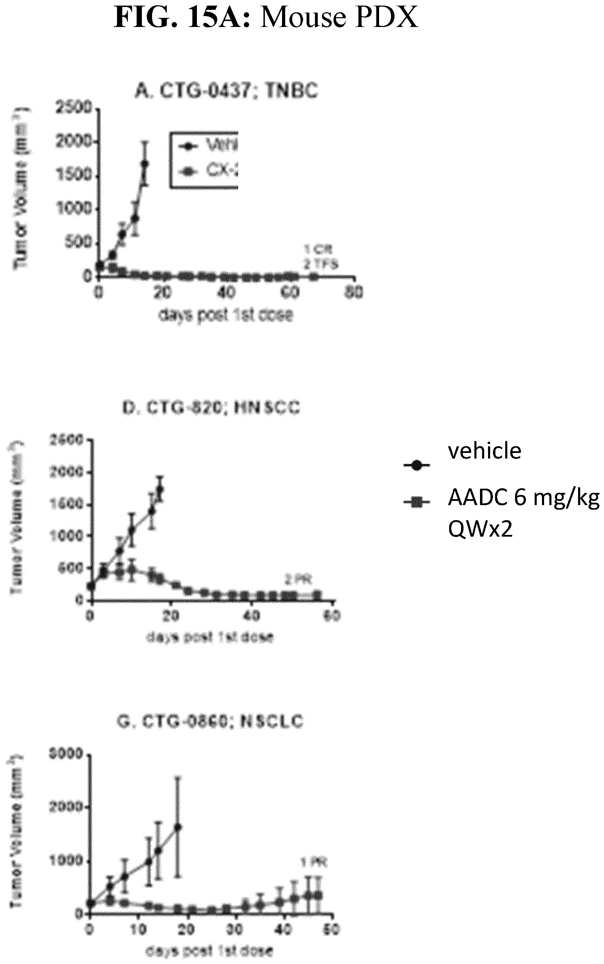

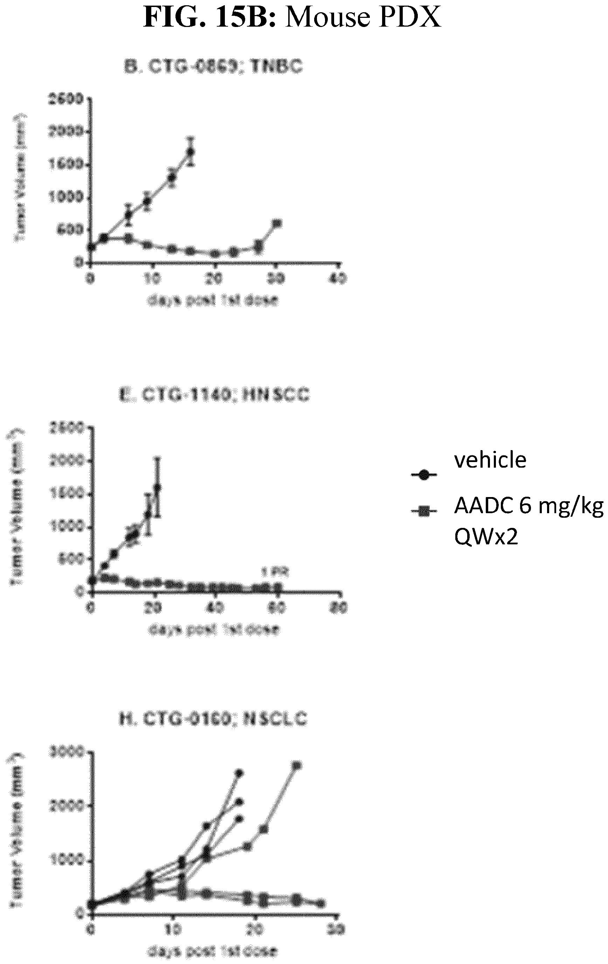

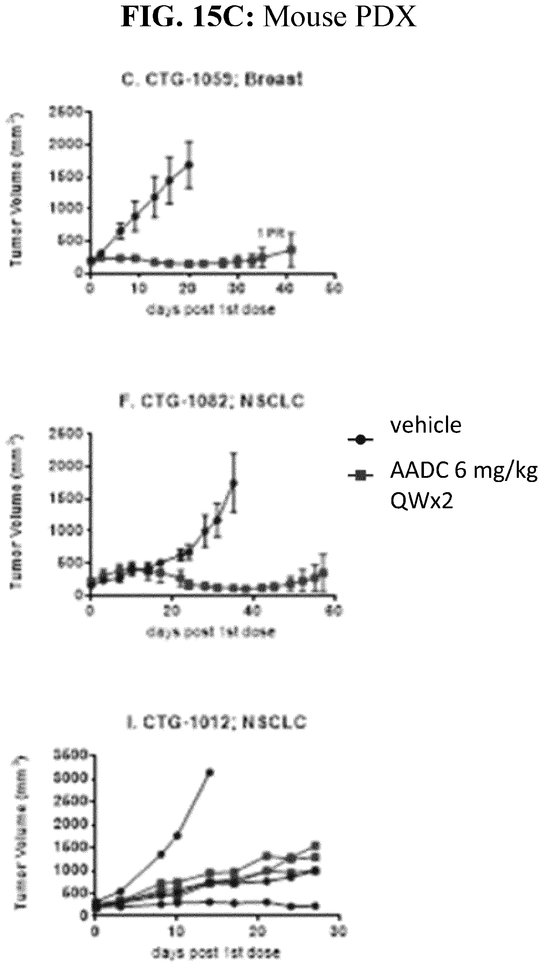

FIGS. 15A, 15B, and 15C, as discussed in Example 21, depicts exemplary efficacy of the AADC of the present disclosure (anti-CD71 TF02.13-2011-vcMMAE E2) in mouse patient-derived xenograft (PDX) models. The exemplary results shown in these figures and example demonstrate that the AADC of the present disclosure demonstrated efficacy, including complete responses in some cases, in PDX models derived from a variety of human cancer types.

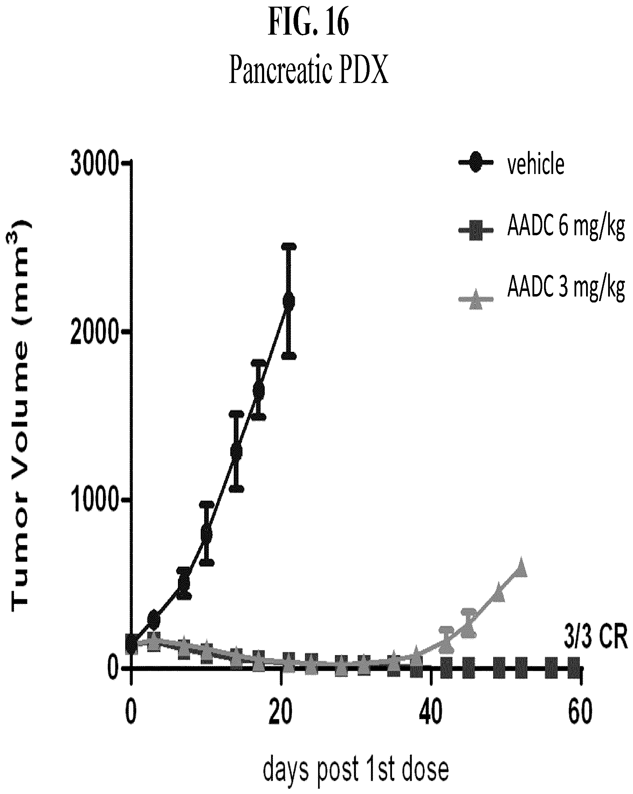

FIG. 16, as discussed in Example 22, depicts exemplary efficacy of the AADC of the present disclosure (anti-CD71 TF02.13-2011-vcMMAE E2) in a mouse patient-derived xenograft model. The exemplary results shown in this figure and example demonstrate that the AADC of the present disclosure demonstrated efficacy, including complete responses in some cases, to PDX pancreatic cancer model.

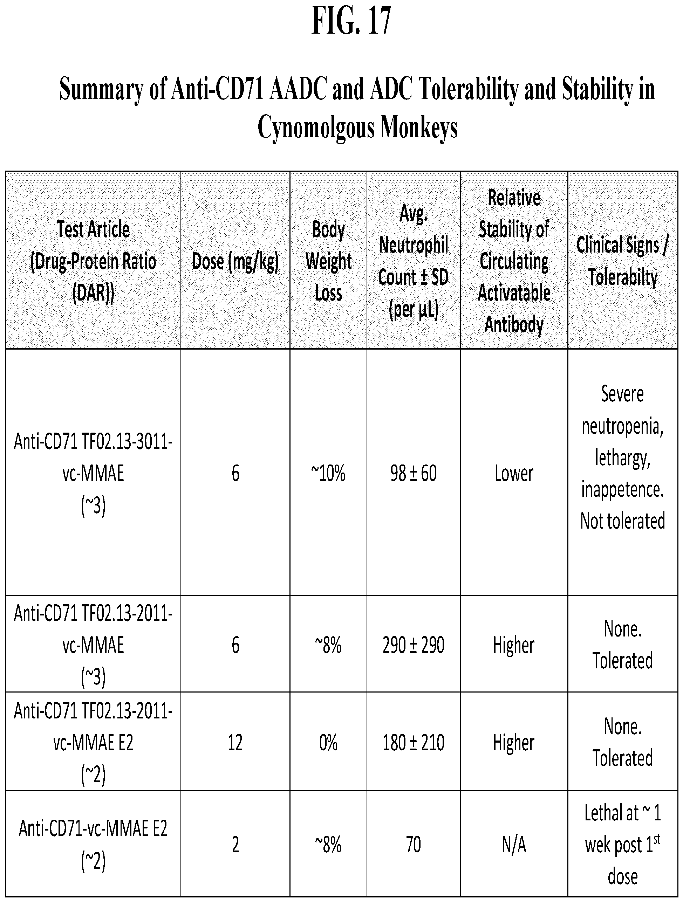

FIG. 17, as discussed in Example 10, summarizes the results that show anti-human CD71 activatable antibodies with conjugated toxins (AADCs) of the present disclosure (anti-CD71 TF02.13-2011-vcMMAE E2) are well-tolerated and stable in cynomolgus monkeys, even at higher relative doses, compared to the corresponding parental anti-CD71 antibody drug conjugate (ADCs), AADCs having a substrate with greater cleavability, and AADCs having a higher DAR

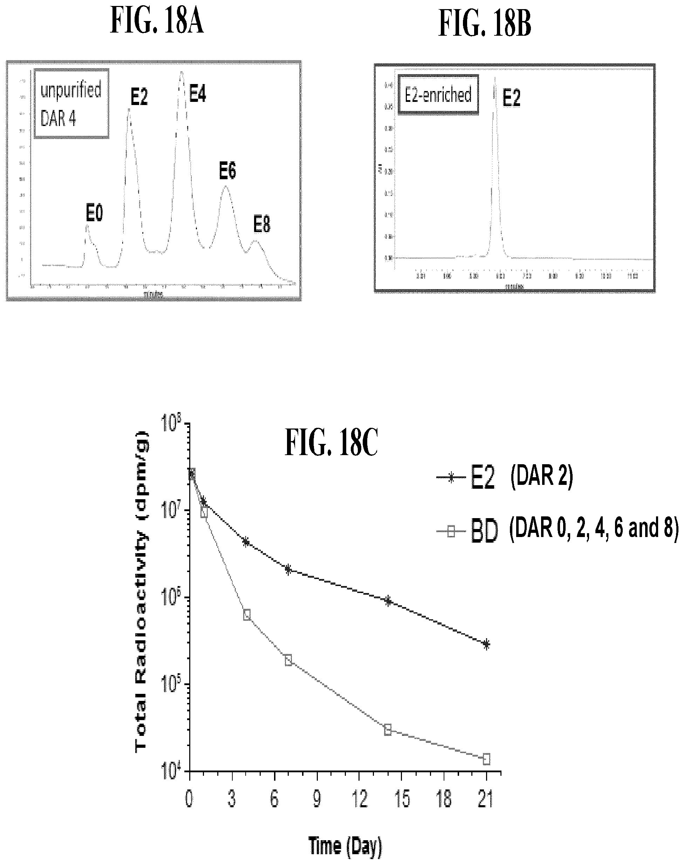

FIGS. 18A, 18B, and 18C, as discussed in Example 27, shows the HIC-separated profile of species present in unpurified anti-CD71-TF02.13-2011-vcMMAE and purified anti-CD71-TF02.13-2011-vcMMAE E2, as well as their rate of clearance in mice. These figures and example show that the anti-CD71-TF02.13-2011-vcMMAE E2 migrates as a single species of conjugate and has a lower clearance rate.

### DETAILED DESCRIPTION OF THE INVENTION

The present disclosure provides anti-CD71 conjugated activatable antibodies that specifically bind CD71 in an activated state. CD71 is also known as transferrin receptor protein 1 (TfR1). Generally, the present disclosure is directed to an anti-CD71 conjugated activatable antibody comprising a mask moiety, a cleavable moiety, a vc linker, and a MMAE toxin. The mask moiety (MM) reduces the ability of the antibody binding site to bind to its CD71 target antigen when the activatable antibody is in an uncleaved state; the cleavable moiety (CM) is a protease-activatable substrate that is cleaved in the tumor microenvironment resulting in removal of the mask moiety and concomitant activation of the anti-CD71 targeting CDRs. Specifically, the present disclosure describes the selection of an anti-CD71 conjugated activatable monoclonal antibody that has superior efficacy and tolerability to other anti-CD71 conjugated activatable monoclonal antibodies otherwise known in the art on account of it having the unique combination of a lower affinity mask moiety, a less cleavable moiety substrate, and a low drug-loading of 2 which collectively results in significantly improved efficacy and tolerability in mouse tumor models. In addition, the anti-CD71 conjugated activatable monoclonal antibody also surprisingly lacks the ability to bind to the inhibitory FcyRIIb receptor, unlike other antibodies containing a wild-type IgG1 Fc, which is important because binding to such receptors is known to inhibit calcium-dependent processes such as degranulation, phagocytosis, antibody dependent cell-mediated cytotoxicity (ADCC), cytokine release, and pro-inflammatory activation, all of which would be expected to result in decreased efficacy if the FcyRIIb receptor was activated.

CD71 is cell-surface receptor that is expressed on dividing cells, including cancer cells. It is expressed at high levels in a wide variety of cancer cell types, and CD71 is internalized, thus making it an attractive target for targeted cancer therapy using antibody-directed conjugated toxins. Masking moieties (MM) having higher and lower affinities, as well as cleavable moieties (CM) having lesser or greater cleavability, were identified and used to construct a variety of activatable antibodies and then vcMMAE conjugated antibodies (*i.e.* activatable antibody drug conjugates or AADC). Exemplary efficacy studies of these conjugated activatable antibodies in a mouse xenograft model showed that AADCs with higher affinity masking moieties (e.g., CD71-TF01-3011-vcMMAE) demonstrated lower efficacy than those with lower affinity masks (e.g., CD71-TF02.13-3011-vcMMAE). Moreover, other efficacy studies using AADCs having the same lower affinity mask but with substrates of different cleavability showed that while the efficacy was the same, in non-human primates the AADC with the less cleavable substrate (CD71-TF02.13-2011-vcMMAE) was better tolerated and with lower levels of circulating AADC in activated form as compared to the AADC with the more cleavable substrate (CD71-TF02.13-3011-vcMMAE), thus providing a higher therapeutic index. Further studies showed that AADCs having a lower drug-to-activatable antibody ratio (*e.g.*, CD71-TF02.13-3011-vcMMAE E2, where DAR is 2) was more tolerated at higher dosages as compared to the same conjugated activatable antibody, with higher DAR (i.e. DAR ∼ 3). Moreover, CD71-TF02.13-3011-vcMMAE E2 showed equivalent efficacy with dose-matched dosages to the higher DAR AADC in a variety of CDX and PDX mouse models of human cancers. Finally, CD71-TF02.13-3011-vcMMAE E2 showed significant tolerability to its unmasked counterpart, which was not tolerated at even low doses.

In some embodiments, the conjugated activatable monoclonal antibodies are internalized by CD71-containing cells. The use of the term "CD71" is intended to cover any variation thereof, such as, by way of non-limiting example, CD-71 and/or CD 71, and all variations are used herein interchangeably.

CD71 is a transmembrane glycoprotein that primarily binds transferrin. CD71 is essential for cell homeostasis. CD71 is continuously recycled through ligand-mediated endocytosis, where the main ligand is transferrin. CD71 is also known to be ubiquitously expressed on dividing cells.

Aberrant expression and/or activity of CD71 and CD71-related signaling have been implicated in the pathogenesis of many diseases and disorders, such as cancer. CD71 is overexpressed in many cancers, including both solid and hematological cancers. CD71 has broad cell surface expression. CD71 in malignant cells mediates higher iron uptake required for cell division. CD71 is also associated with poor prognosis in leukemias. CD71 is desirable target because it is prevalent across multiple cancer indications.

The disclosure provides conjugated activatable anti-CD71 antibodies that are useful in methods of treating, preventing, delaying the progression of, ameliorating and/or alleviating a symptom of a disease or disorder associated with aberrant CD71 expression and/or activity. For example, the activatable anti-CD71 antibodies are used in methods of treating, preventing, delaying the progression of, ameliorating and/or alleviating a symptom of a cancer or other neoplastic condition.

The disclosure provides anti conjugated activatable anti-CD71 antibodies that are useful in methods of treating, preventing, delaying the progression of, ameliorating and/or alleviating a symptom of a disease or disorder associated with cells expressing CD71. In some embodiments, the cells are associated with aberrant CD71 expression and/or activity. In some embodiments, the cells are associated with normal CD71 expression and/or activity. For example, the activatable anti-CD71 antibodies are used in methods of treating, preventing, delaying the progression of, ameliorating and/or alleviating a symptom of a cancer or other neoplastic condition.

The disclosure provides conjugated activatable anti-CD71 antibodies that are useful in methods of treating, preventing, delaying the progression of, ameliorating and/or alleviating a symptom of a disease or disorder in which diseased cells express CD71. In some embodiments, the diseased cells are associated with aberrant CD71 expression and/or activity. In some embodiments, the diseased cells are associated with normal CD71 expression and/or activity. For example, the activatable anti-CD71 antibodies are used in methods of treating, preventing, delaying the progression of, ameliorating and/or alleviating a symptom of a cancer or other neoplastic condition.

The conjugated activatable anti-CD71 antibodies include an antibody or antigen-binding fragment thereof that specifically binds CD71 coupled to a masking moiety (MM), such that coupling of the MM reduces the ability of the antibody or antigen-binding fragment thereof to bind CD71. In some embodiments, the MM is coupled via a sequence that includes a substrate for a protease, for example, a protease that is co-localized with CD71 at a treatment site in a subject.

Exemplary activatable anti-CD71 antibodies of the invention include, for example, activatable antibodies that include a heavy chain and a light chain that are, or are derived from, the heavy chain variable and light chain variable sequences shown below (CDR sequences are shown in bold and underline):

muM21 VH:

EVQLQESGTVLARPGASVKMSCKASGYTFTSYWMHWVKQRPGQGLEWIGAIYPGNSETGYNQNFKGKAKLTAV

TSASTAYMDLSSLTNEDSAVYYCTRENWDPGFAFWGQGTLITVSA (SEQ ID NO: 1)

muM21 VL:
DIVMTQTPAIMSASPGEKVTITCSASSSVYYMYWFQQKPGTSPKLWIYSTSNLASGVPVRFSGSGSGTSYSLT
ISRMEAEDAATYYCQQRRNYPYTFGGGTKLEIKRA (SEQ ID NO: 2)

hu2vHa variable heavy chain

QVQLVQSGAEVKKPGASVKVSCKASGYTFTSYWMHWVRQAPGQGLEWMGAIYPGNSETGYAQKFQGRVTMTRD

TSTSTVYMELSSLRSEDTAVYYCARENWDPGFAFWGQGTLVTVSS (SEQ ID NO: 3)

hu2vHb variable heavy chain

QVQLVQSGAEVKKPGASVKMSCKASGYTFTSYWMHWVRQAPGQGLEWIGAIYPGNSETGYAQKFQGRATLTAD

TSTSTAYMELSSLRSEDTAVYYCTRENWDPGFAFWGQGTLVTVSS (SEQ ID NO: 4)

hu2vHc variable heavy chain

QVQLVQSGAEVKKPGASVKMSCKASGYTFTSYWMHWVRQAPGQGLEWIGAIYPGNSETGYAQKFQGRATLTAD
TSTSTAYMELSSLRSEDTAVYYCTRENWDPGFAFWGQGTLITVSS (SEQ ID NO: 5)

hu21vKa variable light chain
DIQMTQSPSSLSASVGDRVTITCSASSSVYYMYWYQQKPGKAPKLLIYSTSNLASGVPSRFSGSGSGTDFTLT
ISSLQPEDFATYYCQQRRNYPYTFGQGTKLEIK (SEQ ID NO: 6)

hu21vKb variable light chain
DIQMTQSPSSLSASVGDRVTITCSASSSVYYMYWFQQKPGKAPKLWIYSTSNLASGVPSRFSGSGSGTDYTLT
ISSMQPEDFATYYCQQRRNYPYTFGQGTKLEIK (SEQ ID NO: 7)

hu21vKc variable light chain

DIQMTQSPSSLSASVGDRVTITCRASSSVYYMYWFQQKPGKAPKLWIYSTSNLASGVPSRFSGSGSGTDYTLT

ISSMQPEDFATYYCQQRRNYPYTFGQGTKLEIK (SEQ ID NO: 8)

Exemplary activatable anti-CD71 antibodies of the invention include, for example, activatable antibodies that include a combination of a variable heavy chain complementarity determining region 1 (VH CDR1, also referred to herein as CDRH1) sequence, a variable heavy chain complementarity determining region 2 (VH CDR2, also referred to herein as CDRH2) sequence, a variable heavy chain complementarity determining region 3 (VH CDR3, also referred to herein as CDRH3) sequence, a variable light chain complementarity determining region 1 (VL CDR1, also referred to herein as CDRL1) sequence, a variable light chain complementarity determining region 2 (VL CDR2, also referred to herein as CDRL2) sequence, and a variable light chain complementarity determining region 3 (VL CDR3, also referred to herein as CDRL3) sequence, wherein at least one CDR sequence is selected from the group consisting of a VH CDR1 sequence comprising the amino acid sequence GYTFTSYWMH (SEQ ID NO: 9); a VH CDR2 sequence comprising the amino acid sequence AIYPGNSETG (SEQ ID NO: 10); a VH CDR3 sequence comprising the amino acid sequence ENWDPGFAF (SEQ ID NO: 11); a VL CDR1 sequence comprising the amino acid sequence SASSSVYYMY (SEQ ID NO: 12) or CRASSSVYYMY (SEQ ID NO: 13); a VL CDR2 sequence comprising the amino acid sequence STSNLAS (SEQ ID NO: 14); and a VL CDR3 sequence comprising the amino acid sequence QQRRNYPYT (SEQ ID NO: 15).

In some embodiments, the antibody or antigen-binding fragment thereof of the conjugated activatable antibody of the present disclosure comprises a combination of a VH CDR1 sequence, a VH CDR2 sequence, a VH CDR3 sequence, a VL CDR1 sequence, a VL CDR2 sequence, and a VL CDR3 sequence, wherein at least one CDR sequence is selected from the group consisting of a VH CDR1 sequence that includes a sequence that is at least 90%, 91%, 92%, 93%, 94%, 95%, 96%, 97%, 98%, 99% or more identical to a VH CDR1 sequence comprising the amino acid sequence GYTFTSYWMH (SEQ ID NO: 9); a VH CDR2 sequence that includes a sequence that is at least 90%, 91%, 92%, 93%, 94%, 95%, 96%, 97%, 98%, 99% or more identical to a VH CDR2 sequence comprising the amino acid sequence AIYPGNSETG (SEQ ID NO: 10); a VH CDR3 sequence that includes a sequence that is at least 90%, 91%, 92%, 93%, 94%, 95%, 96%, 97%, 98%, 99% or more identical to a VH CDR3 sequence comprising the amino acid sequence ENWDPGFAF (SEQ ID NO: 11); a VL CDR1 sequence that includes a sequence that is at least 90%, 91%, 92%, 93%, 94%, 95%, 96%, 97%, 98%, 99% or more identical to a VL CDR1 sequence comprising the amino acid sequence ASSSVYYMY (SEQ ID NO: 12) or CRASSSVYYMY (SEQ ID NO: 13); a VL CDR2 sequence that includes a sequence that is at least 90%, 91%, 92%, 93%, 94%, 95%, 96%, 97%, 98%, 99% or more identical to a VL CDR2 sequence comprising the amino acid sequence STSNLAS (SEQ ID NO: 14; and a VL CDR3 sequence that includes a sequence that is at least 90%, 91%, 92%, 93%, 94%, 95%, 96%, 97%, 98%, 99% or more identical to a VL CDR3 sequence comprising the amino acid sequence QQRRNYPYT (SEQ ID NO: 15).

In some embodiments, the antibody or antigen-binding fragment thereof of the conjugated activatable antibody of the present disclosure comprises a combination of a VH CDR1 sequence, a VH CDR2 sequence, a VH CDR3 sequence, a VL CDR1 sequence, a VL CDR2 sequence, and a VL CDR3 sequence, wherein the VH CDR1 sequence comprises the amino acid sequence GYTFTSYWMH (SEQ ID NO: 9); the VH CDR2 sequence comprises the amino acid sequence AIYPGNSETG (SEQ ID NO: 10); the VH CDR3 sequence comprises the amino acid sequence ENWDPGFAF (SEQ ID NO: 11); the VL CDR1 sequence comprises the amino acid sequence SASSSVYYMY (SEQ ID NO: 12) or CRASSSVYYMY (SEQ ID NO: 13); the VL CDR2 sequence comprises the amino acid sequence STSNLAS (SEQ ID NO: 14); and the VL CDR3 sequence comprises the amino acid sequence QQRRNYPYT (SEQ ID NO: 15).

In some embodiments, the antibody or antigen-binding fragment thereof of the conjugated activatable antibody of the present disclosure comprises a combination of a VH CDR1 sequence, a VH CDR2 sequence, a VH CDR3 sequence, a VL CDR1 sequence, a VL CDR2 sequence, and a VL CDR3 sequence, wherein the VH CDR1 sequence comprises a sequence that is at least 90%, 91%, 92%, 93%, 94%, 95%, 96%, 97%, 98%, 99% or more identical to the amino acid sequence GYTFTSYWMH (SEQ ID NO: 9); the VH CDR2 sequence comprises a sequence that is at least 90%, 91%, 92%, 93%, 94%, 95%, 96%, 97%, 98%, 99% or more identical to the amino acid sequence AIYPGNSETG (SEQ ID NO: 10); the VH CDR3 sequence comprises a sequence that is at least 90%, 91%, 92%, 93%, 94%, 95%, 96%, 97%, 98%, 99% or more identical to the amino acid sequence ENWDPGFAF (SEQ ID NO: 11); the VL CDR1 sequence comprises a sequence that is at least 90%, 91%, 92%, 93%, 94%, 95%, 96%, 97%, 98%, 99% or more identical to the amino acid sequence SASSSVYYMY (SEQ ID NO: 12) or CRASSSVYYMY (SEQ ID NO: 13); the VL CDR2 sequence comprises a sequence that is at least 90%, 91%, 92%, 93%, 94%, 95%, 96%, 97%, 98%, 99% or more identical to the amino acid sequence STSNLAS (SEQ ID NO: 14); and the VL CDR3 sequence a sequence that is at least 90%, 91%, 92%, 93%, 94%, 95%, 96%, 97%, 98%, 99% or more identical to comprises the amino acid sequence QQRRNYPYT (SEQ ID NO: 15).

In some embodiments, the antibody of the conjugated activatable antibody of the present disclosure includes an antibody that specifically binds CD71. In some embodiments, the conjugated activatable antibody includes a monoclonal antibody that binds CD71. In some embodiments, such a monoclonal antibody that binds CD71 is a humanized or fully human monoclonal antibody.

In some embodiments, the antibody or antigen-binding fragment thereof of the conjugated activatable antibody of the present disclosure comprises a heavy chain variable region amino acid sequence comprising SEQ ID NO: 5. In some embodiments, the antibody or antigen-binding fragment thereof of the conjugated activatable antibody of the present disclosure comprises a light chain variable region amino acid sequence comprising SEQ ID NO: 7. In some embodiments, the antibody or antigen-binding fragment thereof of the conjugated activatable antibody of the present disclosure comprises a heavy chain variable region amino acid sequence comprising SEQ ID NO: 5, and a light chain variable region amino acid sequence or antigen-binding fragment thereof comprising SEQ ID NO: 7.

In some embodiments, the antibody or antigen-binding fragment thereof of the conjugated activatable antibody of the present disclosure comprises a heavy chain variable region amino acid sequence that is at least 90%, 91%, 92%, 93%, 94%, 95%, 96%, 97%, 98% or 99% identical to an amino acid sequence comprising SEQ ID NO: 5. In some embodiments, the antibody or antigen-binding fragment thereof of the conjugated activatable antibody of the present disclosure comprises a light chain variable region amino acid sequence that is at least 90%, 91%, 92%, 93%, 94%, 95%, 96%, 97%, 98% or 99% identical to an amino acid sequence comprising SEQ ID NO: 7. In some embodiments, the antibody or antigen-binding fragment thereof of the conjugated activatable antibody of the present disclosure comprises a heavy chain variable region amino acid sequence that is at least 90%, 91%, 92%, 93%, 94%, 95%, 96%, 97%, 98% or 99% identical to an amino acid sequence comprising SEQ ID NO: 1 and 3-5, and a light chain variable region amino acid sequence that is at least 90%, 91%, 92%, 93%, 94%, 95%, 96%, 97%, 98% or 99% identical to an amino acid sequence comprising SEQ ID NO: 7.

In some embodiments, the antibody or antigen-binding fragment thereof of the conjugated activatable antibody of the present disclosure is encoded by a nucleic acid sequence that comprises a nucleic acid sequence encoding a heavy chain amino acid sequence comprising an amino acid sequence comprising SEQ ID NO: 5. In some embodiments, the antibody or antigen-binding fragment thereof of the conjugated activatable antibody of the present disclosure is encoded by a nucleic acid sequence that comprises a nucleic acid sequence encoding a light chain amino acid sequence comprising an amino acid sequence comprising SEQ ID NO: 7. In some embodiments, the antibody or antigen-binding fragment thereof of the conjugated activatable antibody of the present disclosure is encoded by a nucleic acid sequence that comprises a nucleic acid sequence encoding a heavy chain amino acid sequence comprising an amino acid sequence comprising SEQ ID NO: 5, and a nucleic acid sequence encoding a light chain amino acid sequence comprising an amino acid sequence comprising SEQ ID NO: 7.

In some embodiments, the antibody or antigen-binding fragment thereof of the conjugated activatable antibody of the present disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a heavy chain amino acid sequence comprising an amino acid sequence comprising SEQ ID NO: 5. In some embodiments, the antibody or antigen-binding fragment thereof of the conjugated activatable antibody of the present disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a light chain amino acid sequence comprising an amino acid sequence comprising SEQ ID NO: 7. In some embodiments, the antibody or antigen-binding fragment thereof of the conjugated activatable antibody of the present disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a heavy chain amino acid sequence comprising an amino acid sequence comprising SEQ ID NO: 5, and a nucleic acid sequence that is at least 90%, 91%, 92%, 93%, 94%, 95%, 96%, 97%, 98% or 99% identical to a nucleic acid sequence encoding a light chain amino acid sequence comprising an amino acid sequence comprising SEQ ID NO: 7.

The disclosure also provides methods for producing an activatable antibody of a conjugated activatable antibody of the disclosure by culturing a cell under conditions that lead to expression of the activatable antibody, wherein the cell comprises a nucleic acid molecule of the disclosure or a vector of the disclosure.

The disclosure also provides conjugated activatable antibodies that include an antibody or antigen-binding fragment thereof that specifically binds CD71 coupled to a masking moiety (MM), such that coupling of the MM reduces the ability of the antibody or antigen-binding fragment thereof to bind CD71. In some embodiments, the MM is coupled via a sequence that includes a substrate for a protease, for example, a protease that is active in diseased tissue and/or a protease that is co-localized with CD71 at a treatment site in a subject. The conjugated activatable anti-CD71 antibodies provided herein, also referred to herein interchangeably as anti-CD71 conjugated activatable antibodies or CD71 conjugated activatable antibodies, are stable in circulation, activated at intended sites of therapy and/or diagnosis but not in normal, e.g., healthy tissue or other tissue not targeted for treatment and/or diagnosis, and, when activated, exhibit binding to CD71 that is at least comparable to the corresponding, unmodified antibody, also referred to herein as the parental antibody.

The invention also provides methods of treating, preventing and/or delaying the onset or progression of, or alleviating a symptom associated with aberrant expression and/or activity of CD71 in a subject using conjugated activatable antibodies that bind CD71, particularly conjugated activatable antibodies that bind and neutralize or otherwise inhibit at least one biological activity of CD71 and/or CD71-mediated signaling.

The invention also provides methods of treating, preventing and/or delaying the onset or progression of, or alleviating a symptom associated with the presence, growth, proliferation, metastasis, and/or activity of cells which are expressing CD71 or aberrantly expressing CD71 in a subject using conjugated activatable antibodies that bind CD71, particularly activatable antibodies that bind, target, neutralize, kill, or otherwise inhibit at least one biological activity of cells which are expressing or aberrantly expressing CD71.

The invention also provides methods of treating, preventing and/or delaying the onset or progression of, or alleviating a symptom associated with the presence, growth, proliferation, metastasis, and/or activity of cells which are expressing CD71 in a subject using conjugated activatable antibodies that bind CD71, particularly activatable antibodies that bind, target, neutralize, kill, or otherwise inhibit at least one biological activity of cells which are expressing CD71.

The invention also provides methods of treating, preventing and/or delaying the onset or progression of, or alleviating a symptom associated with the presence, growth, proliferation, metastasis, and/or activity of cells which are aberrantly expressing CD71 in a subject using conjugated activatable antibodies that bind CD71, particularly conjugated activatable antibodies that bind, target, neutralize, kill, or otherwise inhibit at least one biological activity of cells which are aberrantly expressing CD71.

The conjugated activatable antibodies in an activated state bind CD71 and include (i) an antibody (AB) that specifically binds to CD71; (ii) a masking moiety (MM) that, when the activatable antibody is in an uncleaved state, inhibits the binding of the AB to CD71; and (c) a cleavable moiety (CM) coupled to the AB, wherein the CM is a polypeptide that functions as a substrate for a protease.

In some embodiments, the conjugated activatable antibody in the uncleaved state has the structural arrangement from N-terminus to C-terminus as follows: MM-CM-AB or AB-CM-MM

In some embodiments, the conjugated activatable antibody comprises a linking peptide between the MM and the CM.

In some embodiments, the conjugated activatable antibody comprises a linking peptide between the CM and the AB.

In some embodiments, the conjugated activatable antibody comprises a first linking peptide (LP1) and a second linking peptide (LP2), and wherein the conjugated activatable antibody in the uncleaved state has the structural arrangement from N-terminus to C-terminus as follows: MM-LP1-CM-LP2-AB or AB-LP2-CM-LP1-MM. In some embodiments, the two linking peptides need not be identical to each other.

In some embodiments, at least one of LP1 or LP2 comprises an amino acid sequence selected from the group consisting of (GS)ₙ, (GGS)ₙ, (GSGGS)ₙ (SEQ ID NO: 24) and (GGGS)ₙ (SEQ ID NO: 25), where n is an integer of at least one.

In some embodiments, at least one of LP1 or LP2 comprises an amino acid sequence selected from the group consisting of GGSG (SEQ ID NO: 26), GGSGG (SEQ ID NO: 27), GSGSG (SEQ ID NO: 28), GSGGG (SEQ ID NO: 29), GGGSG (SEQ ID NO: 30), and GSSSG (SEQ ID NO: 31).

In some embodiments, LP1 comprises the amino acid sequence GGGSSGGS (SEQ ID NO: 207), GSSGGSGGSGGSG (SEQ ID NO: 32), GSSGGSGGSGG (SEQ ID NO: 33), GSSGGSGGSGGS (SEQ ID NO: 34), GSSGGSGGSGGSGGGS (SEQ ID NO: 35), GSSGGSGGSG (SEQ ID NO: 36), GSSGGSGGSGS (SEQ ID NO: 37).

In some embodiments, LP2 comprises the amino acid sequence GSS, GGS, GGGS (SEQ ID NO: 38), GSSGT (SEQ ID NO: 39) or GSSG (SEQ ID NO: 40).

In some embodiments, the AB has a dissociation constant of about 100 nM or less for binding to mammalian CD71. In some embodiments, the AB has a dissociation constant of about 10 nM or less for binding to mammalian CD71. In some embodiments, the AB has a dissociation constant of about 5 nM or less for binding to CD71. In some embodiments, the AB has a dissociation constant of about 1 nM or less for binding to CD71. In some embodiments, the AB has a dissociation constant of about 0.5 nM or less for binding to CD71. In some embodiments, the AB has a dissociation constant of about 0.1 nM or less for binding to CD71. In some embodiments, the AB has a dissociation constant of 0.01 nM to 100 nM, 0.01 nM to 10 nM, 0.01 nM to 5 nM, 0.01 nM to 1 nM, 0.01 to 0.5 nM, 0.01 nm to 0.1 nM, 0.01 nm to 0.05 nM, 0.05 nM to 100 nM, 0.05 nM to 10 nM, 0.05 nM to 5 nM, 0.05 nM to 1 nM, 0.05 to 0.5 nM, 0.05 nm to 0.1 nM, 0.1 nM to 100 nM, 0.1 nM to 10 nM, 0.1 nM to 5 nM, 0.1 nM to 1 nM, 0.1 to 0.5 nM, 0.5 nM to 100 nM, 0.5 nM to 10 nM, 0.5 nM to 5 nM, 0.5 nM to 1 nM, 1 nM to 100 nM, 1 nM to 10 nM, 1 nM to 5 nM, 5 nM to 100 nM, 5 nM to 10 nM, or 10 nM to 100 nM, for binding to mammalian CD71.

In some embodiments, the conjugated activatable antibody in an uncleaved state specifically binds to the mammalian CD71 with a dissociation constant less than or equal to 1 nM, less than or equal to 5 nM, less than or equal to 10 nM, less than or equal to 15 nM, less than or equal to 20 nM, less than or equal to 25 nM, less than or equal to 50 nM, less than or equal to 100 nM, less than or equal to 150 nM, less than or equal to 250 nM, less than or equal to 500 nM, less than or equal to 750 nM, less than or equal to 1000 nM, and/or less than or equal to 2000 nM.

In some embodiments, the conjugated activatable antibody in an uncleaved state specifically binds to the mammalian CD71 with a dissociation constant in the range of 1 nM to 2000 nM, 1 nM to 1000 nM, 1 nM to 750 nM, 1 nM to 500 nM, 1 nM to 250 nM, 1 nM to 150 nM, 1 nM to 100 nM, 1 nM to 50 nM, 1 nM to 25 nM, 1 nM to 15 nM, 1 nM to 10 nM, 1 nM to 5 nM, 5 nM to 2000 nM, 5 nM to 1000 nM, 5 nM to 750 nM, 5 nM to 500 nM, 5 nM to 250 nM, 5 nM to 150 nM, 5 nM to 100 nM, 5 nM to 50 nM, 5 nM to 25 nM, 5 nM to 15 nM, 5 nM to 10 nM, 10 nM to 2000 nM, 10 nM to 1000 nM, 10 nM to 750 nM, 10 nM to 500 nM, 10 nM to 250 nM, 10 nM to 150 nM, 10 nM to 100 nM, 10 nM to 50 nM, 10 nM to 25 nM, 10 nM to 15 nM, 15 nM to 2000 nM, 15 nM to 1000 nM, 15 nM to 750 nM, 15 nM to 500 nM, 15 nM to 250 nM, 15 nM to 150 nM, 15 nM to 100 nM, 15 nM to 50 nM, 15 nM to 25 nM, 25 nM to 2000 nM, 25 nM to 1000 nM, 25 nM to 750 nM, 25 nM to 500 nM, 25 nM to 250 nM, 25 nM to 150 nM, 25 nM to 100 nM, 25 nM to 50 nM, 50 nM to 2000 nM, 50 nM to 1000 nM, 50 nM to 750 nM, 50 nM to 500 nM, 50 nM to 250 nM, 50 nM to 150 nM, 50 nM to 100 nM, 100 nM to 2000 nM, 100 nM to 1000 nM, 100 nM to 750 nM, 100 nM to 500 nM, 100 nM to 250 nM, 100 nM to 150 nM, 150 nM to 2000 nM, 150 nM to 1000 nM, 150 nM to 750 nM, 150 nM to 500 nM, 150 nM to 250 nM, 250 nM to 2000 nM, 250 nM to 1000 nM, 250 nM to 750 nM, 250 nM to 500 nM, 500 nM to 2000 nM, 500 nM to 1000 nM, 500 nM to 750 nM, 500 nM to 500 nM, 500 nM to 250 nM, 500 nM to 150 nM, 500 nM to 100 nM, 500 nM to 50 nM, 750 nM to 2000 nM, 750 nM to 1000 nM, or 1000 nM to 2000 nM.

In some embodiments, the conjugated activatable antibody in an activated state specifically binds to the mammalian CD71 with a dissociation constant is less than or equal to 0.01 nM, 0.05 nM, 0.1 nM, 0.5 nM, 1 nM, 5 nM, or 10 nM.

In some embodiments, the conjugated activatable antibody in an activated state specifically binds to the mammalian CD71 with a dissociation constant in the range of 0.01 nM to 100 nM, 0.01 nM to 10 nM, 0.01 nM to 5 nM, 0.01 nM to 1 nM, 0.01 to 0.5 nM, 0.01 nm to 0.1 nM, 0.01 nm to 0.05 nM, 0.05 nM to 100 nM, 0.05 nM to 10 nM, 0.05 nM to 5 nM, 0.05 nM to 1 nM, 0.05 to 0.5 nM, 0.05 nm to 0.1 nM, 0.1 nM to 100 nM, 0.1 nM to 10 nM, 0.1 nM to 5 nM, 0.1 nM to 1 nM, 0.1 to 0.5 nM, 0.5 nM to 100 nM, 0.5 nM to 10 nM, 0.5 nM to 5 nM, 0.5 nM to 1 nM, 1 nM to 100 nM, 1 nM to 10 nM, 1 nM to 5 nM, 5 nM to 100 nM, 5 nM to 10 nM, or 10 nM to 100 nM.

In some embodiments, the mammalian CD71 is selected from the group consisting of a human CD71, a murine CD71, a rat CD71, and a cynomolgus monkey CD71. In some embodiments, the AB specifically binds to human CD71, murine CD71 or cynomolgus monkey CD71 with a dissociation constant of less than 1 nM. In some embodiments, the mammalian CD71 is a human CD71.

In some embodiments, the AB has one or more of the following characteristics: (a) the AB specifically binds to human CD71; and (b) the AB specifically binds to human CD71 and cynomolgus monkey CD71.

In some embodiments, the AB has one or more of the following characteristics: (a) the AB specifically binds human CD71 and cynomolgus monkey CD71; (b) the AB inhibits binding of transferrin to mammalian CD71; (c) the AB inhibits binding of human transferrin to human CD71; and (d) the AB inhibits binding of cynomolgus monkey transferrin to cynomolgus monkey CD71.

In some embodiments, the AB blocks the ability of a natural ligand to bind to the mammalian CD71 with an EC₅₀ less than or equal to 5 nM, less than or equal to 10 nM, less than or equal to 50 nM, less than or equal to 100 nM, less than or equal to 500 nM, and/or less than or equal to 1000 nM. In some embodiments, the AB blocks the ability of a transferrin to bind to the mammalian CD71 with an EC₅₀ less than or equal to 5 nM, less than or equal to 10 nM, less than or equal to 50 nM, less than or equal to 100 nM, less than or equal to 500 nM, and/or less than or equal to 1000 nM. In some embodiments, the natural ligand of CD71 is transferrin.

In some embodiments, the AB blocks the ability of a natural ligand to bind to the mammalian CD71 with an EC₅₀ of 5 nM to 1000 nM, 5 nM to 500 nM, 5 nM to 100 nM 5 nM to 50 nM, 5 nM to 10 nM, 10 nM to 1000 nM, 10 nM to 500 nM, 10 nM to 100 nM 10 nM to 50 nM, 50 nM to 1000 nM, 50 nM to 500 nM, 50 nM to 100 nM, 100 nM to 1000 nM, 100 nM to 500 nM, 500 nM to 1000 nM. In some embodiments, the AB blocks the ability of a transferrin to bind to the mammalian CD71 with an EC₅₀ of 5 nM to 1000 nM, 5 nM to 500 nM, 5 nM to 100 nM 5 nM to 50 nM, 5 nM to 10 nM, 10 nM to 1000 nM, 10 nM to 500 nM, 10 nM to 100 nM 10 nM to 50 nM, 50 nM to 1000 nM, 50 nM to 500 nM, 50 nM to 100 nM, 100 nM to 1000 nM, 100 nM to 500 nM, 500 nM to 1000 nM. In some embodiments, the natural ligand of CD71 is transferrin.

In some embodiments, the AB of the present disclosure inhibits or reduces the growth, proliferation, and/or metastasis of cells expressing mammalian CD71. Without intending to be bound by any theory, the AB of the present disclosure may inhibit or reduce the growth, proliferation, and/or metastasis of cells expressing mammalian CD71 by specifically binding to CD71 and inhibiting, blocking, and/or preventing the binding of a natural ligand to mammalian CD71. In some embodiments, the natural ligand of mammalian CD71 is transferrin.

In some embodiments, the MM has a dissociation constant for binding to the AB which is greater than the dissociation constant of the AB to CD71.

In some embodiments, the MM has a dissociation constant for binding to the AB which is no more than the dissociation constant of the AB to CD71.

In some embodiments, the MM has a dissociation constant for binding to the AB which is less than the dissociation constant of the AB to CD71.

In some embodiments, the dissociation constant (K_{d}) of the MM towards the AB is no more than 2, 3, 4, 5, 10, 25, 50, 100, 250, 500, 1,000, 2,500, 5,000, 10,000, 50,000, 100,000, 500,000, 1,000,000, 5,000,000, 10,000,000, 50,000,000 times or greater, or between 1-5, 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times or greater than the dissociation constant of the AB towards the target.

In some embodiments, the MM does not interfere or compete with the AB for binding to CD71 when the activatable antibody is in a cleaved state.

In some embodiments, the MM is a polypeptide of about 2 to 40 amino acids in length. In some embodiments, the MM is a polypeptide of up to about 40 amino acids in length.

In some embodiments, the MM polypeptide sequence is different from that of CD71. In some embodiments, the MM polypeptide sequence is no more than 50% identical to any natural binding partner of the AB. In some embodiments, the MM polypeptide sequence is different from that of CD71 and is no more than 40%, 30%, 25%, 20%, 15%, or 10% identical to any natural binding partner of the AB.

In some embodiments, the coupling of the MM to the AB reduces the ability of the AB to bind CD71 such that the dissociation constant (K_{d}) of the AB when coupled to the MM towards CD71 is at least two times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least five times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 10 times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 20 times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 40 times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 100 times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 1000 times greater than the K_{d} of the AB when not coupled to the MM towards CD71.

In some embodiments, the coupling of the MM to the AB reduces the ability of the AB to bind CD71 such that the dissociation constant (K_{d}) of the AB when coupled to the MM towards CD71 is at least 10,000 times greater than the K_{d} of the AB when not coupled to the MM towards CD71.

In some embodiments, in the presence of CD71, the MM reduces the ability of the AB to bind CD71 by at least 90% when the CM is uncleaved, as compared to when the CM is cleaved when assayed *in vitro* using a target displacement assay such as, for example, the assay described in PCT Publication No. WO 2010/081173, the contents of which are hereby incorporated by reference in their entirety.

In some embodiments, MM comprises an amino acid sequence selected from the group consisting of SEQ ID NO: 16 or 18.

In some embodiments, the protease that cleaves the CM is active, e.g., up-regulated or otherwise unregulated, in diseased tissue, and the protease cleaves the CM in the activatable antibody when the activatable antibody is exposed to the protease.

In some embodiments, the protease is co-localized with CD71 in a tissue, and the protease cleaves the CM in the conjugated activatable antibody when the activatable antibody is exposed to the protease.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least twofold greater than the dissociation constant of an unmodified AB binding to CD71, whereas in the cleaved state (i.e., when the conjugated activatable antibody is in the cleaved state), the AB binds CD71.

In some embodiments, the CM is positioned in the conjugated activatable antibody such that when the conjugated activatable antibody is in the uncleaved state, binding of the activatable antibody to CD71 is reduced to occur with a dissociation constant that is at least fivefold greater than the dissociation constant of an unmodified AB binding to CD71, whereas in the cleaved state (i.e., when the conjugated activatable antibody is in the cleaved state), the AB binds CD71.

In some embodiments, the CM is positioned in the conjugated activatable antibody such that when the conjugated activatable antibody is in the uncleaved state, binding of the activatable antibody to CD71 is reduced to occur with a dissociation constant that is at least 10-fold greater than the dissociation constant of an unmodified AB binding to CD71, whereas in the cleaved state (i.e., when the conjugated activatable antibody is in the cleaved state), the AB binds CD71.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least 20-fold greater than the dissociation constant of an unmodified AB binding to CD71, whereas in the cleaved state (i.e., when the conjugated activatable antibody is in the cleaved state), the AB binds CD71.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least 40-fold greater than the dissociation constant of an unmodified AB binding to CD71, whereas in the cleaved state, the AB binds CD71.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least 50-fold greater than the dissociation constant of an unmodified AB binding to CD71, whereas in the cleaved state, the AB binds CD71.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least 100-fold greater than the dissociation constant of an unmodified AB binding to CD71, whereas in the cleaved state, the AB binds CD71.

In some embodiments, the CM is positioned in the conjugated activatable antibody such that when the conjugated activatable antibody is in the uncleaved state, binding of the conjugated activatable antibody to CD71 is reduced to occur with a dissociation constant that is at least 200-fold greater than the dissociation constant of an unmodified AB binding to CD71, whereas in the cleaved state, the AB binds CD71.

In some embodiments, the CM is a polypeptide of up to 15 amino acids in length.

In some embodiments, the CM is a polypeptide that includes a first cleavable moiety (CM1) that is a substrate for at least one matrix metalloprotease (MMP) and a second cleavable moiety (CM2) that is a substrate for at least one serine protease (SP). In some embodiments, each of the CM1 substrate sequence and the CM2 substrate sequence of the CM1-CM2 substrate is independently a polypeptide of up to 15 amino acids in length.

In some embodiments, the CM is a substrate for at least one protease that is or is believed to be up-regulated or otherwise unregulated in cancer.

In some embodiments, the CM is a substrate for at least one protease selected from the group consisting of a matrix metalloprotease (MMP), thrombin, a neutrophil elastase, a cysteine protease, legumain, and a serine protease, such as matriptase (MT-SP1), and urokinase (uPA). Without being bound by theory, it is believed that these proteases are up-regulated or otherwise unregulated in at least one of cancer.

In some embodiments, the CM is selected for use with a specific protease, for example a protease that is known to be co-localized with the target of the activatable antibody.

In some embodiments, the CM is a substrate for at least one MMP. In some embodiments, the CM is a substrate for a protease selected from the group consisting of MMP 9, MMP14, MMP1, MMP3, MMP13, MMP17, MMP11, and MMP19. In some embodiments the CM is a substrate for MMP9. In some embodiments, the CM is a substrate for MMP14.

In some embodiments, the CM is a substrate that includes the sequence TGRGPSWV (SEQ ID NO: 41); SARGPSRW (SEQ ID NO: 42); TARGPSFK (SEQ ID NO: 43); LSGRSDNH (SEQ ID NO: 44); GGWHTGRN (SEQ ID NO: 45); HTGRSGAL (SEQ ID NO: 46); PLTGRSGG (SEQ ID NO: 47); AARGPAIH (SEQ ID NO: 48); RGPAFNPM (SEQ ID NO: 49); SSRGPAYL (SEQ ID NO: 50); RGPATPIM (SEQ ID NO: 51); RGPA (SEQ ID NO: 52); GGQPSGMWGW (SEQ ID NO: 53); FPRPLGITGL (SEQ ID NO: 54); VHMPLGFLGP (SEQ ID NO: 55); SPLTGRSG (SEQ ID NO: 56); SAGFSLPA (SEQ ID NO: 57); LAPLGLQRR (SEQ ID NO: 58); SGGPLGVR (SEQ ID NO: 59); PLGL (SEQ ID NO: 60); LSGRSGNH (SEQ ID NO: 175); SGRSANPRG (SEQ ID NO: 176); LSGRSDDH (SEQ ID NO: 177); LSGRSDIH (SEQ ID NO: 178); LSGRSDQH (SEQ ID NO: 179); LSGRSDTH (SEQ ID NO: 180); LSGRSDYH (SEQ ID NO: 181); LSGRSDNP (SEQ ID NO: 182); LSGRSANP (SEQ ID NO: 183); LSGRSANI (SEQ ID NO: 184); LSGRSDNI (SEQ ID NO: 185); MIAPVAYR (SEQ ID NO: 186); RPSPMWAY (SEQ ID NO: 187); WATPRPMR (SEQ ID NO: 188); FRLLDWQW (SEQ ID NO: 189); ISSGL (SEQ ID NO: 190); ISSGLLS (SEQ ID NO: 191); and/or ISSGLL (SEQ ID NO: 192).

In some embodiments, the CM comprises the amino acid sequence LSGRSDNH (SEQ ID NO: 44). In some embodiments, the CM comprises the amino acid sequence TGRGPSWV (SEQ ID NO: 41). In some embodiments, the CM comprises the amino acid sequence PLTGRSGG (SEQ ID NO: 47). In some embodiments, the CM comprises the amino acid sequence GGQPSGMWGW (SEQ ID NO: 53). In some embodiments, the CM comprises the amino acid sequence FPRPLGITGL (SEQ ID NO: 54). In some embodiments, the CM comprises the amino acid sequence VHMPLGFLGP (SEQ ID NO: 55). In some embodiments, the CM comprises the amino acid sequence PLGL (SEQ ID NO: 60). In some embodiments, the CM comprises the amino acid sequence SARGPSRW (SEQ ID NO: 42). In some embodiments, the CM comprises the amino acid sequence TARGPSFK (SEQ ID NO: 43). In some embodiments, the CM comprises the amino acid sequence GGWHTGRN (SEQ ID NO: 45). In some embodiments, the CM comprises the amino acid sequence HTGRSGAL (SEQ ID NO: 46). In some embodiments, the CM comprises the amino acid sequence AARGPAIH (SEQ ID NO: 48). In some embodiments, the CM comprises the amino acid sequence RGPAFNPM (SEQ ID NO: 49). In some embodiments, the CM comprises the amino acid sequence SSRGPAYL (SEQ ID NO: 50). In some embodiments, the CM comprises the amino acid sequence RGPATPIM (SEQ ID NO: 51). In some embodiments, the CM comprises the amino acid sequence RGPA (SEQ ID NO: 52). In some embodiments, the CM comprises the amino acid sequence LSGRSGNH (SEQ ID NO: 175). In some embodiments, the CM comprises the amino acid sequence SGRSANPRG (SEQ ID NO: 176). In some embodiments, the CM comprises the amino acid sequence LSGRSDDH (SEQ ID NO: 177). In some embodiments, the CM comprises the amino acid sequence LSGRSDIH (SEQ ID NO: 178). In some embodiments, the CM comprises the amino acid sequence LSGRSDQH (SEQ ID NO: 179). In some embodiments, the CM comprises the amino acid sequence LSGRSDTH (SEQ ID NO: 180). In some embodiments, the CM comprises the amino acid sequence LSGRSDYH (SEQ ID NO: 181). In some embodiments, the CM comprises the amino acid sequence LSGRSDNP (SEQ ID NO: 182). In some embodiments, the CM comprises the amino acid sequence LSGRSANP (SEQ ID NO: 183). In some embodiments, the CM comprises the amino acid sequence LSGRSANI (SEQ ID NO: 184). In some embodiments, the CM comprises the amino acid sequence LSGRSDNI (SEQ ID NO: 185). In some embodiments, the CM comprises the amino acid sequence MIAPVAYR (SEQ ID NO: 186). In some embodiments, the CM comprises the amino acid sequence RPSPMWAY (SEQ ID NO: 187). In some embodiments, the CM comprises the amino acid sequence WATPRPMR (SEQ ID NO: 188). In some embodiments, the CM comprises the amino acid sequence FRLLDWQW (SEQ ID NO: 189). In some embodiments, the CM comprises the amino acid sequence ISSGL (SEQ ID NO: 190). In some embodiments, the CM comprises the amino acid sequence ISSGLLS (SEQ ID NO: 191). In some embodiments, the CM comprises the amino acid sequence and/or ISSGLL (SEQ ID NO: 192).

In some embodiments, the CM is a substrate for an MMP and includes the sequence ISSGLSS (SEQ ID NO: 61); QNQALRMA (SEQ ID NO: 62); AQNLLGMV (SEQ ID NO: 63); STFPFGMF (SEQ ID NO: 64); PVGYTSSL (SEQ ID NO: 65); DWLYWPGI (SEQ ID NO: 66), ISSGLLSS (SEQ ID NO: 67), LKAAPRWA (SEQ ID NO: 68); GPSHLVLT (SEQ ID NO: 69); LPGGLSPW (SEQ ID NO: 70); MGLFSEAG (SEQ ID NO: 71); SPLPLRVP (SEQ ID NO: 72); RMHLRSLG (SEQ ID NO: 73); LAAPLGLL (SEQ ID NO: 74); AVGLLAPP (SEQ ID NO: 75); LLAPSHRA (SEQ ID NO: 76); and/or PAGLWLDP (SEQ ID NO: 77).

In some embodiments, the CM comprises the amino acid sequence ISSGLSS (SEQ ID NO: 61). In some embodiments, the CM comprises the amino acid sequence QNQALRMA (SEQ ID NO: 62). In some embodiments, the CM comprises the amino acid sequence AQNLLGMV (SEQ ID NO: 63). In some embodiments, the CM comprises the amino acid sequence STFPFGMF (SEQ ID NO: 64). In some embodiments, the CM comprises the amino acid sequence PVGYTSSL (SEQ ID NO: 65). In some embodiments, the CM comprises the amino acid sequence DWLYWPGI (SEQ ID NO: 66). In some embodiments, the CM comprises the amino acid sequence ISSGLLSS (SEQ ID NO: 67). In some embodiments, the CM comprises the amino acid sequence LKAAPRWA (SEQ ID NO: 68). In some embodiments, the CM comprises the amino acid sequence GPSHLVLT (SEQ ID NO: 69). In some embodiments, the CM comprises the amino acid sequence LPGGLSPW (SEQ ID NO: 70). In some embodiments, the CM comprises the amino acid sequence MGLFSEAG (SEQ ID NO: 71). In some embodiments, the CM comprises the amino acid sequence SPLPLRVP (SEQ ID NO: 72). In some embodiments, the CM comprises the amino acid sequence RMHLRSLG (SEQ ID NO: 73). In some embodiments, the CM comprises the amino acid sequence LAAPLGLL (SEQ ID NO: 74). In some embodiments, the CM comprises the amino acid sequence AVGLLAPP (SEQ ID NO: 75). In some embodiments, the CM comprises the amino acid sequence LLAPSHRA (SEQ ID NO: 76). In some embodiments, the CM comprises the amino acid sequence PAGLWLDP (SEQ ID NO: 77).

In some embodiments, the CM is a substrate for thrombin. In some embodiments, the CM is a substrate for thrombin and includes the sequence GPRSFGL (SEQ ID NO: 78) or GPRSFG (SEQ ID NO: 79). In some embodiments, the CM comprises the amino acid sequence GPRSFGL (SEQ ID NO: 78). In some embodiments, the CM comprises the amino acid sequence GPRSFG (SEQ ID NO: 79).

In some embodiments, the CM comprises an amino acid sequence selected from the group consisting of NTLSGRSENHSG (SEQ ID NO: 80); NTLSGRSGNHGS (SEQ ID NO: 81); TSTSGRSANPRG (SEQ ID NO: 82); TSGRSANP (SEQ ID NO: 83); VAGRSMRP (SEQ ID NO: 84); VVPEGRRS (SEQ ID NO: 85); ILPRSPAF (SEQ ID NO: 86); MVLGRSLL (SEQ ID NO: 87); QGRAITFI (SEQ ID NO: 88); SPRSIMLA (SEQ ID NO: 89); and SMLRSMPL (SEQ ID NO: 90).

In some embodiments, the CM comprises the amino acid sequence NTLSGRSENHSG (SEQ ID NO: 80). In some embodiments, the CM comprises the amino acid sequence NTLSGRSGNHGS (SEQ ID NO: 81). In some embodiments, the CM comprises the amino acid sequence TSTSGRSANPRG (SEQ ID NO: 82). In some embodiments, the CM comprises the amino acid sequence TSGRSANP (SEQ ID NO: 83). In some embodiments, the CM comprises the amino acid sequence VAGRSMRP (SEQ ID NO: 84). In some embodiments, the CM comprises the amino acid sequence VVPEGRRS (SEQ ID NO: 85). In some embodiments, the CM comprises the amino acid sequence ILPRSPAF (SEQ ID NO: 86). In some embodiments, the CM comprises the amino acid sequence MVLGRSLL (SEQ ID NO: 87). In some embodiments, the CM comprises the amino acid sequence QGRAITFI (SEQ ID NO: 88). In some embodiments, the CM comprises the amino acid sequence SPRSIMLA (SEQ ID NO: 89). In some embodiments, the CM comprises the amino acid sequence SMLRSMPL (SEQ ID NO: 90).

In some embodiments, the CM is a substrate for a neutrophil elastase. In some embodiments, the CM is a substrate for a serine protease. In some embodiments, the CM is a substrate for uPA. In some embodiments, the CM is a substrate for legumain. In some embodiments, the CM is a substrate for matriptase. In some embodiments, the CM is a substrate for a cysteine protease. In some embodiments, the CM is a substrate for a cysteine protease, such as a cathepsin.

In some embodiments, the CM is a CM1-CM2 substrate and includes the sequence ISSGLLSGRSDNH (SEQ ID NO: 91); ISSGLLSSGGSGGSLSGRSDNH (SEQ ID NO: 92); AVGLLAPPGGTSTSGRSANPRG (SEQ ID NO: 93); TSTSGRSANPRGGGAVGLLAPP (SEQ ID NO: 94); VHMPLGFLGPGGTSTSGRSANPRG (SEQ ID NO: 95); TSTSGRSANPRGGGVHMPLGFLGP (SEQ ID NO: 96); AVGLLAPPGGLSGRSDNH (SEQ ID NO: 97); LSGRSDNHGGAVGLLAPP (SEQ ID NO: 98); VHMPLGFLGPGGLSGRSDNH (SEQ ID NO: 99); LSGRSDNHGGVHMPLGFLGP (SEQ ID NO: 100); LSGRSDNHGGSGGSISSGLLSS (SEQ ID NO: 101); LSGRSGNHGGSGGSISSGLLSS (SEQ ID NO: 102); ISSGLLSSGGSGGSLSGRSGNH (SEQ ID NO: 103); LSGRSDNHGGSGGSQNQALRMA (SEQ ID NO: 104); QNQALRMAGGSGGSLSGRSDNH (SEQ ID NO: 105); LSGRSGNHGGSGGSQNQALRMA (SEQ ID NO: 106); QNQALRMAGGSGGSLSGRSGNH (SEQ ID NO: 107); ISSGLLSGRSGNH (SEQ ID NO: 108); ISSGLLSGRSANPRG (SEQ ID NO: 148); AVGLLAPPTSGRSANPRG (SEQ ID NO: 149); AVGLLAPPSGRSANPRG (SEQ ID NO: 150); ISSGLLSGRSDDH (SEQ ID NO: 151); ISSGLLSGRSDIH (SEQ ID NO: 152); ISSGLLSGRSDQH (SEQ ID NO: 153); ISSGLLSGRSDTH (SEQ ID NO: 154); ISSGLLSGRSDYH (SEQ ID NO: 155); ISSGLLSGRSDNP (SEQ ID NO: 156); ISSGLLSGRSANP (SEQ ID NO: 157); ISSGLLSGRSANI (SEQ ID NO: 158); AVGLLAPPGGLSGRSDDH (SEQ ID NO: 159); AVGLLAPPGGLSGRSDIH (SEQ ID NO: 160); AVGLLAPPGGLSGRSDQH (SEQ ID NO: 161); AVGLLAPPGGLSGRSDTH (SEQ ID NO: 162); AVGLLAPPGGLSGRSDYH (SEQ ID NO: 163); AVGLLAPPGGLSGRSDNP (SEQ ID NO: 164); AVGLLAPPGGLSGRSANP (SEQ ID NO: 165); AVGLLAPPGGLSGRSANI (SEQ ID NO: 166), ISSGLLSGRSDNI (SEQ ID NO: 171); AVGLLAPPGGLSGRSDNI (SEQ ID NO: 172); GLSGRSDNHGGAVGLLAPP (SEQ ID NO: 193); and/or GLSGRSDNHGGVHMPLGFLGP (SEQ ID NO: 194).

In some embodiments, the CM1-CM2 substrate includes the sequence ISSGLLSGRSDNH (SEQ ID NO: 91), which is also referred to herein as substrate 2001. In some embodiments, the CM1-CM2 substrate includes the sequence ISSGLLSSGGSGGSLSGRSDNH (SEQ ID NO: 92), which is also referred to herein as substrate 1001/LP'/0001, where LP' as used in this CM1-CM2 substrate is the amino acid sequence GGSGGS (SEQ ID NO: 205). In some embodiments, the CM1-CM2 substrate includes the sequence AVGLLAPPGGTSTSGRSANPRG (SEQ ID NO: 93), which is also referred to herein as substrate 2015 and/or substrate 1004/LP'/0003, where LP' as used in this CM1-CM2 substrate is the amino acid sequence GG. In some embodiments, the CM1-CM2 substrate includes the sequence TSTSGRSANPRGGGAVGLLAPP (SEQ ID NO: 94), which is also referred to herein as substrate 0003/LP'/1004, where LP' as used in this CM1-CM2 substrate is the amino acid sequence GG. In some embodiments, the CM1-CM2 substrate includes the sequence VHMPLGFLGPGGTSTSGRSANPRG (SEQ ID NO: 95), which is also referred to herein as substrate 1003/LP'/0003, where LP' as used in this CM1-CM2 substrate is the amino acid sequence GG. In some embodiments, the CM1-CM2 substrate includes the sequence TSTSGRSANPRGGGVHMPLGFLGP (SEQ ID NO: 96), which is also referred to herein as substrate 0003/LP'/1003, where LP' as used in this CM1-CM2 substrate is the amino acid sequence GG. In some embodiments, the CM1-CM2 substrate includes the sequence AVGLLAPPGGLSGRSDNH (SEQ ID NO: 97), which is also referred to herein as substrate 3001 and/or substrate 1004/LP'/0001, where LP' as used in this CM1-CM2 substrate is the amino acid sequence GG. In some embodiments, the CM1-CM2 substrate includes the sequence LSGRSDNHGGAVGLLAPP (SEQ ID NO: 98), which is also referred to herein as substrate 0001/LP'/1004, where LP' as used in this CM1-CM2 substrate is the amino acid sequence GG. In some embodiments, the CM1-CM2 substrate includes the sequence VHMPLGFLGPGGLSGRSDNH (SEQ ID NO: 99), which is also referred to herein as substrate 1003/LP'/0001, wherein LP' as used in this CM1-CM2 substrate is the amino acid sequence GG. In some embodiments, the CM1-CM2 substrate includes the sequence LSGRSDNHGGVHMPLGFLGP (SEQ ID NO: 100), which is also referred to herein as substrate 0001/LP'/1003, where LP' as used in this CM1-CM2 substrate is the amino acid sequence GG. In some embodiments, the CM1-CM2 substrate includes the sequence LSGRSDNHGGSGGSISSGLLSS (SEQ ID NO: 101), which is also referred to herein as substrate 0001/LP'/1001, where LP' as used in this CM1-CM2 substrate is the amino acid sequence GGSGGS (SEQ ID NO: 205). In some embodiments, the CM1-CM2 substrate includes the sequence LSGRSGNHGGSGGSISSGLLSS (SEQ ID NO: 102), which is also referred to herein as substrate 0002/LP'/1001, where LP' as used in this CM1-CM2 substrate is the amino acid sequence GGSGGS (SEQ ID NO: 205). In some embodiments, the CM1-CM2 substrate includes the sequence ISSGLLSSGGSGGSLSGRSGNH (SEQ ID NO: 103), which is also referred to herein as substrate 1001/LP'/0002, where LP' as used in this CM1-CM2 substrate is the amino acid sequence GGSGGS (SEQ ID NO: 205). In some embodiments, the CM1-CM2 substrate includes the sequence LSGRSDNHGGSGGSQNQALRMA (SEQ ID NO: 104), which is also referred to herein as substrate 0001/LP'/1002, where LP' as used in this CM1-CM2 substrate is the amino acid sequence GGSGGS (SEQ ID NO: 205). In some embodiments, the CM1-CM2 substrate includes the sequence QNQALRMAGGSGGSLSGRSDNH (SEQ ID NO: 105), which is also referred to herein as substrate 1002/LP'/0001, where LP' as used in this CM1-CM2 substrate is the amino acid sequence GGSGGS (SEQ ID NO: 205). In some embodiments, the CM1-CM2 substrate includes the sequence LSGRSGNHGGSGGSQNQALRMA (SEQ ID NO: 106), which is also referred to herein as substrate 0002/LP'/1002, where LP' as used in this CM1-CM2 substrate is the amino acid sequence GGSGGS (SEQ ID NO: 205). In some embodiments, the CM1-CM2 substrate includes the sequence QNQALRMAGGSGGSLSGRSGNH (SEQ ID NO: 107), which is also referred to herein as substrate 1002/LP'/0002, where LP' as used in this CM1-CM2 substrate is the amino acid sequence GGSGGS (SEQ ID NO: 205). In some embodiments, the CM1-CM2 substrate includes the sequence ISSGLLSGRSGNH (SEQ ID NO: 108), which is also referred to herein as substrate 2002. In some embodiments, the CM1-CM2 substrate includes the sequence ISSGLLSGRSANPRG (SEQ ID NO: 148), which is also referred to herein as substrate 2003. In some embodiments, the CM1-CM2 substrate includes the sequence AVGLLAPPTSGRSANPRG (SEQ ID NO: 149), which is also referred to herein as substrate 2004. In some embodiments, the CM1-CM2 substrate includes the sequence AVGLLAPPSGRSANPRG (SEQ ID NO: 150), which is also referred to herein as substrate 2005. In some embodiments, the CM1-CM2 substrate includes the sequence ISSGLLSGRSDDH (SEQ ID NO: 151), which is also referred to herein as substrate 2006. In some embodiments, the CM1-CM2 substrate includes the sequence ISSGLLSGRSDIH (SEQ ID NO: 152), which is also referred to herein as substrate 2007. In some embodiments, the CM1-CM2 substrate includes the sequence ISSGLLSGRSDQH (SEQ ID NO: 153), which is also referred to herein as substrate 2008. In some embodiments, the CM1-CM2 substrate includes the sequence ISSGLLSGRSDTH (SEQ ID NO: 154), which is also referred to herein as substrate 2009. In some embodiments, the CM1-CM2 substrate includes the sequence ISSGLLSGRSDYH (SEQ ID NO: 155), which is also referred to herein as substrate 2010. In some embodiments, the CM1-CM2 substrate includes the sequence ISSGLLSGRSDNP (SEQ ID NO: 156), which is also referred to herein as substrate 2011. In some embodiments, the CM1-CM2 substrate includes the sequence ISSGLLSGRSANP (SEQ ID NO: 157), which is also referred to herein as substrate 2012. In some embodiments, the CM1-CM2 substrate includes the sequence ISSGLLSGRSANI (SEQ ID NO: 158), which is also referred to herein as substrate 2013. In some embodiments, the CM1-CM2 substrate includes the sequence AVGLLAPPGGLSGRSDDH (SEQ ID NO: 159), which is also referred to herein as substrate 3006. In some embodiments, the CM1-CM2 substrate includes the sequence AVGLLAPPGGLSGRSDIH (SEQ ID NO: 160), which is also referred to herein as substrate 3007. In some embodiments, the CM1-CM2 substrate includes the sequence AVGLLAPPGGLSGRSDQH (SEQ ID NO: 161), which is also referred to herein as substrate 3008. In some embodiments, the CM1-CM2 substrate includes the sequence AVGLLAPPGGLSGRSDTH (SEQ ID NO: 162), which is also referred to herein as substrate 3009. In some embodiments, the CM1-CM2 substrate includes the sequence AVGLLAPPGGLSGRSDYH (SEQ ID NO: 163), which is also referred to herein as substrate 3010. In some embodiments, the CM1-CM2 substrate includes the sequence AVGLLAPPGGLSGRSDNP (SEQ ID NO: 164), which is also referred to herein as substrate 3011. In some embodiments, the CM1-CM2 substrate includes the sequence AVGLLAPPGGLSGRSANP (SEQ ID NO: 165), which is also referred to herein as substrate 3012. In some embodiments, the CM1-CM2 substrate includes the sequence AVGLLAPPGGLSGRSANI (SEQ ID NO: 166), which is also referred to herein as substrate 3013. In some embodiments, the CM1-CM2 substrate includes the sequence ISSGLLSGRSDNI (SEQ ID NO: 171), which is also referred to herein as substrate 2014. In some embodiments, the CM1-CM2 substrate includes the sequence AVGLLAPPGGLSGRSDNI (SEQ ID NO: 172), which is also referred to herein as substrate 3014. In some embodiments, the CM1-CM2 substrate includes the sequence GLSGRSDNHGGAVGLLAPP (SEQ ID NO: 193), which is also referred to herein as substrate 0001/LP'/1004, where LP' as used in this CM1-CM2 substrate is the amino acid sequence GG. In some embodiments, the CM1-CM2 substrate includes the sequence GLSGRSDNHGGVHMPLGFLGP (SEQ ID NO: 194), which is also referred to herein as substrate 0001/LP'/1003, where LP' as used in this CM1-CM2 substrate is the amino acid sequence GG

In some embodiments, the CM is a substrate for at least two proteases. In some embodiments, the CM is a substrate for at least two proteases selected from the group consisting of a MMP, thrombin, a neutrophil elastase, a cysteine protease, uPA, legumain and matriptase.

In some embodiments, the conjugated activatable antibody includes at least a first CM and a second CM. In some embodiments, the first CM and the second CM are each polypeptides of no more than 15 amino acids long. In some embodiments, the first CM and the second CM in the conjugated activatable antibody in the uncleaved state have the structural arrangement from N-terminus to C-terminus as follows: MM-CM1-CM2-AB or AB-CM2-CM1-MM. In some embodiments, at least one of the first CM and the second CM is a polypeptide that functions as a substrate for a protease selected from the group consisting of a MMP, thrombin, a neutrophil elastase, a cysteine protease, uPA, legumain, and matriptase. In some embodiments, the first CM is cleaved by a first cleaving agent selected from the group consisting of a MMP, thrombin, a neutrophil elastase, a cysteine protease, uPA, legumain, and matriptase in a target tissue and the second CM is cleaved by a second cleaving agent in a target tissue. In some embodiments, the other protease is selected from the group consisting of those shown in Table (I). In some embodiments, the first cleaving agent and the second cleaving agent are the same protease selected from the group consisting of a MMP, thrombin, a neutrophil elastase, a cysteine protease, uPA, legumain, and matriptase, and the first CM and the second CM are different substrates for the enzyme. In some embodiments, the first cleaving agent and the second cleaving agent are the same protease selected from the group consisting of those shown in Table (I). In some embodiments, the first cleaving agent and the second cleaving agent are different proteases. In some embodiments, the first cleaving agent and the second cleaving agent are co-localized in the target tissue. In some embodiments, the first CM and the second CM are cleaved by at least one cleaving agent in the target tissue.

In some embodiments, the conjugated activatable antibody is exposed to and cleaved by a protease such that, in the activated or cleaved state, the conjugated activated antibody includes a light chain amino acid sequence that includes at least a portion of LP2 and/or CM sequence after the protease has cleaved the CM.

In some embodiments, the activatable antibody is conjugated to one or more equivalents of an agent. In some embodiments, the activatable antibody is conjugated to one equivalent of the agent. In some embodiments, the activatable antibody is conjugated to two, three, or four equivalents of the agent. In some embodiments, the activatable antibody is part of a mixture of activatable antibodies having a homogeneous number of equivalents of conjugated agents. In some embodiments, the activatable antibody is part of a mixture of activatable antibodies having a heterogeneous number of equivalents of conjugated agents. In some embodiments, the mixture of activatable antibodies is such that the average number of agents conjugated to each activatable antibody is between zero to one, between one to two, between two and three, or between three and four. In some embodiments, the mixture of activatable antibodies is such that the average number of agents conjugated to each activatable antibody is one, two, three, or four.

In some embodiments, the activatable antibody comprises one or more site-specific amino acid sequence modifications such that the number of lysine and/or cysteine residues is increased or decreased with respect to the original amino acid sequence of the activatable antibody, thus in some embodiments correspondingly increasing or decreasing the number of agents that can be conjugated to the activatable antibody, or in some embodiments limiting the conjugation of the agents to the activatable antibody in a site-specific manner. In some embodiments, the modified activatable antibody is modified with one or more non-natural amino acids in a site-specific manner, thus in some embodiments limiting the conjugation of the agents to only the sites of the non-natural amino acids.

The conjugated activatable antibodies disclosed herein may comprise drug molecules and antibody moieties in various stoichiometric molar ratios depending on the configuration of the antibody and, at least in part, on the method used to effect conjugation.

The term "drug load" or "drug loading" refers to the molar ratio of drug molecules per antibody in an individual conjugated activatable antibody. In certain embodiments the drug loading may comprise from 1-4 drug molecules, from 2-4 drug molecules, from 1-3 drug molecules, from 2-3 drug molecules, or from 1 to 2 drug molecules. In certain embodiments the drug loading may comprise 1 drug molecule, 2 drug molecules, 3 drug molecules, or 4 drug molecules.

For the purposes of the present invention, one skilled in the art would understand that "drug loading" and "drug to antibody ratio" (also referred to as DAR) are not the same. DAR refers to the average molar ratio of drug molecules per antibody in a population of at least two conjugated activatable antibody molecules, whereas drug loading refers to the molar ratio of drug molecules per antibody in an individual conjugated activatable antibody molecule. Drug loading primarily has relevance for the construction and design of a conjugated activatable antibody, whereas DAR primarily has relevance for the therapeutic conjugated activatable antibody pharmaceutical composition that will be administered to patients.

In some embodiments, activatable antibodies of the present invention may be conjugated to generate relatively homogeneous preparations of conjugated activatable antibodies having a narrow DAR distribution. In some embodiments, a conjugated activatable antibody preparation may be substantially homogeneous with respect to its DAR distribution, meaning that within the preparation is a predominant species of site-specific ADC with a particular DAR (e.g., a DAR of 2 and/or 4). In some embodiments, the substantially homogeneous conjugated activatable antibody preparation may also be uniform with respect to the site of loading (i.e., on the free cysteines).

In some embodiments, the DAR of conjugated activatable antibodies of the disclosure will be about 2 and/or 4. In this context, the term "about" should be construed to mean a conjugated activatable antibody preparation containing greater than 80% of two primary species in aggregate having a DAR equal to 2 and 4 with approximately equal distribution (e.g., greater than 40% each), and the remainder consisting of other DAR species.

In some embodiments, the DAR of the conjugated activatable antibodies of the disclosure will be enriched to be about 2. In this context, the term "about" should be construed to mean a conjugated activatable antibody preparation containing greater than about 90%, 91%, 92%, 93%, 94%, 95%, 96%, 97%, 98% or 99% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 94% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 95% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 96% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 97% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 98% ± 0.1, 0.2, 0.3, 0.4, 0.5, 0.6, 0.7, 0.8, 0.9%.

In some embodiments, the DAR of the conjugated activatable antibodies of the disclosure will be enriched to be about 2, with the term "about" being construed to mean a conjugated activatable antibody preparation containing greater than about 99% ± 0.1, 0.2, 0.3, 0.4, 0.5, 0.6, 0.7, 0.8, 0.9%.

In some embodiments, the DAR of conjugated activatable antibodies of the disclosure will be about 2 and/or 4. In this context, the term "about" should be construed to mean a conjugated activatable antibody preparation containing greater than 80% of two primary species in aggregate having a DAR equal to 2 and 4 with approximately equal distribution (e.g., greater than 40% each), and the remainder consisting of other DAR species.

In some embodiments, it is possible to achieve the desired homogeneity through the use of site-specific activatable antibodies and/or selective reduction and conjugation. In some embodiments, the desired homogeneity may be achieved through the use of site-specific constructs in combination with selective reduction. In some embodiments, the preparations may be further purified, such as by using analytical or preparative chromatography techniques. In these embodiments, the homogeneity of the conjugated activatable antibody preparation can be analyzed using various techniques known in the art including but not limited to mass spectrometry, HPLC (e.g. size exclusion HPLC, RP-HPLC, HIC-HPLC etc.) or capillary electrophoresis.

With regard to the purification of conjugated activatable antibody preparations, it will be appreciated that standard pharmaceutical preparative methods may be employed to obtain the desired purity. As discussed herein liquid chromatography methods such as reverse phase (RP) and hydrophobic interaction chromatography (HIC) may separate compounds in the mixture by drug loading value. In some cases, ion-exchange (IEC) or mixed-mode chromatography (MMC) may also be used to isolate species with a specific drug load.

In some embodiments, a conjugated activatable antibody preparation of the disclosure having a given DAR with a relatively high level of drug load homogeneity may actually comprise a mixture of conjugates with a range or distribution of drug loads, but where the distribution of drug loads in the mixture are centered around (or having a weighted average of) the mean DAR value. Thus, in some embodiments conjugated activatable antibody preparations will include a mixture of conjugates in which the average DAR of the mixture is about 1, 2, or 3, each +/-0.5. It will be appreciated that the range or deviation may be less than 0.4 in certain preferred embodiments. Thus, in other embodiments the compositions will comprise an average DAR of 1, 2, or 3, each +/-0.3.

In some embodiments, the distribution of drugs per antibody in preparations of conjugated activatable antibodies from conjugation reactions may be characterized by conventional means such as UV-Vis spectrophotometry, reverse phase HPLC, HIC, mass spectroscopy, ELISA, and electrophoresis.

In some embodiments, a mixture of conjugated activatable antibodies of the disclosure that includes a mixture of conjugates having different drug loads may be purified or enriched for one or more species of conjugate having a specific drug load. For example, a conjugated activatable antibody mixture that includes a mixture of AADCs having drug load of 0, 2, 4, 6, and 8 may be enriched or purified for the conjugated activatable antibodies having a drug load of 2. In some embodiments, the mixture can be purified or enriched for only the species of conjugated activatable antibodies having a drug load of 4. In some embodiments, the mixture can be purified or enriched for only the species of conjugated activatable antibodies having a drug load of 2 and 4. As used herein, a preparation or purification or conjugated activatable antibody having a relatively homogeneous preparation of species of conjugate species having a drug load of 2 is referred to as "E2", which has a DAR of about 2. As used herein, a preparation or purification or conjugated activatable antibody having a relatively homogeneous preparation of conjugate species having a drug load of 4 is referred to as "E4". As used herein, a preparation or purification or conjugated activatable antibody having a mixture of conjugate species having a drug load of 2 or 4 referred to as "DE" (DAR-enriched), having a DAR of approximately 3. In this context, the term "about" is construed to mean +/- 0.1, 0.2, 0.3, 0.4, 0.5, 0.6, 0.7, 0.8, 0.9 or 1.0 of the proscribed amount.

In some embodiments, a conjugated activatable antibody of the disclosure can be represented by the formula:

AA-(AG)ₚ

where AA is an activatable antibody which has, in an uncleaved state, the structural arrangement from N-terminus to C-terminus of MM-CM-AB. AB is an antibody that specifically binds to mammalian CD71 and includes the heavy chain variable region sequence of SEQ ID NO: 5 and the light chain variable region sequence of SEQ ID NO: 7. MM is a masking moiety that includes the sequence of SEQ ID NO: 18, and the MM is coupled to the AB and inhibits the binding of the AB to CD71 when the AA is in an uncleaved state. CM is a cleavable moiety comprising the sequence of SEQ ID NO: 156, the CM is coupled to the AB, and the CM is a polypeptide that functions as a substrate for a protease. AG is an agent conjugated to the AA, where AA-(AG)ₚ. In some embodiments, p is 0, 1, 2, 3, 4, 5, 6, 7, or 8. In some embodiments, a composition of the disclosure includes a mixture of conjugated activatable antibodies of the disclosure, where each conjugated activatable antibody is represented by the formula AA-(AG)ₚ, where p is an integer from 0 to 8. In some embodiments, the composition includes a mixture of conjugated activatable antibodies of the disclosure where at least 50%, at least 75%, at least 85%, at least 90%, at least 95%, or at least 98% of the species is the conjugated activatable antibody where p is 2.

In some embodiments, a preparation of a conjugated activatable antibody that is purified or enriched for given species of conjugate with a specific drug load includes at least a certain percentage of the given drug load species. For example, in some embodiments, a preparation of E2 conjugated activatable antibody includes at least 50% of the preparation is the conjugated activatable antibody having a drug load of 2. In some embodiments, a preparation of E2 conjugated activatable antibody includes at least 75% of the conjugated activatable antibody having a drug load of 2. In some embodiments, a preparation of E2 conjugated activatable antibody includes at least 85% of the conjugated activatable antibody having a drug load of 2. In some embodiments, a preparation of E2 conjugated activatable antibody includes at least 90% of the conjugated activatable antibody having a drug load of 2. In some embodiments, a preparation of E2 conjugated activatable antibody includes at least 95% of the conjugated activatable antibody having a drug load of 2. In some embodiments, a preparation of E2 conjugated activatable antibody includes at least 98% of the conjugated activatable antibody having a drug load of 2.

In some embodiments, a preparation of conjugated activatable antibody that is purified or enriched for given drug load species includes more molar equivalents of the given drug load species than the total molar equivalents of all other drug load species combined. For example, in some embodiments, a preparation of E2 conjugated activatable antibody is such that the combined equivalents of each of the conjugated activatable antibody species of the composition in which the drug load is not 2 is less than the equivalents of the conjugated activatable antibody with a drug load of 2. In some embodiments, a preparation of E4 conjugated activatable antibody is such that the combined equivalents of each of the conjugated activatable antibody species of the composition in which the drug load is not 4 is less than the equivalents of the conjugated activatable antibody with a drug load of 4. In some embodiments, a preparation of DE conjugated activatable antibody is such that the combined equivalents of each of the conjugated activatable antibody species of the composition in which the drug load is not 2 or 4 is less than the equivalents of the conjugated activatable antibody that have a drug load of 2 or 4.

In some embodiments, a preparation of conjugated activatable antibody that is purified or enriched for given DAR species (e.g. E2 or E4, as discussed herein) includes less than a certain percentage of the conjugated activatable antibody that is not the given drug load species. For example, in some embodiments, a preparation of E2 conjugated activatable antibody includes less than 50% of the conjugated activatable antibody that is not the drug load of 2 species. For example, in some embodiments, a preparation of E2 conjugated activatable antibody includes less than 25% of the conjugated activatable antibody that is not the drug load of 2 species. For example, in some embodiments, a preparation of E2 conjugated activatable antibody includes less than 15% of the conjugated activatable antibody that is not the drug load of 2 species. For example, in some embodiments, a preparation of E2 conjugated activatable antibody includes less than 10% of the conjugated activatable antibody that is not the drug load of 2 species. For example, in some embodiments, a preparation of E2 conjugated activatable antibody includes less than 5% of the conjugated activatable antibody that is not the drug load of 2 species. For example, in some embodiments, a preparation of E2 conjugated activatable antibody includes less than 2% of the conjugated activatable antibody that is not the drug load of 2 species. In some other embodiments, or enriched or purified preparations (e.g. E4 or DE) may have less than 50%, less than 25%, less than 15%, less than 10%, less than 5%, or less than 2% that is not the corresponding drug load species in the enriched or purified conjugated activatable antibody composition.

In some embodiments, the agent is an anti-inflammatory agent.

In some embodiments, the conjugated It activatable antibody also includes a detectable moiety. In some embodiments, the detectable moiety is a diagnostic agent.

In some embodiments, the conjugated activatable antibody also includes a signal peptide. In some embodiments, the signal peptide is conjugated to the activatable antibody via a spacer. In some embodiments, the spacer is conjugated to the activatable antibody in the absence of a signal peptide. In some embodiments, the spacer is joined directly to the MM of the activatable antibody. In some embodiments, the spacer is joined directly to the MM of the activatable antibody in the structural arrangement from N-terminus to C-terminus of spacer-MM-CM-AB. An example of a spacer joined directly to the N-terminus of MM of the activatable antibody is QGQSGQ (SEQ ID NO: 109). Other examples of a spacer joined directly to the N-terminus of MM of the activatable antibody include QGQSGQG (SEQ ID NO: 138), QGQSG (SEQ ID NO: 139), QGQS (SEQ ID NO: 140), QGQ, QG, and Q. Other examples of a spacer joined directly to the N-terminus of MM of the activatable antibody include GQSGQG (SEQ ID NO: 143), QSGQG (SEQ ID NO: 144), SGQG (SEQ ID NO: 145), GQG, and G. In some embodiments, no spacer is joined to the N-terminus of the MM. In some embodiments, the spacer includes at least the amino acid sequence QGQSGQ (SEQ ID NO: 109). In some embodiments, the spacer includes at least the amino acid sequence QGQSGQG (SEQ ID NO: 138). In some embodiments, the spacer includes at least the amino acid sequence QGQSG (SEQ ID NO: 139). In some embodiments, the spacer includes at least the amino acid sequence QGQS (SEQ ID NO: 140). In some embodiments, the spacer includes at least the amino acid sequence QGQ. In some embodiments, the spacer includes at least the amino acid sequence QG. In some embodiments, the spacer includes at least the amino acid residue Q. In some embodiments, the spacer includes at least the amino acid sequence GQSGQG (SEQ ID NO: 143). In some embodiments, the spacer includes at least the amino acid sequence QSGQG (SEQ ID NO: 144). In some embodiments, the spacer includes at least the amino acid sequence SGQG (SEQ ID NO: 145). In some embodiments, the spacer includes at least the amino acid sequence GQG. In some embodiments, the spacer includes at least the amino acid sequence G. In some embodiments, the spacer is absent.

In some embodiments, the AB of the conjugated activatable antibody naturally contains one or more disulfide bonds. In some embodiments, the AB can be engineered to include one or more disulfide bonds.

In some embodiments, the activatable antibody of the conjugated activatable antibody of the disclosure is encoded by a nucleic acid sequence that comprises a nucleic acid sequence encoding a heavy chain variable region amino acid sequence selected from the group consisting of SEQ ID NO: 5. In some embodiments, the activatable antibody of the conjugated activatable antibody of the disclosure is encoded by a nucleic acid sequence that comprises a nucleic acid sequence encoding a light chain variable region amino acid sequence selected from the group consisting of SEQ ID NO: 7. In some embodiments, the activatable antibody of the conjugated activatable antibody of the disclosure is encoded by a nucleic acid sequence that comprises a nucleic acid sequence encoding a heavy chain variable region amino acid sequence selected from the group consisting of SEQ ID NO: 5, and a nucleic acid sequence encoding a light chain variable region amino acid sequence selected from the group consisting of SEQ ID NO: 7.

In some embodiments, the activatable antibody of the conjugated activatable antibody of the disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a heavy chain variable region amino acid sequence selected from the group consisting of SEQ ID NO: 5. In some embodiments, the activatable antibody of the conjugated activatable antibody of the disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a light chain variable region amino acid sequence selected from the group consisting of SEQ ID NO: 7. In some embodiments, the activatable antibody of the conjugated activatable antibody of the disclosure is encoded by a nucleic acid sequence that comprises a nucleic acid sequence that is at least 90%, 91%, 92%, 93%, 94%, 95%, 96%, 97%, 98% or 99% identical to a nucleic acid sequence encoding a heavy chain variable region amino acid sequence selected from the group consisting of SEQ ID NO: 5, and a nucleic acid sequence that is at least 90%, 91%, 92%, 93%, 94%, 95%, 96%, 97%, 98% or 99% identical to a nucleic acid sequence encoding a light chain variable region amino acid sequence selected from the group consisting of SEQ ID NO: 7.

The disclosure also provides methods for producing an activatable antibody of the disclosure by culturing a cell under conditions that lead to expression of the activatable antibody, wherein the cell comprises a nucleic acid molecule of the disclosure or a vector of the disclosure.

The disclosure also provides methods of manufacturing an activatable antibody that, in an activated state, binds CD71, the method comprising: (a) culturing a cell comprising a nucleic acid construct that encodes the activatable antibody under conditions that lead to expression of the activatable antibody, wherein the activatable antibody comprises an activatable antibody of the disclosure; and (b) recovering the activatable antibody.

In some embodiments, the serum half-life of the conjugated activatable antibody is longer than that of the corresponding antibody; e.g., the pK of the conjugated activatable antibody is longer than that of the corresponding antibody. In some embodiments, the serum half-life of the conjugated activatable antibody is similar to that of the corresponding antibody. In some embodiments, the serum half-life of the conjugated activatable antibody is at least 15 days when administered to an organism. In some embodiments, the serum half-life of the conjugated activatable antibody is at least 12 days when administered to an organism. In some embodiments, the serum half-life of the conjugated activatable antibody is at least 11 days when administered to an organism. In some embodiments, the serum half-life of the conjugated activatable antibody is at least 10 days when administered to an organism. In some embodiments, the serum half-life of the conjugated activatable antibody is at least 9 days when administered to an organism. In some embodiments, the serum half-life of the conjugated activatable antibody is at least 8 days when administered to an organism. In some embodiments, the serum half-life of the conjugated activatable antibody is at least 7 days when administered to an organism. In some embodiments, the serum half-life of the activatable antibody is at least 6 days when administered to an organism. In some embodiments, the serum half-life of the activatable antibody is at least 5 days when administered to an organism. In some embodiments, the serum half-life of the activatable antibody is at least 4 days when administered to an organism. In some embodiments, the serum half-life of the activatable antibody is at least 3 days when administered to an organism. In some embodiments, the serum half-life of the activatable antibody is at least 2 days when administered to an organism. In some embodiments, the serum half-life of the activatable antibody is at least 24 hours when administered to an organism. In some embodiments, the serum half-life of the activatable antibody is at least 20 hours when administered to an organism. In some embodiments, the serum half-life of the activatable antibody is at least 18 hours when administered to an organism. In some embodiments, the serum half-life of the activatable antibody is at least 16 hours when administered to an organism. In some embodiments, the serum half-life of the activatable antibody is at least 14 hours when administered to an organism. In some embodiments, the serum half-life of the activatable antibody is at least 12 hours when administered to an organism. In some embodiments, the serum half-life of the activatable antibody is at least 10 hours when administered to an organism. In some embodiments, the serum half-life of the activatable antibody is at least 8 hours when administered to an organism. In some embodiments, the serum half-life of the activatable antibody is at least 6 hours when administered to an organism. In some embodiments, the serum half-life of the activatable antibody is at least 4 hours when administered to an organism. In some embodiments, the serum half-life of the activatable antibody is at least 3 hours when administered to an organism.

The disclosure also provides methods of producing an anti-CD71 antibody and/or activatable anti-CD71 antibody polypeptide by culturing a cell under conditions that lead to expression of the polypeptide, wherein the cell comprises an isolated nucleic acid molecule encoding an antibody and/or an activatable antibody described herein, and/or vectors that include these isolated nucleic acid sequences. The disclosure provides methods of producing an antibody and/or activatable antibody by culturing a cell under conditions that lead to expression of the antibody and/or activatable antibody, wherein the cell comprises an isolated nucleic acid molecule encoding an antibody and/or an activatable antibody described herein, and/or vectors that include these isolated nucleic acid sequences.

The invention provides methods of preventing, delaying the progression of, treating, alleviating a symptom of, or otherwise ameliorating an CD71 mediated disease in a subject by administering a therapeutically effective amount of a conjugated activatable anti-CD71 antibody described herein to a subject in need thereof.

The invention also provides methods of preventing, delaying the progression of, treating, alleviating a symptom of, or otherwise ameliorating cancer in a subject by administering a therapeutically effective amount of a conjugated activatable anti-CD71 antibody described herein to a subject in need thereof. CD71 is known to be expressed in a variety of cancers, such as, by way of non-limiting example, adenocarcinoma, bile duct (biliary) cancer, bladder cancer, breast cancer, e.g., triple-negative breast cancer and Her2-negative breast cancer; carcinoid cancer; cervical cancer; cholangiocarcinoma; colorectal; endometrial; glioma; head and neck cancer, e.g., head and neck squamous cell cancer; leukemia; liver cancer; lung cancer, e.g., NSCLC, SCLC; lymphoma; melanoma; osopharyngeal cancer; ovarian cancer; pancreatic cancer; prostate cancer, *e.g.,* metastatic castration-resistant prostate carcinoma; renal cancer; skin cancer; squamous cell cancer, stomach cancer; testis cancer; thyroid cancer; and urothelial cancer.

In some embodiments, the cancer is associated with a CD71-expressing tumor. In some embodiments, the cancer is due to a CD71-expressing tumor.

A conjugated activatable anti-CD71 antibody used in any of the embodiments of these methods and uses can be administered at any stage of the disease. For example, such a conjugated activatable anti-CD71 antibody can be administered to a patient suffering cancer of any stage, from early to metastatic. The terms subject and patient are used interchangeably herein.

In some embodiments, the subject is a mammal, such as a human or non-human primate.

The conjugated activatable anti-CD71 antibody and therapeutic formulations thereof are administered to a subject suffering from or susceptible to a disease or disorder associated with aberrant CD71 expression and/or activity. A subject suffering from or susceptible to a disease or disorder associated with aberrant CD71 expression and/or activity is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, e.g., blood, urine and/or stool analysis, to evaluate health status.

Administration of a conjugated activatable anti-CD71 antibody to a patient suffering from a disease or disorder associated with aberrant CD71 expression and/or activity is considered successful if any of a variety of laboratory or clinical objectives is achieved. For example, administration of a conjugated activatable anti-CD71 antibody to a patient suffering from a disease or disorder associated with aberrant CD71 expression and/or activity is considered successful if one or more of the symptoms associated with the disease or disorder is alleviated, reduced, inhibited or does not progress to a further, *i.e*., worse, state. Administration of a conjugated activatable anti-CD71 antibody to a patient suffering from a disease or disorder associated with aberrant CD71 expression and/or activity is considered successful if the disease or disorder enters remission or does not progress to a further, *i.e.,* worse, state.

In some embodiments, the conjugated activatable anti-CD71 antibody and therapeutic formulations thereof are administered to a subject suffering from or susceptible to a disease or disorder, such as subjects suffering from cancer or other neoplastic condition, wherein the subject's diseased cells are expressing CD71. In some embodiments, the diseased cells are associated with aberrant CD71 expression and/or activity. In some embodiments, the diseased cells are associated with normal CD71 expression and/or activity. A subject suffering from or susceptible to a disease or disorder wherein the subject's diseased cells express CD71 is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, e.g., blood, urine and/or stool analysis, to evaluate health status.

In some embodiments, the conjugated activatable anti-CD71 antibody and therapeutic formulations thereof are administered to a subject suffering from or susceptible to a disease or disorder associated with cells expressing CD71 or the presence, growth, proliferation, metastasis, and/or activity of such cells, such as subjects suffering from cancer or other neoplastic conditions. In some embodiments, the cells are associated with aberrant CD71 expression and/or activity. In some embodiments, the cells are associated with normal CD71 expression and/or activity. A subject suffering from or susceptible to a disease or disorder associated with cells that express CD71 is identified using any of a variety of methods known in the art. For example, subjects suffering from cancer or other neoplastic condition are identified using any of a variety of clinical and/or laboratory tests such as, physical examination and blood, urine and/or stool analysis to evaluate health status. For example, subjects suffering from inflammation and/or an inflammatory disorder are identified using any of a variety of clinical and/or laboratory tests such as physical examination and/or bodily fluid analysis, e.g., blood, urine and/or stool analysis, to evaluate health status.

Administration of a conjugated activatable anti-CD71 antibody to a patient suffering from a disease or disorder associated with cells expressing CD71 is considered successful if any of a variety of laboratory or clinical objectives is achieved. For example, administration of a conjugated activatable anti-CD71 antibody to a patient suffering from a disease or disorder associated with cells expressing CD71 is considered successful if one or more of the symptoms associated with the disease or disorder is alleviated, reduced, inhibited or does not progress to a further, *i.e*., worse, state. Administration of a conjugated activatable anti-CD71 antibody to a patient suffering from a disease or disorder associated with cells expressing CD71 is considered successful if the disease or disorder enters remission or does not progress to a further, *i.e*., worse, state.

The disclosure also provides methods of treating, alleviating a symptom of, or delaying the progression of a disorder or disease in which diseased cells express CD71 comprising administering a therapeutically effective amount of a conjugated antibody of the disclosure or a pharmaceutical composition comprising an antibody of the disclosure or a pharmaceutical composition comprising a conjugated antibody of the disclosure to a subject in need thereof. In some embodiments, the disorder or disease is cancer.

The disclosure also provides methods of treating, alleviating a symptom of, or delaying the progression of a disorder or disease associated with cells expressing CD71 comprising administering a therapeutically effective amount of a conjugated antibody of the disclosure or a pharmaceutical composition comprising a pharmaceutical composition comprising a conjugated antibody of the disclosure to a subject in need thereof. In some embodiments, the disorder or disease associated with cells expressing CD71 is cancer. In some embodiments, the cancer is an adenocarcinoma, a bile duct (biliary) cancer, a bladder cancer, a bone cancer, a breast cancer, a triple-negative breast cancer, a Her2-negative breast cancer, a carcinoid cancer, a cervical cancer, a cholangiocarcinoma, a colorectal cancer, a colon cancer, an endometrial cancer, a glioma, a head and neck cancer, a head and neck squamous cell cancer, a leukemia, a liver cancer, a lung cancer, a non-small cell lung cancer, a small cell lung cancer, a lymphoma, a melanoma, an oropharyngeal cancer, an ovarian cancer, a pancreatic cancer, a prostate cancer, a metastatic castration-resistant prostate carcinoma, a renal cancer, a sarcoma, a skin cancer, a squamous cell cancer, a stomach cancer, a testis cancer, a thyroid cancer, a urogenital cancer, or a urothelial cancer. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

The disclosure also provides methods of inhibiting or reducing the growth, proliferation, or metastasis of cells expressing mammalian CD71 comprising administering a therapeutically effective amount of an antibody of the disclosure or a conjugated antibody of the disclosure or a pharmaceutical composition comprising an antibody of the disclosure or a pharmaceutical composition comprising a conjugated antibody of the disclosure to a subject in need thereof. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

The disclosure also provides methods of inhibiting, blocking, or preventing the binding of a natural ligand to mammalian CD71, comprising administering a therapeutically effective amount of an antibody of the disclosure or a conjugated antibody of the disclosure or a pharmaceutical composition comprising an antibody of the disclosure or a pharmaceutical composition comprising a conjugated antibody of the disclosure to a subject in need thereof. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

The disclosure also provides methods of treating, alleviating a symptom of, or delaying the progression of a disorder or disease in which diseased cells express CD71 comprising administering a therapeutically effective amount of an activatable antibody of the disclosure or a conjugated activatable antibody of the disclosure or a pharmaceutical composition comprising an activatable antibody of the disclosure or a pharmaceutical composition comprising a conjugated activatable antibody of the disclosure to a subject in need thereof. In some embodiments, the disorder or disease is cancer.

The disclosure also provides methods of treating, alleviating a symptom of, or delaying the progression of a disorder or disease associated with cells expressing CD71 comprising administering a therapeutically effective amount of a conjugated activatable antibody of the disclosure or a pharmaceutical composition comprising a conjugated activatable antibody of the disclosure to a subject in need thereof. In some embodiments, the disorder or disease associated with cells expressing CD71 is cancer. In some embodiments, the cancer is an adenocarcinoma, a bile duct (biliary) cancer, a bladder cancer, a bone cancer, a breast cancer, a triple-negative breast cancer, a Her2-negative breast cancer, a carcinoid cancer, a cervical cancer, a cholangiocarcinoma, a colorectal cancer, a colon cancer, an endometrial cancer, a glioma, a head and neck cancer, a head and neck squamous cell cancer, a leukemia, a liver cancer, a lung cancer, a non-small cell lung cancer, a small cell lung cancer, a lymphoma, a melanoma, an oropharyngeal cancer, an ovarian cancer, a pancreatic cancer, a prostate cancer, a metastatic castration-resistant prostate carcinoma, a renal cancer, a sarcoma, a skin cancer, a squamous cell cancer, a stomach cancer, a testis cancer, a thyroid cancer, a urogenital cancer, or a urothelial cancer. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

The disclosure also provides methods of inhibiting or reducing the growth, proliferation, or metastasis of cells expressing mammalian CD71 comprising administering a therapeutically effective amount of an activatable antibody of the disclosure or a conjugated activatable antibody of the disclosure or a pharmaceutical composition comprising an activatable antibody of the disclosure or a pharmaceutical composition comprising a conjugated activatable antibody of the disclosure to a subject in need thereof. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

The disclosure also provides methods of inhibiting, blocking, or preventing the binding of a natural ligand to mammalian CD71, comprising administering a therapeutically effective amount of a conjugated activatable antibody of the disclosure or a pharmaceutical composition comprising a conjugated activatable antibody of the disclosure to a subject in need thereof. In some embodiments, the natural ligand is transferrin. In some embodiments, the expression and/or activity of the mammalian CD71 is aberrant. In some embodiments, the method comprises administering an additional agent. In some embodiments, the additional agent is a therapeutic agent.

In some embodiments of these methods and kits, the anti-CD71 conjugated activatable antibody includes a detectable label. In some embodiments of these methods and kits, the detectable label includes an imaging agent, a contrasting agent, an enzyme, a fluorescent label, a chromophore, a dye, one or more metal ions, or a ligand-based label. In some embodiments of these methods and kits, the imaging agent comprises a radioisotope. In some embodiments of these methods and kits, the radioisotope is indium or technetium. In some embodiments of these methods and kits, the contrasting agent comprises iodine, gadolinium or iron oxide. In some embodiments of these methods and kits, the enzyme comprises horseradish peroxidase, alkaline phosphatase, or β-galactosidase. In some embodiments of these methods and kits, the fluorescent label comprises yellow fluorescent protein (YFP), cyan fluorescent protein (CFP), green fluorescent protein (GFP), modified red fluorescent protein (mRFP), red fluorescent protein tdimer2 (RFP tdimer2), HCRED, or a europium derivative. In some embodiments of these methods and kits, the luminescent label comprises an N-methylacrydium derivative. In some embodiments of these methods, the label comprises an Alexa Fluor® label, such as Alex Fluor® 680 or Alexa Fluor® 750. In some embodiments of these methods and kits, the ligand-based label comprises biotin, avidin, streptavidin or one or more haptens.

In some embodiments of these methods and kits, the subject is a mammal. In some embodiments of these methods, the subject is a human. In some embodiments, the subject is a non-human mammal, such as a non-human primate, companion animal (e.g., cat, dog, horse), farm animal, work animal, or zoo animal. In some embodiments, the subject is a rodent.

In some embodiments of these methods and kits, the method is an *in vivo* method. In some embodiments of these methods, the method is an *in situ* method. In some embodiments of these methods, the method is an *ex vivo* method. In some embodiments of these methods, the method is an *in vitro* method.

In some embodiments of the methods and kits, the method is used to identify or otherwise refine a patient population suitable for treatment with an anti-CD71 conjugated activatable antibody of the disclosure, followed by treatment by administering that conjugated activatable anti-CD71 antibody to a subject in need thereof. For example, patients that test positive for both the target (e.g., CD71) and a protease that cleaves the substrate in the cleavable moiety (CM) of the anti-CD71 activatable antibody being tested in these methods are identified as suitable candidates for treatment with such an anti-CD71 activatable antibody comprising such a CM, and the patient is then administered a therapeutically effective amount of the activatable anti-CD71 antibody and/or conjugated activatable anti-CD71 antibody that was tested. Likewise, patients that test negative for either or both of the target (*e.g.,* CD71) and the protease that cleaves the substrate in the CM in the activatable antibody being tested using these methods might be identified as suitable candidates for another form of therapy. In some embodiments, such patients can be tested with other anti-CD71 activatable antibodies until a suitable anti-CD71 activatable antibody for treatment is identified (e.g., an anti-CD71 activatable antibody comprising a CM that is cleaved by the patient at the site of disease). In some embodiments, the patient is then administered a therapeutically effective amount of the activatable anti-CD71 antibody and/or conjugated for which the patient tested positive. Suitable AB, MM, and/or CM include any of the AB, MM, and/or CM disclosed herein.

Pharmaceutical compositions according to the invention can include an antibody of the invention and optionally a pharmaceutically acceptable carrier. These pharmaceutical compositions can be included in kits, such as, for example, diagnostic kits.

In some embodiments, the pharmaceutical composition comprises a conjugated activatable antibody of the disclosure, and optionally a pharmaceutically acceptable carrier. In some embodiments, the pharmaceutical composition comprises an additional agent. In some embodiments, the additional agent is a therapeutic agent.

The anti-CD71 antibodies and the ABs in the activatable antibodies of the disclosure specifically bind a CD71 target, such as, for example, mammalian CD71, and/or human CD71. Also included in the disclosure are anti-CD71 antibodies and ABs that bind to the same CD71 epitope as an antibody of the disclosure and/or an activated activatable antibody described herein. Also included in the disclosure are anti-CD71 antibodies and ABs that compete with an anti-CD71 antibody and/or an activated anti-CD71 activatable antibody described herein for binding to a CD71 target, e.g., human CD71. Also included in the disclosure are anti-CD71 antibodies and ABs that cross-compete with an anti-CD71 antibody and/or an activated anti-CD71 activatable antibody described herein for binding to a CD71 target, e.g., human CD71.

The activatable anti-CD71 antibodies provided herein include a masking moiety. In some embodiments, the masking moiety is an amino acid sequence that is coupled or otherwise attached to the anti-CD71 antibody and is positioned within the activatable anti-CD71 antibody construct such that the masking moiety reduces the ability of the anti-CD71 antibody to specifically bind CD71. Suitable masking moieties are identified using any of a variety of known techniques. For example, peptide masking moieties are identified using the methods described in PCT Publication No. WO 2009/025846 by Daugherty et al., the contents of which are hereby incorporated by reference in their entirety.

The activatable anti-CD71 antibodies provided herein include a cleavable moiety. In some embodiments, the cleavable moiety includes an amino acid sequence that is a substrate for a protease, usually an extracellular protease. Suitable substrates are identified using any of a variety of known techniques. For example, peptide substrates are identified using the methods described in U.S. Patent No. 7,666,817 by Daugherty et al.; in U.S. Patent No. 8,563,269 by Stagliano et al.; and in PCT Publication No. WO 2014/026136 by La Porte et al., the contents of each of which are hereby incorporated by reference in their entirety. (*See also* Boulware et al. "Evolutionary optimization of peptide substrates for proteases that exhibit rapid hydrolysis kinetics." Biotechnol. Bioeng. 106.3 (2010): 339-46).

Exemplary substrates include but are not limited to substrates cleavable by one or more of the following enzymes or proteases listed in Table (I).

**Table (I): Exemplary Proteases and/or Enzymes**

| | | |
|---|---|---|
| ADAMS, ADAMTS, *e.g*. | Cysteine proteinases, *e.g*., | Serine proteases, *e.g*., |
| ADAM8 | Cruzipain | activated protein C |
| ADAM9 | Legumain | Cathepsin A |
| ADAM10 | Otubain-2 | Cathepsin G |
| ADAM12 | | Chymase |
| ADAM15 | KLKs, *e.g*., | coagulation factor proteases |
| ADAM17/TACE | KLK4 | (*e.g.,* FVIIa, FIXa, FXa, FXIa, FXIIa) |
| ADAMDEC1 | KLK5 | |
| ADAMTS1 | KLK6 | Elastase |
| ADAMTS4 | KLK7 | Granzyme B |
| ADAMTS5 | KLK8 | Guanidinobenzoatase |
| | KLK10 | HtrA1 |
| Aspartate proteases, *e.g*., | KLK11 | Human Neutrophil Elastase |
| BACE | KLK13 | Lactoferrin |
| Renin | KLK14 | Marapsin |
| | | NS3/4A |
| Aspartic cathepsins, *e.g.*, | Metallo proteinases, *e.g.,* | PACE4 |
| Cathepsin D | Meprin | Plasmin |
| Cathepsin E | Neprilysin | PSA |
| | PSMA | tPA |
| Caspases, *e.g.*, | BMP-1 | Thrombin |
| Caspase 1 | | Tryptase |
| Caspase 2 | MMPs, *e.g.,* | uPA |
| Caspase 3 | MMP1 | |
| Caspase 4 | MMP2 | Type II Transmembrane |
| Caspase 5 | MMP3 | Serine Proteases (TTSPs), *e.g.,* |
| Caspase 6 | MMP7 | DESC1 |
| Caspase 7 | MMP8 | DPP-4 |
| Caspase 8 | MMP9 | FAP |
| Caspase 9 | MMP10 | Hepsin |
| Caspase 10 | MMP11 | Matriptase-2 |
| Caspase 14 | MMP12 | MT-SP1/Matriptase |
| | MMP13 | TMPRSS2 |
| Cysteine cathepsins, *e.g.*, | MMP14 | TMPRSS3 |
| Cathepsin B | MMP15 | TMPRSS4 |
| Cathepsin C | MMP16 | |
| Cathepsin K | MMP17 | |
| Cathepsin L | MMP19 | |
| Cathepsin S | MMP20 | |
| Cathepsin V/L2 | MMP23 | |
| Cathepsin X/Z/P | MMP24 | |
| | MMP26 | |
| | MMP27 | |

The activatable anti-CD71 antibodies described herein overcome a limitation of antibody therapeutics, particularly antibody therapeutics that are known to be toxic to at least some degree *in vivo.* Target-mediated toxicity constitutes a major limitation for the development of therapeutic antibodies. The activatable anti-CD71 antibodies provided herein are designed to address the toxicity associated with the inhibition of the target in normal tissues by traditional therapeutic antibodies. These activatable anti-CD71 antibodies remain masked until proteolytically activated at the site of disease. Starting with an anti-CD71 antibody as a parental therapeutic antibody, the activatable anti-CD71 antibodies of the invention were engineered by coupling the antibody to an inhibitory mask through a linker that incorporates a protease substrate.

When the AB is modified with a MM and is in the presence of the target, specific binding of the AB to its target is reduced or inhibited, as compared to the specific binding of the AB not modified with an MM or the specific binding of the parental AB to the target.

The K_{d} of the AB modified with a MM towards the target is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times greater than the K_{d} of the AB not modified with an MM or of the parental AB towards the target. Conversely, the binding affinity of the AB modified with a MM towards the target is at least 2, 3, 4, 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times lower than the binding affinity of the AB not modified with an MM or of the parental AB towards the target.

The dissociation constant (K_{d}) of the MM towards the AB is generally greater than the K_{d} of the AB towards the target. The K_{d} of the MM towards the AB can be at least 5, 10, 25, 50, 100, 250, 500, 1,000, 2,500, 5,000, 10,000, 100,000, 1,000,000 or even 10,000,000 times greater than the K_{d} of the AB towards the target. Conversely, the binding affinity of the MM towards the AB is generally lower than the binding affinity of the AB towards the target. The binding affinity of MM towards the AB can be at least 5, 10, 25, 50, 100, 250, 500, 1,000, 2,500, 5,000, 10,000, 100,000, 1,000,000 or even 10,000,000 times lower than the binding affinity of the AB towards the target.

In some embodiments, the dissociation constant (K_{d}) of the MM towards the AB is approximately equal to the K_{d} of the AB towards the target. In some embodiments, the dissociation constant (K_{d}) of the MM towards the AB is no more than the dissociation constant of the AB towards the target.

In some embodiments, the dissociation constant (K_{d}) of the MM towards the AB is less than the dissociation constant of the AB towards the target.

In some embodiments, the dissociation constant (K_{d}) of the MM towards the AB is greater than the dissociation constant of the AB towards the target.

In some embodiments, the MM has a K_{d} for binding to the AB that is no more than the K_{d} for binding of the AB to the target.

In some embodiments, the MM has a K_{d} for binding to the AB that is no less than the K_{d} for binding of the AB to the target.

In some embodiments, the MM has a K_{d} for binding to the AB that is approximately equal to the K_{d} for binding of the AB to the target.

In some embodiments, the MM has a K_{d} for binding to the AB that is less than the K_{d} for binding of the AB to the target.

In some embodiments, the MM has a K_{d} for binding to the AB that is greater than the K_{d} for binding of the AB to the target.

In some embodiments, the MM has a K_{d} for binding to the AB that is no more than 2, 3, 4, 5, 10, 25, 50, 100, 250, 500, or 1,000 fold greater than the K_{d} for binding of the AB to the target. In some embodiments, the MM has a K_{d} for binding to the AB that is between 1-5, 2-5, 2-10, 5-10, 5-20, 5-50, 5-100, 10-100, 10-1,000, 20-100, 20-1000, or 100-1,000 fold greater than the K_{d} for binding of the AB to the target.

In some embodiments, the MM has an affinity for binding to the AB that is less than the affinity of binding of the AB to the target.

In some embodiments, the MM has an affinity for binding to the AB that is no more than the affinity of binding of the AB to the target.

In some embodiments, the MM has an affinity for binding to the AB that is approximately equal of the affinity of binding of the AB to the target.

In some embodiments, the MM has an affinity for binding to the AB that is no less than the affinity of binding of the AB to the target.

In some embodiments, the MM has an affinity for binding to the AB that is greater than the affinity of binding of the AB to the target.

In some embodiments, the MM has an affinity for binding to the AB that is 2, 3, 4, 5, 10, 25, 50, 100, 250, 500, or 1,000 less than the affinity of binding of the AB to the target. I In some embodiments, the MM has an affinity for binding to the AB that is between 1-5, 2-5, 2-10, 5-10, 5-20, 5-50, 5-100, 10-100, 10-1,000, 20-100, 20-1000, or 100-1,000 fold less than the affinity of binding of the AB to the target. In some embodiments, the MM has an affinity for binding to the AB that is 2 to 20 fold less than the affinity of binding of the AB to the target. In some embodiments, a MM not covalently linked to the AB and at equimolar concentration to the AB does not inhibit the binding of the AB to the target.

When the AB is modified with a MM and is in the presence of the target specific binding of the AB to its target is reduced or inhibited, as compared to the specific binding of the AB not modified with an MM or the specific binding of the parental AB to the target. When compared to the binding of the AB not modified with an MM or the binding of the parental AB to the target the AB's ability to bind the target when modified with an MM can be reduced by at least 50%, 60%, 70%, 80%, 90%, 92%, 93%, 94%, 95%, 96%, 97%, 98%, 99% and even 100% for at least 2, 4, 6, 8, 12, 28, 24, 30, 36, 48, 60, 72, 84, or 96 hours, or 5, 10, 15, 30, 45, 60, 90, 120, 150, or 180 days, or 1, 2, 3, 4, 5, 6, 7, 8, 9, 10, 11, or 12 months or more when measured *in vivo* or in an *in vitro* assay.

The MM inhibits the binding of the AB to the target. The MM binds the antigen binding domain of the AB and inhibits binding of the AB to the target. The MM can sterically inhibit the binding of the AB to the target. The MM can allosterically inhibit the binding of the AB to its target. In these embodiments when the AB is modified or coupled to a MM and in the presence of target there is no binding or substantially no binding of the AB to the target, or no more than 0.001%, 0.01%, 0.1%, 1%, 2%, 3%, 4%, 5%, 6%, 7%, 8%, 9%, 10%, 15%, 20%, 25%, 30%, 35%, 40%, or 50% binding of the AB to the target, as compared to the binding of the AB not modified with an MM, the parental AB, or the AB not coupled to an MM to the target, for at least 2, 4, 6, 8, 12, 28, 24, 30, 36, 48, 60, 72, 84, or 96 hours, or 5, 10, 15, 30, 45, 60, 90, 120, 150, or 180 days, or 1, 2, 3, 4, 5, 6, 7, 8, 9, 10, 11, or 12 months or longer when measured *in vivo* or in an *in vitro* assay.

When an AB is coupled to or modified by a MM, the MM 'masks' or reduces or otherwise inhibits the specific binding of the AB to the target. When an AB is coupled to or modified by a MM, such coupling or modification can effect a structural change that reduces or inhibits the ability of the AB to specifically bind its target.

An AB coupled to or modified with an MM can be represented by the following formulae (in order from an amino (N) terminal region to carboxyl (C) terminal region:
(MM)-(AB)
(AB)-(MM)
(MM)-L-(AB)
(AB)-L-(MM)
where MM is a masking moiety, the AB is an antibody or antibody fragment thereof, and the L is a linker. In many embodiments, it can be desirable to insert one or more linkers, e.g., flexible linkers, into the composition so as to provide for flexibility.

In certain embodiments, the MM is not a natural binding partner of the AB. In some embodiments, the MM contains no or substantially no homology to any natural binding partner of the AB. In some embodiments, the MM is no more than 5%, 10%, 15%, 20%, 25%, 30%, 35%, 40%, 45%, 50%, 55%, 60%, 65%, 70%, 75%, or 80% similar to any natural binding partner of the AB. In some embodiments, the MM is no more than 5%, 10%, 15%, 20%, 25%, 30%, 35%, 40%, 45%, 50%, 55%, 60%, 65%, 70%, 75%, or 80% identical to any natural binding partner of the AB. In some embodiments, the MM is no more than 25% identical to any natural binding partner of the AB. In some embodiments, the MM is no more than 50% identical to any natural binding partner of the AB. In some embodiments, the MM is no more than 20% identical to any natural binding partner of the AB. In some embodiments, the MM is no more than 10% identical to any natural binding partner of the AB.

In some embodiments, the activatable antibodies include an AB that is modified by an MM and also includes one or more cleavable moieties (CM). Such activatable antibodies exhibit activatable/switchable binding, to the AB's target. Activatable antibodies generally include an antibody or antibody fragment (AB), modified by or coupled to a masking moiety (MM) and a modifiable or cleavable moiety (CM). In some embodiments, the CM contains an amino acid sequence that serves as a substrate for at least one protease.

The elements of the activatable antibodies are arranged so that the MM and CM are positioned such that in a cleaved (or relatively active) state and in the presence of a target, the AB binds a target while the activatable antibody is in an uncleaved (or relatively inactive) state in the presence of the target, specific binding of the AB to its target is reduced or inhibited. The specific binding of the AB to its target can be reduced due to the inhibition or masking of the AB's ability to specifically bind its target by the MM

The K_{d} of the AB modified with a MM and a CM towards the target is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times greater than the K_{d} of the AB not modified with an MM and a CM or of the parental AB towards the target. Conversely, the binding affinity of the AB modified with a MM and a CM towards the target is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times lower than the binding affinity of the AB not modified with an MM and a CM or of the parental AB towards the target.

When the AB is modified with a MM and a CM and is in the presence of the target but not in the presence of a modifying agent (for example at least one protease), specific binding of the AB to its target is reduced or inhibited, as compared to the specific binding of the AB not modified with an MM and a CM or of the parental AB to the target. When compared to the binding of the parental AB or the binding of an AB not modified with an MM and a CM to its target, the AB's ability to bind the target when modified with an MM and a CM can be reduced by at least 50%, 60%, 70%, 80%, 90%, 92%, 93%, 94%, 95%, 96%, 97%, 98%, 99% and even 100% for at least 2, 4, 6, 8, 12, 28, 24, 30, 36, 48, 60, 72, 84, or 96 hours or 5, 10, 15, 30, 45, 60, 90, 120, 150, or 180 days, or 1, 2, 3, 4, 5, 6, 7, 8, 9, 10, 11, or 12 months or longer when measured *in vivo* or in an *in vitro* assay.

As used herein, the term cleaved state refers to the condition of the activatable antibodies following modification of the CM by at least one protease. The term uncleaved state, as used herein, refers to the condition of the activatable antibodies in the absence of cleavage of the CM by a protease. As discussed above, the term "activatable antibodies" is used herein to refer to an activatable antibody in both its uncleaved (native) state, as well as in its cleaved state. It will be apparent to the ordinarily skilled artisan that in some embodiments a cleaved activatable antibody may lack an MM due to cleavage of the CM by protease, resulting in release of at least the MM (*e.g.,* where the MM is not joined to the activatable antibodies by a covalent bond (*e.g.,* a disulfide bond between cysteine residues).

By activatable or switchable is meant that the activatable antibody exhibits a first level of binding to a target when the activatable antibody is in a inhibited, masked or uncleaved state (*i.e.,* a first conformation), and a second level of binding to the target in the uninhibited, unmasked and/or cleaved state (*i.e.,* a second conformation), where the second level of target binding is greater than the first level of binding. In general, the access of target to the AB of the activatable antibody is greater in the presence of a cleaving agent capable of cleaving the CM, i.e., a protease, than in the absence of such a cleaving agent. Thus, when the activatable antibody is in the uncleaved state, the AB is inhibited from target binding and can be masked from target binding (*i.e.,* the first conformation is such the AB cannot bind the target), and in the cleaved state the AB is not inhibited or is unmasked to target binding.

The CM and AB of the activatable antibodies are selected so that the AB represents a binding moiety for a given target, and the CM represents a substrate for a protease. In some embodiments, the protease is co-localized with the target at a treatment site or diagnostic site in a subject. As used herein, co-localized refers to being at the same site or relatively close nearby. In some embodiments, a protease cleaves a CM yielding an activated antibody that binds to a target located nearby the cleavage site. The activatable antibodies disclosed herein find particular use where, for example, a protease capable of cleaving a site in the CM, i.e., a protease, is present at relatively higher levels in target-containing tissue of a treatment site or diagnostic site than in tissue of non-treatment sites (for example in healthy tissue). In some embodiments, a CM of the disclosure is also cleaved by one or more other proteases. In some embodiments, it is the one or more other proteases that is co-localized with the target and that is responsible for cleavage of the CM *in vivo.*

In some embodiments activatable antibodies provide for reduced toxicity and/or adverse side effects that could otherwise result from binding of the AB at non-treatment sites if the AB were not masked or otherwise inhibited from binding to the target.

In general, an activatable antibody can be designed by selecting an AB of interest and constructing the remainder of the activatable antibody so that, when conformationally constrained, the MM provides for masking of the AB or reduction of binding of the AB to its target. Structural design criteria can be to be taken into account to provide for this functional feature.

Activatable antibodies exhibiting a switchable phenotype of a desired dynamic range for target binding in an inhibited versus an uninhibited conformation are provided. Dynamic range generally refers to a ratio of (a) a maximum detected level of a parameter under a first set of conditions to (b) a minimum detected value of that parameter under a second set of conditions. For example, in the context of an activatable antibody, the dynamic range refers to the ratio of (a) a maximum detected level of target protein binding to an activatable antibody in the presence of at least one protease capable of cleaving the CM of the activatable antibodies to (b) a minimum detected level of target protein binding to an activatable antibody in the absence of the protease. The dynamic range of an activatable antibody can be calculated as the ratio of the dissociation constant of an activatable antibody cleaving agent (*e.g.*, enzyme) treatment to the dissociation constant of the activatable antibodies cleaving agent treatment. The greater the dynamic range of an activatable antibody, the better the switchable phenotype of the activatable antibody. Activatable antibodies having relatively higher dynamic range values (*e.g.*, greater than 1) exhibit more desirable switching phenotypes such that target protein binding by the activatable antibodies occurs to a greater extent (*e.g.,* predominantly occurs) in the presence of a cleaving agent (*e.g.,* enzyme) capable of cleaving the CM of the activatable antibodies than in the absence of a cleaving agent.

Activatable antibodies can be provided in a variety of structural configurations. Exemplary formulae for activatable antibodies are provided below. It is specifically contemplated that the N- to C-terminal order of the AB, MM and CM can be reversed within an activatable antibody. It is also specifically contemplated that the CM and MM may overlap in amino acid sequence, *e.g.*, such that the CM is contained within the MM

For example, activatable antibodies can be represented by the following formula (in order from an amino (N) terminal region to carboxyl (C) terminal region:
(MM)-(CM)-(AB)
(AB)-(CM)-(MM)
where MM is a masking moiety, CM is a cleavable moiety, and AB is an antibody or fragment thereof. It should be noted that although MM and CM are indicated as distinct components in the formulae above, in all exemplary embodiments (including formulae) disclosed herein it is contemplated that the amino acid sequences of the MM and the CM could overlap, *e.g.*, such that the CM is completely or partially contained within the MM. In addition, the formulae above provide for additional amino acid sequences that can be positioned N-terminal or C-terminal to the activatable antibodies elements.

In certain embodiments, the MM is not a natural binding partner of the AB. In some embodiments, the MM contains no or substantially no homology to any natural binding partner of the AB. In some embodiments, the MM is no more than 5%, 10%, 15%, 20%, 25%, 30%, 35%, 40%, 45%, 50%, 55%, 60%, 65%, 70%, 75%, or 80% similar to any natural binding partner of the AB. In some embodiments, the MM is no more than 5%, 10%, 15%, 20%, 25%, 30%, 35%, 40%, 45%, 50%, 55%, 60%, 65%, 70%, 75%, or 80% identical to any natural binding partner of the AB. In some embodiments, the MM is no more than 50% identical to any natural binding partner of the AB. In some embodiments, the MM is no more than 25% identical to any natural binding partner of the AB. In some embodiments, the MM is no more than 20% identical to any natural binding partner of the AB. In some embodiments, the MM is no more than 10% identical to any natural binding partner of the AB.

In many embodiments it may be desirable to insert one or more linkers, *e.g.*, flexible linkers, into the activatable antibody construct so as to provide for flexibility at one or more of the MM-CM junction, the CM-AB junction, or both. For example, the AB, MM, and/or CM may not contain a sufficient number of residues (*e.g.*, Gly, Ser, Asp, Asn, especially Gly and Ser, particularly Gly) to provide the desired flexibility. As such, the switchable phenotype of such activatable antibody constructs may benefit from introduction of one or more amino acids to provide for a flexible linker. In addition, as described below, where the activatable antibody is provided as a conformationally constrained construct, a flexible linker can be operably inserted to facilitate formation and maintenance of a cyclic structure in the uncleaved activatable antibody.

For example, in certain embodiments an activatable antibody comprises one of the following formulae (where the formula below represent an amino acid sequence in either N- to C-terminal direction or C- to N-terminal direction):
(MM)-LP1-(CM)-(AB)
(MM)-(CM)-LP2-(AB)
(MM)-LP1-(CM)-LP2-(AB)
wherein MM, CM, and AB are as defined above; wherein LP1 and LP2 are each independently and optionally present or absent, are the same or different flexible linkers that include at least 1 flexible amino acid (*e.g.,* Gly). In addition, the formulae above provide for additional amino acid sequences that can be positioned N-terminal or C-terminal to the activatable antibodies elements. Examples include, but are not limited to, targeting moieties (*e.g.*, a ligand for a receptor of a cell present in a target tissue) and serum half-life extending moieties (*e.g.*, polypeptides that bind serum proteins, such as immunoglobulin (*e.g.,* IgG) or serum albumin (*e.g.,* human serum albumin (HAS)).

The CM is specifically cleaved by at least one protease at a rate of about 0.001-1500 x 10⁴ M⁻¹S⁻¹ or at least 0.001, 0.005, 0.01, 0.05, 0.1, 0.5, 1, 2.5, 5, 7.5, 10, 15, 20, 25, 50, 75, 100, 125, 150, 200, 250, 500, 750, 1000, 1250, or 1500 x 10⁴ M⁻¹S⁻¹. In some embodiments, the CM is specifically cleaved at a rate of about 100,000 M⁻¹S⁻¹. In some embodiments, the CM is specifically cleaved at a rate from about 1x10E2 to about 1x10E6 M⁻¹S⁻¹ (i.e., from about 1x10² to about 1x10⁶ M⁻¹S⁻¹).

For specific cleavage by an enzyme, contact between the enzyme and CM is made. When the activatable antibody comprising an AB coupled to a MM and a CM is in the presence of target and sufficient enzyme activity, the CM can be cleaved. Sufficient enzyme activity can refer to the ability of the enzyme to make contact with the CM and effect cleavage. It can readily be envisioned that an enzyme may be in the vicinity of the CM but unable to cleave because of other cellular factors or protein modification of the enzyme.

Linkers suitable for use in compositions described herein are generally ones that provide flexibility of the modified AB or the activatable antibodies to facilitate the inhibition of the binding of the AB to the target. Such linkers are generally referred to as flexible linkers. Suitable linkers can be readily selected and can be of any of a suitable of different lengths, such as from 1 amino acid (*e.g.,* Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and can be 1, 2, 3, 4, 5, 6, 7, 8, 9, 10, 11, 12, 13, 14, 15, 16, 17, 18, 19, or 20 amino acids in length.

Exemplary flexible linkers include glycine polymers (G)n, glycine-serine polymers (including, for example, (GS)n, (GSGGS)n (SEQ ID NO: 24) and (GGGS)n (SEQ ID NO: 25), where n is an integer of at least one), glycine-alanine polymers, alanine-serine polymers, and other flexible linkers known in the art. Glycine and glycine-serine polymers are relatively unstructured, and therefore may be able to serve as a neutral tether between components. Glycine accesses significantly more phi-psi space than even alanine, and is much less restricted than residues with longer side chains (see Scheraga, Rev. Computational Chem. 11173-142 (1992)). Exemplary flexible linkers include, but are not limited to Gly-Gly-Ser-Gly (SEQ ID NO: 26), Gly-Gly-Ser-Gly-Gly (SEQ ID NO: 27), Gly-Ser-Gly-Ser-Gly (SEQ ID NO: 28), Gly-Ser-Gly-Gly-Gly (SEQ ID NO: 29), Gly-Gly-Gly-Ser-Gly (SEQ ID NO: 30), Gly-Ser-Ser-Ser-Gly (SEQ ID NO: 31), and the like. The ordinarily skilled artisan will recognize that design of an activatable antibodies can include linkers that are all or partially flexible, such that the linker can include a flexible linker as well as one or more portions that confer less flexible structure to provide for a desired activatable antibodies structure.

The disclosure also provides compositions and methods that include an activatable anti-CD71 antibody that includes an antibody or antibody fragment (AB) that specifically binds CD71, where the AB is coupled to a masking moiety (MM) that decreases the ability of the AB to bind its target. In some embodiments, the activatable anti-CD71 antibody further includes a cleavable moiety (CM) that is a substrate for a protease. The compositions and methods provided herein enable the attachment of one or more agents to one or more cysteine residues in the AB without compromising the activity (e.g., the masking, activating or binding activity) of the activatable anti-CD71 antibody. In some embodiments, the compositions and methods provided herein enable the attachment of one or more agents to one or more cysteine residues in the AB without reducing or otherwise disturbing one or more disulfide bonds within the MM. The compositions and methods provided herein produce an activatable anti-CD71 antibody that is conjugated to one or more agents, *e.g.,* any of a variety of therapeutic, diagnostic and/or prophylactic agents, for example, in some embodiments, without any of the agent(s) being conjugated to the MM of the activatable anti-CD71 antibody. The compositions and methods provided herein produce conjugated activatable anti-CD71 antibodies in which the MM retains the ability to effectively and efficiently mask the AB of the activatable antibody in an uncleaved state. The compositions and methods provided herein produce conjugated activatable anti-CD71 antibodies in which the activatable antibody is still activated, *i.e*., cleaved, in the presence of a protease that can cleave the CM.

The activatable anti-CD71 antibodies have at least one point of conjugation for an agent, but in the methods and compositions provided herein less than all possible points of conjugation are available for conjugation to an agent. In some embodiments, the one or more points of conjugation are sulfur atoms involved in disulfide bonds. In some embodiments, the one or more points of conjugation are sulfur atoms involved in interchain disulfide bonds. In some embodiments, the one or more points of conjugation are sulfur atoms involved in interchain sulfide bonds, but not sulfur atoms involved in intrachain disulfide bonds. In some embodiments, the one or more points of conjugation are sulfur atoms of cysteine or other amino acid residues containing a sulfur atom. Such residues may occur naturally in the antibody structure or can be incorporated into the antibody by site-directed mutagenesis, chemical conversion, or mis-incorporation of non-natural amino acids.

Also provided are methods of preparing a conjugate of an activatable anti-CD71 antibody having one or more interchain disulfide bonds in the AB and one or more intrachain disulfide bonds in the MM, and a drug reactive with free thiols is provided. The method generally includes partially reducing interchain disulfide bonds in the activatable antibody with a reducing agent, such as, for example, TCEP; and conjugating the drug reactive with free thiols to the partially reduced activatable antibody. As used herein, the term partial reduction refers to situations where an activatable anti-CD71 antibody is contacted with a reducing agent and less than all disulfide bonds, *e.g.,* less than all possible sites of conjugation are reduced. In some embodiments, less than 99%, 98%, 97%, 96%, 95%, 90%, 85%, 80%, 75%, 70%, 65%, 60%, 55%, 50%, 45%, 40%, 35%, 30%, 25%, 20%, 15%, 10% or less than 5% of all possible sites of conjugation are reduced.

In yet other embodiments, a method of reducing and conjugating an agent, e.g., a drug, to an activatable anti-CD71 antibody resulting in selectivity in the placement of the agent is provided. The method generally includes partially reducing the activatable anti-CD71 antibody with a reducing agent such that any conjugation sites in the masking moiety or other non-AB portion of the activatable antibody are not reduced, and conjugating the agent to interchain thiols in the AB. The conjugation site(s) are selected so as to allow desired placement of an agent to allow conjugation to occur at a desired site. The reducing agent is, for example, TCEP. The reduction reaction conditions such as, for example, the ratio of reducing agent to activatable antibody, the length of incubation, the temperature during the incubation, the pH of the reducing reaction solution, etc., are determined by identifying the conditions that produce a conjugated activatable antibody in which the MM retains the ability to effectively and efficiently mask the AB of the activatable antibody in an uncleaved state. The ratio of reduction agent to activatable anti-CD71 antibody will vary depending on the activatable antibody. In some embodiments, the ratio of reducing agent to activatable anti-CD71 antibody will be in a range from about 20:1 to 1:1, from about 10:1 to 1:1, from about 9:1 to 1:1, from about 8:1 to 1:1, from about 7:1 to 1:1, from about 6:1 to 1:1, from about 5:1 to 1:1, from about 4:1 to 1:1, from about 3:1 to 1:1, from about 2:1 to 1:1, from about 20:1 to 1:1.5, from about 10:1 to 1:1.5, from about 9:1 to 1:1.5, from about 8:1 to 1:1.5, from about 7:1 to 1:1.5, from about 6:1 to 1:1.5, from about 5:1 to 1:1.5, from about 4:1 to 1:1.5, from about 3:1 to 1:1.5, from about 2:1 to 1:1.5, from about 1.5:1 to 1:1.5, or from about 1:1 to 1:1.5. In some embodiments, the ratio is in a range of from about 5:1 to 1:1. In some embodiments, the ratio is in a range of from about 5:1 to 1.5:1. In some embodiments, the ratio is in a range of from about 4:1 to 1:1. In some embodiments, the ratio is in a range from about 4:1 to 1.5:1. In some embodiments, the ratio is in a range from about 8:1 to about 1:1. In some embodiments, the ratio is in a range of from about 2.5:1 to 1:1.

In some embodiments, a method of reducing interchain disulfide bonds in the AB of an activatable anti-CD71 antibody and conjugating an agent, e.g., a thiol-containing agent such as a drug, to the resulting interchain thiols to selectively locate agent(s) on the AB is provided. The method generally includes partially reducing the AB with a reducing agent to form at least two interchain thiols without forming all possible interchain thiols in the activatable antibody; and conjugating the agent to the interchain thiols of the partially reduced AB. For example, the AB of the activatable antibody is partially reduced for about 1 hour at about 37°C at a desired ratio of reducing agent: activatable antibody. In some embodiments, the ratio of reducing agent to activatable antibody will be in a range from about 20:1 to 1:1, from about 10:1 to 1:1, from about 9:1 to 1:1, from about 8:1 to 1:1, from about 7:1 to 1:1, from about 6:1 to 1:1, from about 5:1 to 1:1, from about 4:1 to 1:1, from about 3:1 to 1:1, from about 2:1 to 1:1, from about 20:1 to 1:1.5, from about 10:1 to 1:1.5, from about 9:1 to 1:1.5, from about 8:1 to 1:1.5, from about 7:1 to 1:1.5, from about 6:1 to 1:1.5, from about 5:1 to 1:1.5, from about 4:1 to 1:1.5, from about 3:1 to 1:1.5, from about 2: 1 to 1:1.5, from about 1.5:1 to 1:1.5, or from about 1:1 to 1:1.5. In some embodiments, the ratio is in a range of from about 5:1 to 1:1. In some embodiments, the ratio is in a range of from about 5:1 to 1.5:1. In some embodiments, the ratio is in a range of from about 4:1 to 1:1. In some embodiments, the ratio is in a range from about 4:1 to 1.5:1. In some embodiments, the ratio is in a range from about 8:1 to about 1:1. In some embodiments, the ratio is in a range of from about 2.5:1 to 1:1.

The thiol-containing reagent can be, for example, cysteine or N-acetyl cysteine. The reducing agent can be, for example, TCEP. In some embodiments, the reduced activatable antibody can be purified prior to conjugation, using for example, column chromatography, dialysis, or diafiltration. Alternatively, the reduced antibody is not purified after partial reduction and prior to conjugation.

The invention also provides partially reduced activatable anti-CD71 antibodies in which at least one interchain disulfide bond in the activatable antibody has been reduced with a reducing agent without disturbing any intrachain disulfide bonds in the activatable antibody, wherein the activatable antibody includes an antibody or an antigen binding fragment thereof (AB) that specifically binds to CD71, a masking moiety (MM) that inhibits the binding of the AB of the activatable antibody in an uncleaved state to the CD71 target, and a cleavable moiety (CM) coupled to the AB, wherein the CM is a polypeptide that functions as a substrate for a protease. In some embodiments the MM is coupled to the AB via the CM. In some embodiments, one or more intrachain disulfide bond(s) of the activatable antibody is not disturbed by the reducing agent. In some embodiments, one or more intrachain disulfide bond(s) of the MM within the activatable antibody is not disturbed by the reducing agent. In some embodiments, the activatable antibody in the uncleaved state has the structural arrangement from N-terminus to C-terminus as follows: MM-CM-AB or AB-CM-MM. In some embodiments, reducing agent is TCEP.

In yet other embodiments, a method of reducing and conjugating an agent, e.g., a drug, to an activatable anti-CD71 antibody resulting in selectivity in the placement of the agent by providing an activatable anti-CD71 antibody with a defined number and positions of lysine and/or cysteine residues. In some embodiments, the defined number of lysine and/or cysteine residues is higher or lower than the number of corresponding residues in the amino acid sequence of the parent antibody or activatable antibody. In some embodiments, the defined number of lysine and/or cysteine residues may result in a defined number of agent equivalents that can be conjugated to the anti-CD71 antibody or activatable anti-CD71 antibody. In some embodiments, the defined number of lysine and/or cysteine residues may result in a defined number of agent equivalents that can be conjugated to the anti-CD71 antibody or activatable anti-CD71 antibody in a site-specific manner. In some embodiments, the modified activatable antibody is modified with one or more non-natural amino acids in a site-specific manner, thus in some embodiments limiting the conjugation of the agents to only the sites of the non-natural amino acids. In some embodiments, the anti-CD71 antibody or activatable anti-CD71 antibody with a defined number and positions of lysine and/or cysteine residues can be partially reduced with a reducing agent as discussed herein such that any conjugation sites in the masking moiety or other non-AB portion of the activatable antibody are not reduced, and conjugating the agent to interchain thiols in the AB.

The disclosure also provides partially reduced activatable antibodies in which at least one interchain disulfide bond in the activatable antibody has been reduced with a reducing agent without disturbing any intrachain disulfide bonds in the activatable antibody, wherein the activatable antibody includes an antibody or an antigen binding fragment thereof (AB) that specifically binds to the target, e.g., CD71, a masking moiety (MM) that inhibits the binding of the AB of the activatable antibody in an uncleaved state to the target, and a cleavable moiety (CM) coupled to the AB, wherein the CM is a polypeptide that functions as a substrate for at least one protease. In some embodiments, the MM is coupled to the AB via the CM. In some embodiments, one or more intrachain disulfide bond(s) of the activatable antibody is not disturbed by the reducing agent. In some embodiments, one or more intrachain disulfide bond(s) of the MM within the activatable antibody is not disturbed by the reducing agent. In some embodiments, the activatable antibody in the uncleaved state has the structural arrangement from N-terminus to C-terminus as follows: MM-CM-AB or AB-CM-MM. In some embodiments, reducing agent is TCEP.

In some embodiments, the activatable antibodies described herein also include an agent conjugated to the activatable antibody. In some embodiments, the conjugated agent is a therapeutic agent, such as an anti-inflammatory and/or an antineoplastic agent. In such embodiments, the agent is conjugated to a carbohydrate moiety of the activatable antibody, for example, in some embodiments, where the carbohydrate moiety is located outside the antigen-binding region of the antibody or antigen-binding fragment in the activatable antibody. In some embodiments, the agent is conjugated to a sulfhydryl group of the antibody or antigen-binding fragment in the activatable antibody.

In some embodiments, the agent is a cytotoxic agent such as a toxin (*e.g.,* an enzymatically active toxin of bacterial, fungal, plant, or animal origin, or fragments thereof), or a radioactive isotope (*i.e.,* a radio-conjugate).

In some embodiments, the agent is a detectable moiety such as, for example, a label or other marker. In some embodiments, the detectable moiety is a diagnostic agent. For example, the agent is or includes a radiolabeled amino acid, one or more biotinyl moieties that can be detected by marked avidin (*e.g.*, streptavidin containing a fluorescent marker or enzymatic activity that can be detected by optical or calorimetric methods), one or more radioisotopes or radionuclides, one or more fluorescent labels, one or more enzymatic labels, and/or one or more chemiluminescent agents. In some embodiments, detectable moieties are attached by spacer molecules.

The disclosure also pertains to immunoconjugates comprising an antibody conjugated to a cytotoxic agent such as a toxin (*e.g.,* an enzymatically active toxin of bacterial, fungal, plant, or animal origin, or fragments thereof), or a radioactive isotope (*i.e.,* a radio-conjugate). Suitable cytotoxic agents include, for example, dolastatins and derivatives thereof (*e.g.* auristatin E, AFP, MMAF, MMAE, MMAD, DMAF, DMAE). For example, the agent is monomethyl auristatin E (MMAE) or monomethyl auristatin D (MMAD). In some embodiments, the agent is a dolastatin. In some embodiments, the agent is an auristatin or derivative thereof. In some embodiments, the agent is auristatin E or a derivative thereof. In some embodiments, the agent is monomethyl auristatin E (MMAE). In some embodiments, the agent is monomethyl auristatin D (MMAD). In some embodiments, the agent is a maytansinoid or maytansinoid derivative.

In some embodiments, the linker and toxin conjugated to the AB comprises an SPDB-DM4 moiety, a vc-MMAD moiety, a vc-MMAE moiety, vc-duocarmycin, or a PEG2-vc-MMAD moiety. In some embodiments, the linker is a cleavable linker. In some embodiments, the linker is a non-cleavable linker. In some embodiments, the agent is a detectable moiety.

In some embodiments, the agent is linked to the AB using a maleimide caproyl-valine-citrulline linker or a maleimide PEG-valine-citrulline linker. In some embodiments, the agent is linked to the AB using a maleimide caproyl-valine-citrulline linker. In some embodiments, the agent is monomethyl auristatin E (MMAE) linked to the AB using a maleimide caproyl-valine-citrulline linker, and this linker payload construct is referred to herein as "vc-MMAE." In some embodiments, the agent is linked to the AB using a maleimide PEG-valine-citrulline linker. In some embodiments, the agent is monomethyl auristatin D (MMAD) linked to the AB using a maleimide tetra-PEG-valine-citrulline-para-aminobenzyloxycarbonyl linker, and this linker payload construct is referred to herein as "PEG4-vc-MMAD." In some embodiments, the agent is monomethyl auristatin E (MMAE) linked to the AB using a maleimide tetra-PEG-valine-citrulline-para-aminobenzyloxycarbonyl linker, and this linker payload construct is referred to herein as "PEG4-vc-MMAE." In some embodiments, the agent is linked to the AB using a maleimide PEG-valine-citrulline linker In some embodiments, the agent is monomethyl auristatin D (MMAD) linked to the AB using a maleimide bis-PEG-valine-citrulline-para-aminobenzyloxycarbonyl linker, and this linker payload construct is referred to herein as "PEG2-vc-MMAD." The structures of PEG4-vc-MMAD, PEG4-vc-MMAE, PEG2-vc-MMAD, and vc-MMAE are shown below:
PEG4-vc-MMAD: 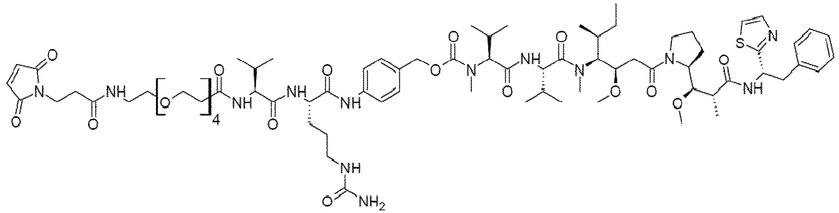
PEG4-vc-MMAE: 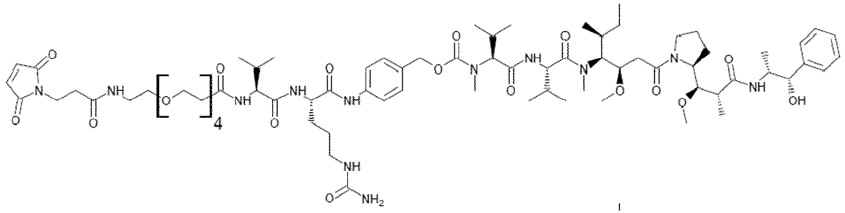
PEG2-vc-MMAD: 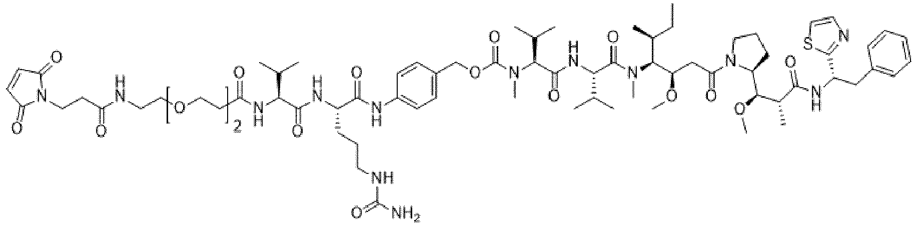
vc-MMAE

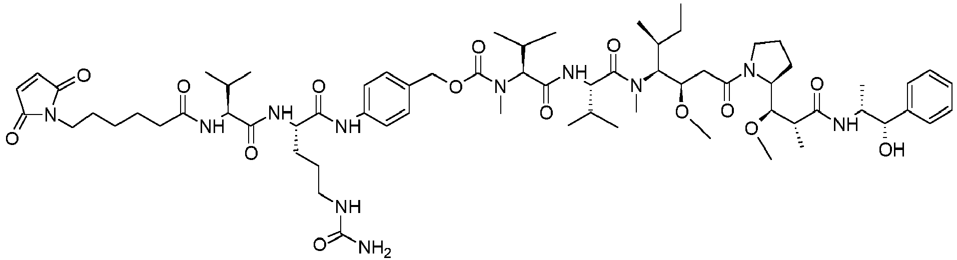

The disclosure also provides conjugated activatable antibodies that include an activatable antibody linked to vcMMAE, which is a monomethyl auristatin E (MMAE) payload with a val-cit (vc) linker, wherein the activatable antibody includes an antibody (AB) that specifically binds to a target, a masking moiety (MM) coupled to the AB that inhibits the binding of the AB to the target when the conjugated activatable antibody in an uncleaved state, and cleavable moiety (CM) coupled to the AB, and the CM is a polypeptide that functions as a substrate for a protease. In some embodiments, conjugated activatable antibodies of the present disclosure have the structure of Formula (I) or a salt thereof: 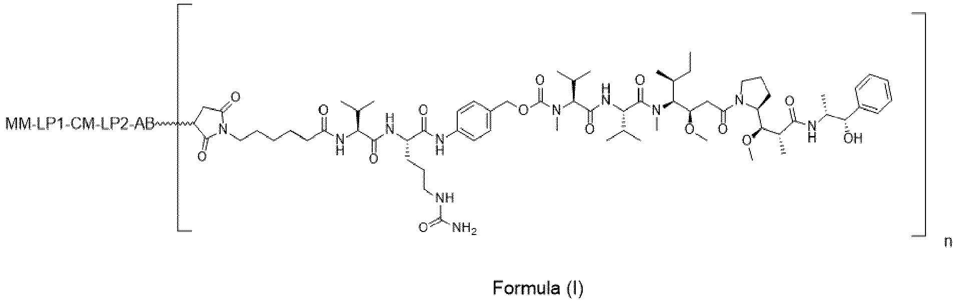 where AB is an antibody that specifically binds to a target and includes a heavy chain variable region, a heavy chain, and/or one or more heavy chain CDR (CDRH) regions. The AB also includes a light chain variable region, a light chain, and/or one or more light chain CDR (CDRL) regions. The conjugated activatable antibody of Formula (I) also includes a masking moiety (MM) where the MM inhibits the binding of the AB to its target when the conjugated activatable antibody is in an uncleaved state, a first linking moiety (LP1), a cleavable moiety (CM) where the CM, and a second linking moiety (LP2). In a particular embodiment of Formula (I), "n" is 2. In some embodiments, the conjugated activatable antibody of Formula (I) wherein the AB comprises an IgG1 isotype. In some embodiments, the conjugated activatable antibody of Formula (I) wherein the AB is an antibody having a heavy chain constant region, and wherein the C-terminal residue of the heavy chain constant region is not a lysine. In some embodiments, the conjugated activatable antibody of Formula (I), wherein the N-terminal glutamate on either the heavy chain and/or light chain is optionally either pyroglutamate or post-translationally modified to pyroglutamate.

In some embodiments, conjugated activatable antibodies of the present disclosure have the structure of Formula (II) or a salt thereof: 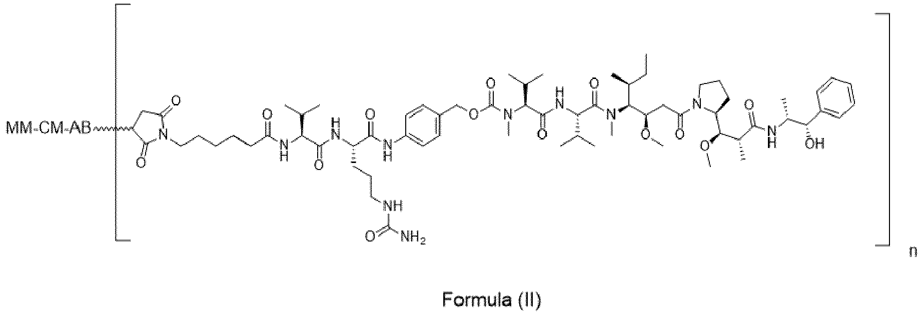 where AB is an antibody that specifically binds to a target and includes a heavy chain variable region, a heavy chain, and/or one or more heavy chain CDR (CDRH) regions. The AB also includes a light chain variable region, a light chain, and/or one or more light chain CDR (CDRL) regions. The conjugated activatable antibody of Formula (I) also includes a masking moiety (MM) where the MM inhibits the binding of the AB to its target when the conjugated activatable antibody is in an uncleaved state, and a cleavable moiety (CM) where the CM. In a particular embodiment of Formula (II), "n" is 2. In some embodiments, the conjugated activatable antibody of Formula (II) wherein the AB comprises an IgG1 isotype. In some embodiments, the conjugated activatable antibody of Formula (II) wherein the AB is an antibody having a heavy chain constant region, and wherein the C-terminal residue of the heavy chain constant region is not a lysine. In some embodiments, the conjugated activatable antibody of Formula (II), wherein the N-terminal glutamate on either the heavy chain and/or light chain is optionally either pyroglutamate or post-translationally modified to pyroglutamate.

In some embodiments of conjugated activatable antibodies of the present disclosure of Formulas (I) or (II), the target is mammalian CD71. In some embodiments of conjugated activatable antibodies of the present disclosure of Formulas (I) or (II), the target is human CD71.

In some embodiments of conjugated activatable antibodies of the present disclosure of Formulas (I) or (II), AB includes a heavy chain variable region comprising a CDRH1 sequence comprising SEQ ID NO: 9, a CDRH2 sequence comprising SEQ ID NO: 10, and a CDRH3 sequence comprising SEQ ID NO: 11, and a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15, a MM including the amino acid sequence of SEQ ID NO: 18, and a CM including the sequence of SEQ ID NO: 156, and "n" is 2. In some embodiments of conjugated activatable antibodies of the present disclosure of Formula (I), AB includes a heavy chain variable region comprising a CDRH1 sequence comprising SEQ ID NO: 9, a CDRH2 sequence comprising SEQ ID NO: 10, and a CDRH3 sequence comprising SEQ ID NO: 11, and a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15, a MM including the amino acid sequence of SEQ ID NO: 18, a LP1 including the amino acid sequence of SEQ ID NO: 207, a CM including the sequence of SEQ ID NO: 156, and a LP2 including the amino acid sequence of SEQ ID NO: 38, and "n" is 2.

In some embodiments of conjugated activatable antibodies of the present disclosure of Formulas (I) or (II), AB includes a heavy chain variable region including a sequence of SEQ ID NO: 5 and a light chain variable region including a sequence of SEQ ID NO: 7, a MM including the amino acid sequence of SEQ ID NO: 18, and a CM including the sequence of SEQ ID NO: 156, and "n" is 2. In some embodiments of conjugated activatable antibodies of the present disclosure of Formula (I), AB includes a heavy chain variable region including a sequence of SEQ ID NO: 5 and a light chain variable region including a sequence of SEQ ID NO: 7, a MM including the amino acid sequence of SEQ ID NO: 18, a LP1 including the amino acid sequence of SEQ ID NO: 207, a CM including the sequence of SEQ ID NO: 156, and a LP2 including the amino acid sequence of SEQ ID NO: 38, and "n" is 2.

In some embodiments of conjugated activatable antibodies of the present disclosure of Formulas (I) or (II), AB includes a heavy chain including a sequence of SEQ ID NO: 167 and a light chain including a sequence of SEQ ID NO: 19, a MM including the amino acid sequence of SEQ ID NO: 18, and a CM including the sequence of SEQ ID NO: 156, and "n" is 2. In some embodiments of conjugated activatable antibodies of the present disclosure of Formula (I), AB includes a heavy chain including a sequence of SEQ ID NO: 167 and a light chain including a sequence of SEQ ID NO: 19, a MM including the amino acid sequence of SEQ ID NO: 18, a LP1 including the amino acid sequence of SEQ ID NO: 207, a CM including the sequence of SEQ ID NO: 156, and a LP2 including the amino acid sequence of SEQ ID NO: 38, and "n" is 2.

In some embodiments of conjugated activatable antibodies of the present disclosure of Formulas (I) or (II), AB includes a heavy chain including a sequence of SEQ ID NO: 167 and a light chain including a sequence of SEQ ID NO: 169, and "n" is 2. In some embodiments of conjugated activatable antibodies of the present disclosure of Formula (I), AB includes a heavy chain including a sequence of SEQ ID NO: 167 and a light chain including a sequence of SEQ ID NO: 169, and "n" is 2. In some embodiments of conjugated activatable antibodies of the present disclosure of Formulas (I) or (II), AB includes a heavy chain including a sequence of SEQ ID NO: 167 and a light chain including a sequence of SEQ ID NO: 201, and "n" is 2.

In some embodiments of conjugated activatable antibodies of the present disclosure of Formulas (I) or (II), AB includes a heavy chain variable region comprising a sequence of SEQ ID NO: 5 and a light chain variable region comprising a sequence of SEQ ID NO: 7. In some embodiments of conjugated activatable antibodies of the present disclosure of Formulas (I) or (II), AB includes a heavy chain variable region comprising a sequence of SEQ ID NO: 5 and a light chain variable region comprising a sequence of SEQ ID NO: 7.

The disclosure also provides conjugated activatable antibodies that include an activatable antibody linked to monomethyl auristatin D (MMAD) payload, wherein the activatable antibody includes an antibody or an antigen binding fragment thereof (AB) that specifically binds to a target, a masking moiety (MM) that inhibits the binding of the AB of the activatable antibody in an uncleaved state to the target, and cleavable moiety (CM) coupled to the AB, and the CM is a polypeptide that functions as a substrate for at least one MMP protease.

In some embodiments, the MMAD-conjugated activatable antibody can be conjugated using any of several methods for attaching agents to ABs: (a) attachment to the carbohydrate moieties of the AB, or (b) attachment to sulfhydryl groups of the AB, or (c) attachment to amino groups of the AB, or (d) attachment to carboxylate groups of the AB.
In some embodiments, the MMAD payload is conjugated to the AB via a linker. In some embodiments, the MMAD payload is conjugated to a cysteine in the AB via a linker. In some embodiments, the MMAD payload is conjugated to a lysine in the AB via a linker. In some embodiments, the MMAD payload is conjugated to another residue of the AB via a linker, such as those residues disclosed herein. In some embodiments, the linker is a thiol-containing linker. In some embodiments, the linker is a cleavable linker. In some embodiments, the linker is a non-cleavable linker. In some embodiments, the linker is selected from the group consisting of the linkers shown in Tables (II) and (III). In some embodiments, the activatable antibody and the MMAD payload are linked via a maleimide caproyl-valine-citrulline linker. In some embodiments, the activatable antibody and the MMAD payload are linked via a maleimide PEG-valine-citrulline linker. In some embodiments, the activatable antibody and the MMAD payload are linked via a maleimide caproyl-valine-citrulline-para-aminobenzyloxycarbonyl linker. In some embodiments, the activatable antibody and the MMAD payload are linked via a maleimide PEG-valine-citrulline-para-aminobenzyloxycarbonyl linker. In some embodiments, the MMAD payload is conjugated to the AB using the partial reduction and conjugation technology disclosed herein.

In some embodiments, the polyethylene glycol (PEG) component of a linker of the present disclosure is formed from 2 ethylene glycol monomers, 3 ethylene glycol monomers, 4 ethylene glycol monomers, 5 ethylene glycol monomers, 6 ethylene glycol monomers, 7 ethylene glycol monomers 8 ethylene glycol monomers, 9 ethylene glycol monomers, or at least 10 ethylene glycol monomers. In some embodiments of the present disclosure, the PEG component is a branched polymer. In some embodiments of the present disclosure, the PEG component is an unbranched polymer. In some embodiments, the PEG polymer component is functionalized with an amino group or derivative thereof, a carboxyl group or derivative thereof, or both an amino group or derivative thereof and a carboxyl group or derivative thereof.

In some embodiments, the PEG component of a linker of the present disclosure is an amino-tetra-ethylene glycol-carboxyl group or derivative thereof. In some embodiments, the PEG component of a linker of the present disclosure is an amino-tri-ethylene glycol-carboxyl group or derivative thereof. In some embodiments, the PEG component of a linker of the present disclosure is an amino-di-ethylene glycol-carboxyl group or derivative thereof. In some embodiments, an amino derivative is the formation of an amide bond between the amino group and a carboxyl group to which it is conjugated. In some embodiments, a carboxyl derivative is the formation of an amide bond between the carboxyl group and an amino group to which it is conjugated. In some embodiments, a carboxyl derivative is the formation of an ester bond between the carboxyl group and a hydroxyl group to which it is conjugated.

Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. (*See* WO94/11026).

Those of ordinary skill in the art will recognize that a large variety of possible moieties can be coupled to the resultant antibodies of the disclosure. (*See, for example,* "Conjugate Vaccines", Contributions to Microbiology and Immunology, J. M. Cruse and R. E. Lewis, Jr (eds), Carger Press, New York, (1989), the entire contents of which are incorporated herein by reference).

Coupling can be accomplished by any chemical reaction that will bind the two molecules so long as the antibody and the other moiety retain their respective activities. This linkage can include many chemical mechanisms, for instance covalent binding, affinity binding, intercalation, coordinate binding and complexation. In some embodiments, the binding is, however, covalent binding. Covalent binding can be achieved either by direct condensation of existing side chains or by the incorporation of external bridging molecules. Many bivalent or polyvalent linking agents are useful in coupling protein molecules, such as the antibodies of the present disclosure, to other molecules. For example, representative coupling agents can include organic compounds such as thioesters, carbodiimides, succinimide esters, diisocyanates, glutaraldehyde, diazobenzenes and hexamethylene diamines. This listing is not intended to be exhaustive of the various classes of coupling agents known in the art but, rather, is exemplary of the more common coupling agents. (*See* Killen and Lindstrom, Jour. Immun. 133:1335-2549 (1984); Jansen et al., Immunological Reviews 62:185-216 (1982); and Vitetta et al., Science 238:1098 (1987).

In some embodiments, in addition to the compositions and methods provided herein, the conjugated activatable antibody can also be modified for site-specific conjugation through modified amino acid sequences inserted or otherwise included in the activatable antibody sequence. These modified amino acid sequences are designed to allow for controlled placement and/or dosage of the conjugated agent within a conjugated activatable antibody. For example, the activatable antibody can be engineered to include cysteine substitutions at positions on light and heavy chains that provide reactive thiol groups and do not negatively impact protein folding and assembly, nor alter antigen binding. In some embodiments, the activatable antibody can be engineered to include or otherwise introduce one or more non-natural amino acid residues within the activatable antibody to provide suitable sites for conjugation. In some embodiments, the activatable antibody can be engineered to include or otherwise introduce enzymatically activatable peptide sequences within the activatable antibody sequence.

Suitable linkers are described in the literature. (*See, for example,* Ramakrishnan, S. et al., Cancer Res. 44:201-208 (1984) describing use of MBS (M-maleimidobenzoyl-N-hydroxysuccinimide ester). *See also,* U.S. Patent No. 5,030,719, describing use of halogenated acetyl hydrazide derivative coupled to an antibody by way of an oligopeptide linker. In some embodiments, suitable linkers include: (i) EDC (1-ethyl-3-(3-dimethylamino-propyl) carbodiimide hydrochloride; (ii) SMPT (4-succinimidyloxycarbonyl-alpha-methyl-alpha-(2-pridyl-dithio)-toluene (Pierce Chem. Co., Cat. (21558G); (iii) SPDP (succinimidyl-6 [3-(2-pyridyldithio) propionamido]hexanoate (Pierce Chem. Co., Cat #21651G); (iv) Sulfo-LC-SPDP (sulfosuccinimidyl 6 [3-(2-pyridyldithio)-propianamide] hexanoate (Pierce Chem. Co. Cat. #2165-G); and (v) sulfo-NHS (N-hydroxysulfo-succinimide: Pierce Chem. Co., Cat. #24510) conjugated to EDC. Additional linkers include, but are not limited to, SMCC ((succinimidyl 4-(N-maleimidomethyl)cyclohexane-1-carboxylate), sulfo-SMCC (sulfosuccinimidyl 4-(N-maleimidomethyl)cyclohexane-1-carboxylate), SPDB (N-succinimidyl-4-(2-pyridyldithio) butanoate), or sulfo-SPDB (N-succinimidyl-4-(2-pyridyldithio)-2-sulfo butanoate).

The linkers described above contain components that have different attributes, thus leading to conjugates with differing physio-chemical properties. For example, sulfo-NHS esters of alkyl carboxylates are more stable than sulfo-NHS esters of aromatic carboxylates. NHS-ester containing linkers are less soluble than sulfo-NHS esters. Further, the linker SMPT contains a sterically hindered disulfide bond, and can form conjugates with increased stability. Disulfide linkages, are in general, less stable than other linkages because the disulfide linkage is cleaved *in vitro,* resulting in less conjugate available. Sulfo-NHS, in particular, can enhance the stability of carbodimide couplings. Carbodimide couplings (such as EDC) when used in conjunction with sulfo-NHS, forms esters that are more resistant to hydrolysis than the carbodimide coupling reaction alone.

In some embodiments, the linkers are cleavable. In some embodiments, the linkers are non-cleavable. In some embodiments, two or more linkers are present. The two or more linkers are all the same, *i.e*., cleavable or non-cleavable, or the two or more linkers are different, *i.e*., at least one cleavable and at least one non-cleavable.

The present disclosure utilizes several methods for attaching agents to ABs: (a) attachment to the carbohydrate moieties of the AB, or (b) attachment to sulfhydryl groups of the AB, or (c) attachment to amino groups of the AB, or (d) attachment to carboxylate groups of the AB. According to the disclosure, ABs can be covalently attached to an agent through an intermediate linker having at least two reactive groups, one to react with AB and one to react with the agent. The linker, which may include any compatible organic compound, can be chosen such that the reaction with AB (or agent) does not adversely affect AB reactivity and selectivity. Furthermore, the attachment of linker to agent might not destroy the activity of the agent. Suitable linkers for reaction with oxidized antibodies or oxidized antibody fragments include those containing an amine selected from the group consisting of primary amine, secondary amine, hydrazine, hydrazide, hydroxylamine, phenylhydrazine, semicarbazide and thiosemicarbazide groups. Such reactive functional groups may exist as part of the structure of the linker, or can be introduced by suitable chemical modification of linkers not containing such groups.

According to the present disclosure, suitable linkers for attachment to reduced ABs include those having certain reactive groups capable of reaction with a sulfhydryl group of a reduced antibody or fragment. Such reactive groups include, but are not limited to: reactive haloalkyl groups (including, for example, haloacetyl groups), p-mercuribenzoate groups and groups capable of Michael-type addition reactions (including, for example, maleimides and groups of the type described by Mitra and Lawton, 1979, J. Amer. Chem. Soc. 101: 3097-3110).

According to the present disclosure, suitable linkers for attachment to neither oxidized nor reduced Abs include those having certain functional groups capable of reaction with the primary amino groups present in unmodified lysine residues in the Ab. Such reactive groups include, but are not limited to, NHS carboxylic or carbonic esters, sulfo-NHS carboxylic or carbonic esters, 4-nitrophenyl carboxylic or carbonic esters, pentafluorophenyl carboxylic or carbonic esters, acyl imidazoles, isocyanates, and isothiocyanates.

According to the present disclosure, suitable linkers for attachment to neither oxidized nor reduced Abs include those having certain functional groups capable of reaction with the carboxylic acid groups present in aspartate or glutamate residues in the Ab, which have been activated with suitable reagents. Suitable activating reagents include EDC, with or without added NHS or sulfo-NHS, and other dehydrating agents utilized for carboxamide formation. In these instances, the functional groups present in the suitable linkers would include primary and secondary amines, hydrazines, hydroxylamines, and hydrazides.

The agent can be attached to the linker before or after the linker is attached to the AB. In certain applications it may be desirable to first produce an AB-linker intermediate in which the linker is free of an associated agent. Depending upon the particular application, a specific agent may then be covalently attached to the linker. In some embodiments, the AB is first attached to the MM, CM and associated linkers and then attached to the linker for conjugation purposes.

*Branched Linkers:* In specific embodiments, branched linkers that have multiple sites for attachment of agents are utilized. For multiple site linkers, a single covalent attachment to an AB would result in an AB-linker intermediate capable of binding an agent at a number of sites. The sites can be aldehyde or sulfhydryl groups or any chemical site to which agents can be attached.

In some embodiments, higher specific activity (or higher ratio of agents to AB) can be achieved by attachment of a single site linker at a plurality of sites on the AB. This plurality of sites can be introduced into the AB by either of two methods. First, one may generate multiple aldehyde groups and/or sulfhydryl groups in the same AB. Second, one may attach to an aldehyde or sulfhydryl of the AB a "branched linker" having multiple functional sites for subsequent attachment to linkers. The functional sites of the branched linker or multiple site linker can be aldehyde or sulfhydryl groups, or can be any chemical site to which linkers can be attached. Still higher specific activities can be obtained by combining these two approaches, that is, attaching multiple site linkers at several sites on the AB.

*Cleavable Linkers:* Peptide linkers that are susceptible to cleavage by enzymes of the complement system, such as but not limited to u-plasminogen activator, tissue plasminogen activator, trypsin, plasmin, or another enzyme having proteolytic activity can be used in one embodiment of the present disclosure. According to one method of the present disclosure, an agent is attached via a linker susceptible to cleavage by complement. The antibody is selected from a class that can activate complement. The antibody-agent conjugate, thus, activates the complement cascade and releases the agent at the target site. According to another method of the present disclosure, an agent is attached via a linker susceptible to cleavage by enzymes having a proteolytic activity such as a u-plasminogen activator, a tissue plasminogen activator, plasmin, or trypsin.

Non-limiting examples of cleavable linker sequences are provided in Table (II).

**Table (II): Exemplary Linker Sequences for Conjugation**

| Types of Cleavable Sequences | Amino Acid Sequence |
|---|---|
| Plasmin cleavable sequences | |
| Pro-urokinase | PRFKIIGG (SEQ ID NO: 110) |
| | PRFRIIGG (SEQ ID NO: 111) |
| TGFβ | SSRHRRALD (SEQ ID NO: 112) |
| Plasminogen | RKSSIIIRMRDVVL (SEQ ID NO: 113) |
| Staphylokinase | SSSFDKGKYKKGDDA (SEQ ID NO: 114) |
| | SSSFDKGKYKRGDDA (SEQ ID NO: 115) |
| Factor Xa cleavable sequences | IEGR (SEQ ID NO: 116) |
| | IDGR (SEQ ID NO: 117) |
| | GGSIDGR (SEQ ID NO: 118) |

| MMP cleavable sequences | |
|---|---|
| Gelatinase A | PLGLWA (SEQ ID NO: 119) |
| Collagenase cleavable sequences | |
| Calf skin collagen (α1(I) chain) | GPQGIAGQ (SEQ ID NO: 120) |
| Calf skin collagen (α2(I) chain) | GPQGLLGA (SEQ ID NO: 121) |
| Bovine cartilage collagen (α1(II) chain) | GIAGQ (SEQ ID NO: 122) |
| Human liver collagen (α1(III) chain) | GPLGIAGI (SEQ ID NO: 123) |
| Human α₂M | GPEGLRVG (SEQ ID NO: 124) |
| Human PZP | YGAGLGVV (SEQ ID NO: 125) |
| | AGLGVVER (SEQ ID NO: 126) |
| | AGLGISST (SEQ ID NO: 127) |
| Rat α₁M | EPQALAMS (SEQ ID NO: 128) |
| | QALAMSAI (SEQ ID NO: 129) |
| Rat α₂M | AAYHLVSQ (SEQ ID NO: 130) |
| | MDAFLESS (SEQ ID NO: 131) |
| Rat α₁I₃(2J) | ESLPVVAV (SEQ ID NO: 132) |
| Rat α₁I₃(27J) | SAPAVESE (SEQ ID NO: 133) |
| Human fibroblast collagenase | DVAQFVLT (SEQ ID NO: 134) |
| (autolvtic cleavages) | VAQFVLTE (SEQ ID NO: 135) |
| | AQFVLTEG (SEQ ID NO: 136) |
| | PVQPIGPQ (SEQ ID NO: 137) |

In addition, agents can be attached via disulfide bonds (for example, the disulfide bonds on a cysteine molecule) to the AB. Since many tumors naturally release high levels of glutathione (a reducing agent) this can reduce the disulfide bonds with subsequent release of the agent at the site of delivery. In some embodiments, the reducing agent that would modify a CM would also modify the linker of the conjugated activatable antibody.

*Spacers and Cleavable Elements:* In some embodiments, it may be necessary to construct the linker in such a way as to optimize the spacing between the agent and the AB of the activatable antibody. This can be accomplished by use of a linker of the general structure:

W - (CH₂)n - Q

wherein
W is either --NH--CH₂-- or --CH₂--;
Q is an amino acid, peptide; and
n is an integer from 0 to 20.

In some embodiments, the linker may comprise a spacer element and a cleavable element. The spacer element serves to position the cleavable element away from the core of the AB such that the cleavable element is more accessible to the enzyme responsible for cleavage. Certain of the branched linkers described above may serve as spacer elements.

Throughout this discussion, it should be understood that the attachment of linker to agent (or of spacer element to cleavable element, or cleavable element to agent) need not be particular mode of attachment or reaction. Any reaction providing a product of suitable stability and biological compatibility is acceptable.

*Serum Complement and Selection of Linkers:* According to one method of the present disclosure, when release of an agent is desired, an AB that is an antibody of a class that can activate complement is used. The resulting conjugate retains both the ability to bind antigen and activate the complement cascade. Thus, according to this embodiment of the present disclosure, an agent is joined to one end of the cleavable linker or cleavable element and the other end of the linker group is attached to a specific site on the AB. For example, if the agent has an hydroxy group or an amino group, it can be attached to the carboxy terminus of a peptide, amino acid or other suitably chosen linker via an ester or amide bond, respectively. For example, such agents can be attached to the linker peptide via a carbodimide reaction. If the agent contains functional groups that would interfere with attachment to the linker, these interfering functional groups can be blocked before attachment and deblocked once the product conjugate or intermediate is made. The opposite or amino terminus of the linker is then used either directly or after further modification for binding to an AB that is capable of activating complement.

Linkers (or spacer elements of linkers) can be of any desired length, one end of which can be covalently attached to specific sites on the AB of the activatable antibody. The other end of the linker or spacer element can be attached to an amino acid or peptide linker.

Thus when these conjugates bind to antigen in the presence of complement the amide or ester bond that attaches the agent to the linker will be cleaved, resulting in release of the agent in its active form. These conjugates, when administered to a subject, will accomplish delivery and release of the agent at the target site, and are particularly effective for the *in vivo* delivery of pharmaceutical agents, antibiotics, antimetabolites, antiproliferative agents and the like.

*Linkers for Release without Complement Activation:* In yet another application of targeted delivery, release of the agent without complement activation is desired since activation of the complement cascade will ultimately lyse the target cell. Hence, this approach is useful when delivery and release of the agent should be accomplished without killing the target cell. Such is the goal when delivery of cell mediators such as hormones, enzymes, corticosteroids, neurotransmitters, genes or enzymes to target cells is desired. These conjugates can be prepared by attaching the agent to an AB that is not capable of activating complement via a linker that is mildly susceptible to cleavage by serum proteases. When this conjugate is administered to an individual, antigen-antibody complexes will form quickly whereas cleavage of the agent will occur slowly, thus resulting in release of the compound at the target site.

*Biochemical Cross Linkers:* In some embodiments, the activatable antibody can be conjugated to one or more therapeutic agents using certain biochemical cross-linkers. Cross-linking reagents form molecular bridges that tie together functional groups of two different molecules. To link two different proteins in a step-wise manner, hetero-bifunctional cross-linkers can be used that eliminate unwanted homopolymer formation.

Peptidyl linkers cleavable by lysosomal proteases are also useful, for example, Val-Cit, Val-Ala or other dipeptides. In addition, acid-labile linkers cleavable in the low-pH environment of the lysosome can be used, for example: bis-sialyl ether. Other suitable linkers include cathepsin-labile substrates, particularly those that show optimal function at an acidic pH.

Exemplary hetero-bifunctional cross-linkers are referenced in Table (III).

**Table (III): Exemplary Hetero-Bifunctional Cross Linkers**

| **HETERO-BIFUNCTIONAL CROSS-LINKERS** | | | |
|---|---|---|---|
| Linker | Reactive Toward | Advantages and Applications | Spacer Arm Length after cross-linking (Angstroms) |
| SMPT | Primary amines | Greater stability | 11.2 Å |
| | Sulfhydryls | | |
| SPDP | Primary amines | Thiolation | 6.8 Å |
| | Sulfhydryls | Cleavable cross-linking | |
| LC-SPDP | Primary amines | Extended spacer arm | 15.6 Å |
| | Sulfhydryls | | |
| Sulfo-LC-SPDP | Primary amines | Extender spacer arm | 15.6 Å |
| | Sulfhydryls | Water-soluble | |
| SMCC | Primary amines | Stable maleimide reactive group | 11.6 Å |
| | Sulfhydryls | Enzyme-antibody conjugation | |
| | | Hapten-carrier protein conjugation | |
| Sulfo-SMCC | Primary amines | Stable maleimide reactive group | 11.6 Å |
| | Sulfhydryls | Water-soluble Enzyme-antibody conjugation | |
| MBS | Primary amines | Enzyme-antibody conjugation | 9.9 Å |
| | Sulfhydryls | Hapten-carrier protein conjugation | |
| Sulfo-MBS | Primary amines | Water-soluble | 9.9 Å |
| | Sulfhydryls | | |
| SIAB | Primary amines | Enzyme-antibody conjugation | 10.6 Å |
| | Sulfhydryls | | |
| Sulfo-SIAB | Primary amines | Water-soluble | 10.6 Å |
| | Sulfhydryls | | |
| SMPB | Primary amines | Extended spacer arm | 14.5 Å |
| | Sulfhydryls | Enzyme-antibody conjugation | |
| Sulfo-SMPB | Primary amines | Extended spacer arm | 14.5 Å |
| | Sulfhydryls | Water-soluble | |
| EDE/Sulfo-NHS | Primary amines | Hapten-Carrier conjugation | 0 |
| | Carboxyl groups | | |
| ABH | Carbohydrates | Reacts with sugar groups | 11.9 Å |
| | Nonselective | | |

*Non-Cleavable Linkers or Direct Attachment:* In some embodiments of the disclosure, the conjugate can be designed so that the agent is delivered to the target but not released. This can be accomplished by attaching an agent to an AB either directly or via a non-cleavable linker.

These non-cleavable linkers may include amino acids, peptides, D-amino acids or other organic compounds that can be modified to include functional groups that can subsequently be utilized in attachment to ABs by the methods described herein. A-general formula for such an organic linker could be

W - (CH₂)n - Q

wherein
W is either --NH--CH₂-- or --CH₂--;
Q is an amino acid, peptide; and
n is an integer from 0 to 20.

*Non-Cleavable Conjugates:* In some embodiments, a compound can be attached to ABs that do not activate complement. When using ABs that are incapable of complement activation, this attachment can be accomplished using linkers that are susceptible to cleavage by activated complement or using linkers that are not susceptible to cleavage by activated complement.

### Definitions:

Unless otherwise defined, scientific and technical terms used in connection with the present disclosure shall have the meanings that are commonly understood by those of ordinary skill in the art. The term "a" entity or "an" entity refers to one or more of that entity. For example, a compound refers to one or more compounds. As such, the terms "a", "an", "one or more" and "at least one" can be used interchangeably. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well-known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:
As used herein, the term "antibody" refers to immunoglobulin molecules and immunologically active portions of immunoglobulin (Ig) molecules, *i.e.,* molecules that contain an antigen binding site that specifically binds (immunoreacts with) an antigen. By "specifically bind" or "immunoreacts with" or "immunospecifically bind" is meant that the antibody reacts with one or more antigenic determinants of the desired antigen and does not react with other polypeptides or binds at much lower affinity (K_{d} > 10⁻⁶).

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. In general, antibody molecules obtained from humans relate to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain can be a kappa chain or a lambda chain.

The term "monoclonal antibody" (mAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs contain an antigen binding site capable of immunoreacting with a particular epitope of the antigen characterized by a unique binding affinity for it.

The term "antigen-binding site" or "binding portion" refers to the part of the immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains, referred to as "hypervariable regions," are interposed between more conserved flanking stretches known as "framework regions," or "FRs". Thus, the term "FR" refers to amino acid sequences that are naturally found between, and adjacent to, hypervariable regions in immunoglobulins. In an antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." The assignment of amino acids to each domain is in accordance with the definitions of Kabat Sequences of Proteins of Immunological Interest (National Institutes of Health, Bethesda, Md. (1987 and 1991)), or Chothia & Lesk J. Mol. Biol. 196:901-917 (1987), Chothia et al. Nature 342:878-883 (1989).

As used herein, the term "epitope" includes any protein determinant capable of specific binding to an immunoglobulin, an scFv, or a T-cell receptor. The term "epitope" includes any protein determinant capable of specific binding to an immunoglobulin or T-cell receptor. Epitopic determinants usually consist of chemically active surface groupings of molecules such as amino acids or sugar side chains and usually have specific three-dimensional structural characteristics, as well as specific charge characteristics. For example, antibodies can be raised against N-terminal or C-terminal peptides of a polypeptide. An antibody is said to specifically bind an antigen when the dissociation constant is ≤ 1 µM; in some embodiments, ≤ 100 nM and in some embodiments, ≤ 10 nM

As used herein, the terms "specific binding," "immunological binding," and "immunological binding properties" refer to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and geometric parameters that equally influence the rate in both directions. Thus, both the "on rate constant" (Kₒₙ) and the "off rate constant" (K_{off}) can be determined by calculation of the concentrations and the actual rates of association and dissociation. (*See* Nature 361:186-87 (1993)). The ratio of K_{off}/Kₒₙ enables the cancellation of all parameters not related to affinity, and is equal to the dissociation constant K_{d}. (*See, generally,* Davies et al. (1990) Annual Rev Biochem 59:439-473). An antibody of the present disclosure is said to specifically bind to the target, when the binding constant (K_{d}) is ≤1 µM, in some embodiments ≤ 100 nM, in some embodiments ≤ 10 nM, and in some embodiments ≤ 100 pM to about 1 pM, as measured by assays such as radioligand binding assays or similar assays known to those skilled in the art.

The term "isolated polynucleotide" as used herein shall mean a polynucleotide of genomic, cDNA, or synthetic origin or some combination thereof, which by virtue of its origin the "isolated polynucleotide" (1) is not associated with all or a portion of a polynucleotide in which the "isolated polynucleotide" is found in nature, (2) is operably linked to a polynucleotide which it is not linked to in nature, or (3) does not occur in nature as part of a larger sequence. Polynucleotides in accordance with the disclosure include the nucleic acid molecules encoding the heavy chain immunoglobulin molecules shown herein, and nucleic acid molecules encoding the light chain immunoglobulin molecules shown herein.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, *e.g.,* free of murine proteins, (3) is expressed by a cell from a different species, or (4) does not occur in nature.

The term "polypeptide" is used herein as a generic term to refer to native protein, fragments, or analogs of a polypeptide sequence. Hence, native protein fragments, and analogs are species of the polypeptide genus. Polypeptides in accordance with the disclosure comprise the heavy chain immunoglobulin molecules shown herein, and the light chain immunoglobulin molecules shown herein, as well as antibody molecules formed by combinations comprising the heavy chain immunoglobulin molecules with light chain immunoglobulin molecules, such as kappa light chain immunoglobulin molecules, and vice versa, as well as fragments and analogs thereof.

The term "naturally-occurring" as used herein as applied to an object refers to the fact that an object can be found in nature. For example, a polypeptide or polynucleotide sequence that is present in an organism (including viruses) that can be isolated from a source in nature and that has not been intentionally modified by man in the laboratory or otherwise is naturally-occurring.

The term "operably linked" as used herein refers to positions of components so described are in a relationship permitting them to function in their intended manner. A control sequence "operably linked" to a coding sequence is ligated in such a way that expression of the coding sequence is achieved under conditions compatible with the control sequences.

The term "control sequence" as used herein refers to polynucleotide sequences that are necessary to effect the expression and processing of coding sequences to which they are ligated. The nature of such control sequences differs depending upon the host organism in prokaryotes, such control sequences generally include promoter, ribosomal binding site, and transcription termination sequence in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences. The term "polynucleotide" as referred to herein means nucleotides of at least 10 bases in length, either ribonucleotides or deoxynucleotides or a modified form of either type of nucleotide. The term includes single and double stranded forms of DNA.

The term oligonucleotide referred to herein includes naturally occurring, and modified nucleotides linked together by naturally occurring, and non-naturally occurring oligonucleotide linkages. Oligonucleotides are a polynucleotide subset generally comprising a length of 200 bases or fewer. In some embodiments, oligonucleotides are 10 to 60 bases in length and in some embodiments, 12, 13, 14, 15, 16, 17, 18, 19, or 20 to 40 bases in length. Oligonucleotides are usually single stranded, *e.g.,* for probes, although oligonucleotides may be double stranded, *e.g.,* for use in the construction of a gene mutant. Oligonucleotides of the disclosure are either sense or antisense oligonucleotides.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotide linkages such as phosphorothioate, phosphorodithioate, phosphoroselerloate, phosphorodiselenoate, phosphoroanilothioate, phoshoraniladate, phosphoronmidate, and the like. *See e.g.,* LaPlanche et al. Nucl. Acids Res. 14:9081 (1986); Stec et al. J. Am. Chem. Soc. 106:6077 (1984), Stein et al. Nucl. Acids Res. 16:3209 (1988), Zon et al. Anti Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Patent No. 5,151,510; Uhlmann and Peyman Chemical Reviews 90:543 (1990). An oligonucleotide can include a label for detection, if desired.

As used herein, the twenty conventional amino acids and their abbreviations follow conventional usage. *See* Immunology - A Synthesis (2nd Edition, E.S. Golub and D.R. Green, Eds., Sinauer Associates, Sunderland, Mass. (1991)). Stereoisomers (*e.g.*, D-amino acids) of the twenty conventional amino acids, unnatural amino acids such as α-, α-disubstituted amino acids, N-alkyl amino acids, lactic acid, and other unconventional amino acids may also be suitable components for polypeptides of the present disclosure. Examples of unconventional amino acids include: 4 hydroxyproline, γ-carboxyglutamate, ε-N,N,N-trimethyllysine, ε -N-acetyllysine, O-phosphoserine, N-acetylserine, N-formylmethionine, 3-methylhistidine, 5-hydroxylysine, σ-N-methylarginine, and other similar amino acids and imino acids (*e.g.*, 4-hydroxyproline). In the polypeptide notation used herein, the left-hand direction is the amino terminal direction and the right-hand direction is the carboxy-terminal direction, in accordance with standard usage and convention.

Similarly, unless specified otherwise, the left-hand end of single-stranded polynucleotide sequences is the 5' end the left-hand direction of double-stranded polynucleotide sequences is referred to as the 5' direction. The direction of 5' to 3' addition of nascent RNA transcripts is referred to as the transcription direction sequence regions on the DNA strand having the same sequence as the RNA and that are 5' to the 5' end of the RNA transcript are referred to as "upstream sequences", sequence regions on the DNA strand having the same sequence as the RNA and that are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, in some embodiments, at least 90 percent sequence identity, in some embodiments, at least 95 percent sequence identity, and in some embodiments, at least 99 percent sequence identity.

In some embodiments, residue positions that are not identical differ by conservative amino acid substitutions.

As discussed herein, minor variations in the amino acid sequences of antibodies or immunoglobulin molecules are contemplated as being encompassed by the present disclosure, providing that the variations in the amino acid sequence maintain at least 75%, in some embodiments, at least 80%, 90%, 95%, and in some embodiments, 99%. In particular, conservative amino acid replacements are contemplated. Conservative replacements are those that take place within a family of amino acids that are related in their side chains. Genetically encoded amino acids are generally divided into families: (1) acidic amino acids are aspartate, glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. The hydrophilic amino acids include arginine, asparagine, aspartate, glutamine, glutamate, histidine, lysine, serine, and threonine. The hydrophobic amino acids include alanine, cysteine, isoleucine, leucine, methionine, phenylalanine, proline, tryptophan, tyrosine and valine. Other families of amino acids include (i) serine and threonine, which are the aliphatic-hydroxy family; (ii) asparagine and glutamine, which are the amide containing family; (iii) alanine, valine, leucine and isoleucine, which are the aliphatic family; and (iv) phenylalanine, tryptophan, and tyrosine, which are the aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. Suitable amino- and carboxy-termini of fragments or analogs occur near boundaries of functional domains. Structural and functional domains can be identified by comparison of the nucleotide and/or amino acid sequence data to public or proprietary sequence databases. In some embodiments, computerized comparison methods are used to identify sequence motifs or predicted protein conformation domains that occur in other proteins of known structure and/or function. Methods to identify protein sequences that fold into a known three-dimensional structure are known. Bowie et al. Science 253:164 (1991). Thus, the foregoing examples demonstrate that those of skill in the art can recognize sequence motifs and structural conformations that can be used to define structural and functional domains in accordance with the disclosure.

Suitable amino acid substitutions are those that: (1) reduce susceptibility to proteolysis, (2) reduce susceptibility to oxidation, (3) alter binding affinity for forming protein complexes, (4) alter binding affinities, and (5) confer or modify other physicochemical or functional properties of such analogs. Analogs can include various muteins of a sequence other than the naturally-occurring peptide sequence. For example, single or multiple amino acid substitutions (for example, conservative amino acid substitutions) can be made in the naturally-occurring sequence (for example, in the portion of the polypeptide outside the domain(s) forming intermolecular contacts. A conservative amino acid substitution should not substantially change the structural characteristics of the parent sequence (*e.g.*, a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991).

The term "polypeptide fragment" as used herein refers to a polypeptide that has an amino terminal and/or carboxy-terminal deletion and/or one or more internal deletion(s), but where the remaining amino acid sequence is identical to the corresponding positions in the naturally-occurring sequence deduced, for example, from a full length cDNA sequence. Fragments typically are at least 5, 6, 8 or 10 amino acids long, in some embodiments, at least 14 amino acids long, in some embodiments, at least 20 amino acids long, usually at least 50 amino acids long, and in some embodiments, at least 70 amino acids long. The term "analog" as used herein refers to polypeptides that are comprised of a segment of at least 25 amino acids that has substantial identity to a portion of a deduced amino acid sequence and that has specific binding to the target, under suitable binding conditions. Typically, polypeptide analogs comprise a conservative amino acid substitution (or addition or deletion) with respect to the naturally-occurring sequence. Analogs typically are at least 20 amino acids long, in some embodiments, at least 50 amino acids long or longer, and can often be as long as a full-length naturally-occurring polypeptide.

The term "agent" is used herein to denote a chemical compound, a mixture of chemical compounds, a biological macromolecule, or an extract made from biological materials.

As used herein, the terms "label" or "labeled" refers to incorporation of a detectable marker, *e.g.*, by incorporation of a radiolabeled amino acid or attachment to a polypeptide of biotinyl moieties that can be detected by marked avidin (*e.g.*, streptavidin containing a fluorescent marker or enzymatic activity that can be detected by optical or calorimetric methods). In certain situations, the label or marker can also be therapeutic. Various methods of labeling polypeptides and glycoproteins are known in the art and can be used. Examples of labels for polypeptides include, but are not limited to, the following: radioisotopes or radionuclides (*e.g.*, ³H, ¹⁴C, ¹⁵N, ³⁵S, ⁹⁰Y, ⁹⁹Tc, ¹¹¹In, ¹²⁵I, ¹³¹I), fluorescent labels (*e.g.,* FITC, rhodamine, lanthanide phosphors), enzymatic labels (*e.g.*, horseradish peroxidase, p-galactosidase, luciferase, alkaline phosphatase), chemiluminescent, biotinyl groups, predetermined polypeptide epitopes recognized by a secondary reporter (*e.g.*, leucine zipper pair sequences, binding sites for secondary antibodies, metal binding domains, epitope tags). In some embodiments, labels are attached by spacer arms of various lengths to reduce potential steric hindrance. The term "pharmaceutical agent or drug" as used herein refers to a chemical compound or composition capable of inducing a desired therapeutic effect when properly administered to a patient.

Other chemistry terms herein are used according to conventional usage in the art, as exemplified by The McGraw-Hill Dictionary of Chemical Terms (Parker, S., Ed., McGraw-Hill, San Francisco (1985)).

As used herein, "substantially pure" means an object species is the predominant species present (*i.e.,* on a molar basis it is more abundant than any other individual species in the composition), and in some embodiments, a substantially purified fraction is a composition wherein the object species comprises at least about 50 percent (on a molar basis) of all macromolecular species present.

Generally, a substantially pure composition will comprise more than about 80 percent of all macromolecular species present in the composition, in some embodiments, more than about 85%, 90%, 95%, and 99%. In some embodiments, the object species is purified to essential homogeneity (contaminant species cannot be detected in the composition by conventional detection methods) wherein the composition consists essentially of a single macromolecular species.

The term patient includes human and veterinary subjects.

Antibodies and/or activatable antibodies of the disclosure specifically bind a given target, e.g., a human target protein such as human CD71. Also included in the disclosure are antibodies and/or activatable antibodies that bind to the same epitope as the antibodies and/or activatable antibodies described herein. Also included in the disclosure are antibodies and/or antibodies activatable antibodies that compete with an anti-CD71 antibody and/or an anti-CD71 activatable antibody described herein for binding to CD71, e.g., human CD71. Also included in the disclosure are antibodies and/or antibodies activatable antibodies that cross-compete with an anti-CD71 antibody and/or an anti-CD71 activatable antibody described herein for binding to CD71, e.g., human CD71.

Those skilled in the art will recognize that it is possible to determine, without undue experimentation, if a monoclonal antibody (*e.g.*, a murine monoclonal or humanized antibody) has the same specificity as a monoclonal antibody used in the methods described herein by ascertaining whether the former prevents the latter from binding to the target. If the monoclonal antibody being tested competes with the monoclonal antibody of the disclosure, as shown by a decrease in binding by the monoclonal antibody of the disclosure, then the two monoclonal antibodies bind to the same, or a closely related, epitope. An alternative method for determining whether a monoclonal antibody has the specificity of a monoclonal antibody of the disclosure is to pre-incubate the monoclonal antibody of the disclosure with the target and then add the monoclonal antibody being tested to determine if the monoclonal antibody being tested is inhibited in its ability to bind the target. If the monoclonal antibody being tested is inhibited then, in all likelihood, it has the same, or functionally equivalent, epitopic specificity as the monoclonal antibody of the disclosure.

### Use Of Conjugated Activatable Antibodies

It will be appreciated that administration of therapeutic entities in accordance with the disclosure will be administered with suitable pharmaceutically acceptable carriers, excipients, and other agents that are incorporated into formulations to provide improved transfer, delivery, tolerance, and the like.

Therapeutic formulations of the disclosure, which include by way of non-limiting example, a conjugated activatable antibody, are used to prevent, treat or otherwise ameliorate a disease or disorder associated with aberrant target expression and/or activity. For example, therapeutic formulations of the disclosure, which include a conjugated activatable antibody, are used to treat or otherwise ameliorate a cancer or other neoplastic condition, inflammation, an inflammatory disorder, and/or an autoimmune disease. In some embodiments, the cancer is a solid tumor or a hematologic malignancy where the target is expressed. In some embodiments, the cancer is a solid tumor where the target is expressed. In some embodiments, the cancer is a hematologic malignancy where the target is expressed. In some embodiments, the target is expressed on parenchyma (e.g., in cancer, the portion of an organ or tissue that often carries out function(s) of the organ or tissue). In some embodiments, the target is expressed on a cell, tissue, or organ. In some embodiments, the target is expressed on stroma (i.e., the connective supportive framework of a cell, tissue, or organ). In some embodiments, the target is expressed on an osteoblast. In some embodiments, the target is expressed on the endothelium (vasculature). In some embodiments, the target is expressed on a cancer stem cell. In some embodiments, the agent to which the antibody and/or the activatable antibody is conjugated is a microtubule inhibitor. In some embodiments, the agent to which the antibody and/or the activatable antibody is conjugated is a nucleic acid damaging agent.

Efficaciousness of prevention, amelioration or treatment is determined in association with any known method for diagnosing or treating the disease or disorder associated with target expression and/or activity, such as, for example, aberrant target expression and/or activity. Prolonging the survival of a subject or otherwise delaying the progression of the disease or disorder associated with target expression and/or activity, e.g., aberrant target expression and/or activity, in a subject indicates that the antibody, conjugated antibody, activatable antibody and/or conjugated activatable antibody confers a clinical benefit.

An antibody, a conjugated antibody, an activatable antibody and/or a conjugated activatable antibody can be administered in the form of pharmaceutical compositions. In some embodiments where antibody fragments are used, the smallest fragment that specifically binds to the binding domain of the target protein is selected. For example, based upon the variable-region sequences of an antibody, peptide molecules can be designed that retain the ability to bind the target protein sequence. Such peptides can be synthesized chemically and/or produced by recombinant DNA technology. (*See, e.g.,* Marasco et al., Proc. Natl. Acad. Sci. USA, 90: 7889-7893 (1993)).

The formulations to be used for *in vivo* administration must be sterile. This is readily accomplished by filtration through sterile filtration membranes.

In some embodiments, the antibody, the conjugated antibody, activatable antibody and/or conjugated activatable antibody contains a detectable label. An intact antibody, or a fragment thereof (*e.g.*, Fab, scFv, or F(ab)₂) is used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. Included within the usage of the term "biological sample", therefore, is blood and a fraction or component of blood including blood serum, blood plasma, or lymph. That is, the detection method of the disclosure can be used to detect an analyte mRNA, protein, or genomic DNA in a biological sample *in vitro* as well as *in vivo.* For example, *in vitro* techniques for detection of an analyte mRNA include Northern hybridizations and *in situ* hybridizations. *In vitro* techniques for detection of an analyte protein include enzyme linked immunosorbent assays (ELISAs), Western blots, immunoprecipitations, immunochemical staining, and immunofluorescence. *In vitro* techniques for detection of an analyte genomic DNA include Southern hybridizations. Procedures for conducting immunoassays are described, for example in "ELISA: Theory and Practice: Methods in Molecular Biology", Vol. 42, J. R. Crowther (Ed.) Human Press, Totowa, NJ, 1995; "Immunoassay", E. Diamandis and T. Christopoulus, Academic Press, Inc., San Diego, CA, 1996; and "Practice and Theory of Enzyme Immunoassays", P. Tijssen, Elsevier Science Publishers, Amsterdam, 1985. Furthermore, *in vivo* techniques for detection of an analyte protein include introducing into a subject a labeled anti-analyte protein antibody. For example, the antibody can be labeled with a radioactive marker whose presence and location in a subject can be detected by standard imaging techniques.

The antibodies, conjugated antibodies, activatable antibodies and/or conjugated activatable antibodies of the disclosure are also useful in a variety of diagnostic and prophylactic formulations. In one embodiment, a conjugated activatable antibody is administered to patients that are at risk of developing one or more of the aforementioned disorders. A patient's or organ's predisposition to one or more of the aforementioned disorders can be determined using genotypic, serological or biochemical markers.

In some embodiments of the disclosure, a conjugated activatable antibody is administered to human individuals diagnosed with a clinical indication associated with one or more of the aforementioned disorders. Upon diagnosis, a conjugated activatable antibody is administered to mitigate or reverse the effects of the clinical indication.

An antibody, a conjugated antibody, an activatable antibody, and/or a conjugated activatable antibody of the disclosure is also useful in the detection of a target in patient samples and accordingly are useful as diagnostics. For example, the antibodies and/or activatable antibodies, and conjugated versions thereof, of the disclosure are used in *in vitro* assays, *e.g.,* ELISA, to detect target levels in a patient sample.

In one embodiment, an antibody, a conjugated antibody, an activatable antibody and/or a conjugated activatable antibody of the disclosure is immobilized on a solid support (e.g., the well(s) of a microtiter plate). The immobilized antibody, conjugated antibody, activatable antibody and/or conjugated activatable antibody serves as a capture antibody for any target that may be present in a test sample. Prior to contacting the immobilized antibody and/or activatable antibody, and/or conjugated versions thereof, with a patient sample, the solid support is rinsed and treated with a blocking agent such as milk protein or albumin to prevent nonspecific adsorption of the analyte.

Subsequently the wells are treated with a test sample suspected of containing the antigen, or with a solution containing a standard amount of the antigen. Such a sample is, *e.g.,* a serum sample from a subject suspected of having levels of circulating antigen considered to be diagnostic of a pathology. After rinsing away the test sample or standard, the solid support is treated with a second antibody that is detectably labeled. The labeled second antibody serves as a detecting antibody. The level of detectable label is measured, and the concentration of target antigen in the test sample is determined by comparison with a standard curve developed from the standard samples.

It will be appreciated that based on the results obtained using the antibodies and activatable antibodies of the disclosure, and conjugated versions thereof, in an *in vitro* diagnostic assay, it is possible to stage a disease in a subject based on expression levels of the target antigen. For a given disease, samples of blood are taken from subjects diagnosed as being at various stages in the progression of the disease, and/or at various points in the therapeutic treatment of the disease. Using a population of samples that provides statistically significant results for each stage of progression or therapy, a range of concentrations of the antigen that may be considered characteristic of each stage is designated.

An antibody, a conjugated antibody, an activatable antibody and/or a conjugated activatable antibody can also be used in diagnostic and/or imaging methods. In some embodiments, such methods are *in vitro* methods. In some embodiments, such methods are *in vivo* methods. In some embodiments, such methods are *in situ* methods. In some embodiments, such methods are *ex vivo* methods. For example, activatable antibodies having an enzymatically cleavable CM can be used to detect the presence or absence of an enzyme that is capable of cleaving the CM. Such activatable antibodies can be used in diagnostics, which can include *in vivo* detection (*e.g.,* qualitative or quantitative) of enzyme activity (or, in some embodiments, an environment of increased reduction potential such as that which can provide for reduction of a disulfide bond) through measured accumulation of activated antibodies (i.e., antibodies resulting from cleavage of an activatable antibody) in a given cell or tissue of a given host organism. Such accumulation of activated antibodies indicates not only that the tissue expresses enzymatic activity (or an increased reduction potential depending on the nature of the CM) but also that the tissue expresses target to which the activated antibody binds.

For example, the CM can be selected to be substrate for at least one protease found at the site of a tumor, at the site of a viral or bacterial infection at a biologically confined site (*e.g.,* such as in an abscess, in an organ, and the like), and the like. The AB can be one that binds a target antigen. Using methods as disclosed herein, or when appropriate, methods familiar to one skilled in the art, a detectable label (*e.g.,* a fluorescent label or radioactive label or radiotracer) can be conjugated to an AB or other region of an antibody and/or activatable antibody. Suitable detectable labels are discussed in the context of the above screening methods and additional specific examples are provided below. Using an AB specific to a protein or peptide of the disease state, along with at least one protease whose activity is elevated in the disease tissue of interest, activatable antibodies will exhibit an increased rate of binding to disease tissue relative to tissues where the CM specific enzyme is not present at a detectable level or is present at a lower level than in disease tissue or is inactive (e.g., in zymogen form or in complex with an inhibitor). Since small proteins and peptides are rapidly cleared from the blood by the renal filtration system, and because the enzyme specific for the CM is not present at a detectable level (or is present at lower levels in non-disease tissues or is present in inactive conformation), accumulation of activated antibodies in the disease tissue is enhanced relative to non-disease tissues.

In another example, activatable antibodies can be used to detect the presence or absence of a cleaving agent in a sample. For example, where the activatable antibodies contain a CM susceptible to cleavage by an enzyme, the activatable antibodies can be used to detect (either qualitatively or quantitatively) the presence of an enzyme in the sample. In another example, where the activatable antibodies contain a CM susceptible to cleavage by reducing agent, the activatable antibodies can be used to detect (either qualitatively or quantitatively) the presence of reducing conditions in a sample. To facilitate analysis in these methods, the activatable antibodies can be detectably labeled, and can be bound to a support (*e.g.*, a solid support, such as a slide or bead). The detectable label can be positioned on a portion of the activatable antibody that is not released following cleavage, for example, the detectable label can be a quenched fluorescent label or other label that is not detectable until cleavage has occurred. The assay can be conducted by, for example, contacting the immobilized, detectably labeled activatable antibodies with a sample suspected of containing an enzyme and/or reducing agent for a time sufficient for cleavage to occur, then washing to remove excess sample and contaminants. The presence or absence of the cleaving agent (*e.g.*, enzyme or reducing agent) in the sample is then assessed by a change in detectable signal of the activatable antibodies prior to contacting with the sample *e.g.*, the presence of and/or an increase in detectable signal due to cleavage of the activatable antibody by the cleaving agent in the sample.

Such detection methods can be adapted to also provide for detection of the presence or absence of a target that is capable of binding the AB of the activatable antibodies when cleaved. Thus, the assays can be adapted to assess the presence or absence of a cleaving agent and the presence or absence of a target of interest. The presence or absence of the cleaving agent can be detected by the presence of and/or an increase in detectable label of the activatable antibodies as described above, and the presence or absence of the target can be detected by detection of a target-AB complex *e.g.*, by use of a detectably labeled anti-target antibody.

Activatable antibodies are also useful in *in situ* imaging for the validation of activatable antibody activation, *e.g.,* by protease cleavage, and binding to a particular target. *In situ* imaging is a technique that enables localization of proteolytic activity and target in biological samples such as cell cultures or tissue sections. Using this technique, it is possible to confirm both binding to a given target and proteolytic activity based on the presence of a detectable label (e.g., a fluorescent label).

These techniques are useful with any frozen cells or tissue derived from a disease site (*e.g.* tumor tissue) or healthy tissues. These techniques are also useful with fresh cell or tissue samples.

In these techniques, an activatable antibody is labeled with a detectable label. The detectable label can be a fluorescent dye, (e.g. a fluorophore, Fluorescein Isothiocyanate (FITC), Rhodamine Isothiocyanate (TRITC), an Alexa Fluor® label), a near infrared (NIR) dye (e.g., Qdot® nanocrystals), a colloidal metal, a hapten, a radioactive marker, biotin and an amplification reagent such as streptavidin, or an enzyme (e.g. horseradish peroxidase or alkaline phosphatase).

Detection of the label in a sample that has been incubated with the labeled, activatable antibody indicates that the sample contains the target and contains a protease that is specific for the CM of the activatable antibody. In some embodiments, the presence of the protease can be confirmed using broad spectrum protease inhibitors such as those described herein, and/or by using an agent that is specific for the protease, for example, an antibody such as A11, which is specific for the protease matriptase and inhibits the proteolytic activity of matriptase; see e.g., International Publication Number WO 2010/129609, published 11 November 2010. The same approach of using broad spectrum protease inhibitors such as those described herein, and/or by using a more selective inhibitory agent can be used to identify a protease that is specific for the CM of the activatable antibody. In some embodiments, the presence of the target can be confirmed using an agent that is specific for the target, *e.g.*, another antibody, or the detectable label can be competed with unlabeled target. In some embodiments, unlabeled activatable antibody could be used, with detection by a labeled secondary antibody or more complex detection system.

Similar techniques are also useful for *in vivo* imaging where detection of the fluorescent signal in a subject, *e.g.,* a mammal, including a human, indicates that the disease site contains the target and contains a protease that is specific for the CM of the activatable antibody.

These techniques are also useful in kits and/or as reagents for the detection, identification or characterization of protease activity in a variety of cells, tissues, and organisms based on the protease-specific CM in the activatable antibody.

The disclosure provides methods of using the antibodies and/or activatable antibodies in a variety of diagnostic and/or prophylactic indications. For example, the disclosure provides methods of detecting presence or absence of a cleaving agent and a target of interest in a subject or a sample by (i) contacting a subject or sample with an activatable antibody, wherein the activatable antibody comprises a masking moiety (MM), a cleavable moiety (CM) that is cleaved by the cleaving agent, e.g., a protease, and an antigen binding domain or fragment thereof (AB) that specifically binds the target of interest, wherein the activatable antibody in an uncleaved, non-activated state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB and is not a modified form of a natural binding partner of the AB; and (b) wherein, in an uncleaved, non-activated state, the MM interferes with specific binding of the AB to the target, and in a cleaved, activated state the MM does not interfere or compete with specific binding of the AB to the target; and (ii) measuring a level of activated activatable antibody in the subject or sample, wherein a detectable level of activated activatable antibody in the subject or sample indicates that the cleaving agent and the target are present in the subject or sample and wherein no detectable level of activated activatable antibody in the subject or sample indicates that the cleaving agent, the target or both the cleaving agent and the target are absent and/or not sufficiently present in the subject or sample. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides methods of detecting presence or absence of a cleaving agent in a subject or a sample by (i) contacting a subject or sample with an activatable antibody in the presence of a target of interest, e.g., the target, wherein the activatable antibody comprises a masking moiety (MM), a cleavable moiety (CM) that is cleaved by the cleaving agent, e.g., a protease, and an antigen binding domain or fragment thereof (AB) that specifically binds the target of interest, wherein the activatable antibody in an uncleaved, non-activated state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB and is not a modified form of a natural binding partner of the AB; and (b) wherein, in an uncleaved, non-activated state, the MM interferes with specific binding of the AB to the target, and in a cleaved, activated state the MM does not interfere or compete with specific binding of the AB to the target; and (ii) measuring a level of activated activatable antibody in the subject or sample, wherein a detectable level of activated activatable antibody in the subject or sample indicates that the cleaving agent is present in the subject or sample and wherein no detectable level of activated activatable antibody in the subject or sample indicates that the cleaving agent is absent and/or not sufficiently present in the subject or sample. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides kits for use in methods of detecting presence or absence of a cleaving agent and the target in a subject or a sample, where the kits include at least an activatable antibody comprises a masking moiety (MM), a cleavable moiety (CM) that is cleaved by the cleaving agent, e.g., a protease, and an antigen binding domain or fragment thereof (AB) that specifically binds the target of interest, wherein the activatable antibody in an uncleaved, non-activated state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB and is not a modified form of a natural binding partner of the AB; and (b) wherein, in an uncleaved, non-activated state, the MM interferes with specific binding of the AB to the target, and in a cleaved, activated state the MM does not interfere or compete with specific binding of the AB to the target; and (ii) measuring a level of activated activatable antibody in the subject or sample, wherein a detectable level of activated activatable antibody in the subject or sample indicates that the cleaving agent is present in the subject or sample and wherein no detectable level of activated activatable antibody in the subject or sample indicates that the cleaving agent is absent and/or not sufficiently present in the subject or sample. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides methods of detecting presence or absence of a cleaving agent in a subject or a sample by (i) contacting a subject or sample with an activatable antibody, wherein the activatable antibody comprises a masking moiety (MM), a cleavable moiety (CM) that is cleaved by the cleaving agent, e.g., a protease, an antigen binding domain (AB) that specifically binds the target, and a detectable label, wherein the activatable antibody in an uncleaved, non-activated state comprises a structural arrangement from N-terminus to C-terminus as follows: MM-CM-AB or AB-CM-MM; wherein the MM is a peptide that inhibits binding of the AB to the target, and wherein the MM does not have an amino acid sequence of a naturally occurring binding partner of the AB and is not a modified form of a natural binding partner of the AB; wherein, in an uncleaved, non-activated state, the MM interferes with specific binding of the AB to the target, and in a cleaved, activated state the MM does not interfere or compete with specific binding of the AB to the target; and wherein the detectable label is positioned on a portion of the activatable antibody that is released following cleavage of the CM; and (ii) measuring a level of detectable label in the subject or sample, wherein a detectable level of the detectable label in the subject or sample indicates that the cleaving agent is absent and/or not sufficiently present in the subject or sample and wherein no detectable level of the detectable label in the subject or sample indicates that the cleaving agent is present in the subject or sample. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides kits for use in methods of detecting presence or absence of a cleaving agent and the target in a subject or a sample, where the kits include at least an activatable antibody and/or conjugated activatable antibody (e.g., an activatable antibody to which a therapeutic agent is conjugated) described herein for use in contacting a subject or biological sample and means for detecting the level of activated activatable antibody and/or conjugated activatable antibody in the subject or biological sample, wherein a detectable level of activated activatable antibody in the subject or biological sample indicates that the cleaving agent and the target are present in the subject or biological sample and wherein no detectable level of activated activatable antibody in the subject or biological sample indicates that the cleaving agent, the target or both the cleaving agent and the target are absent and/or not sufficiently present in the subject or biological sample, such that the target binding and/or protease cleavage of the activatable antibody cannot be detected in the subject or biological sample.

The disclosure also provides methods of detecting presence or absence of a cleaving agent in a subject or a sample by (i) contacting a subject or biological sample with an activatable antibody in the presence of the target, and (ii) measuring a level of activated activatable antibody in the subject or biological sample, wherein a detectable level of activated activatable antibody in the subject or biological sample indicates that the cleaving agent is present in the subject or biological sample and wherein no detectable level of activated activatable antibody in the subject or biological sample indicates that the cleaving agent is absent and/or not sufficiently present in the subject or biological sample at a detectable level, such that protease cleavage of the activatable antibody cannot be detected in the subject or biological sample. Such an activatable antibody includes a masking moiety (MM), a cleavable moiety (CM) that is cleaved by the cleaving agent, e.g., a protease, and an antigen binding domain or fragment thereof (AB) that specifically binds the target, wherein the activatable antibody in an uncleaved (*i.e.,* non-activated) state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB; and (b) wherein the MM of the activatable antibody in an uncleaved state interferes with specific binding of the AB to the target, and wherein the MM of an activatable antibody in a cleaved (*i.e.,* activated) state does not interfere or compete with specific binding of the AB to the target. In some embodiments, the activatable antibody is an activatable antibody to which a therapeutic agent is conjugated. In some embodiments, the activatable antibody is not conjugated to an agent. In some embodiments, the detectable label is attached to the masking moiety. In some embodiments, the detectable label is attached to the cleavable moiety N-terminal to the protease cleavage site. In some embodiments, a single antigen binding site of the AB is masked. In some embodiments wherein an antibody of the disclosure has at least two antigen binding sites, at least one antigen binding site is masked and at least one antigen binding site is not masked. In some embodiments all antigen binding sites are masked. In some embodiments, the measuring step includes use of a secondary reagent comprising a detectable label.

The disclosure also provides kits for use in methods of detecting presence or absence of a cleaving agent and the target in a subject or a sample, where the kits include at least an activatable antibody and/or conjugated activatable antibody described herein for use in contacting a subject or biological sample with an activatable antibody in the presence of the target, and measuring a level of activated activatable antibody in the subject or biological sample, wherein a detectable level of activated activatable antibody in the subject or biological sample indicates that the cleaving agent is present in the subject or biological sample and wherein no detectable level of activated activatable antibody in the subject or biological sample indicates that the cleaving agent is absent and/or not sufficiently present in the subject or biological sample at a detectable level, such that protease cleavage of the activatable antibody cannot be detected in the subject or biological sample. Such an activatable antibody includes a masking moiety (MM), a cleavable moiety (CM) that is cleaved by the cleaving agent, e.g., a protease, and an antigen binding domain or fragment thereof (AB) that specifically binds the target, wherein the activatable antibody in an uncleaved (*i.e.,* non-activated) state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB; and (b) wherein the MM of the activatable antibody in an uncleaved state interferes with specific binding of the AB to the target, and wherein the MM of an activatable antibody in a cleaved (*i.e.,* activated) state does not interfere or compete with specific binding of the AB to the target. In some embodiments, the activatable antibody is an activatable antibody to which a therapeutic agent is conjugated. In some embodiments, the activatable antibody is not conjugated to an agent. In some embodiments, the detectable label is attached to the masking moiety. In some embodiments, the detectable label is attached to the cleavable moiety N-terminal to the protease cleavage site. In some embodiments, a single antigen binding site of the AB is masked. In some embodiments wherein an antibody of the disclosure has at least two antigen binding sites, at least one antigen binding site is masked and at least one antigen binding site is not masked. In some embodiments all antigen binding sites are masked. In some embodiments, the measuring step includes use of a secondary reagent comprising a detectable label.

The disclosure also provides kits for use in methods of detecting presence or absence of a cleaving agent in a subject or a sample, where the kits include at least an activatable antibody and/or conjugated activatable antibody described herein for use in contacting a subject or biological sample and means for detecting the level of activated activatable antibody and/or conjugated activatable antibody in the subject or biological sample, wherein the activatable antibody includes a detectable label that is positioned on a portion of the activatable antibody that is released following cleavage of the CM, wherein a detectable level of activated activatable antibody in the subject or biological sample indicates that the cleaving agent is absent and/or not sufficiently present in the subject or biological sample such that the target binding and/or protease cleavage of the activatable antibody cannot be detected in the subject or biological sample, and wherein no detectable level of activated activatable antibody in the subject or biological sample indicates that the cleaving agent is present in the subject or biological sample at a detectable level.

The disclosure provides methods of detecting presence or absence of a cleaving agent and the target in a subject or a sample by (i) contacting a subject or biological sample with an activatable antibody, wherein the activatable antibody includes a detectable label that is positioned on a portion of the activatable antibody that is released following cleavage of the CM and (ii) measuring a level of activated activatable antibody in the subject or biological sample, wherein a detectable level of activated activatable antibody in the subject or biological sample indicates that the cleaving agent, the target or both the cleaving agent and the target are absent and/or not sufficiently present in the subject or biological sample, such that the target binding and/or protease cleavage of the activatable antibody cannot be detected in the subject or biological sample, and wherein a reduced detectable level of activated activatable antibody in the subject or biological sample indicates that the cleaving agent and the target are present in the subject or biological sample. A reduced level of detectable label is, for example, a reduction of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% and/or about 100%. Such an activatable antibody includes a masking moiety (MM), a cleavable moiety (CM) that is cleaved by the cleaving agent, and an antigen binding domain or fragment thereof (AB) that specifically binds the target, wherein the activatable antibody in an uncleaved (*i.e.,* non-activated) state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB; and (b) wherein the MM of the activatable antibody in an uncleaved state interferes with specific binding of the AB to the target, and wherein the MM of an activatable antibody in a cleaved (*i.e.,* activated) state does not interfere or compete with specific binding of the AB to the target. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides kits for use in methods of detecting presence or absence of a cleaving agent and the target in a subject or a sample, where the kits include at least an activatable antibody and/or conjugated activatable antibody described herein for use in contacting a subject or biological sample and means for detecting the level of activated activatable antibody and/or conjugated activatable antibody in the subject or biological sample, wherein a detectable level of activated activatable antibody in the subject or biological sample indicates that the cleaving agent, the target or both the cleaving agent and the target are absent and/or not sufficiently present in the subject or biological sample, such that the target binding and/or protease cleavage of the activatable antibody cannot be detected in the subject or biological sample, and wherein a reduced detectable level of activated activatable antibody in the subject or biological sample indicates that the cleaving agent and the target are present in the subject or biological sample. A reduced level of detectable label is, for example, a reduction of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% and/or about 100%.

The disclosure also provides methods of detecting presence or absence of a cleaving agent in a subject or a sample by (i) contacting a subject or biological sample with an activatable antibody, wherein the activatable antibody includes a detectable label that is positioned on a portion of the activatable antibody that is released following cleavage of the CM; and (ii) measuring a level of detectable label in the subject or biological sample, wherein a detectable level of the detectable label in the subject or biological sample indicates that the cleaving agent is absent and/or not sufficiently present in the subject or biological sample at a detectable level, such that protease cleavage of the activatable antibody cannot be detected in the subject or biological sample, and wherein a reduced detectable level of the detectable label in the subject or biological sample indicates that the cleaving agent is present in the subject or biological sample. A reduced level of detectable label is, for example, a reduction of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% and/or about 100%. Such an activatable antibody includes a masking moiety (MM), a cleavable moiety (CM) that is cleaved by the cleaving agent, and an antigen binding domain or fragment thereof (AB) that specifically binds the target, wherein the activatable antibody in an uncleaved (*i.e.,* non-activated) state comprises a structural arrangement from N-terminus to C-terminus as follows: MM-CM-AB or AB-CM-MM; (a) wherein the MM is a peptide that inhibits binding of the AB to the target, and wherein the MM does not have an amino acid sequence of a naturally occurring binding partner of the AB; and (b) wherein the MM of the activatable antibody in an uncleaved state interferes with specific binding of the AB to the target, and wherein the MM of an activatable antibody in a cleaved (*i.e.,* activated) state does not interfere or compete with specific binding of the AB to the target. In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

The disclosure also provides kits for use in methods of detecting presence or absence of a cleaving agent of interest in a subject or a sample, where the kits include at least an activatable antibody and/or conjugated activatable antibody described herein for use in contacting a subject or biological sample and means for detecting the level of activated activatable antibody and/or conjugated activatable antibody in the subject or biological sample, wherein the activatable antibody includes a detectable label that is positioned on a portion of the activatable antibody that is released following cleavage of the CM, wherein a detectable level of the detectable label in the subject or biological sample indicates that the cleaving agent, the target, or both the cleaving agent and the target are absent and/or not sufficiently present in the subject or biological sample, such that the target binding and/or protease cleavage of the activatable antibody cannot be detected in the subject or biological sample, and wherein a reduced detectable level of the detectable label in the subject or biological sample indicates that the cleaving agent and the target are present in the subject or biological sample. A reduced level of detectable label is, for example, a reduction of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% and/or about 100%.

In some embodiments of these methods and kits, the activatable antibody includes a detectable label. In some embodiments of these methods and kits, the detectable label includes an imaging agent, a contrasting agent, an enzyme, a fluorescent label, a chromophore, a dye, one or more metal ions, or a ligand-based label. In some embodiments of these methods and kits, the imaging agent comprises a radioisotope. In some embodiments of these methods and kits, the radioisotope is indium or technetium. In some embodiments of these methods and kits, the contrasting agent comprises iodine, gadolinium or iron oxide. In some embodiments of these methods and kits, the enzyme comprises horseradish peroxidase, alkaline phosphatase, or β-galactosidase. In some embodiments of these methods and kits, the fluorescent label comprises yellow fluorescent protein (YFP), cyan fluorescent protein (CFP), green fluorescent protein (GFP), modified red fluorescent protein (mRFP), red fluorescent protein tdimer2 (RFP tdimer2), HCRED, or a europium derivative. In some embodiments of these methods and kits, the luminescent label comprises an N-methylacrydium derivative. In some embodiments of these methods, the label comprises an Alexa Fluor® label, such as Alex Fluor® 680 or Alexa Fluor® 750. In some embodiments of these methods and kits, the ligand-based label comprises biotin, avidin, streptavidin or one or more haptens.

In some embodiments of these methods and kits, the subject is a mammal. In some embodiments of these methods and kits, the subject is a human. In some embodiments, the subject is a non-human mammal, such as a non-human primate, companion animal (e.g., cat, dog, horse), farm animal, work animal, or zoo animal. In some embodiments, the subject is a rodent.

In some embodiments of these methods, the method is an *in vivo* method. In some embodiments of these methods, the method is an *in situ* method. In some embodiments of these methods, the method is an *ex vivo* method. In some embodiments of these methods, the method is an *in vitro* method.

In some embodiments, *in situ* imaging and/or *in vivo* imaging are useful in methods to identify which patients to treat. For example, in *in situ* imaging, the activatable antibodies are used to screen patient samples to identify those patients having the appropriate protease(s) and target(s) at the appropriate location, *e.g.*, at a tumor site.

In some embodiments *in situ* imaging is used to identify or otherwise refine a patient population suitable for treatment with an activatable antibody of the disclosure. For example, patients that test positive for both the target (e.g., the target) and a protease that cleaves the substrate in the cleavable moiety (CM) of the activatable antibody being tested (e.g., accumulate activated antibodies at the disease site) are identified as suitable candidates for treatment with such an activatable antibody comprising such a CM. Likewise, patients that test negative for either or both of the target (*e.g.,* the target) and the protease that cleaves the substrate in the CM in the activatable antibody being tested using these methods might be identified as suitable candidates for another form of therapy. In some embodiments, such patients that test negative with respect to a first activatable antibody can be tested with other activatable antibodies comprising different CMs until a suitable activatable antibody for treatment is identified (e.g., an activatable antibody comprising a CM that is cleaved by the patient at the site of disease). In some embodiments, the patient is then administered a therapeutically effective amount of the activatable antibody for which the patient tested positive.

In some embodiments *in vivo* imaging is used to identify or otherwise refine a patient population suitable for treatment with an activatable antibody of the disclosure. For example, patients that test positive for both the target (e.g., the target) and a protease that cleaves the substrate in the cleavable moiety (CM) of the activatable antibody being tested (e.g., accumulate activated antibodies at the disease site) are identified as suitable candidates for treatment with such an activatable antibody comprising such a CM. Likewise, patients that test negative might be identified as suitable candidates for another form of therapy. In some embodiments, such patients that test negative with respect to a first activatable antibody can be tested with other activatable antibodies comprising different CMs until a suitable activatable antibody for treatment is identified (e.g., an activatable antibody comprising a CM that is cleaved by the patient at the site of disease). In some embodiments, the patient is then administered a therapeutically effective amount of the activatable antibody for which the patient tested positive.

In some embodiments of the methods and kits, the method or kit is used to identify or otherwise refine a patient population suitable for treatment with an activatable antibody of the disclosure. For example, patients that test positive for both the target (e.g., the target) and a protease that cleaves the substrate in the cleavable moiety (CM) of the activatable antibody being tested in these methods are identified as suitable candidates for treatment with such an activatable antibody comprising such a CM. Likewise, patients that test negative for both of the targets (e.g., the target) and the protease that cleaves the substrate in the CM in the activatable antibody being tested using these methods might be identified as suitable candidates for another form of therapy. In some embodiments, such patients can be tested with other activatable antibodies until a suitable activatable antibody for treatment is identified (e.g., an activatable antibody comprising a CM that is cleaved by the patient at the site of disease). In some embodiments, patients that test negative for either of the target (*e.g.,* the target) are identified as suitable candidates for treatment with such an activatable antibody comprising such a CM. In some embodiments, patients that test negative for either of the target (*e.g.,* the target) are identified as not being suitable candidates for treatment with such an activatable antibody comprising such a CM. In some embodiments, such patients can be tested with other activatable antibodies until a suitable activatable antibody for treatment is identified (e.g., an activatable antibody comprising a CM that is cleaved by the patient at the site of disease). In some embodiments, the activatable antibody is an activatable antibody to which a therapeutic agent is conjugated. In some embodiments, the activatable antibody is not conjugated to an agent. In some embodiments, the activatable antibody comprises a detectable label. In some embodiments, the detectable label is positioned on the AB. In some embodiments, measuring the level of activatable antibody in the subject or sample is accomplished using a secondary reagent that specifically binds to the activated antibody, wherein the reagent comprises a detectable label. In some embodiments, the secondary reagent is an antibody comprising a detectable label.

In some embodiments, a method or kit is used to identify or otherwise refine a patient population suitable for treatment with an anti-the target activatable antibody and/or conjugated activatable antibody (e.g., activatable antibody to which a therapeutic agent is conjugated) of the disclosure, followed by treatment by administering that activatable antibody and/or conjugated activatable antibody to a subject in need thereof. For example, patients that test positive for both the targets (e.g., the target) and a protease that cleaves the substrate in the cleavable moiety (CM) of the activatable antibody and/or conjugated activatable antibody being tested in these methods are identified as suitable candidates for treatment with such antibody and/or such a conjugated activatable antibody comprising such a CM, and the patient is then administered a therapeutically effective amount of the activatable antibody and/or conjugated activatable antibody that was tested. Likewise, patients that test negative for either or both of the target (e.g., the target) and the protease that cleaves the substrate in the CM in the activatable antibody being tested using these methods might be identified as suitable candidates for another form of therapy. In some embodiments, such patients can be tested with other antibody and/or conjugated activatable antibody until a suitable antibody and/or conjugated activatable antibody for treatment is identified (*e.g.,* an activatable antibody and/or conjugated activatable antibody comprising a CM that is cleaved by the patient at the site of disease). In some embodiments, the patient is then administered a therapeutically effective amount of the activatable antibody and/or conjugated activatable antibody for which the patient tested positive.

In some embodiments of these methods and kits, the MM is a peptide having a length from about 4 to 40 amino acids. In some embodiments of these methods and kits, the activatable antibody comprises a linker peptide, wherein the linker peptide is positioned between the MM and the CM. In some embodiments of these methods and kits, the activatable antibody comprises a linker peptide, where the linker peptide is positioned between the AB and the CM. In some embodiments of these methods and kits, the activatable antibody comprises a first linker peptide (LP1) and a second linker peptide (LP2), wherein the first linker peptide is positioned between the MM and the CM and the second linker peptide is positioned between the AB and the CM. In some embodiments of these methods and kits, each of LP1 and LP2 is a peptide of about 1 to 20 amino acids in length, and wherein each of LP1 and LP2 need not be the same linker. In some embodiments of these methods and kits, one or both of LP1 and LP2 comprises a glycine-serine polymer. In some embodiments of these methods and kits, at least one of LP1 and LP2 comprises an amino acid sequence selected from the group consisting of (GS)n, (GSGGS)n (SEQ ID NO: 24) and (GGGS)n (SEQ ID NO: 25), where n is an integer of at least one. In some embodiments of these methods and kits, at least one of LP1 and LP2 comprises an amino acid sequence having the formula (GGS)n, where n is an integer of at least one. In some embodiments of these methods and kits, at least one of LP1 and LP2 comprises an amino acid sequence selected from the group consisting of Gly-Gly-Ser-Gly (SEQ ID NO: 26), Gly-Gly-Ser-Gly-Gly (SEQ ID NO: 27), Gly-Ser-Gly-Ser-Gly (SEQ ID NO: 28), Gly-Ser-Gly-Gly-Gly (SEQ ID NO: 29), Gly-Gly-Gly-Ser-Gly (SEQ ID NO: 30), and Gly-Ser-Ser-Ser-Gly (SEQ ID NO: 31).

In some embodiments of these methods and kits, the AB comprises an antibody or antibody fragment sequence selected from the cross-reactive antibody sequences presented herein. In some embodiments of these methods and kits, the AB comprises a Fab fragment, a scFv or a single chain antibody (scAb).

In some embodiments of these methods and kits, the cleaving agent is a protease that is co-localized in the subject or sample with the target and the CM is a polypeptide that functions as a substrate for the protease, wherein the protease cleaves the CM in the activatable antibody when the activatable antibody is exposed to the protease. In some embodiments of these methods and kits, the CM is a polypeptide of up to 15 amino acids in length. In some embodiments of these methods and kits, the CM is coupled to the N-terminus of the AB. In some embodiments of these methods and kits, the CM is coupled to the C-terminus of the AB. In some embodiments of these methods and kits, the CM is coupled to the N-terminus of a VL chain of the AB.

The N- and C-termini of antibody polypeptide chains of the present invention may differ from the sequences described herein due to commonly observed post-translational modifications. For example, C-terminal lysine residues are often missing from antibody heavy chains. Dick et al. (2008) Biotechnol. Bioeng. 100:1132. N-terminal glutamine residues, and to a lesser extent glutamate residues, are frequently converted to pyroglutamate residues on both light and heavy chains of therapeutic antibodies. Dick et al. (2007) Biotechnol. Bioeng. 97:544; Liu et al. (2011) JBC 28611211; Liu et al. (2011) J. Biol. Chem. 286:11211. Accordingly, the conjugated activatable antibody of Formula (I) and/or Formula (II) may have an antibody (AB) where the C-terminal residue of the heavy chain constant region either lacks one or more amino acids at the terminus, lacks the C-terminal lysine, has the C-terminal lysine removed due to post-translationally processing, or the C-terminal lysine is an amino acid other than lysine. If the C-terminal residue of the heavy chain constant region is an amino acid other than lysine, in one embodiment, it is an amino acid not generally amenable to forming a disulfide bond or otherwise not amenable to conjugation with a cytotoxic agent.

In certain embodiments, the conjugated activatable antibody of Formula (I) and/or Formula (II), may have an antibody (AB) in which the N-terminal glutamate on either the heavy chain and/or light chain is optionally either pyroglutamate or post-translationally modified to pyroglutamate.

In certain embodiments, the heavy chain constant region of the conjugated activatable antibody of Formula (I) and/or Formula (II) comprises a lysine or another amino acid at the C-terminus, e.g., it comprises the following last amino acids: PGK for the heavy chain. In certain embodiments, the heavy chain constant region is lacking one or more amino acids at the C-terminus, and has, e.g., the C-terminal sequence PG or P.

In certain embodiments, the heavy chain and/or light chain variable regions of the conjugated activatable antibody of Formula (I) and/or Formula (II) comprises a glutamate or pyroglutamate amino acid at the N-terminus, e.g., it comprises the following last amino acids: pQGQ for the light chain, and/or pQVQ for the heavy chain, where "pQ" represents pyroglutamate.

The antibodies, conjugated antibodies, activatable antibodies and/or conjugated activatable antibodies of the disclosure are used in diagnostic and prophylactic formulations. In one embodiment, an activatable antibody is administered to patients that are at risk of developing one or more of the aforementioned inflammation, inflammatory disorders, cancer or other disorders.

A patient's or organ's predisposition to one or more of the aforementioned disorders can be determined using genotypic, serological or biochemical markers.

In some embodiments of the disclosure, an antibody, a conjugated antibody, an activatable antibody and/or a conjugated activatable antibody is administered to human individuals diagnosed with a clinical indication associated with one or more of the aforementioned disorders. Upon diagnosis, an antibody, a conjugated antibody, an activatable antibody and/or a conjugated activatable antibody is administered to mitigate or reverse the effects of the clinical indication.

Antibodies, conjugated antibodies, activatable antibodies and/or conjugated activatable antibodies of the disclosure are also useful in the detection of the target in patient samples and accordingly are useful as diagnostics. For example, the antibodies, conjugated antibodies, the activatable antibodies and/or conjugated activatable antibodies of the disclosure are used in *in vitro* assays, *e.g.*, ELISA, to detect target levels in a patient sample.

In one embodiment, an antibody and/or activatable antibody of the disclosure is immobilized on a solid support (*e.g.*, the well(s) of a microtiter plate). The immobilized antibody and/or activatable antibody serves as a capture antibody for any target that may be present in a test sample. Prior to contacting the immobilized antibody and/or activatable antibody with a patient sample, the solid support is rinsed and treated with a blocking agent such as milk protein or albumin to prevent nonspecific adsorption of the analyte.

Subsequently the wells are treated with a test sample suspected of containing the antigen, or with a solution containing a standard amount of the antigen. Such a sample is, *e.g.,* a serum sample from a subject suspected of having levels of circulating antigen considered to be diagnostic of a pathology. After rinsing away the test sample or standard, the solid support is treated with a second antibody that is detectably labeled. The labeled second antibody serves as a detecting antibody. The level of detectable label is measured, and the concentration of target antigen in the test sample is determined by comparison with a standard curve developed from the standard samples.

It will be appreciated that based on the results obtained using the antibodies and/or activatable antibodies of the disclosure in an *in vitro* diagnostic assay, it is possible to stage a disease in a subject based on expression levels of the Target antigen. For a given disease, samples of blood are taken from subjects diagnosed as being at various stages in the progression of the disease, and/or at various points in the therapeutic treatment of the disease. Using a population of samples that provides statistically significant results for each stage of progression or therapy, a range of concentrations of the antigen that may be considered characteristic of each stage is designated.

Antibodies, conjugated antibodies, activatable antibodies and/or conjugated activatable antibodies can also be used in diagnostic and/or imaging methods. In some embodiments, such methods are *in vitro* methods. In some embodiments, such methods are *in vivo* methods. In some embodiments, such methods are *in situ* methods. In some embodiments, such methods are *ex vivo* methods. For example, activatable antibodies having an enzymatically cleavable CM can be used to detect the presence or absence of an enzyme that is capable of cleaving the CM. Such activatable antibodies can be used in diagnostics, which can include *in vivo* detection *(e.g.,* qualitative or quantitative) of enzyme activity (or, in some embodiments, an environment of increased reduction potential such as that which can provide for reduction of a disulfide bond) through measured accumulation of activated antibodies (i.e., antibodies resulting from cleavage of an activatable antibody) in a given cell or tissue of a given host organism. Such accumulation of activated antibodies indicates not only that the tissue expresses enzymatic activity (or an increased reduction potential depending on the nature of the CM) but also that the tissue expresses target to which the activated antibody binds.

For example, the CM can be selected to be a protease substrate for a protease found at the site of a tumor, at the site of a viral or bacterial infection at a biologically confined site (*e.g.,* such as in an abscess, in an organ, and the like), and the like. The AB can be one that binds a target antigen. Using methods familiar to one skilled in the art, a detectable label (*e.g.,* a fluorescent label or radioactive label or radiotracer) can be conjugated to an AB or other region of an activatable antibody. Suitable detectable labels are discussed in the context of the above screening methods and additional specific examples are provided below. Using an AB specific to a protein or peptide of the disease state, along with a protease whose activity is elevated in the disease tissue of interest, activatable antibodies will exhibit an increased rate of binding to disease tissue relative to tissues where the CM specific enzyme is not present at a detectable level or is present at a lower level than in disease tissue or is inactive (e.g., in zymogen form or in complex with an inhibitor). Since small proteins and peptides are rapidly cleared from the blood by the renal filtration system, and because the enzyme specific for the CM is not present at a detectable level (or is present at lower levels in non-disease tissues or is present in inactive conformation), accumulation of activated antibodies in the disease tissue is enhanced relative to non-disease tissues.

In another example, activatable antibodies can be used to detect the presence or absence of a cleaving agent in a sample. For example, where the activatable antibodies contain a CM susceptible to cleavage by an enzyme, the activatable antibodies can be used to detect (either qualitatively or quantitatively) the presence of an enzyme in the sample. In another example, where the activatable antibodies contain a CM susceptible to cleavage by reducing agent, the activatable antibodies can be used to detect (either qualitatively or quantitatively) the presence of reducing conditions in a sample. To facilitate analysis in these methods, the activatable antibodies can be detectably labeled, and can be bound to a support (*e.g.*, a solid support, such as a slide or bead). The detectable label can be positioned on a portion of the activatable antibody that is not released following cleavage, for example, the detectable label can be a quenched fluorescent label or other label that is not detectable until cleavage has occurred. The assay can be conducted by, for example, contacting the immobilized, detectably labeled activatable antibodies with a sample suspected of containing an enzyme and/or reducing agent for a time sufficient for cleavage to occur, then washing to remove excess sample and contaminants. The presence or absence of the cleaving agent (*e.g.*, enzyme or reducing agent) in the sample is then assessed by a change in detectable signal of the activatable antibodies prior to contacting with the sample *e.g.*, the presence of and/or an increase in detectable signal due to cleavage of the activatable antibody by the cleaving agent in the sample.

Such detection methods can be adapted to also provide for detection of the presence or absence of a target that is capable of binding the AB of the activatable antibodies when cleaved. Thus, the assays can be adapted to assess the presence or absence of a cleaving agent and the presence or absence of a target of interest. The presence or absence of the cleaving agent can be detected by the presence of and/or an increase in detectable label of the activatable antibodies as described above, and the presence or absence of the target can be detected by detection of a target-AB complex *e.g.,* by use of a detectably labeled anti-target antibody.

Activatable antibodies are also useful in *in situ* imaging for the validation of activatable antibody activation, *e.g.,* by protease cleavage, and binding to a particular target. *In situ* imaging is a technique that enables localization of proteolytic activity and target in biological samples such as cell cultures or tissue sections. Using this technique, it is possible to confirm both binding to a given target and proteolytic activity based on the presence of a detectable label (e.g., a fluorescent label).

These techniques are useful with any frozen cells or tissue derived from a disease site (*e.g.* tumor tissue) or healthy tissues. These techniques are also useful with fresh cell or tissue samples.

In these techniques, an activatable antibody is labeled with a detectable label. The detectable label can be a fluorescent dye, (e.g. Fluorescein Isothiocyanate (FITC), Rhodamine Isothiocyanate (TRITC), a near infrared (NIR) dye (e.g., Qdot® nanocrystals), a colloidal metal, a hapten, a radioactive marker, biotin and an amplification reagent such as streptavidin, or an enzyme (e.g. horseradish peroxidase or alkaline phosphatase).

Detection of the label in a sample that has been incubated with the labeled, activatable antibody indicates that the sample contains the target and contains a protease that is specific for the CM of the activatable antibody. In some embodiments, the presence of the protease can be confirmed using broad spectrum protease inhibitors such as those described herein, and/or by using an agent that is specific for the protease, for example, an antibody such as A11, which is specific for the protease matriptase and inhibits the proteolytic activity of matriptase; see e.g., International Publication Number WO 2010/129609, published 11 November 2010. The same approach of using broad spectrum protease inhibitors such as those described herein, and/or by using a more selective inhibitory agent can be used to identify a protease or class of proteases specific for the CM of the activatable antibody. In some embodiments, the presence of the target can be confirmed using an agent that is specific for the target, *e.g.,* another antibody, or the detectable label can be competed with unlabeled target. In some embodiments, unlabeled activatable antibody could be used, with detection by a labeled secondary antibody or more complex detection system.

Similar techniques are also useful for *in vivo* imaging where detection of the fluorescent signal in a subject, *e.g.,* a mammal, including a human, indicates that the disease site contains the target and contains a protease that is specific for the CM of the activatable antibody.

These techniques are also useful in kits and/or as reagents for the detection, identification or characterization of protease activity in a variety of cells, tissues, and organisms based on the protease-specific CM in the activatable antibody.

In some embodiments, *in situ* imaging and/or *in vivo* imaging are useful in methods to identify which patients to treat. For example, in *in situ* imaging, the activatable antibodies are used to screen patient samples to identify those patients having the appropriate protease(s) and target(s) at the appropriate location, e.g., at a tumor site.

In some embodiments *in situ* imaging is used to identify or otherwise refine a patient population suitable for treatment with an activatable antibody of the disclosure. For example, patients that test positive for both the target and a protease that cleaves the substrate in the cleavable moiety (CM) of the activatable antibody being tested (e.g., accumulate activated antibodies at the disease site) are identified as suitable candidates for treatment with such an activatable antibody comprising such a CM. Likewise, patients that test negative for either or both of the target and the protease that cleaves the substrate in the CM in the activatable antibody being tested using these methods are identified as suitable candidates for another form of therapy (*i.e.,* not suitable for treatment with the activatable antibody being tested). In some embodiments, such patients that test negative with respect to a first activatable antibody can be tested with other activatable antibodies comprising different CMs until a suitable activatable antibody for treatment is identified (e.g., an activatable antibody comprising a CM that is cleaved by the patient at the site of disease).

In some embodiments *in vivo* imaging is used to identify or otherwise refine a patient population suitable for treatment with an activatable antibody of the disclosure. For example, patients that test positive for both the target and a protease that cleaves the substrate in the cleavable moiety (CM) of the activatable antibody being tested (e.g., accumulate activated antibodies at the disease site) are identified as suitable candidates for treatment with such an activatable antibody comprising such a CM. Likewise, patients that test negative are identified as suitable candidates for another form of therapy (*i.e.,* not suitable for treatment with the activatable antibody being tested). In some embodiments, such patients that test negative with respect to a first activatable antibody can be tested with other activatable antibodies comprising different CMs until a suitable activatable antibody for treatment is identified (e.g., an activatable antibody comprising a CM that is cleaved by the patient at the site of disease).

### Pharmaceutical compositions

The antibodies, conjugated antibodies, activatable antibodies and/or conjugated activatable antibodies of the disclosure (also referred to herein as "active compounds"), and derivatives, fragments, analogs and homologs thereof, can be incorporated into pharmaceutical compositions suitable for administration.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutically acceptable carrier. The specification for the dosage unit forms of the disclosure are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration.

The invention will be further described in the following enumerated embodiments and examples, which do not limit the scope of the invention described in the claims.

### ENUMERATED EMBODIMENTS

The invention may be defined by reference to the following enumerated, illustrative embodiments.

### Set I

I-1. A conjugated activatable antibody comprising:
   (a) an activatable antibody (AA) comprising in an uncleaved state the structural arrangement from N-terminus to C-terminus as follows: MM-CM-AB, wherein:
      (i) AB is an antibody that specifically binds to mammalian CD71 and comprises the heavy chain variable region sequence of SEQ ID NO: 5 and the light chain variable region sequence of SEQ ID NO: 7;
      (ii) MM is a masking moiety comprising the amino acid sequence of SEQ ID NO: 18, wherein the MM coupled to the AB inhibits the binding of the AB to CD71 when the conjugated activatable antibody is in an uncleaved state;
      (iii) CM is a cleavable moiety comprising the sequence of SEQ ID NO: 156 coupled to the AB wherein the CM is a polypeptide that functions as a substrate for a protease; and
   (b) monomethyl auristatin E (MMAE), wherein the activatable antibody is conjugated to two equivalents of MMAE
I-2. A composition comprising:a conjugated activatable antibody having the formula AA-(AG)p wherein
   (a) AA is an activatable antibody comprising in an uncleaved state the structural arrangement from N-terminus to C-terminus as follows: MM-CM-AB, wherein:
      (i) AB is an antibody that specifically binds to mammalian CD71 and comprises the heavy chain variable region sequence of SEQ ID NO: 5 and the light chain variable region sequence of SEQ ID NO: 7,
      (ii) MM is a masking moiety comprising the sequence of SEQ ID NO: 18, wherein the MM coupled to the AB inhibits the binding of the AB to CD71 when the conjugated activatable antibody is in an uncleaved state, and
      (iii) CM is a cleavable moiety comprising the sequence of SEQ ID NO: 156 coupled to the AB wherein the CM is a polypeptide that functions as a substrate for a protease; and
   (b) AG is an agent conjugated to the AA, wherein the agent is MMAE and wherein p is 2.
I-3. A method of manufacturing a conjugated activatable antibody comprising: conjugating at least one MMAE to an activatable antibody (AA) thereby producing a composition comprising AA-(MMAE)p, wherein p is 1 to 8; and enriching the composition for the conjugated activatable antibody species in which p is 2, wherein AA comprises in an uncleaved state comprises the structural arrangement from N-terminus to C-terminus as follows: MM-CM-AB wherein:
   (i) AB is an antibody that specifically binds to mammalian CD71 and comprises the heavy chain variable region sequence of SEQ ID NO: 5 and the light chain variable region sequence of SEQ ID NO: 7,
   (ii) MM is a masking moiety comprising the amino acid sequence of SEQ ID NO: 18, wherein the MM coupled to the AB that inhibits the binding of the AB to CD71 when the conjugated activatable antibody is in an uncleaved state, and
   (iii) CM is a cleavable moiety comprising the sequence of SEQ ID NO: 156 coupled to the AB wherein the CM is a polypeptide that functions as a substrate for a protease.
I-4. The conjugated activatable antibody of embodiment I-1 or the composition of embodiment I-2 or the method of embodiment I-3, wherein the AB comprises the heavy chain sequence of SEQ ID NO: 20.
I-5. The conjugated activatable antibody of embodiment I-1 or the composition of embodiment I-2 or the method of embodiment I-3, wherein the AB comprises the heavy chain sequence of SEQ ID NO: 167.
I-6. The conjugated activatable antibody of any one of embodiments I-1, I-4, and I-5 or the composition of any one of embodiments I-2, I-4, and I-5 or the method of any one of embodiments I-3 to I-5, wherein the AB comprises the light chain sequence of SEQ ID NO: 19.
I-7. The conjugated activatable antibody of any one of embodiments I-1 and I-4 to I-6 or the composition of any one of embodiments I-2 and I-4 to I-6 or the method of any one of embodiments I- 3 to 6, wherein the AA comprises a linking peptide LP1 between the AB and the MM
I-8. The conjugated activatable antibody or composition or method of embodiment I-7, wherein the LP1 comprises the amino acid sequence of GGGSSGGS (SEQ ID NO: 207).
I-9. The conjugated activatable antibody of any one of embodiments I- 1 and 4 to 8 or the composition of any one of embodiments I-2 and I-4 to I-8 or the method of any one of embodiments I-3 to I-8, wherein the AA comprises a linking peptide LP2 between the AB and the CM.
I-10. The conjugated activatable antibody or composition or method of embodiment 1-9, wherein the LP2 comprises the amino acid sequence of GGGS (SEQ ID NO: 38).
I-11. The conjugated activatable antibody of any one of embodiments I-1 and I-4 to I-10 or the composition of any one of embodiments I-2 and I-4 to I-10 or the method of any one of embodiments I-3 to I-10, wherein the AA comprises a first linking peptide (LP1) and a second linking peptide (LP2), and wherein the AA in the uncleaved state has the structural arrangement from N-terminus to C-terminus as follows: MM-LP1-CM-LP2-AB.
I-12. The conjugated activatable antibody of any one of embodiments I-1 and I-4 to I-11 or the composition of any one of embodiments I-2 and I-4 to I-11 or the method of any one of embodiments I-3 to I-11, wherein the activatable antibody comprises a light chain comprising a spacer sequence selected from the group consisting of SEQ ID NOs: 138 and 143-145, and GQG.
I-13. The conjugated activatable antibody or composition or method of embodiment I-12, wherein the spacer sequence is N-terminal to the MM
I-14. The conjugated activatable antibody of any one of embodiments I-1 and I-4 to I-13 or the composition of any one of embodiments I-2 and I-4 to I-13 or the method of any one of embodiments I-3 to I-13, wherein the activatable antibody comprises the light chain variable region sequence of SEQ ID NOs: 201.
I-15. The conjugated activatable antibody of any one of embodiments I-1 and I-4 to I-13 or the composition of any one of embodiments I-2 and I-4 to I-13 or the method of any one of embodiments I-3 to I-13, wherein the activatable antibody comprises the light chain variable region sequence of SEQ ID NOs: 202.
1-16. The conjugated activatable antibody of any one of embodiments I-1 and I-4 to I-13 or the composition of any one of embodiments I-2 and I-4 to I-13 or the method of any one of embodiments I-3 to I-13, wherein the activatable antibody comprises the light chain sequence of SEQ ID NO: 169.
I-17. The conjugated activatable antibody of any one of embodiments I-1 and I-4 to I-13 or the composition of any one of embodiments I-2 and I-4 to I-13 or the method of any one of embodiments I-3 to I-13, wherein the activatable antibody comprises the light chain sequence of SEQ ID NO: 170.
I-18. The conjugated activatable antibody of any one of embodiments I-1 and I-4 to 1-17 or the composition of any one of embodiments I-2 and I-4 to I-17 or the method of any one of embodiments I-3 to I-17, wherein the AB specifically binds human CD71 and cynomolgus monkey CD71.
I-19. The conjugated activatable antibody of any one of embodiments I-1 and I-4 to I-18 or the composition of any one of embodiments I-2 and 4 to 18 or the method of any one of embodiments I-3 to I-18, wherein the MM has a dissociation constant for binding to the AB that is greater than the dissociation constant of the AB to CD71.
I-20. The conjugated activatable antibody of any one of embodiments I-1 and I-4 to I-19 or the composition of any one of embodiments I-2 and I-4 to I-19 or the method of any one of embodiments I-3 to I-19, wherein the MM does not interfere or compete with the AB for binding to CD71 when the activatable antibody is in a cleaved state.
I-21. The conjugated activatable antibody of any one of embodiments I-1 and I-4 to I-20 or the composition of any one of embodiments I-2 and I-4 to I-20 or the method of any one of embodiments I-3 to I-20, wherein the MM is a polypeptide of no more than 40 amino acids in length.
I-22. The conjugated activatable antibody of any one of embodiments I-1 and I-4 to I-21 or the composition of any one of embodiments I-2 and I-4 to I-21 or the method of any one of embodiments I-3 to I-21, wherein the CM is a substrate for a protease that is active in diseased tissue.
I-23. The conjugated activatable antibody of any one of embodiments I-1 and I-4 to I-22 or the composition of any one of embodiments I-2 and I-4 to I-22 or the method of any one of embodiments I-3 to I-22, wherein the agent is conjugated to the AB via a linker.
I-24. The conjugated activatable antibody of any one of embodiments I-1 and I-4 to I-23 or the composition of any one of embodiments I-2 and I-4 to I-23 or the method of any one of embodiments I-3 to I-23, wherein the linker with which the agent is conjugated to the AB comprises a valine-citrulline moiety.
I-25. The conjugated activatable antibody of any one of embodiments I-1 and I-4 to I-24 or the composition of any one of embodiments I-2 and I-4 to I-24 or the method of any one of embodiments I-3 to I-24, wherein the linker with which the agent is conjugated to the AB comprises a maleimide caproyl-valine-citrulline moiety.
I-26. The conjugated activatable antibody of any one of embodiments I-1 and I-4 to I-25 or the composition of any one of embodiments I-2 and I-4 to I-25 or the method of any one of embodiments I-3 to I-25, wherein the linker with which the agent is conjugated to the AB comprises a maleimide caproyl-valine-citrulline para aminobenzyloxycarbonyl moiety.
**I**-27. The composition of any one of embodiments I-2 and I-4 to I-26 or the method of any one of embodiments I-3 to I-26, wherein at least 50% of the conjugated activatable antibody of the composition is of the species when p is 2.
**I**-28. The composition of any one of embodiments I-2 and I-4 to I-26 or the method of any one of embodiments I-3 to I-26, wherein at least 75% of the conjugated activatable antibody of the composition is of the species when p is 2.
I-29. The composition of any one of embodiments I-2 and I-4 to I-26 or the method of any one of embodiments I-3 to I-26, wherein at least 90% of the conjugated activatable antibody of the composition is of the species when p is 2.
**I**-30. The composition of any one of embodiments I-2 and I-4 to I-26 or the method of any one of embodiments I-3 to I-26, wherein at least 95% of the conjugated activatable antibody of the composition is of the species when p is 2.
I-31. The composition of any one of embodiments I-2 and I-4 to I-26 or the method of any one of embodiments I-3 to I-26, wherein at least 98% of the conjugated activatable antibody of the composition is of the species when p is 2.
I-32. The composition of any one of embodiments I-2 and I-4 to I-26 or the method of any one of embodiments I-3 to I-26, wherein the equivalents of each of the conjugated activatable antibody species of the composition in which p is 1 or 3 to 8 is less than the equivalents of the conjugated activatable antibody species of the composition in which p is 2.
I-33. The composition of any one of embodiments I-2 and I-4 to I-26 or the method of any one of embodiments I-3 to I-26, wherein the conjugated activatable antibody species of the composition in which p is 1 or 3 to 8 is less than the equivalents of the conjugated activatable antibody species of the composition in which p is 2.
I-34. The composition of any one of embodiments I-2 and I-4 to I-26 or the method of any one of embodiments I-3 to I-26, wherein less than 50% of the conjugated activatable antibody species of the composition is of the species when p is 1 or 3 to 8.
I-35. The composition of any one of embodiments I-2 and I-4 to I-26 or the method of any one of embodiments I-3 to I-26, wherein less than 25% of the conjugated activatable antibody species of the composition is of the species when p is 1 or 3 to 8.
I-36. The composition of any one of embodiments I-2 and I-4 to I-26 or the method of any one of embodiments I-3 to I-26, wherein less than 10% of the conjugated activatable antibody species of the composition is of the species when p is 1 or 3 to 8.
I-37. The composition of any one of embodiments I-2 and I-4 to I-26 or the method of any one of embodiments I-3 to I-26, wherein less than 5% of the conjugated activatable antibody species of the composition is of the species when p is 1 or 3 to 8.
I-38. The composition of any one of embodiments I-2 and I-4 to I-26 or the method of any one of embodiments I-3 to I-26, wherein less than 2% of the conjugated activatable antibody species of the composition is of the species when p is 1 or 3 to 8.
I-39. A pharmaceutical composition comprising:
   the conjugated activatable antibody of any one of embodiments I-1 and I-4 to I-26 or the composition of any one of embodiments I-2 and I-4 to I-38; and a carrier.
I-40. A method of treating, alleviating a symptom of, or delaying the progression of a cancer in a subject, the method comprising administering a therapeutically effective amount of the conjugated activatable antibody of any one of embodiments I-1 and I-4 to I-26 or the composition of any one of embodiments I-2 and I-4 to I-38 or the pharmaceutical composition of embodiment I-39 to a subject in need thereof.
I-41. The method of embodiment I-40, wherein the cancer is a gastric cancer.
I-42. The method of embodiment I-40, wherein the cancer is an ovarian cancer.
I-43. The method of embodiment I-40, wherein the cancer is an esophageal cancer.
I-44. The method of embodiment I-40, wherein the cancer is a non-small cell lung cancer.
I-45. The method of embodiment I-40, wherein the cancer is a breast cancer.
I-46. The method of embodiment I-40, wherein the cancer is a colorectal cancer.
I-47. The method of embodiment I-40, wherein the cancer is a melanoma.
I-48. The method of embodiment I-40, wherein the cancer is a prostate cancer.
I-49. The method of embodiment I-40, wherein the cancer is a mesothelioma.

### Set II

II-1. A conjugated activatable antibody comprising the structure of Formula (I) or a salt thereof: 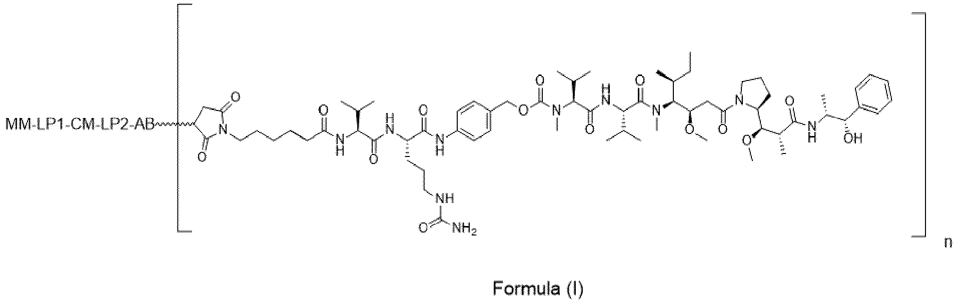
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
II-2. The conjugated activatable antibody of embodiment II-1, wherein the AB comprises an IgG1 isotype.
II-3. The conjugated activatable antibody of embodiment II-1 or II-2, wherein the AB is an antibody having a heavy chain constant region, and wherein the C-terminal residue of the heavy chain constant region is not a lysine.
II-4. A conjugated activatable antibody comprising the structure of Formula (I) or a salt thereof: 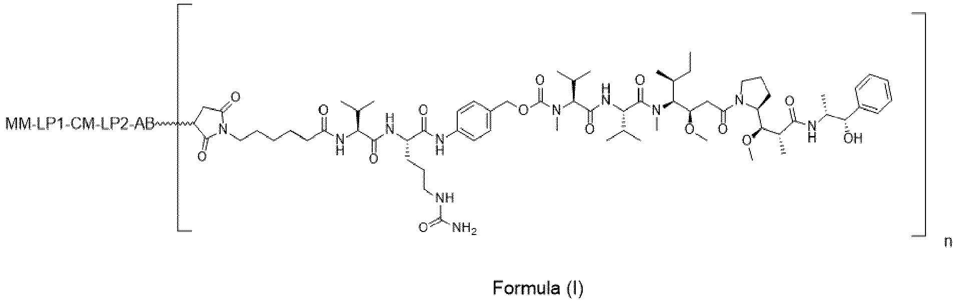
   (a) wherein
      (i) AB is an antibody that specifically binds to human CD71 and comprises a heavy chain variable region comprising a sequence of SEQ ID NO: 5 and a light chain variable region comprising a sequence of SEQ ID NO: 7;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
II-5. The conjugated activatable antibody of embodiment II-4, wherein the AB comprises an IgG1 isotype.
II-6. The conjugated activatable antibody of embodiment II-4 or II-5, wherein the AB is an antibody having a heavy chain constant region, and wherein the C-terminal residue of the heavy chain constant region is not a lysine.
II-7. A conjugated activatable antibody comprising the structure of Formula (I) or a salt thereof: 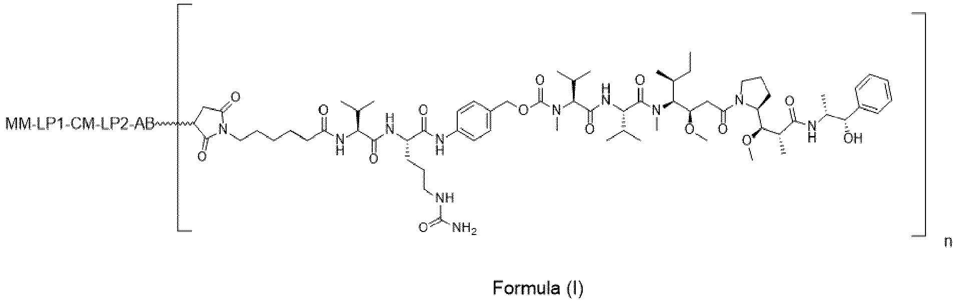
   (a) wherein
      (i) AB is an antibody that specifically binds to human CD71 and comprises a heavy chain comprising a sequence of SEQ ID NO: 167 and a light chain comprising a sequence of SEQ ID NO: 19;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
II-8. The conjugated activatable antibody of embodiment II-7, wherein the AB comprises an IgG1 isotype.
II-9. The conjugated activatable antibody of embodiment II-7 or II-8, wherein the AB is an antibody having a heavy chain constant region, and wherein the C-terminal residue of the heavy chain constant region is not a lysine.
II-10. A conjugated activatable antibody comprising the structure of Formula (I) or a salt thereof: 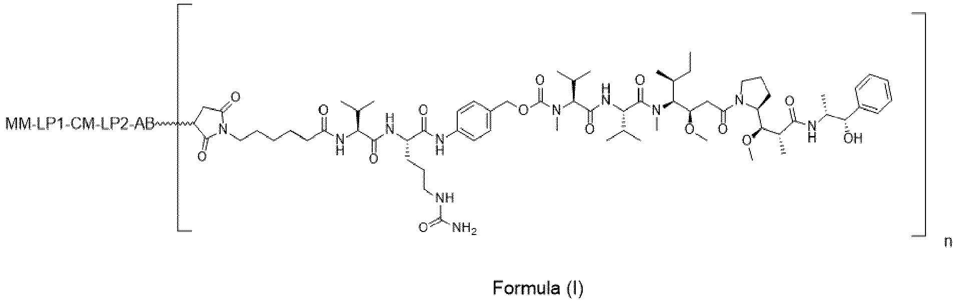
   wherein MM-LP1-CM-LP2-AB is an activatable antibody, wherein the AB is an antibody that specifically binds to human CD71,
   wherein the activatable antibody comprises a heavy chain comprising a sequence of SEQ ID NO: 167 and a light chain comprising a sequence of SEQ ID NO: 170, and
      wherein "n" is 2.
II-11. The conjugated activatable antibody of embodiment II-10, wherein the AB comprises an IgG1 isotype.
II-12. The conjugated activatable antibody of embodiment II-10 or II-11, wherein the AB is an antibody having a heavy chain constant region, and wherein the C-terminal residue of the heavy chain constant region is not a lysine.
11-13. A conjugated activatable antibody comprising the structure of Formula (II) or a salt thereof: 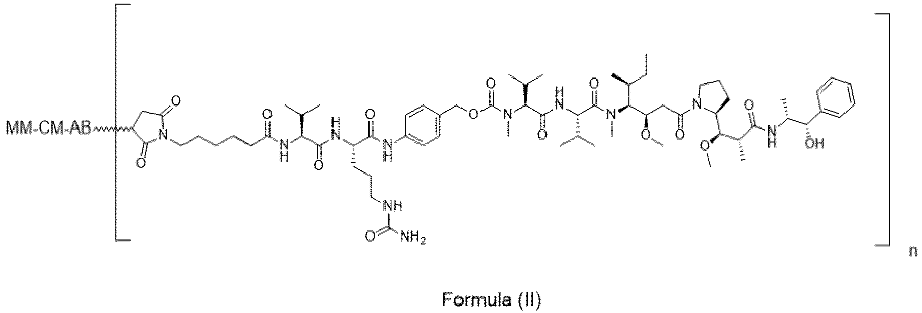
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and
   (b) wherein "n" is 2.
II-14. The conjugated activatable antibody of embodiment II-13, wherein the AB comprises an IgG1 isotype.
II-15. The conjugated activatable antibody of embodiment II-13 or II-14, wherein the AB is an antibody having a heavy chain constant region, and wherein the C-terminal residue of the heavy chain constant region is not a lysine.
II-16. A conjugated activatable antibody comprising the structure of Formula (II) or a salt thereof: 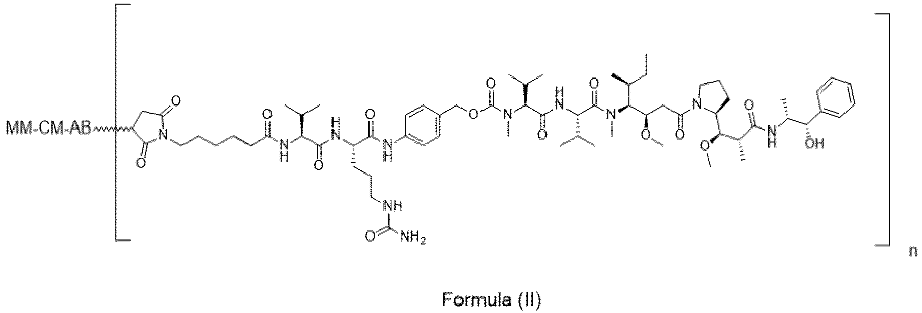
   (a) wherein
      (i) AB is an antibody that specifically binds to human CD71 and comprises a heavy chain variable region comprising a sequence of SEQ ID NO: 5 and a light chain variable region comprising a sequence of SEQ ID NO: 7;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and
   (b) wherein "n" is 2.
II-17. The conjugated activatable antibody of embodiment II-16, wherein the AB comprises an IgG1 isotype.
II-18. The conjugated activatable antibody of embodiment II-16 or II-17, wherein the AB is an antibody having a heavy chain constant region, and wherein the C-terminal residue of the heavy chain constant region is not a lysine.
II-19. A conjugated activatable antibody comprising the structure of Formula (II) or a salt thereof: 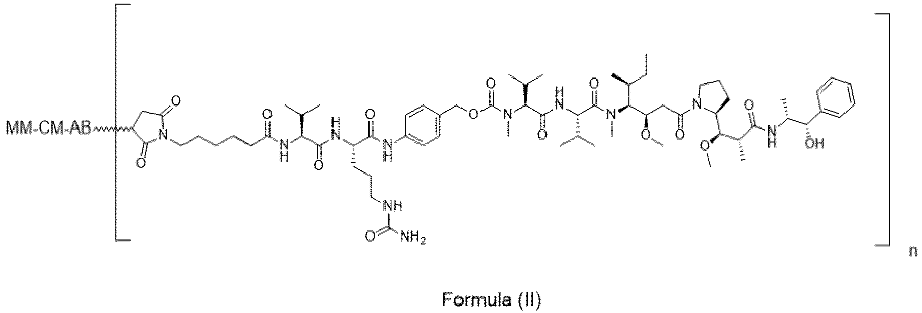
   (a) wherein
      (i) AB is an antibody that specifically binds to human CD71 and comprises a heavy chain comprising a sequence of SEQ ID NO: 167 and a light chain comprising a sequence of SEQ ID NO: 19;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and
   (b) wherein "n" is 2.
II-20. The conjugated activatable antibody of embodiment II-19, wherein the AB comprises an IgG1 isotype.
II-21. The conjugated activatable antibody of embodiment II-19 or II-20, wherein the AB is an antibody having a heavy chain constant region, and wherein the C-terminal residue of the heavy chain constant region is not a lysine.
II-22. A conjugated activatable antibody comprising the structure of Formula (II) or a salt thereof: 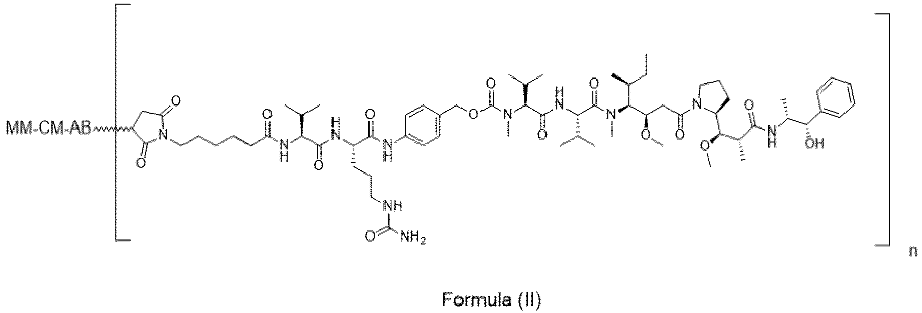
   wherein MM-CM-AB is an activatable antibody, wherein the AB is an antibody that specifically binds to human CD71,
   wherein the activatable antibody comprises a heavy chain comprising a sequence of SEQ ID NO: 167 and a light chain comprising a sequence of SEQ ID NO: 169, and
      wherein "n" is 2.
II-23. The conjugated activatable antibody of embodiment II-22, wherein the AB comprises an IgG1 isotype.
II-24. The conjugated activatable antibody of embodiment II-22 or II-23, wherein the AB is an antibody having a heavy chain constant region, and wherein the C-terminal residue of the heavy chain constant region is not a lysine.
II-25. A method of manufacturing a conjugated activatable antibody comprising the structure of Formula (I) or a salt thereof: 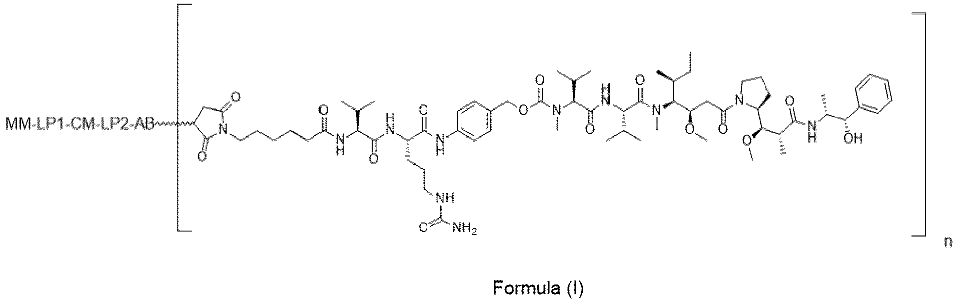
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38;
      and wherein "n" is 2;
   (b) the method comprising
      (i) reducing an activatable antibody comprising MM-LP1-CM-LP2-AB with a reducing agent; and
      (ii) conjugating one or more vcMMAE to the reduced activatable antibody.
11-26. A method of manufacturing a conjugated activatable antibody comprising the structure of Formula (II) or a salt thereof: 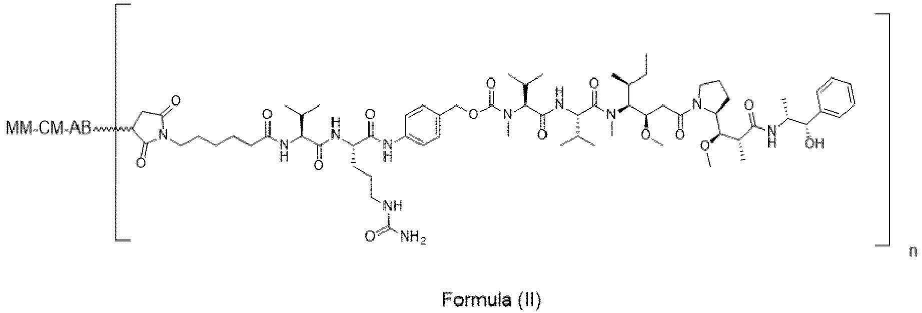
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO: 13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (ii) CM is a cleavable moiety comprising the sequence of SEQ ID NO: 156, wherein the CM is a polypeptide that functions as a substrate for a protease; and
         and wherein "n" is 2;
   (b) the method comprising
      (i) reducing an activatable antibody comprising MM-CM-AB with a reducing agent; and
      (ii) conjugating one or more vcMMAE to the reduced activatable antibody.
II-27. A pharmaceutical composition comprising: the conjugated activatable antibody of any one of embodiments II-1 to 11-24; and optionally a pharmaceutically acceptable carrier.
11-28. A method of treating, alleviating a symptom of, or delaying the progression of a cancer in a subject, the method comprising administering a therapeutically effective amount of the conjugated activatable antibody of any one of embodiments II-1 to II-24 or the pharmaceutical composition of embodiment 11-27 to a subject in need thereof for a cancer selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.
II-29. A conjugated activatable antibody of any one of embodiments II-1 to II-24 or a pharmaceutical composition of embodiment II-27, for use as a medicament.
II-30. A conjugated activatable antibody of any one of embodiments II-1 to II-24 or a pharmaceutical composition of embodiment II-27, for use in the treatment of cancer, optionally wherein the cancer is selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.

### EXAMPLES

### EXAMPLE 1. Anti-CD71 Conjugated Activatable Antibodies (AADCs)

The studies provided herein were designed to make some of the anti-CD71 conjugated activatable antibodies of the disclosure.

As described in U.S. Patent Application No. US 2016/0355599 A1, which is incorporated herein by reference in its entirety, an anti-CD71 M21 monoclonal antibody was obtained using mouse hybridoma technology in accordance with techniques known in the art. Mice were immunized with human CD71 extracellular domain (ECD) and subsequent hybridomas were screened using a cytotoxicity piggyback assay, and cytotoxicity positive clones from this assay were confirmed by ELISA to bind the human CD71 ECD polypeptide and confirmed to bind cell surfaces by FACS. The anti-CD71 M21 monoclonal antibody includes a heavy chain variable region (VH) of SEQ ID NO: 1, and a light chain variable region (VL) of SEQ ID NO: 2.

The following humanized anti-CD71 antibodies, which were based on the anti-CD71 mouse monoclonal antibody M21, were also tested: Ab21.10 LcB:HcA (VH of SEQ ID NO: 3 and VL of SEQ ID NO: 7), Ab21.11 LcB:HcB (VH of SEQ ID NO: 4 and VL of SEQ ID NO: 7), Ab21.12 LcB:HcC (VH of SEQ ID NO: 5 and VL of SEQ ID NO: 7), and M21 (VH of SEQ ID NO: 1 and VL of SEQ ID NO: 2). The ability of various anti-CD71 antibodies of the disclosure to bind cell-surface CD71 was confirmed by FACS.

All of the humanized anti-CD71 antibodies showed binding to human and cynomolgus CD71 that was comparable to the binding demonstrated by the CD71 M21 mouse antibody. Binding of the humanized anti-CD71 antibodies was confirmed on the BxPC3 cell line by FACS. Briefly, BxPC3 cells were labeled with mouse monoclonal Mab21 or huCD71 (Ab21.10, Ab21.11, and Ab21.12) antibody at the indicated concentrations and subsequently detected with an Alexa Fluor 647 labeled goat anti-mouse or anti-human IgG Alexa Fluor 647, respectively.

In an exemplary study, the binding activity of anti-CD71 Ab21.12 LcB:HcC (VH of SEQ ID NO: 5 and VL of SEQ ID NO: 7) to recombinant CD71 (using ELISA) and CD71-expressing cells (using flow cytometry) from human, cynomolgus monkey, mouse, and rat sources were measured. As shown in the exemplary results of Table 1, the anti-CD71 antibody bound human and monkey recombinant CD71 with equivalent affinity as measured by ELISA, and which were equivalent to the affinity of human holo-transferrin to human CD71. The antibody bound human and monkey CD71-expressing cell lines (human BxPC3 pancreatic cancer cells and cynomolgus monkey primary kidney epithelial cells, respectively) with equivalent affinity as measured by flow cytometry. No significant bind by the anti-CD71 antibody was measured to recombinant mouse CD71 or a rat CD71-expressing cell line (rate H-4-11-E hepatoma cells).

**Table 1: Binding of Anti-CD71 to Human, Monkey, Rat, and Mouse CD71 by ELISA and Flow Cytometry, and Binding of Holo-transferrin to Human CD71 by ELISA**

| **Test article** | | **ELISA (ECD)** | **Flow cytometry (cell lines)** |
|---|---|---|---|
| | **CD71 Species** | **Kapp (nM)** | **Kapp (nM)** |
| anti-CD71 Ab21.12 antibody | Human | 0.3 | 3.0 |
| | Monkey | 0.4 | 3.0 |
| | Rat | Not tested | No binding |
| | Mouse | No binding | Not tested |
| *Holo-transferrin | Human | 0.5 | Not tested |

### EXAMPLE 2. Mask Discovery

The studies provided herein were designed to identify and characterize masking moieties for use in activatable anti-CD71 antibodies of the disclosure.

As described in U.S. Patent Application No. US 2016/0355599 A1, anti-CD71 21.12 antibody, comprising a VH of SEQ ID NO: 5 and a VL of SEQ ID NO: 7, was used to screen a random X₁₅ peptide library with a total diversity of 6x10¹⁰, where X is any amino acid, using a method similar to that described in PCT International Publication Number WO 2010/081173, published 15 July 2010. The screening consisted of one round of MACS and five rounds of FACS sorting. The initial MACS sorting was done with protein-A Dynabeads (Invitrogen) and the anti-CD71 21.12 antibody at a concentration of 200 nM. For MACS, approximately 1x10¹² cells were screened for binding and 1x 10⁷ cells were collected. Anti-CD71 21.12 was conjugated with DyLight-488 (ThermoFisher), CD71 binding activity was confirmed and anti-CD71 21.12-488 was used as a fluorescent probe for all FACS rounds. Bacterial cells were stained and positive clones were collected as follows: 20 nM anti-CD71 21.12-488 with 1x10⁶ cells collected in FACS round 1, 5nM anti-CD71 21.12-488 with 6.2x10⁴ cells collected in FACS round 2 and 5 nM anti-CD71 21.12-488 with 5x10³ cells and 1 nM anti-CD71 21.12-488 with 5x10² cells collected in FACS round 3, 1 nM anti-CD71 21.12-488 with > 2x10² cells collected in FACS rounds 4 and 5. The positive population from the second FACS round was verified to inhibit binding of the anti-CD71 21.12-488 antibody to recombinant CD71 protein. Individual peptide clones were identified by sequence analysis from the 5 nM binders from FACS round 3 and the 1 nM binders from FACS rounds 3, 4 and 5.

The masking moieties identified include TF01 (QFCPWSYYLIGDCDI; SEQ ID NO: 16) and TF02 (NLCTEHSFALDCRSY; SEQ ID NO: 17). The TF01 and TF02 masks were truncated and alanine-scanned to generate families of activatable antibodies with different masking efficiencies, including the masking moiety TF02.13 (NLCTEHSAALDCRSY; SEQ ID NO: 18).

These masking peptides were used to generate anti-CD71 activatable antibodies of the disclosure. The sequences for certain of these anti-CD71 activatable antibodies are shown below in Table A. In some embodiments, these anti-CD71 activatable antibodies include cleavable moiety 2001 (ISSGLLSGRSDNH; SEQ ID NO: 91), cleavable moiety 3001 (AVGLLAPPGGLSGRSDNH; SEQ ID NO: 97), cleavable moiety 2011 (ISSGLLSGRSDNP; SEQ ID NO: 156) or the cleavable moiety 3011 (AVGLLAPPGGLSGRSDNP; SEQ ID NO: 164) as indicated.

While certain sequences shown below include the spacer sequence of SEQ ID NO: 138, those of ordinary skill in the art appreciate that the activatable anti-CD71 antibodies of the disclosure can include any suitable spacer sequence, such as, for example, a spacer sequence selected from the group consisting of QGQSGQG (SEQ ID NO: 138), QGQSGQ (SEQ ID NO: 109), QGQSG (SEQ ID NO: 139), QGQS (SEQ ID NO: 140), QGQ, QG, GQSGQG (SEQ ID NO: 143), QSGQG (SEQ ID NO: 144), SGQG (SEQ ID NO: 145), GQG, G, or Q. In some embodiments, the activatable anti-CD71 antibodies of the disclosure can have no spacer sequence joined to its N-terminus.

**Table A. Anti-CD71 Activatable Antibody Sequences**

| |
|---|
| HuCD71_HcC heavy chain variable region |
| Amino Acid Sequence |
| QVQLVQSGAEVKKPGASVKMSCKASGYTFTSYWMHWVRQAPGQGLEWIGAIYPGNSETGYAQKFQGRATLTAD TSTSTAYMELSSLRSEDTAVYYCTRENWDPGFAFWGQGTLITVSS (SEQ ID NO: 5) |
| Nucleotide sequence |
| CAGGTGCAGCTGGTGCAGTCTGGCGCCGAAGTGAAGAAACCTGGCGCCTCCGTGAAGATGTCCTGCAAGGCCT CCGGCTACACCTTCACCAGCTACTGGATGCACTGGGTGCGACAGGCTCCAGGCCAGGGCCTCGAATGGATCGG CGCCATCTACCCCGGCAACTCCGAGACAGGCTACGCCCAGAAGTTCCAGGGCAGAGCCACCCTGACCGCCGAC ACCTCCACCTCCACCGCCTACATGGAACTGTCCAGCCTGCGGAGCGAGGACACCGCCGTGTACTACTGCACCA GAGAGAACTGGGACCCCGGCTTCGCCTTCTGGGGCCAGGGCACCCTGATCACCGTGTCCTCC (SEQ ID NO: 206) |
| HuCD71_HcC-des heavy chain |
| Amino Acid Sequence |
| QVQLVQSGAEVKKPGASVKMSCKASGYTFTSYWMHWVRQAPGQGLEWIGAIYPGNSETGYAQKFQGRATLTAD TSTSTAYMELSSLRSEDTAVYYCTRENWDPGFAFWGQGTLITVSSASTKGPSVFPLAPSSKSTSGGTAALGCL VKDYFPEPVTVSWNSGALTSGVHTFPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDKSRWQQGNVFSCSVMHEALHNHYT QKSLSLSPG (SEQ ID NO: 167) |
| Nucleotide sequence |
| CAGGTGCAGCTGGTGCAGTCTGGCGCCGAAGTGAAGAAACCTGGCGCCTCCGTGAAGATGTCCTGCAAGGCCT CCGGCTACACCTTCACCAGCTACTGGATGCACTGGGTGCGACAGGCTCCAGGCCAGGGCCTCGAATGGATCGG CGCCATCTACCCCGGCAACTCCGAGACAGGCTACGCCCAGAAGTTCCAGGGCAGAGCCACCCTGACCGCCGAC ACCTCCACCTCCACCGCCTACATGGAACTGTCCAGCCTGCGGAGCGAGGACACCGCCGTGTACTACTGCACCA GAGAGAACTGGGACCCCGGCTTCGCCTTCTGGGGCCAGGGCACCCTGATCACCGTGTCCTCCGCCAGCACCAA GGGCCCCTCCGTGTTCCCTCTGGCCCCTTCCAGCAAGTCCACCTCTGGCGGCACAGCTGCCCTGGGCTGCCTG GTGAAAGACTACTTCCCCGAGCCCGTGACCGTGTCCTGGAACTCTGGCGCCCTGACCAGCGGAGTGCACACCT TCCCTGCCGTGCTGCAGTCCTCCGGCCTGTACTCCCTGTCCTCCGTGGTGACAGTGCCCTCCTCCAGCCTGGG CACCCAGACCTACATCTGCAACGTGAACCACAAGCCCTCCAACACCAAGGTGGACAAGAAGGTGGAACCCAAG TCCTGCGACAAGACCCACACCTGTCCTCCCTGCCCTGCCCCTGAACTGCTGGGCGGACCTTCCGTGTTTCTGT TCCCCCCAAAGCCCAAGGACACCCTGATGATCTCCCGGACCCCCGAAGTGACCTGCGTGGTGGTGGACGTGTC CCACGAGGACCCTGAAGTGAAGTTCAATTGGTACGTGGACGGCGTGGAAGTGCACAACGCCAAGACCAAGCCC AGAGAGGAACAGTACAACTCCACCTACCGGGTGGTGTCCGTGCTGACCGTGCTGCACCAGGACTGGCTGAACG GCAAAGAGTACAAGTGCAAGGTGTCCAACAAGGCCCTGCCTGCCCCCATCGAAAAGACCATCTCCAAGGCCAA |
| GGGCCAGCCCCGCGAGCCCCAGGTGTACACACTGCCACCTAGCCGGGAAGAGATGACCAAGAACCAGGTGTCC CTGACCTGTCTGGTGAAAGGCTTCTACCCCTCCGATATCGCCGTGGAATGGGAGAGCAACGGCCAGCCCGAGA ACAACTACAAGACCACCCCACCTGTGCTGGACTCCGACGGCTCATTCTTCCTGTACTCCAAGCTGACCGTGGA CAAGTCCCGGTGGCAGCAGGGCAACGTGTTCTCCTGCAGCGTGATGCACGAGGCCCTGCACAACCACTACACC CAGAAGTCCCTGTCCCTGAGCCCCGGC (SEQ ID NO: 168) |
| HuCD71_HcC heavy chain |
| Amino Acid Sequence |
| QVQLVQSGAEVKKPGASVKMSCKASGYTFTSYWMHWVRQAPGQGLEWIGAIYPGNSETGYAQKFQGRATLTAD TSTSTAYMELSSLRSEDTAVYYCTRENWDPGFAFWGQGTLITVSSASTKGPSVFPLAPSSKSTSGGTAALGCL VKDYFPEPVTVSWNSGALTSGVHTFPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSREEMTKNQVS LTCLVKGFYPSDIAVEWESNGQPENNYKTTPPVLDSDGSFFLYSKLTVDKSRWQQGNVFSCSVMHEALHNHYT QKSLSLSPGK (SEQ ID NO: 20) |
| Nucleotide sequence |
| CAGGTGCAGCTGGTGCAGTCTGGCGCCGAAGTGAAGAAACCTGGCGCCTCCGTGAAGATGTCCTGCAAGGCCT CCGGCTACACCTTCACCAGCTACTGGATGCACTGGGTGCGACAGGCTCCAGGCCAGGGCCTCGAATGGATCGG CGCCATCTACCCCGGCAACTCCGAGACAGGCTACGCCCAGAAGTTCCAGGGCAGAGCCACCCTGACCGCCGAC ACCTCCACCTCCACCGCCTACATGGAACTGTCCAGCCTGCGGAGCGAGGACACCGCCGTGTACTACTGCACCA GAGAGAACTGGGACCCCGGCTTCGCCTTCTGGGGCCAGGGCACCCTGATCACCGTGTCCTCCGCCAGCACCAA GGGCCCCTCCGTGTTCCCTCTGGCCCCTTCCAGCAAGTCCACCTCTGGCGGCACAGCTGCCCTGGGCTGCCTG GTGAAAGACTACTTCCCCGAGCCCGTGACCGTGTCCTGGAACTCTGGCGCCCTGACCAGCGGAGTGCACACCT TCCCTGCCGTGCTGCAGTCCTCCGGCCTGTACTCCCTGTCCTCCGTGGTGACAGTGCCCTCCTCCAGCCTGGG CACCCAGACCTACATCTGCAACGTGAACCACAAGCCCTCCAACACCAAGGTGGACAAGAAGGTGGAACCCAAG TCCTGCGACAAGACCCACACCTGTCCTCCCTGCCCTGCCCCTGAACTGCTGGGCGGACCTTCCGTGTTTCTGT TCCCCCCAAAGCCCAAGGACACCCTGATGATCTCCCGGACCCCCGAAGTGACCTGCGTGGTGGTGGACGTGTC CCACGAGGACCCTGAAGTGAAGTTCAATTGGTACGTGGACGGCGTGGAAGTGCACAACGCCAAGACCAAGCCC AGAGAGGAACAGTACAACTCCACCTACCGGGTGGTGTCCGTGCTGACCGTGCTGCACCAGGACTGGCTGAACG GCAAAGAGTACAAGTGCAAGGTGTCCAACAAGGCCCTGCCTGCCCCCATCGAAAAGACCATCTCCAAGGCCAA GGGCCAGCCCCGCGAGCCCCAGGTGTACACACTGCCACCTAGCCGGGAAGAGATGACCAAGAACCAGGTGTCC CTGACCTGTCTGGTGAAAGGCTTCTACCCCTCCGATATCGCCGTGGAATGGGAGAGCAACGGCCAGCCCGAGA |
| ACAACTACAAGACCACCCCACCTGTGCTGGACTCCGACGGCTCATTCTTCCTGTACTCCAAGCTGACCGTGGA CAAGTCCCGGTGGCAGCAGGGCAACGTGTTCTCCTGCAGCGTGATGCACGAGGCCCTGCACAACCACTACACC CAGAAGTCCCTGTCCCTGAGCCCCGGCAAG (SEQ ID NO: 21) |
| HuCD71_LcB |
| Amino Acid Sequence |
| DIQMTQSPSSLSASVGDRVTITCSASSSVYYMYWFQQKPGKAPKLWIYSTSNLASGVPSRFSGSGSGTDYTLT ISSMQPEDFATYYCQQRRNYPYTFGQGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC (SEQ ID NO: 19) |
| HuCD71_LcB |
| Nucleotide sequence |
| GACATCCAGATGACCCAGTCCCCATCCAGCCTGTCCGCCTCCGTGGGCGACAGAGTGACAATCACCTGTTCCG CCAGCTCCTCCGTGTACTACATGTACTGGTTCCAGCAGAAGCCCGGCAAGGCCCCCAAGCTGTGGATCTACTC CACCTCCAACCTGGCCTCCGGCGTGCCCTCCAGATTCTCCGGCTCTGGCTCCGGCACCGACTACACCCTGACC ATCTCCAGCATGCAGCCCGAGGACTTCGCCACCTACTACTGCCAGCAGCGGCGGAACTACCCCTACACCTTCG GCCAGGGCACCAAGCTGGAAATCAAGCGGACCGTGGCCGCTCCCAGCGTGTTCATCTTCCCACCCTCCGACGA GCAGCTGAAGTCCGGCACCGCCAGCGTCGTGTGCCTGCTGAACAACTTCTACCCCCGCGAGGCCAAGGTGCAG TGGAAGGTGGACAACGCCCTGCAGTCCGGCAACTCCCAGGAATCCGTCACCGAGCAGGACTCCAAGGACAGCA CCTACTCCCTGTCCTCCACCCTGACCCTGTCCAAGGCCGACTACGAGAAGCACAAGGTGTACGCCTGCGAAGT GACCCACCAGGGCCTGTCCAGCCCCGTGACCAAGTCCTTCAACCGCGGCGAGTGC (SEQ ID NO: 208) |
| Activatable Antibody Light Chain anti-CD71-TF01-2001 |
| [spacer (SEQ ID NO: 138)] [huCD71Lc_TF01_2001 (SEQ ID NO: 141)] |
| Amino Acid Sequence |
| [QGQSGQG][QFCPWSYYLIGDCDIGGGSSGGSISSGLLSGRSDNHGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIKRTVAAPSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC] (SEQ ID NO: 22) |
| Activatable Antibody Light Chain anti-CD71-TF02.13-2001 |
| [spacer (SEQ ID NO: 138)] [huCD71Lc_TF02.13_2001 (SEQ ID NO: 142)] |
| Amino Acid sequence |
| [QGQSGQG] [NLCTEHSAALDCRSYGGGSSGGSISSGLLSGRSDNHGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIKRTVAAPSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC] (SEQ ID NO: 23) |
| Activatable Antibody Light Chain anti-CD71-TF02.13-3011 |
| [spacer (SEQ ID NO: 138)] [huCD71Lc_TF02.13_3011 (SEQ ID NO: 146)] |
| Amino Acid sequence |
| [QGQSGQG][NLCTEHSAALDCRSYGGGSSGGSAVGLLAPPGGLSGRSDNPGGSDIQMTQSPSSLSASVGDRV TITCSASSSVYYMYWFQQKPGKAPKLWIYSTSNLASGVPSRFSGSGSGTDYTLTISSMQPEDFATYYCQQRRN YPYTFGQGTKLEIKRTVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC] (SEQ ID NO: 147) |
| Activatable Antibody Light Chain anti-CD71-TF02.13-2011 |
| [spacer (SEQ ID NO: 138)] [huCD71Lc_TF02.13_2011 (SEQ ID NO: 169)] |
| Amino Acid sequence |
| [QGQSGQG] [NLCTEHSAALDCRSYGGGSSGGSISSGLLSGRSDNPGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIKRTVAAPSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC] (SEQ ID NO: 170) |
| Activatable Antibody Light Chain anti-CD71-TF01-3011 |
| [spacer (SEQ ID NO: 138)] [huCD71Lc_TF01_3011 (SEQ ID NO: 173)] |
| Amino Acid sequence |
| [QGQSGQG][QFCPWSYYLIGDCDIGGGSSGGSAVGLLAPPGGLSGRSDNPGGSDIQMTQSPSSLSASVGDRV TITCSASSSVYYMYWFQQKPGKAPKLWIYSTSNLASGVPSRFSGSGSGTDYTLTISSMQPEDFATYYCQQRRN YPYTFGQGTKLEIKRTVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC] (SEQ ID NO: 174) |
| Activatable Antibody Light Chain Variable Region anti-CD71-TF02.13-2001 |
| [spacer (SEQ ID NO: 138)] [huCD71Lc_TF02.13_2001 VL domain (SEQ ID NO: 197)] |
| Amino Acid sequence |
| [QGQSGQG] [NLCTEHSAALDCRSYGGGSSGGSISSGLLSGRSDNHGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIK] (SEQ ID NO: 198) |
| Activatable Antibody Light Chain Variable Region anti-CD71-TF02.13-3011 |
| [spacer (SEQ ID NO: 138)] [huCD71Lc_TF02.13_3011 VL domain (SEQ ID NO: 199)] |
| Amino Acid sequence |
| [QGQSGQG] [NLCTEHSAALDCRSYGGGSSGGSAVGLLAPPGGLSGRSDNPGGSDIQMTQSPSSLSASVGDRV TITCSASSSVYYMYWFQQKPGKAPKLWIYSTSNLASGVPSRFSGSGSGTDYTLTISSMQPEDFATYYCQQRRN YPYTFGQGTKLEIK] (SEQ ID NO: 200) |
| Activatable Antibody Light Chain Variable Region anti-CD71-TF02.13-2011 |
| [spacer (SEQ ID NO: 138)] [huCD71Lc_TF02.13_2011 VL domain (SEQ ID NO: 201)] |
| Amino Acid sequence |
| [QGQSGQG] [NLCTEHSAALDCRSYGGGSSGGSISSGLLSGRSDNPGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIK] (SEQ ID NO: 202) |
| Activatable Antibody Light Chain Variable Region anti-CD71-TF01-2001 |
| [spacer (SEQ ID NO: 138)] [huCD71Lc_TF01_2001 VL domain (SEQ ID NO: 195)] |
| Amino Acid Sequence |
| [QGQSGQG][QFCPWSYYLIGDCDIGGGSSGGSISSGLLSGRSDNHGGGSDIQMTQSPSSLSASVGDRVTITC SASSSVYYMYWFQQKPGKAPKLWIYSTSNLASGVPSRFSGSGSGTDYTLTISSMQPEDFATYYCQQRRNYPYT FGQGTKLEIK] (SEQ ID NO: 196) |
| Activatable Antibody Light Chain Variable Region anti-CD71-TF01-3011 |
| [spacer (SEQ ID NO: 138)] [huCD71Lc_TF01_3011 VL domain (SEQ ID NO: 203)] |
| Amino Acid sequence |
| [QGQSGQG] [QFCPWSYYLIGDCDIGGGSSGGSAVGLLAPPGGLSGRSDNPGGSDIQMTQSPSSLSASVGDRV TITCSASSSVYYMYWFQQKPGKAPKLWIYSTSNLASGVPSRFSGSGSGTDYTLTISSMQPEDFATYYCQQRRN YPYTFGQGTKLEIK] (SEQ ID NO: 204) |

### EXAMPLE 3. Generation and Characterization of Activatable Anti-CD71 Activatable Antibodies and Anti-CD71 Conjugated Activatable Antibodies

The studies provided herein were designed to generate some anti-CD71 activatable antibodies of the disclosure.

Anti-CD71 activatable antibodies were generated with different masking efficiencies (i.e., a measurement of the ability of the MM of the activatable antibody to block binding of the AB of the activatable antibody to its target). The peptides TF01 and TF02 were mutated by truncation and alanine scanning as described in Example 2, and these masking peptide variants were used to generate families of anti-CD71 activatable antibodies of the present disclosure with a range of masking efficiencies.

Binding of anti-CD71 activatable antibodies of the present disclosure to the NCI H292 (also referred to herein as H292) cell line was evaluated using FACS. Briefly, cells were labeled with huCD71 antibody or activatable antibody at a range of concentrations and subsequently detected with an Alexa Fluor 647 labeled goat anti-human IgG secondary antibody to determine a binding curve. As summarized in the exemplary binding data below in Table 2, anti-CD71 activatable antibodies of the present disclosure show a range of masking efficiencies compared to the parental anti-CD71 antibody (huCD71 21.12 Ab).

**Table 2: Masking Efficiencies of Masking Moieties**

| **Activatable Antibody Masking Moiety** | **Light Chain VL SEQ ID NO** | **Kₐₚₚ (nM)** | **Masking Efficiency** |
|---|---|---|---|
| None (Ab 21.12) | 7 | 2.288 | 1 |
| TF01 | 809 | 809.5 | 352 |
| TF02.13 | 813 | 127.4 | 55 |

These exemplary data in Table 2 show that both masks (TF01 and TF02.13) inhibit binding of the anti-CD71 antibody to CD71 when the activatable antibody is in an intact or uncleaved state. These exemplary data also demonstrate that the TF01 masking moiety demonstrates a higher masking efficiency than the TF02.13 masking moiety.

### EXAMPLE 4: Characterization of the Binding Activity of the CD71 Activatable Antibody and the CD71 Conjugated Activatable Antibody

This Example shows that anti-CD71 activatable antibodies and anti-CD71 conjugated activatable antibodies of the present disclosure demonstrated lower binding affinity to recombinant and cell surface human and cynomolgus CD71 protein when in their intact, uncleaved form as compared to their corresponding protease-cleaved forms.

As shown in FIGS. 3A and 3B, a solid-phase binding assay (ELISA) was used to demonstrate the binding affinity of anti-CD71 activatable antibodies and anti-CD71 conjugated activatable antibodies of the present disclosure to recombinant CD71, both in their intact, uncleaved forms and their protease-activated, cleaved forms. In these examples, recombinant cynomolgus (FIG. 3B) or human CD71 (FIG. 3A) protein (R&D Systems) was coated on ELISA plates at a concentration of 1 µg/mL, and then incubated with the indicated concentration of intact, uncleaved anti-CD71 activatable antibody ("anti-CD71-TF02.13-2011") of the present disclosure or intact, uncleaved E2 (i.e. having a DAR of ∼2) anti-CD71 conjugated activatable antibody ("anti-CD71-TF02.13-2011-vc-MMAE E2") of the present disclosure. Also assayed were the anti-CD71 activatable antibody ("anti-CD71-TF02.13-2011 (Act)") or E2 (i.e. having a DAR of ∼2) anti-CD71 conjugated activatable antibody ("anti-CD71-TF02.13-2011 (Act)") of the present disclosure following treatment with a protease that cleaved the cleavable moiety of the activatable antibody component.

As discussed herein, "E2" refers to a composition of a given anti-CD71 conjugated activatable antibody of the present disclosure that includes essentially only the species of the conjugated activatable antibody where the drug load is 2 drug molecules for each activatable antibody molecule, and essentially does not include any or minimal amounts of the other possible species (*i.e.* where the drug load is 0, 1, 3, 4, 5, 6, 7, 8, etc.) As discussed herein, "DAR" refers to the average ratio between the drug (in this case, MMAE) to the activatable antibody (*i.e.* CD71-TF02.13-2011) in a population of such conjugated activatable antibodies. Thus, in this example, anti-CD71-TF02.13-2011-vc-MMAE E2 refers to a composition that includes only anti-CD71-TF02.13-2011 activatable antibody in which each activatable antibody had a drug load of 2 equivalents of vc-MMAE and a DAR of about 2 as described in Example 26.

The amount of bound antibody in each sample was detected by incubation and detection by goat anti-human antibody conjugated to horseradish peroxidase and Ultra TMB (Thermo Fisher Scientific) detection. A summary of the binding affinities is shown below:

**Table 3A: Exemplary Observed CD71 Binding Activity of Uncleaved and Cleaved Anti-CD71 Activatable Antibodies and Anti-CD71 Conjugated Activatable Antibodies**

| **Test Article** | **Human CD71 K_{d(app)} nM** | **Cynomolgus CD71 K_{d(app)} nM** |
|---|---|---|
| a nti-CD71-TF02.13-2011 | 0.42 | 1.24 |
| a nti-CD71-TF02.13-2011 (Activated) | 0.04 | 0.06 |
| anti-CD71-TF02.13-2011-vcMMAE E2 | 0.48 | 1.49 |
| anti-CD71-TF02.13-2011-vcMMAE E2 (Activated) | 0.05 | 0.07 |

FIGS. 3C and 3D show in a FACS assay the exemplary results that anti-CD71 activatable antibodies of the present disclosure and anti-CD71 conjugated activatable antibodies of the present disclosure bind cells expressing human CD71 (FIG. 3C) and cynomolgus CD71 (FIG. 3D) with a higher dissociation constant and lower affinity than that of the corresponding protease-activated anti-CD71 activatable antibody or protease-activated anti-CD71 conjugated activatable antibody of the present disclosure. These exemplary results show that the binding affinity of the cleaved, protease-activated anti-CD71 activatable antibodies of the present disclosure and the cleaved, protease-activated anti-CD71 conjugated activatable antibodies of the present disclosure bound to the cells at an affinity similar to that of the parental anti-CD71 antibody, thus demonstrating the effect of the masking moiety in inhibiting binding of the anti-CD71 to its target in the intact activatable antibody or intact conjugated activatable antibody.

In the exemplary study shown in FIGS. 3C and 3D, the binding of the antibodies of the present disclosure to the indicated cell lines were performed using a standard FACS labelling method. Briefly, cells were labeled with the indicated antibodies or activatable antibodies of the present disclosure: a control antibody (AB095), a human anti-CD71 antibody (Ab 21.12, having a VH of SEQ ID NO: 5 and a VL of SEQ ID NO: 7), an anti-CD71 activatable antibody (anti-CD71 TF02.13-2011), or an E2 (i.e. having a DAR of ∼2) anti-CD71 conjugated activatable antibody (anti-CD71 TF02.13-2011-vcMMAE E2). In addition, the binding affinity of the anti-CD71 activatable antibody (anti-CD71 TF02.13-2011 (Act)) and the E2 (i.e. having a DAR of ∼2) anti-CD71 conjugated activatable antibody (anti-CD71 TF02.13-2011-vcMMAE E2 (Act)) were also assayed following protease-activation that cleaved the cleavable moiety therein. Each test article was applied at the indicated concentrations and subsequently detected with an Alexa Fluor 647 labeled goat anti-human IgG secondary antibody.

Table 3B below shows the EC50 values based on the binding curves depicted in FIGS. 3C to 3D. These results show that intact activatable antibody and intact conjugated activatable antibody bound to its target with lower affinity than their protease-activated counterparts, and that the protease-activated activatable antibodies bound with an affinity that was similar to that of the parental anti-CD71 antibody.

**Table 3B: Exemplary Observed CD71 Binding Activity of Anti-CD71 Binders**

| **Test Article** | **Human H292 cells FACS EC50** | **CHO w/ Cynomolgus CD71 FACS EC50** |
|---|---|---|
| Ab 21.12 | 1.021 | |
| Ab 21.12-vc-MMAE | - | 0.2912 |
| Anti-CD71-TF02.13-2011 | 46.06 | 27.83 |
| Anti-CD71-TF02.13-2011 (Activated) | 1.429 | 0.4639 |
| Anti-CD71-TF02.13-2011-vcMMAE E2 | 50.97 | 35.30 |
| Anti-CD71-TF02.13-2011-vcMMAE E2 (Activated) | 1.854 | 0.7292 |

In a similar exemplary study, ELISA and flow cytometry assays were used to demonstrate the binding affinity of anti-CD71 antibodies, anti-CD71 conjugated antibodies, anti-CD71 activatable antibodies (intact, uncleaved form and protease-activated, cleaved form) and anti-CD71 conjugated activatable antibodies (intact, uncleaved form and protease-activated, cleaved form) of the present disclosure to recombinant CD71 to recombinant human and cynomolgus CD71 and human and cynomolgus CD71-expressing cell lines. The test articles were the anti-CD71 Ab21.12 antibody (VH of SEQ ID NO: 5 and a VL of SEQ ID NO: 7), the conjugated antibody anti-CD71 Ab21.12-vcMMAE E2 (i.e. having a DAR of ∼2), the activatable antibody anti-CD71-TF02.13-2011, and the DAR2 conjugated activatable antibody anti-CD71-TF02.13-2011-vcMMAE E2 (i.e. having a DAR of ∼2).

In the ELISA examples, recombinant cynomolgus or human CD71 ECD proteins were coated on ELISA plates, and then incubated with one of a range of concentrations of the test articles of the present disclosure, followed by measurement of the amount of bound test article with a secondary antibody. Test articles that were activated were incubated with matriptase protease enzyme. The exemplary apparent Kd values for each test article are shown in Table 3C.

In the exemplary study also shown in Table 3C, the binding of the antibodies of the present disclosure to the indicated cell lines were performed using a standard FACS labelling method. The flow cytometry results reflect the average measured Kapp from four human cell lines (HCC1806 human breast cancer, HT-29 human colorectal cancer, NCI-H292 human lung cancer, and NCI-H520 human lung cancer) and one CHO-K1 cell line expressing cynomolgus monkey CD71. The exemplary apparent Kd values for each test article are shown in Table 3C.

**Table 3C: Exemplary Observed CD71 Binding Activity of Anti-CD71 Antibodies, Anti-CD71 Conjugated Antibodies, Uncleaved and Cleaved Anti-CD71 Activatable Antibodies and Anti-CD71 Conjugated Activatable Antibodies**

| | **Test article** | **ELISA rhCD71** | **ELISA rcCD71** | **Flow cytometry Average of 4 human cell lines** | **Flow cytometry CHO-K1 cyno CD71** |
|---|---|---|---|---|---|
| | | **Kapp (nM)** | **Kapp (nM)** | **Kapp (nM)** | **Kapp (nM)** |
| 1 | Ab21.12 | 0.03 | 0.03 | 0.8 | 0.3 |
| 2 | Ab21.12-vc-MMAE E2 | 0.04 | 0.03 | Not tested | Not tested |
| 3 | anti-CD71-TF02.13-2011 | 1.94 | 1.11 | 105.5 | 28 |
| 4 | anti-CD71-TF02.13-2011, matriptase activated | 0.06 | 0.04 | 2.0 | 0.5 |
| 5 | anti-CD71-TF02.13-2011-vcMMAE E2 | 2.58 | 1.02 | 98.1 | 35 |
| 6 | anti-CD71-TF02.13-2011-vcMMAE E2, matriptase activated | 0.05 | 0.04 | 2.0 | 0.7 |

| | **Ratio** | | | | |
|---|---|---|---|---|---|
| | anti-CD71-TF02.13-2011/Ab21.12 | 65 | 37 | 127 | 93 |
| | anti-CD71-TF02.13-2011-vcMMAE E2 / Ab21.12 | 86 | 34 | 117 | 117 |
| | anti-CD71-TF02.13-2011/ anti-CD71-TF02.13-2011, matriptase activated | 32 | 28 | 55 | 56 |
| | anti-CD71-TF02.13-2011-vcMMAE E2 / anti-CD71-TF02.13-2011-vcMMAE E2, matriptase activated | 52 | 26 | 48 | 50 |

These exemplary results show that intact activatable antibodies and intact conjugated activatable antibodies showed a lower apparent affinity to CD71 as compared to their activated counterparts or their parental antibodies.

### EXAMPLE 5: CD71 Expression in Multiple Patient-Derived Primary and Metastatic Tumors

This Example shows that CD71 is expressed in a large variety of primary and metastatic tumor types by immunohistochemical (IHC) staining using an anti-CD71 antibody.

FIGS. 1 and 2 show that CD71 is highly expressed in a large number of primary and metastatic tumor samples, using IHC staining with a commercially-purchased anti-CD71 antibody on multiple patient-derived primary tumors and patient-derived metastatic tissue microarrays (TMA). FIG. 1 shows an IHC staining of CD71 in head and neck cancer (1), cervical cancer (2), an breast cancer (3), non-Hodgkin's lymphoma (NHL) in lymph nodes (4), a lung cancer (5), a bladder cancer (6), an ovarian cancer (7), and an esophageal cancer (8). FIG. 2 shows an IHC staining of CD71 in a tissue microarray (TMA) consisting of cores from metastatic tumors demonstrated a moderate to high level of expression of CD71 in the majority of the cores. A summary of CD71 expression in human tumor samples by immunohistochemistry (IHC) is shown in Table 4A.

**Table 4A: Summary of CD71 Expression in Human Tumor Samples by IHC**

| **Indication** | **Site** | **Total cases** | **% Strong/ Moderate (2+ or greater IHC)** |
|---|---|---|---|
| Esophageal | primary | 56 | 82.1 |
| | metastases | 69 | 75.4 |
| Gastric | primary | 209 | 63.6 |
| | metastases | 74 | 83.7 |
| Pancreatic | primary | 151 | 57 |
| | metastases | 15 | 80 |
| non-Hodgkin's lymphoma | primary | 132 | 66.7 |
| | metastases | 59 | 88.1 |
| non-small cell lung carcinomas | primary | 127 | 74.8 |
| | metastases | 87 | 70.1 |
| Breast | primary | 45 | 66.7 |
| | metastases | 80 | 85 |
| Endometrial | primary | 147 | 98 |
| | metastases | 9 | 88.9 |
| Colorectal | primary | 146 | 77.4 |
| | metastases | 98 | 74.5 |
| head and neck squamous cell carcinomas | primary | 160 | 53.8 |
| | metastases | 25 | 60 |

### EXAMPLE 6: Masking Efficiency and Activatable Anti-CD71-AADC in vivo Efficacy in CRC Xenograft Model

This Example shows that the masking efficiency of anti-human CD71 activatable antibodies with conjugated toxins (AADCs) of the present disclosure is a factor in its efficacy in an HT29 colorectal cancer (CRC) mouse xenograft model.

In these studies, HT29 xenograft tumors in mice were grown to an average volume of 150 mm³. The mice were then randomized into groups and dosed on day 1 with 3 mg/kg (or otherwise indicated) of each indicated test article. The mean tumor volume ± SEM was plotted for each time point.

FIG. 4A shows that an exemplary anti-CD71 AADC of the present disclosure (anti-CD71 TF01-3011-vc-MMAE) with a higher affinity mask showed some tumor growth inhibition relative to a vehicle control after a single 3 mg/kg dose. In comparison, FIG. 4B showed an exemplary anti-CD71 AADC of the present disclosure (anti-CD71 TF02.13-3011-vc-MMAE) with a lower affinity mask but with the same cleavable substrate showed essentially complete regression of the xenograft after a single 3 mg/kg dose. FIG. 4C shows an exemplary anti-CD71 AADC of the present disclosure (anti-CD71 TF02.13-3011-vc-MMAE) with the lower affinity mask but with the same cleavable substrate also showed essentially complete regression of the HT29 xenograft at both a single dosage of 2 mg/kg or 3 mg/kg. These exemplary results show that an AADC with the lower affinity masking moiety demonstrated a higher efficacy than an AADC with a higher affinity masking moiety.

### EXAMPLE 7: Cleavable Substrates and Activatable Anti-CD71-AADC in vivo Efficacy in a CRC Xenograft Models

This Example shows the effect of the cleavable substrate on the efficacy of exemplary anti-human CD71 conjugated activatable antibodies (AADCs) of the present disclosure in a mouse xenograft model.

In these studies, HT29 (colorectal cancer-derived) xenograft tumors in mice were grown to an average volume of 150 mm³. The mice were then randomized into groups and dosed on day 1 with the indicated amount of the indicated test article. The mean tumor volume ± SEM was plotted for each time point.

FIG. 5 shows that an exemplary anti-CD71 AADCs of the present disclosure (CD71 TF02.13-2011-vc-MMAE) shows complete or near complete regression in the mouse xenograft model at two different dosages. These exemplary data demonstrate that the efficacy of the indicated AADC (anti-CD71 TF02.13-2011-vc-MMAE) with a less cleavable substrate is substantially the same as the efficacy of the AADC (anti-CD71 TF02.13-3011-vc-MMAE) with a more cleavable substrate shown in FIGS. 4B and 4C.

### EXAMPLE 8: Activatable Anti-CD71-AADC in vivo Efficacy in Multiple Xenograft Models

This Example shows the efficacies of the anti-CD71 TF02.13-2011-vc-MMAE AADC of the present disclosure at two different drug-to-antibody ratios (DARs) in various mouse xenograft models.

In these studies, ovarian cancer (OV-90; FIG. 6B), stomach cancer (NCI-N87; FIG. 6A), ER+ breast cancer (BT474; FIG. 6C), or triple-negative breast cancer (HCC-70; FIG. 6D) xenograft tumors in mice were grown to an average volume of 150 mm³. The mice were then randomized into groups and dosed on day 1 with the indicated test article. The amount of each AADC that was administered was dose-matched based on the amount of MMAE toxin, such that the anti-CD71 TF02.13-2011-vc-MMAE (having an average DAR of ∼3) was administered at 3 mg/kg and anti-CD71 TF02.13-2011-vc-MMAE E2 (having an average DAR of ∼2) was administered at 4.3 mg/kg. The mean tumor volume ± SEM was plotted for each time point.

FIGS. 6A-6B show that an exemplary anti-CD71 AADCs of the present disclosure (anti-CD71 TF02.13-2011-vc-MMAE) shows complete or near complete regression in an ovarian and stomach cancer mouse xenograft model. The anti-CD71 TF02.13-2011-vc-MMAE E2 (i.e. having a DAR of ∼2), which included purified AADCs each with two equivalents of MMAE toxin, also showed completed regressions in the same models. In all cases, such as shown in FIGS. 6C and 6D, even when complete or near-complete regression was not observed, the activities of the E2 (i.e. having a DAR of ∼2) and the higher DAR AADCs were equivalent or nearly-equivalent. These exemplary data demonstrate that the dose-matched efficacy of the E2 (i.e. having a DAR of ∼2) AADC (anti-CD71 TF02.13-2011-vc-MMAE E2) is comparable to the corresponding higher DAR AADC.

As shown in Table 4B, the efficacy of anti-CD71 TF02.13-2011-vc-MMAE E2 AADC having a DAR of ∼2 were tested against a variety of patient-derived (PDX) or cancer (CDX) cell lines in a mouse xenograft model, and were dosed with less than or equal to 6 mg/kg once or twice. The results, which are summarized below, show that the AADC demonstrated at least some efficacy, ranging from regression to stasis to tumor growth inhibition in almost all of the tested models.

**Table 4B: Efficacy of Anti-CD71 TF02.13-2011-vc-MMAE E2 In Cell-Line Derived Xenografts**

| **Tumor Types** | **CDX or PDC** | **Regressions or Stasis** | **Tumor Growth Inhibition** | **No Response** |
|---|---|---|---|---|
| Gastric | CDX | 1 | | |
| | PDX | 4 | | 1 |
| Esophageal | CDX | 3 | 1 | |
| | CDX | 4 | | |
| Ovarian | PDX | 2 | 1 | |
| Non-Small Cell Lung Cancer | CDX | 3 | 1 | |
| | PDX | 2 | 1 | 1 |
| Breast | CDX | 3 | 2 | |
| | PDX | 5 | | |
| Colorectal | CDX | 2 | | 1 |
| Multiple Myeloma | | | 1 | |
| Prostate | CDX | 1 | | |
| Mesothelioma | | | 1 | |
| Diffuse Large B-Cell Lymphoma | PDX | 4 | 1 | |
| Head & Neck Small Cell Carcinoma | PDX | 5 | | |
| Pancreatic | PDX | 6 | | 1 |

### EXAMPLE 9: Bioanalytical Assays to Determine Levels of Conjugated and Free Toxin, and Intact and Total AADC In Samples From Treated Animals

This Example shows exemplary workflows for assays to measure levels of metabolites in test samples, including the levels of conjugated and free toxin, and the levels of intact and total conjugated activatable antibodies.

FIGS. 7A, 7B, and 7C show schematic workflows for the exemplary bioanalytic assays to determine the amount of intact and total (*i.e.* combined intact and cleaved) activatable antibody in the treated monkeys, as well as the amount of intact AADC (with uncleaved toxin) and cleaved toxin in the treated monkeys. Using the exemplary assay protocol depicted in FIG. 7A, the total amount of intact and cleaved activatable antibody (*e.g.* anti-CD71 TF02.13-3001) in the sample can be captured by magnetic Protein A beads, and subsequently denatured (RapiGest™), reduced with dithiothreitol, alkylated, and trypsin digested. The resulting peptide fragments can be analyzed by using reverse phase LC-MS/MS to identify and quantitate characteristic peptide fragments from the antibody heavy and light chains. In this particular assay, one or more peptides that are characteristic for the antibody portion of the activatable antibody or conjugated activatable antibody can identified and quantitated using reverse phase LC-MS/MS to determine the total amount of both intact and cleaved activatable antibody, thus measuring the amount of total intact and cleaved activatable antibody in the sample.

In the same assay, one or more peptides that are characteristic for uncleaved cleavable moiety (CM) and masking moiety (MM) portion of the activatable antibody (*e.g.* from the N-terminus of the light chain of anti-CD71-TF02.13-2011) can identified and quantitated using reverse phase LC-MS/MS to determine the total amount of intact activatable antibody, thus measuring the amount of intact activatable antibody in the sample.

Using the exemplary assay protocol depicted in FIG. 7B, the total amount of AADC (*e.g.* anti-CD71 TF02.13-3001-vc-MMAE) that remains conjugated to its toxin (MMAE) in the sample can be determined. In this assay, the total amount of activatable antibody, both conjugated and unconjugated, is captured by MabSelect® Protein A. This fraction is treated a cysteine protease to cleave the linker-toxin, thus releasing any toxin that remained conjugated to its activatable antibody. Using reverse phase LC-MS/MS to analyze the cysteine protease-cleaved fraction, the characteristic toxin (*e.g.* MMAE) that remained conjugated to the captured activatable antibody can be identified and quantitated, thus measuring the amount of conjugated toxin in the sample.

Using the exemplary assay protocol depicted in FIG. 7C, the total amount of toxin in the sample, which include toxins cleaved from the AADC in the animal, can be determined. In this exemplary assay depicted in FIG. 7C, the total protein from the sample is precipitated, leaving free, unconjugated toxin (MMAE) in the supernatant. The supernatant can be analyzed using reverse phase LC-MS/MS to identify and quantitating the characteristic toxin (*e.g.* MMAE) that did not associate with the precipitated protein, thus measuring the amount of unconjugated toxin in the sample. In these assays, internal standards (*e.g.* SILu™ Mab, Sigma-Aldrich) were included to allow quantitation.

### EXAMPLE 10: Tolerability and Stability of Anti-CD71-AADC and Anti-CD71 ADCs in Cynomolgus Monkeys

This Example shows that anti-human CD71 activatable antibodies with conjugated toxins (AADCs) of the present disclosure are well-tolerated in cynomolgus monkeys compared to the corresponding parental anti-CD71 antibody drug conjugate (ADCs) based on one or more hematology readouts and clinical symptoms. The results are summarized below in Table 5 and summarized in Figure 17.

In this study, cynomolgus monkeys were treated intravenously with the indicated AADC or ADC with the indicated dosage at days 1 and 21. Only one dose was tolerated for anti-CD71 TF02.13-3011-vc-MMAE AADC and only one dose was administered for anti-CD71-vc-MMAE E2 (i.e. having a DAR of ∼2) ADC. The stability of the activatable antibodies (*i.e.* total activatable antibody vs intact activatable antibody) in the samples obtained from each animals was performed as described in Example 9 using reverse phase LC/MS/MS)

**Table 5: Summary of Anti-CD71 AADC and ADC Tolerability and Stability in Cynomolgous Monkeys**

| **Test Article (Drug-Protein Ratio (DAR))** | **Dose (mg/kg)** | **Body Weight Loss** | **Avg. Neutrophil Count ± SD (per µl.)** | **Relative Stability of Circulating Activatable Antibody** | **Clinical Signs / Tolerabilty** |
|---|---|---|---|---|---|
| Anti-CD71 TF02.13-3011-vc-MMAE (∼3) | 6 | ∼10% | 98 ± 60 | Lower | Severe neutropenia, lethargy, inappetence. Not tolerated |
| Anti-CD71 TF02.13-2011-vc-MMAE (∼3) | 6 | ∼8% | 290 ± 290 | Higher | None. Tolerated |
| Anti-CD71 TF02.13-2011-vc-MMAE E2 (∼2) | 12 | 0% | 180 ± 210 | Higher | None. Tolerated |
| Ab21.12-vc-MMAE E2 (∼2) | 2 | ∼8% | 70 | N/A | Lethal at ∼1 week post 1^{st} dose |

The body weight loss was determined on the lowest measure weight following the 1^{st} administered dose. The baseline neutrophil count was at least 1000 per µL.

As summarized in Table 5, the non-masked E2 (i.e. having a DAR of ∼2) ADC (Anti-CD71-vc-MMAE E2) demonstrated lethality within 8 days of the first dose.

The exemplary results summarized in Table 5 also shows that AADCs with the less cleavable substrate (*i.e.* anti-CD71 TF02.13-2011-vc-MMAE and anti-CD71 TF02.13-2011-vc-MMAE E2) were better tolerated and demonstrated a higher level of circulatory stability in a non-human primate than the corresponding AADC with the more cleavable substrate (*i.e.* anti-CD71 TF02.13-3011-vc-MMAE). Finally, the exemplary results show that the AADC (anti-CD71 TF02.13-2011-vc-MMAE E2 (i.e. having a DAR of ∼2)) demonstrated a measurably improved tolerability in a non-human primate than the corresponding higher DAR AADC (anti-CD71 TF02. 13-2011-vc-MMAE).

### EXAMPLE 11: Stability and Pharmacokinetics of Anti-CD71-AADC in Cynomolgus Monkeys

This Example shows the stability and pharmacokinetics of anti-human CD71 activatable antibodies with conjugated toxins (AADCs) of the present disclosure in cynomolgus monkeys. In this study,

In this study, the results of which are described in further detail in Examples 12 to 14, four groups of cynomolgus monkeys were treated intravenously with the indicated the E2 (i.e. having a DAR of ∼2) anti-CD71 TF02.13-2011-vc-MMAE or a vehicle control with the indicated dosage and schedule shown in Table 6. Samples from each animal were obtained on the days indicated in Table 6, which were assayed for total and intact activatable antibody and conjugated and unconjugated MMAE toxin in manner described in Example 9.

**Table 6: Anti-CD71 AADC Toxicity Study Design**

| **Group** | **Test Article** | **Dose (mg/kg)** | **# Doses** | **# Animals** | **Sampling Time Points Relative to Dose (days)** |
|---|---|---|---|---|---|
| 1 | Vehicle | N/A | 2 (Q3W) | 3 (2 male; 1 female) | Day 1: + 0.003, 0.17, 1, 2, 4, 7, 11, 14, 18, 21 Day 22: + 0.003, 0.17, 1, 2, 4, 7 |
| 2 | anti-CD71 TF02.13-2011-vc-MMAE E2 | 6 | 2 (Q3W) | 3 (2 male; 1 female) | Day 1: + 0.003, 0.17, 1, 2, 4, 7, 11, 14, 18, 21 Day 22: + 0.003, 0.17, 1, 2, 4, 7 |
| 3 | anti-CD71 TF02.13-2011-vc-MMAE E2 | 12 | 2 (Q3W) | 3 (2 male; 1 female) | Day 1: + 0.003, 0.17, 1, 2, 4, 7, 11, 14, 18, 21 Day 22: + 0.003, 0.17, 1, 2, 4, 7 |
| 4 | anti-CD71 TF02.13-2011-vc-MMAE E2 | 18 | 1 | 3 (2 male; 1 female) | Day 1: + 0.003, 0.17, 1, 2, 4, 7, 11, 14, 18, 21 |

As summarized in Table 6, cynomolgus monkeys were dosed with either vehicle or anti-CD71 TF02.13-2011-vc-MMAE E2 AADC by slow IV bolus at the dose levels and schedules shown. Blood samples for toxicokinetic analysis were processed to plasma and stored at -80°C prior to analysis. Serum samples were obtained for anti-drug analysis pre-dose and 7 days after the second dose for Groups 1-3 and 22 days after the first dose for Group 4. All groups contained three animals (2 males and 1 female).

### EXAMPLE 12: Stability of Anti-CD71-AADC in Cynomolgus Monkeys

This Example shows the stability of anti-human CD71 activatable antibodies with conjugated toxins (AADCs) of the present disclosure in cynomolgus monkeys.

As shown in in the exemplary results of FIGS. 8A and 8B, the plasma concentrations of total and intact activatable antibody (anti-CD71 TF02.13-2011) of the administered AADC were determined in the manner described in Examples 9 and 11. The lower limits of quantitation (LLOQ) for both intact and total activatable antibody were 0.633 nM as indicated. These exemplary results demonstrate that the plasma concentration of intact activatable antibodies was generally maintained during the 21-day dosing interval, and at a level that was proportional to the originally-administered dose. Even 7 days post-dose, approximately 80% of conjugated activatable antibody (anti-CD71-TF02.13-2011-vc-MMAE E2) in plasma was in the intact, prodrug form.

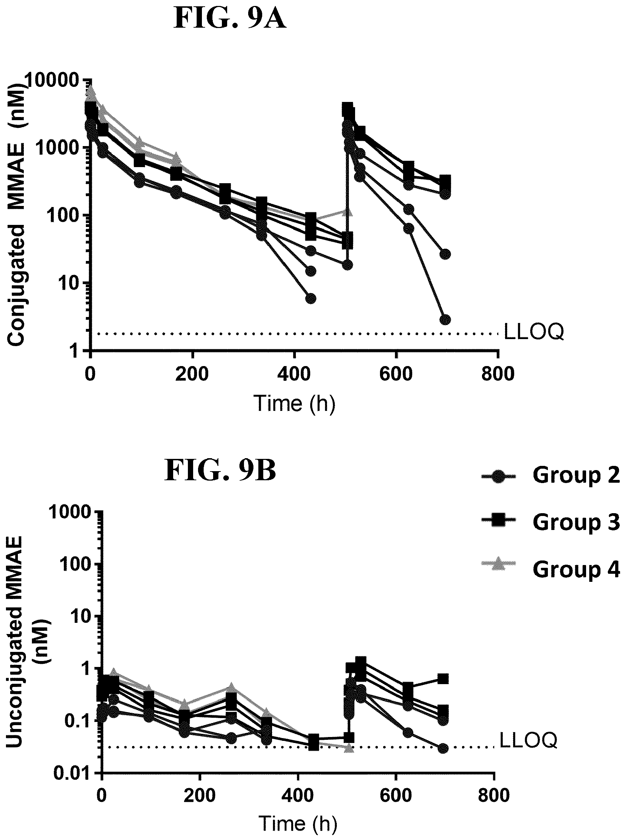

As shown in the exemplary results of FIGS. 9A and 9B, the plasma concentrations of conjugated and unconjugated MMAE toxin were determined in the manner described in Examples 9 and 11. The lower limit of quantitation (LLOQ) was 1.77 nM for conjugated MMAE and 0.31 nM for unconjugated MMAE as indicated. These exemplary results demonstrate that less than 1% of the total MMAE measured in the plasma concentration of was in an unconjugated form.

### EXAMPLE 13: Change of Drug to Activatable Antibody Ratios After Anti-CD71-AADC Dosing in Cynomolgus Monkeys

This Example shows the observed ratio of drug (MMAE) to activatable antibody (anti-CD71 TF02.13-2011) in plasma after dosage of cynomolgus monkeys with anti-human CD71 activatable antibodies with conjugated toxins (AADCs) of the present disclosure.

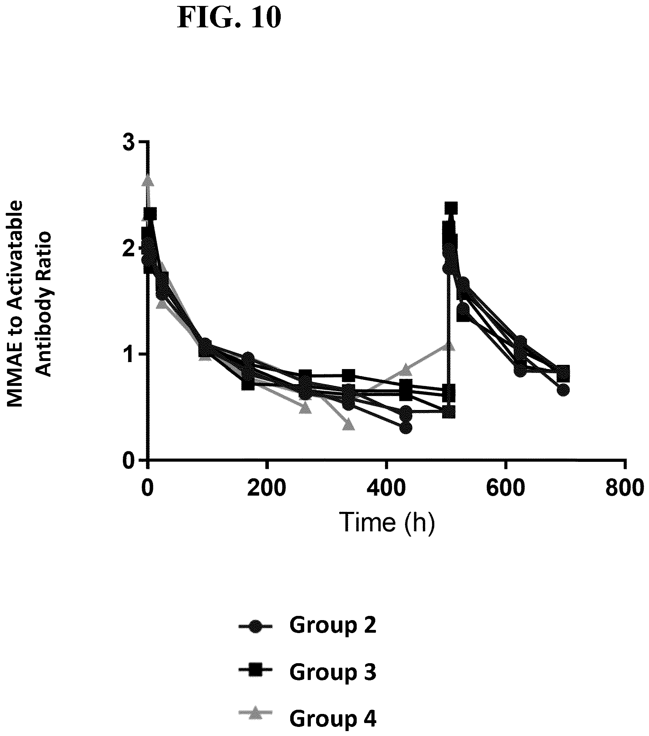

The plasma concentrations of total and intact activatable antibody (anti-CD71 TF02.13-2011) were determined in the manner described in Examples 9 and 11. As shown in FIG. 10, the drug to activatable antibody ratio was calculated by dividing the concentration of conjugated MMAE (see Example 12 and FIG. 9A) by the concentration of total activatable antibody (see Example 11 and FIG. 8A) at each indicated time point. The average ratios and standard deviations for specific time points are shown in Table 7. These exemplary results demonstrate that the observed ratio of MMAE drug to activatable antibody changed over time in a manner that was consistent between each of the three (3) dosages. In each dosage, the calculated DAR of intact AADC started at ∼2 immediately after and within 4 hours of administration, and that this ratio dropped to about 1 approximately 96 hours (*i.e.* about 4 days) post-dose.

**Table 7: Drug to Activatable Antibody Ratio**

| **Test Article** | **Dose (mg/kg)** | **Ratio (5 min)** | **Ratio (4 hr)** | **Ratio (24 hr)** | **Ratio (4 days)** | **Ratio (7 days)** |
|---|---|---|---|---|---|---|
| anti-CD71 TF02.13-2011-vc-MMAE E2 | 6 | 1.98 ± 0.081 | 1.95 ± 0.008 | 1.65 ± 0.075 | 1.08 ± 0.029 | 0.874 ± 0.077 |
| anti-CD71 TF02.13-2011-vc-MMAE E2 | 12 | 2.05 ± 0.080 | 2.00 ± 0.283 | 1.69 ± 0.030 | 1.06 ± 0.023 | 0.835 ± 0.100 |
| anti-CD71 TF02.13-2011-vc-MMAE E2 | 18 | 2.34 ± 0.295 | 2.01 ± 0.072 | 1.63 ± 0.164 | 1.03 ± 0.030 | 0.835 ± 0.119 |

### EXAMPLE 14: Pharmacokinetic Parameters of Anti-CD71-AADC in Cynomolgus Monkeys

This Example shows the pharmacokinetic parameters of anti-human CD71 activatable antibodies with conjugated toxins (AADCs) of the present disclosure in cynomolgus monkeys.

The plasma concentrations of total and intact activatable antibody (anti-CD71 TF02.13-2011) were determined in the manner described in Examples 9 and 11. As shown in Table 8, the mean Cₘₐₓ for a given analyte was based on the maximum observed plasma concentration. The mean AUC₀₋₇ was based on the area under the plasma concentration-time curve from days 0 to 7. Half-Life estimates for total conjugated activatable antibody, intact conjugated activatable antibody, and conjugate MMAE ranged from 2.5 - 6.3 days. Half-life estimates were not generated for unconjugated MMAE.

**Table 8: Drug to Activatable Antibody Ratio**

| **Analyte** | **Mean Cₘₐₓ (nM)** | | | **Mean AUC_{0.7} (day·nM)** | | |
|---|---|---|---|---|---|---|
| | **18 mg/kg** | **12 mg/kg** | **6 mg/kg** | **18 mg/kg** | **12 mg/kg** | **6 mg/kg** |
| Total Activatable Antibody | 2610 | 1810 | 1060 | 5560 | 5490 | 2850 |
| Intact Activatable Antibody | 2530 | 1190 | 1080 | 4804 | 4970 | 2520 |
| Conjugated MMAE | 6090 | 3820 | 2090 | 12500 | 7850 | 4000 |
| Unconjugated MMAE | 0.696 | 0.487 | 0.195 | 1680 | 1170 | 531 |

### EXAMPLE 15: Assay of Anti-Drug Antibodies

This Example describes the use of a bridging assay to monitor the formation of anti-drug antibodies (ADA) in the tested animals.

Plasma samples for ADA analysis were collected pre-study and 7 days after the second dose. Anti-drug antibodies were detected in 3 of the 9 animals dosed in the study.

### EXAMPLE 16: In Vitro Cytotoxicity of Anti-CD71 Conjugated Activatable Antibody

This Example describes *in vitro* cytotoxicity assays on human tumor-derived cell lines of intact and activated anti-CD71 conjugated activatable antibodies of the present disclosure compared to an isotype control ADC.

An exemplary *in vitro* cytotoxic activity of the conjugated activatable antibody of the present disclosure anti-CD71-TF02.13-2011-vcMMAE E2 (HC of SEQ ID NO: 167 and LC of SEQ ID NO: 169), with or without pretreatment with matriptase, was evaluated and compared to a isotype non-CD71 binding conjugated antibody control (AB095-vcMMAE with DAR of 2, where AB095 is specific for tetanus toxin). The cytotoxicity of the test articles were tested on HT29 (human colorectal cancer cell line), H292 (human lung cancer cell line), H520 (human lung cancer cell line), and HCC1806 (human breast cancer cell line) by incubating each test article with each cell line over 5 days, and then measuring cell viability over a range of concentrations of each test article to determine the EC50 for each test article. The results are summarized below in Table 9.

**Table 9: EC50 Values for In Vitro Cytotoxicity Assays in Human Tumor Cells**

| **Test article** | **HT29 EC₅₀ (nM)** | **HCC1806 EC₅₀ (nM)** | **H520 EC₅₀ (nM)** | **H292 EC₅₀ (nM)** |
|---|---|---|---|---|
| anti-CD71 TF02.13-2011-vc-MMAE E2 | ≥50 | ≥50 | ≥50 | ≥50 |
| anti-CD71 TF02.13-2011-vc-MMAE E2, matriptase activated | 1.3 | 3.2 | 1.2 | 3.3 |
| Isotype ADC | No response | >50 | >50 | >50 |

These exemplary results show that the *in vitro* cytotoxicity of the intact anti-CD71 conjugated activatable antibody was similar to that of the isotypc control, but was substantially increased upon matriptase activation.

### EXAMPLE 17: Fc Binding and Complement Activation by Anti-CD71 Conjugated Activatable Antibody

This Example demonstrated the ability of anti-CD71 conjugated activatable antibodies of the present disclosure to mediate effector function-based mechanisms such as antibody-dependent cell-mediated cytotoxicity (ADCC) and complement-dependent cytotoxicity (CDC).

The fragment crystallizable (Fc) portion of an IgG antibody can mediate a variety of functions, some of which occur via binding to Fc receptors and C1q. These functions include cytokine induction, antibody-dependent cell-mediated cytotoxicity (ADCC), complement dependent cytotoxicity (CDC) through activation of the classical pathway of the complement system, phagocytosis, and antibody homeostasis through IgG recycling. (Ravetch et al., Annu Rev Immunol., 2001;19:275-290; Roopenian et al., Nat Rev Immunol. 2007;7:715-725).

Antibody homeostasis is regulated in part by the Fc-related neonatal receptor, FcRn, which is present on intestinal epithelial cells, vascular endothelial cells, and professional antigen presenting cells. Intracellularly, FcRn is present in vesicles; in acidic endosomes at low pH it may bind the Fc portion of an IgG that has been taken in by pinocytosis. The IgG is then released when the endosomes recycle to the cell surface as a result of exposure to extracellular neutral pH. This process allows the control of IgG trafficking across single-layered epithelial barriers, and protects IgG molecules from catabolism, extending the IgG serum half-life. (Roopenian). Indeed, mutations that enhance FcRn binding at low pH but maintain low binding at physiological pH have been shown to have longer serum half-life. (Hinton et al., J Biol Chem. 2004;279:6213-6216; Hinton et al., J Immunol. 2006;176:346-356)

The human FcγR system is composed of both activating (FcγRI, FcγRIIa, and FcγRIIIa) and inhibitory (FcγRIIb) receptors. FcγRI, a high-affinity IgG1 receptor, is expressed by cells of monocytic lineage. (Li et al., J Immunol. 2008;181:1012-1018) FcγRIIa is a more widely expressed low-affinity IgG receptor that preferentially binds immune complexes. (Littaua et al., J Immunol., 1990;144:3183-3186) FcγRIIb is mostly expressed on monocytic lineage and B cells and is a low-affinity inhibitory IgG receptor. Upon FcγRIIb binding to IgG complexes, calcium-dependent processes such as degranulation, phagocytosis, ADCC, cytokine release, and pro-inflammatory activation are all blocked. (Clynes et al., Nat. Med., 6:446-446 (2000); Minard-Colin et al., Blood, 112:1205-1213 (2008); and Hamaguchi et al, J. Exp. Med., 203:743-753 (2006)). FcγRIIIa is a low-affinity receptor responsible for the ADCC activity of NK cells. (Ravetch) To add to the diversity of Fcγ receptors, there are polymorphic variants of FcγRIIa (H131 vs. R131) and FcγRIIIa (V158 vs. F158) that have differences in affinities for IgG molecules. (Bruhns et al., Blood, 2008;113:3716-3725).

The ability of antibodies to mediate a variety of functions through FcγR or complement effector systems allows the possibility of unintended exaggerated pharmacology in IgG based engineered biologics. Unknown cross-reactivity on peripheral blood lymphocytes is capable of triggering cross-linking of Fcγ receptors. Because cross-linking Fcγ receptor interactions can mediate a variety of functions including cytokine induction, ADCC, and phagocytosis (Ravetch), it is important to determine the capacity of therapeutic antibodies to bind unintended targets in blood or tissues.

### Methods - Surface Plasmon Resonance FcγRIIa, FcγRIIb, FcγRIIIa, and FcRn Binding Assay

Recombinant human FcγRII & FcγRIII were captured via 6xHis-tag to assess their binding to Anti-CD71 TF02.13-2011-vc-MMAE, anti-CD71 Ab21.12 (IgG1/κ), AB095-MMAE-E2, and AB095 (IgG1/κ) by surface plasmon resonance (Biacore). Mouse anti-6xHis antibodies were directly immobilized on a CM5 chip by amine coupling according to manufacturer's protocol to the density of 10000 RU. Human FcγRs were then captured on Flow Cells 2, 3 and 4 to achieve capture level of the FcyRs of 250-500 RU. Flow Cell 1 was used as a reference surface. HBS-EP+ buffer (GE, Healthcare) was used as the running buffer. Anti-CD71 TF02.13-2011-vc-MMAE E2, anti-CD71 Ab21.12 (IgG1/κ), AB095-MMAE-E2, AB095 (IgG1/κ), and trastuzumab (IgG1/κ) were injected over all the flow cells at a flow rate of 50 µL/minute for one to two minutes (one minute for FcγRIIb and FcγRIIa H131, and two minutes for FcγRIIIa F158 and FcγRIIIa V158) at concentrations ranging from 46.9 to 12000 nM for FcγRII and 7.8 to 4000 nM for FcγRIII (2-fold serial dilution for both), followed by one to five minutes dissociation time (one minute for FcγRIIb, FcγRIIa H131, and five minutes for FcγRIIIa F158 and FcγRIIIa V158). The chip surfaces were regenerated with an injection of 100 mM HCl at a flow rate of 100 µL/minute for two seconds over all four flow cells. Three experiments with the use of three different CM5 chips were run for each sample (each in duplicate) to accommodate all types of experimental error (instrument, surface and operator). The results of these three experiments were averaged.

For FcRn binding analysis, Anti-CD71 TF02.13-2011-vc-MMAE E2, Anti-CD71 TF02.13-2011 (IgG1/κ without vc-MMAE), AB095-vcMMAE-E2 (a non-specific, monoclonal human anti-tetanus toxoid IgG1/κ antibody containing MMAE with enriched DAR=2), AB095 (a non-specific, monoclonal human anti-tetanus toxoid IgG1/κ antibody) and trastuzumab (IgG1/κ) were directly immobilized on a CM5 chip by amine coupling according to manufacturer's protocol to the density of 500 RU. Hu FcRn was injected over all the flow cells at a flow rate of 50 µL/minute for one minute at concentrations ranging from 5.5 to 12000 nM (3-fold serial dilution), followed by a two-minute dissociation time. The surfaces were regenerated with an injection of 100 mM HCl for two seconds followed by HBS-EP+, pH 7.4. Samples were prepared and run in two running buffer systems, MES-EP+ pH 6.0 and HBS-EP pH 7.4. Three experiments with the use of three different CM5 chips were run for each sample (each in duplicate) to account for instrument, surface and operator error. The results of these three experiments were averaged.

All recombinant human FcγRIIIa V158 data and FcγRIIIa F158 data were fitted to 1:1 binding fit model with Rmax fixed local to account for variability in capture levels. Recombinant human FcγRIIb, FcγRIIa H131, and FcRn binding data were fitted to a steady state affinity model. Biacore T200 Evaluation Software Version 2.0 was used to fit all the data.

### Results

Anti-CD71 TF02.13-2011-vc-MMAE E2 contains a human IgG1/κ isotype Fc. It was expected to bind to FcγRI, FcγIIa, FcγIIb, FcγIII, consistent with other wild type IgG1/κ antibodies. Binding of Anti-CD71 TF02.13-2011-vc-MMAE E2 to FcγR was analyzed by surface plasmon resonance, an FcyRI competition binding ELISA (results not shown), and FcγRIII competition binding ELISA (results not shown), and binding of Anti-CD71 TF02.13-2011-vc-MMAE E2 to complement was analyzed by functional human and cynomolgus complement activation assays (results not shown). These assays were benchmarked by commercial and in-house generated human antibodies. Clinical grade human anti-HER2, IgG1/κ, trastuzumab material was used as a positive control for FcγR binding assays. Fc receptors and complement are known to bind wild type IgG1 antibodies. (Ravetch; Roopenian; Burton et al., Nature. 1980;288:338-344; Hughes-Jones et al., Mol Immunol., 1979;16:697-701; and Hezareh et al., J Virol. 2001;75:12161-12168). Examination of Anti-CD71 TF02.13-2011-vc-MMAE revealed that it did bind FcγRI, FcγIIa H131, FcγRIII, and FcRn, although modestly reduced binding to FcγRIII (by SPR) was observed when compared to the positive control, trastuzumab. Anti-CD71 TF02.13-2011-vc-MMAE E2 binding to FcγRIIb was too weak to determine because it fell below the limit of detection of the Biacore instrument at 1.0 e-05 KD. (see Tables 10A and 10B).

**Table 10A: Binding of Anti-CD71 Conjugated Activatable Antibody to FcyRIIa, FcyRIIb, FcγRIIIa by Surface Plasmon Resonance**

| | **Kd(M)** | | | | |
|---|---|---|---|---|---|
| **Captured FcγR** | **Anti-CD71-TF02.13-2011-vcMMAE E2** | **Anti-CD71-TF02.13-2011 (IgG1/κ)** | **AB095-vcMMAE--E2** | **AB095 (IgG1/κ)** | **Trastuzumab** |
| huFcγRIIb | Binding too weak to determine | Binding too weak to determine | Binding too weak to determine | 7.2 ± 1.2 E-06 | 8.6 ± 1.4 E-06 |
| huFcyRlla H131 | 6.2 ± 2.3 E-06 | 4.7 ± 0.5 E-06 | 2.9 ± 0.3 E-06 | 2.1 ± 0.1 E-06 | 3.8 ± 0.2 E-06 |
| huFcγRIIIa f158 | 2.1 ± 0.3 E-06 | 1.8 ± 0.1 E-06 | 8.9 ± 0.6 E-07 | 1.5 ± 0.1 E-06 | 7.5 ± 0.9 E-07 |
| huFcγRIIIa V158 | 2.0 ± 0.4 E-07 | 1.8 ± 0.2 E-07 | 1.2 ± 0.2 E-07 | 1.6 ± 0.8 E-07 | 8.5 ± 1.3 E-08 |

**Table 10B: Human FcRn Surface Plasmon Resonance**

| **Test Article** | KD (M) | |
|---|---|---|
| | FcRn at pH 6.0 | FcRn at pH 7.4 |
| **Anti-CD71-TF02.13-2011-vcMMAE E2** | 5.3 ± 0.3 E-06 | No significant binding |
| **Anti-CD71-TF02.13-2011 (IgG1/κ)** | 4.7 ± 0.3 E-06 | No significant binding |
| **AB095-vcMMAE-E2** | 5.7 ± 1.3 E-06 | No significant binding |
| **AB095 (IgG1/κ)** | 5.5 ± 1.2 E-06 | No significant binding |
| **Trastuzumab** | 4.5 ± 1.0 E-06 | No significant binding |

Complement-dependent cytotoxicity (CDC) was evaluated by assessing the capacity for anti-CD71 conjugated activatable antibody to activate human and cynomolgus serum complement by capturing iC3b (human) or C3 (cyno) by ELISA, for CDC.

As shown in the exemplary results in Figure 12, the binding of the indicated test articles at the indicated concentrations to iC3b are shown as an assay for human complement activation. Anti-CD71-TF02.13-2011-vcMMAE E2 bound iC3b in a complement activation assay in both human and cynomolgus serum, but to a lesser degree than did the positive control, 7C6 antibody, that was used in the CDC assay. The 7C6.7 antibody with the indicated mutations were used as a negative control. The iC3b binding of anti-CD71-TF02.13-2011-vcMMAE E2 was also lesser than its unconjugated activatable antibody counterpart (anti-CD71-TF02.13-2011).

### EXAMPLE 18: Cytokine Release in Human PBMCs

This Example showed that although anti-CD71-TF02.13-2011-vcMMAE E2 does not bind human or cynomolgus peripheral blood mononuclear cells (PBMCs), IgG1 antibodies are capable of cross linking Fcγ receptors on peripheral blood lymphocytes, thereby mediating a variety of functions including cytokine induction.

The ability of anti-CD71-TF02.13-2011-vcMMAE E2 or anti-CD71-TF02.13-2011 to induce *in vitro* cytokine release in human PBMCs was evaluated in a soluble or solid-phase (plate-bound) format for the following cytokines: interleukin (IL)-1β, IL 6, IL 2, interferon (IFN)y, and tumor necrosis factor alpha (TNFα). For the solid-phase experiment, PBMCs from 8 healthy human donors were added to polypropylene plates previously coated with 0.96 µg of test article and washed off after 1.5 hours. Supernatant was collected after 48 hours for analysis. Trastuzumab and a nonbinding isotype control ADC, AB095-vcMMAE E2, were used as negative controls. Three positive controls were used: T cell stimulating antibodies, TGN1412 or visilizumab and B cell, and myeloid cell stimulating lipopolysaccharide (LPS). In 1 of 8 donors tested, a substantial release of 3 of the 5 measured cytokines, IL-1β, IL-6, and TNFα, was observed in response to both anti-CD71-TF02.13-2011-vcMMAE E2 and AB095-vcMMAE-E2. IL-6 and TNFα release was observed in an additional 2 of 8 donors in response to both anti-CD71-TF02.13-2011-vcMMAE E2 and AB095-vcMMAE E2. Three donors were tested in the soluble presentation assay, which was a 24-hour, high cell density presentation assay.

In this study, cytokine release from human donor PBMCs was measured following stimulation with a precoat of 0.96 µg/well with anti-CD71-TF02.13-2011-vcMMAE E2 and anti-CD71-TF02.13-2011 (IgG1κ). Trastuzumab was used as a negative control IgG1κ antibody. AB095-vcMMAE-E2 and AB095 (IgG1κ) were used as isotype control antibodies. TGN1412, visilizumab, and lipopolysaccharide (LPS) were added as positive controls for cytokine release. Values in bold and boxed highlight test article that elicited cytokine release > 2 times the release by trastuzumab.

As shown in the exemplary results in Table 11, anti-CD71-TF02.13-2011-vcMMAE E2 elicited no cytokine release above the level induced by the negative control trastuzumab. Cytokine release was also assessed in 3 cynomolgus monkey donors in the solid phase format for the following cytokines: IL-1β, IL-6, IL-2, IFNγ, IL-8, and IL 10. Anti-CD71-TF02.13-2011-vcMMAE E2elicited IL-8 production slightly above background level, as defined by trastuzumab, in 1 of the 3 donors tested. In the cynomolgus monkey non-GLP and GLP toxicology studies for anti-CD71-TF02.13-2011-vcMMAE E2, acute clinical symptoms representative of cytokine release were not observed at any dose level.

**Table 11: Cytokine Release of Human PBMCs in Response to Solid Phase Anti-CD71 Conjugated Activatable Antibody and Other Test Articles**

| **pg/mL Cytokine** Listed **by** Donor No. | | | | | | | | |
|---|---|---|---|---|---|---|---|---|
| **Donor No.:** | **32150** | **32151** | **32289** | **32165** | **32314** | **32147** | **32156** | **32169** |
| **IL-1β** | | | | | | | | |
| anti-CD71-TF02.13-2011-vcMMAE E2 | 735 | 89 | 39 | 77 | 4 | 96 | **5594** | 493 |
| anti-CD71-TF02.13-2011 (IgG1κ) | 294 | 90 | 40 | 64 | 6 | 111 | **4088** | 299 |
| AB095-vcMMAE-E2 | 49 | 24 | 7 | 68 | 0 | 51 | **2704** | 141 |
| AB095 (IgG1κ) | 121 | 44 | 10 | 8 | 1 | 72 | 70 | 50 |
| Buffer | 1 | 0 | 0 | 0 | 0 | 0 | 0 | 0 |
| Trastuzumab | 570 | 111 | 29 | 14 | 2 | 103 | 381 | 525 |
| TGN1412 | 288 | 208 | 148 | 128 | 58 | 875 | 104 | 200 |
| Visilizumab | 439 | 231 | 616 | 465 | 270 | 172 | 61 | 35 |
| LPS | 3007 | 1570 | 1994 | 803 | 2272 | 0 | 0 | 0 |

| **IL-6** | | | | | | | | |
|---|---|---|---|---|---|---|---|---|
| anti-CD71-TF02.13-2011-vcMMAE E2 | 2022 | 176 | 110 | **102** | 8 | **71** | **>10000** | 1182 |
| anti-CD71-TF02.13-2011 (IgG1κ) | 763 | 160 | 116 | **111** | 15 | **83** | **>10000** | 1213 |
| AB095-vcMMAE-E2 | 278 | 59 | 37 | **103** | 5 | **78** | **9781** | 423 |
| AB095 (IgG1κ) | 195 | 63 | 42 | 19 | 2 | 37 | 305 | 132 |
| Buffer | 15 | 1 | 1 | 0 | 1 | 1 | 1 | 8 |
| Trastuzumab | 1262 | 119 | 60 | 23 | 3 | 33 | 2776 | 879 |
| TGN1412 | 655 | 521 | 296 | 214 | 207 | 151 | 208 | 248 |
| Visilizumab | 292 | 103 | 253 | 76 | 291 | 397 | 234 | 202 |
| LPS | 9668 | 9244 | >10000 | 5655 | >10000 | 1 | 1 | 10 |

| **TNFα** | | | | | | | | |
|---|---|---|---|---|---|---|---|---|
| anti-CD71-TF02.13-2011-vcMMAE E2 | 3741 | 497 | 583 | **2110** | 133 | **2830** | **8581** | 3854 |
| anti-CD71-TF02.13-2011 (IgG1κ) | 3438 | 693 | 881 | **1425** | 114 | **1554** | **5163** | 2309 |
| AB095-vcMMAE-E2 | 1660 | 568 | 296 | **1458** | 51 | **1221** | **4815** | 815 |
| AB095 (IgG1κ) | 3703 | 634 | 349 | 443 | 50 | 1906 | 664 | 658 |
| Buffer | 93 | 0 | 1 | 0 | 1 | 0 | 2 | 15 |
| Trastuzumab | 5276 | 931 | 506 | 553 | 70 | 1131 | 2180 | 3701 |
| TGN1412 | >10000 | 9651 | 8913 | >10000 | >10000 | >10000 | 9126 | >10000 |
| Visilizumab | >10000 | 6593 | >10000 | >10000 | >10000 | >10000 | 3157 | 1642 |
| LPS | 470 | 308 | 293 | 322 | 257 | 2 | 0 | 12 |

| **IFNγ** | | | | | | | | |
|---|---|---|---|---|---|---|---|---|
| anti-CD71-TF02.13-2011-vcMMAE E2 | 1950 | 19 | 3 | 12 | 7 | 349 | **354** | 52 |
| anti-CD71-TF02.13-2011 (IgG1κ) | 7099 | 44 | 8 | 10 | 2 | 129 | **139** | 245 |
| AB095-vcMMAE-E2 | 6119 | 35 | 4 | 4 | 1 | 161 | **53** | 14 |
| AB095 (IgG1κ) | >10000 | 125 | 13 | 14 | 3 | 433 | 30 | 33 |
| Buffer | 26 | 0 | 2 | 1 | 0 | 0 | 0 | 8 |
| Trastuzumab | 9173 | 59 | 6 | 7 | 1 | 300 | 58 | 4100 |
| TGN1412 | >10000 | >10000 | >10000 | >10000 | 3488 | >10000 | 8685 | >10000 |

| **pg/mL Cytokine Listed by Donor No.** | | | | | | | | |
|---|---|---|---|---|---|---|---|---|
| **Donor** No.: | **32150** | **32151** | **32289** | **32165** | **32314** | **32147** | **32156** | **32169** |
| Visilizumab | >10000 | 6159 | 7957 | >10000 | 7965 | >10000 | 2048 | 873 |
| LPS | 376 | 607 | 30 | 7 | 29 | 0 | 0 | 14 |
| **IL-2** anti-CD71-TF02.13-2011-vcMMAE E2 | 145 | 4 | 3 | 3 | 3 | 2 | 3 | 3 |
| anti-CD71-TF02.13-2011 (IgG1κ) | 217 | 2 | 3 | 5 | 1 | 1 | 3 | 13 |
| AB095-vcMMAE-E2 | 126 | 2 | 3 | 2 | 1 | 1 | 8 | 2 |
| AB095 (IgG1κ) | 301 | 8 | 3 | 4 | 2 | 1 | 5 | 3 |
| Buffer | 447 | 6 | 8 | 30 | 7 | 2 | 24 | 33 |
| Trastuzumab | 262 | 5 | 4 | 8 | 1 | 2 | 3 | 12 |
| TGN1412 | >10000 | 8485 | 9018 | 8223 | >10000 | 821 | 636 | 1318 |
| Visilizumab | 396 | 459 | 1863 | 609 | 4643 | >10000 | 1517 | 1038 |
| LPS | 78 | 3 | 6 | 4 | 4 | 7 | 15 | 34 |

### EXAMPLE 19: Antitumor Efficacy of Anti-CD71 AADC in SW-48 Xenograft Mouse Model

This Example shows the efficacy of anti-CD71 conjugated activated antibody of the present disclosure in a mouse tumor xenograft model, using SW-48 cell (human colorectal cancer).

In this exemplary study, single intraperitoneal injections of anti-CD71-TF02.13-2011-vcMMAE E2 (AADC) at the indicated dosages were administered to groups of 8 mice, and the mean tumor volume was observed over time. As shown in Figure 13 and Table 12, a range of total growth inhibition (TGI) at 23 days as compared to vehicle control was observed in all dosages, with complete tumor regression observed at 6 and 12 mg/kg dosages.

**Table 12: Xenograft Tumor Growth Inhibition Following Anti-CD71 AADC Treatment**

| **AADC Dose (mg/kg)** | **TGI (%)** | **TGD (%)** | **PR (%)** | **CR(%)** | **TR (%)** |
|---|---|---|---|---|---|
| 0.75 | 35 | 21 | 0 | 0 | 0 |
| 1.5 | 66 | 71 | 0 | 0 | 0 |
| 3 | 96 | >521 | 12.5 | 50 | 62.5 |
| 6 | 100 | >521 | 0 | 100 | 100 |
| 12 | 100 | >521 | 0 | 100 | 100 |

| | | | | | |
|---|---|---|---|---|---|
| CR = complete response; PR = partial response; TGI = tumor growth inhibition; TGD = tumor growth delay; TR = total response. | | | | | |

### EXAMPLE 20: Antitumor Efficacy of Anti-CD71 AADC in a DLBCL PDX Mouse Model

This Example shows the efficacy of anti-CD71 conjugated activated antibody of the present disclosure in five (5) mouse tumor patient-derived xenograft (PDX) model, using HuPrime patient-derived diffuse large B-cell lymphoma (DLBCL) in female SCID or NOG mice.

In this exemplary study, groups of 3 mice were established with subcutaneous tumors (100 - 200 mm³) and administered with 6 mg/kg of anti-CD71-TF02.13-2011-vcMMAE E2 (AADC) or a vehicle control on days 0 and 7. As shown in the exemplary results of Figure 14, the AADC test article achieved > 100% TGI in 4 of 5 models and 72% TFI in 1 model compared to the vehicle control group. Complete responses (*i.e.,* absence of measurable tumor at study termination) in the models administered with the AADC were observed in 2 of 3 mice for LY2345, 3 of 3 mice for model LY6933, 2 of 3 mice for model LY6934, and 2 or 3 mice for model LY2318. No complete responses were observed for model LY3604. These exemplary results demonstrate that the AADC of the present disclosure demonstrated efficacy, up to complete response in some models.

### EXAMPLE 21: Antitumor Efficacy of Anti-CD71 AADC in TumorGraft PDX Mouse Models

This Example shows the efficacy of anti-CD71 conjugated activated antibody of the present disclosure in nine (9) mouse tumor patient-derived xenograft (PDX) model, using TumorGraft patient-derived human breast cancer (CTG-0437, CTG-0869, CTG-1059), non-small cell lung cancer (NSCLC) (CTG-1082, CTG-0860, CTG-0160, CTG-1012), and head and neck small cell carcinoma (HNSCC) (CTG-1140, CTG-1082) in nude mice.

In this exemplary study, groups of 3 mice for each PDX model were established with subcutaneous tumors (150 - 300 mm³) and administered with 6 mg/kg of anti-CD71-TF02.13-2011-vcMMAE E2 (AADC) or a vehicle control on days 0 and 7. As shown in the exemplary results of Figures 15A, 15B, and 15C, the AADC test article achieved > 100% tumor growth inhibition (TGI) in 6 of 9 models across the three cancer indications. In these 6 models in which >100% TGI was observed, 6 of 18 mice receiving the AADC achieved complete response (*i.e.,* absence of measurable tumor at study termination). In 2 additional model, 92% and 78% TGI was observed for HNSCC and NSCLC, respectively. One NSCLC model did not respond to the AADC. As shown in these exemplary results, the AADC of the present disclosure demonstrated efficacy, including complete responses in some cases, to PDX model derived from a variety of human cancer types.

### EXAMPLE 22: Antitumor Efficacy of Anti-CD71 AADC in HuPrime Pancreatic PDX Mouse Model

This Example shows the efficacy of anti-CD71 conjugated activated antibody of the present disclosure in a patient-derived xenograft (PDX) mouse model, using HuPrime patient-derived human pancreatic cancer (PA6237) in SCID mice.

In this exemplary study, a group of 3 mice was established with subcutaneous tumors (100 - 200 mm³) and administered with 3 or 6 mg/kg of anti-CD71-TF02.13-2011-vcMMAE E2 (AADC) or a vehicle control on days 0 and 7. As shown in the exemplary results of Figure 16, the AADC test article achieved > 100% tumor growth inhibition (TGI) at 3 and 6 mg/kg. In these 6 mg/kg group, 3 of 3 complete responses were observed out to day 59, while the 3 mg/kg group maintained complete response out to day 31, followed by tumor regrowth.

### EXAMPLE 23: Non-GLP Pilot Toxicity Studies of Anti-CD71 Conjugated Activatable Antibody

This Example shows the relative toxicities of the anti-CD71 conjugated activatable antibody (AADC) anti-CD71-TF02.13-2011-vcMMAE E2 of the present disclosure at dose levels ranging from 6 to 18 mg/kg to anti-CD71 conjugated antibodies (ADC) Ab21.12-vcMMAE E2 at dose levels ranging from 0.6 to 6 mg/kg in cynomolgus monkeys (1-2 monkeys per sex per group) after IV bolus injection either once every 3 weeks (Q3W) or once every 2 weeks (Q2W). Terminal necropsies were performed on the test animals on day 29 (7 days after the last dose). Assessments during the study included clinical signs, body weight, food consumption, clinical pathology, anatomic pathology, TK, and immunogenicity.A summary of the pilot toxicity study is shown in Table 13.

**Table 13: Non-GLP Pilot Toxicity Study In Cynomolgus Monkeys**

| **Test Article** | **Dose Level (mg/kg)** | **Drug-to-Protein Ratio** | **Dose Day** | **Male/Female** |
|---|---|---|---|---|
| **anti-CD71-TF02.13-3011-vcMMAE** | 6 | 2.7 | 1 (no 2^{nd} dose) | 1/1 |
| **anti-CD71-TF02.13-3011-vcMMAE** | 3 | 2.7 | 1, 22 | 1/1 |
| **anti-CD71-TF01-3011-vcMMAE** | 6 | 2.9 | 1, 22 | 1/1 |
| **anti-CD71-TF02.13-2011-vcMMAE** | 6 | 2.9 | 1, 22 | 1/1 |
| **anti-CD71-TF02.13-2011-vcMMAE E2** | 12 | 2.0 | 1, 22 | 1/1 |
| | | | | |

| **Test Article** | **Dose Level (mg/kg)** | **Drug-to-Protein Ratio** | **Dose / Necropsy Day** | **Male/Female** |
|---|---|---|---|---|
| **Vehicle Control** | 0 | N/A | 1, 22/29 | 2/1 |
| **anti-CD71-TF02.13-2011-vcMMAE E2** | 6 | 2.0 | 1, 22/29 | 2/1 |
| **anti-CD71-TF02.13-2011-vcMMAE E2** | 12 | 2.0 | 1, 22/29 | 2/1 |
| **anti-CD71-TF02.13-2011-vcMMAE E2** | 18 | 2.0 | 1/22 (no 2^{nd} dose) | 2/1 |
| **anti-CD71-TF02.13-3011-vcMMAE E2** | 9 | 2.0 | 1, 22/29 | 1/1 |
| **anti-CD71-TF02.13-2011-vcMMAE E2** | 8 | 2.0 | 1,15/50 | 1/1 |
| Ab21.12-vcMMAE E2 | 6 | 2.0 | 1 / 10 (no 2^{nd} dose) | 1/0 |
| **Ab21.12-vcMMAE E2** | 2 | 2.0 | 1 (no 2^{nd} dose) | 0/1 |
| **Ab21.12-vcMMAE E2** | 0.6 | 2.0 | 1, 22/29 | 1/1 |

Severe toxicity was observed as manifested by hunched posture, decreased activity, and body temperature elevation in groups that received a high dose of CD71-TF02.13-2011-vcMMAE E2 (18 mg/kg), frequent doses of CD71-TF02.13-2011-vcMMAE E2 (dosing on days 1 and 15), or the unmasked parental anti-CD71 antibody drug conjugate (ADC) (Ab21.12-vcMMAE E2) at 6 and 2 mg/kg. When a cause of death could be established, it was determined to be infection secondary to drug-related immune suppression.

Test article-related findings were similar for CD71-TF02.13-2011-vcMMAE E2 and Ab21.12-vcMMAE E2 and consisted primarily of hematologic toxicity. Key findings included (1) mortality attributed to secondary infection was observed in groups that received a single dose of CD71-TF02.13-2011-vcMMAE E2 at 18 mg/kg, CD71-TF02.13-2011-vcMMAE E2 on days 1 and 15 at 8 mg/kg, or a single dose of the ADC (Ab21.12-vcMMAE E2) at ≥ 2 mg/kg, (2) body weight decrease 2 weeks post dose, with recovery by 3 weeks post dose at ≥ 12 mg/kg of CD71-TF02.13-2011-vcMMAE E2, (3) dose-dependent decreases in red blood cell mass and all leukocyte populations with partial to complete recovery observed 3 weeks post dose, (4) transient increase in platelet levels 2 weeks post dose, (5) mild to moderate increases in fibrinogen, decreased albumin, and increased globulin, (6) mild to moderate decreases in bone marrow, spleen, and thymic cellularity, with corresponding weight reductions in the thymus and spleen. Decreased cellularity was also observed in several lymphoid tissues.

In this exemplary study, the highest tolerated doses of CD71-TF02.13-2011-vcMMAE E2 and Ab21.12-vcMMAE E2 (the corresponding ADC) in these studies were 12 mg/kg and 0.6 mg/kg, respectively, administered once every 3 weeks for 2 doses.

### EXAMPLE 24: GLP Toxicity Studies of Anti-CD71 Conjugated Activatable Antibody

This Example shows a GLP repeat dose toxicity study in cynomolgus monkeys of the anti-CD71 conjugated activatable antibody (AADC) anti-CD71 -TF02.13-2011-vcMMAE E2 of the present disclosure at dose levels of 0, 2, 6, or 12 mg/kg on days 1 and 22 via IV bolus injection. The dose groups are shown in Table 14.

**Table 14: GLP Toxicity Study In Cynomolgus Monkeys**

| **Test Article** | **Dose Level (mg/kg)** | **Drug-to-Protein Ratio** | **Dose Day** | **Male/Female** |
|---|---|---|---|---|
| **Vehicle** | 0 | N/A | 1, 22 | 6/6 |
| **anti-CD71- TF02.13-2011-vcMMAE E2** | 2 | 2.0 | 1, 22 | 6/6 |
| **anti-CD71-TF02.13-2011-vcMMAE E2** | 6 | 2.0 | 1, 22 | 6/6 |
| **anti-CD71-TF02.13-2011-vcMMAE E2** | 12 | 2.0 | 1 (no 2^{nd} dose) | 6/6 |

In this exemplary study, each group of 6 male and 6 female monkeys were dosed with anti-CD71-TF02.13-2011-vcMMAE E2 or vehicle (IV bolus of the indicated dosage on days 1 and 22) as shown in Table 14. Terminal necropsies were performed on day 29, and recovery necropsies were performed on day 64. Toxicity assessments included mortality, clinical signs, body weight changes, food consumption, clinical pathology, anatomic pathology, ophthalmology examinations, and assessments of cardiovascular, respiratory, and central nervous system function. The study included TK analysis and an assessment of ADA formation. The following exemplary observations were made. A summary of selected results from the GLP toxicity study of anti-CD71 TF02.13-2011-vcMMAE E2, having a DAR of ∼2, compared to corresponding non-GLP studies of an unmasked anti-CD71 antibody drug conjugate (ADC) Ab21.12-vcMMAE E2 (also having a DAR of ∼2), is summarized in Table 15.

**Table 15: GLP Toxicity Study Results In Cynomolgus Monkeys**

| **Test Article (q3wx2)** | **Dose Level (mg/kg)** | **Body weight loss** | **Average neutrophil count ± SD** | **Tolerability** |
|---|---|---|---|---|
| **anti-CD71-TF02.13-2011-vcMMAE E2** | 12 | 0 % | 61 ± 44 | Not tolerated |
| **anti-CD71-TF02.13-2011-vcMMAE E2** | 6 | 0 % | 277 ± 132 | Tolerated |
| **Ab21.12-vcMMAE E2** | 2 | 6% | 70 | Not tolerated |
| **Ab21.12-vcMMAE E2** | 0.6 | 4% | 280 ± 42 | Tolerated |

There were no AADC-related effects on body weights, food consumption, respiratory rate, electrocardiography, coagulation, and urinalysis parameters, nor were there any neurologic effects, ocular effects, or gross pathology findings.

AADC-related mortality was observed at 12 mg/kg doses; 1 female was found dead on day 10 and 2 females were euthanized on day 11 in declining clinical condition. AADC-related clinical pathology and microscopic findings in these animals were similar to those present in animals that survived to scheduled necropsy (day 29), with the exception of microscopic evidence of bacterial infection, which was considered to be a consequence of AADC-related immunosuppression.

As shown in Table 15, the anti-CD71-TF02.13-2011-vcMMAE E2 demonstrated superior tolerability relative to Ab21.12-vcMMAE E2 with no detectable loss of body weight. As shown, Ab21.12-vcMMAE E2 lacked tolerability within 8 days of a single dose at 2 mpk, while anti-CD71-TF02.13-2011-vcMMAE E2 provided ≥ 10 fold protection over the non-masked, Ab21.12-vcMMAE E2.

AADC-related clinical signs in 12 mg/kg-dosed animals surviving until scheduled necropsy were skin lesions/wounds consistent with infection that correlated to decreased leukocyte populations and signs of acute phase response (decreased albumin and cholesterol and increased globulins, total bilirubin, and triglycerides).

Changes in hematology parameters at ≥ 6 mg/kg following the first dose consisted of decreases in red blood cell mass, reticulocytes, and all leukocyte subsets, along with increases in platelets. Hematology changes at 2 mg/kg were limited to mildly increased platelets following the first and second dose.

AADC-related change in clinical chemistry parameters following the first dose were most notable at 12 mg/kg and consisted of an acute phase response (decreases in albumin and cholesterol, and increases in globulins, triglycerides [females only], total bilirubin), and increases in urea nitrogen (individuals only) and creatinine, and decreases in phosphorus. Clinical chemistry changes at 6 mg/kg were only seen following the first dose and were limited to minimally increased bilirubin and increased globulins in individual females.

AADC-related hematologic and clinical chemistry findings recovered by the end of the recovery phase.

AADC-related microscopic findings at terminal euthanasia were present in the thymus, adrenal gland, and bone marrow at ≥ 6 mg/kg/dose. Findings at the injection site were observed in all groups, and are interpreted to be related to the administration procedure and not specifically to AADC. Thymic weights were decreased in male monkeys dosed with 12 mg/kg and female monkeys dosed with 6 mg/kg, which correlated histologically to decreased cellularity, particularly of the thymic cortex. Adrenal gland weights were increased in male monkeys dosed with 12 mg/kg and female monkeys dosed with 6 mg/kg and the histologic correlate was adrenocortical hypertrophy. Decreased cellularity was present in the bone marrow, specifically in the femur, in male monkeys dosed with ≥ 6 mg/kg. After a 6 week dose-free interval the decreased cellularity of the thymus and the adrenocortical hypertrophy were not present, consistent with complete recovery of these changes. The increased cellularity of the bone marrow persisted but at decreased severity, consistent with partial recovery.

### EXAMPLE 25: Conjugation of MMAE to the CD71 Activatable Antibody and Methods of Lowering the DAR of the Resulting CD71 Conjugated Activatable Antibody to About DAR 3

This Example outlines the methods that were used to conjugate vc-MMAE to the anti-CD71 activatable antibody, anti-CD71 TF02.13-2011, to create anti-CD71 TF02.13-2011-vc-MMAE containing a drug load of 2 MMAE molecules for each anti-CD71 TF02.13-2011 molecule, in addition to the methods used to enrich the DAR to about 3.

### Conjugation Method

E2-enriched anti-CD71 TF02.13-2011-vc-MMAE - To a solution of anti-CD71 TF02.13-2011 (13.95 mg/mL), 39 g solution, 544 mg, 3.5 nmol) was added 0.5 M EDTA (0.39 mL) and TCEP (0.77 mL, 10 mM in WFI, 7.7 nmol, 2.2 equivalents) at 2-8 °C overnight. The reduced antibody was then diluted with DMSO (2.29 mL) treated with vcMMAE in DMSO (1.89 mL of 9.49 mM vcMMAE in DMSO, 17.9 nmol, 5.1 equivalents) at room temperature for 1 h. The conjugated antibody was treated with N-acetyl cysteine (0.29 mL of 100 mM in WFI, 29 nmol, 8.2 equivalents) to quench excess vcMMAE. The quenched solution was analyzed by HIC (hydrophobic interaction chromatography) for DAR species > E4 (higher DAR species, 20.9% in the above preparation).

### Method for Enriching to Lower DAR

The conjugation reaction was suspended with HIC resin, like those commercially available from Tosoh Biosciences (Toyopearl alkyl as an example) in portions at room temperature for 2 h to reduce the higher DAR species to a targeted level (generally < 2%). The treated solution was filtered and the resin was washed successively with PBSE (125 mM potassium phosphate, 150 mM NaCl, 6.3 mM EDTA, pH 7.2, 22 g) followed by PBS (5 mM potassium phosphate, 200 mM NaCl, pH 7, 35 g). The combined filtrate and rinses were concentrated and buffer exchanged using a 30 kD molecular weight cut off centrifuge filter then diluted with formulation components for storage.

The resulting product contained 228 mg (A280 6.5 mg/mL) and was shown to have a DAR of about 2.9 as measured from a commercially available HIC column and to constitute the following characteristics and DAR species: SEC 98.7% monomer, 0.2% higher molecular weight, 1.1% lower molecular weight, Endotoxin < 0.06 EU/mg, DAR species greater than E4 were less than 2 area%; DAR E0 was about 6.2 area%, DAR E2 was about 40.7 area%, and DAR E4 was about 46.8 area%).

In the examples described herein, this enriched DAR product is referred to as "anti-CD71 TF02.13-2011-vc-MMAE (having an average DAR of ∼3)''

### EXAMPLE 26: Conjugation of MMAE to the CD71 Activatable Antibody and Methods of Enriching the DAR of the Resulting CD71 Conjugated Activatable Antibody to About DAR 2 ("E2")

This Example outlines the methods that were used to conjugate vc-MMAE to the anti-CD71 activatable antibody, anti-CD71 TF02.13-2011, to create anti-CD71 TF02.13-2011-vc-MMAE containing a drug load of 2 MMAE molecules for each anti-CD71 TF02.13-2011 molecule, in addition to the methods used to enrich the DAR to about 2.

### Conjugation Method

Anti-CD71 TF02.13-2011-vc-MMAE E2 - To a solution of anti-CD71 TF02.13-2011 (20.3 mg/mL) was diluted with 1457 g PBSE (125 mM potassium phosphate, 150 mM NaCl, 6.3 mM EDTA, pH 7.7) and cooled to 4 °C. To the cooled solution, TCEP (10 mM in WFI, 30.2 mL, 0.30 mmol, 1.5 equivalents) was added and maintained at 4 °C for 15 hours. To the reduced antibody, a solution of vc-MMAE (1.056 g, 0.80 mmol, 4 equivalents) in DMSO (300 g) was added with cooling followed by a DMSO rinse (65 g) after which the solution was allowed to warm to room temperature. After 3.5 h, N-acetyl cysteine (0.262 g, 1.60 mmol, 8 equivalents) was added to quench excess vc-MMAE and the reaction mixture was stored at 2-8 °C until purification.

### Method for Enriching to Lower DAR

The product was purified in portions on commercially available HIC purification columns like those commercially available from GE Life Sciences (alkyl Sepharose) using a gradient of ammonium sulfate / phosphate buffers at pH 7.1 with monitoring at 280 nm by UV. The collected DAR 2 product pool (approximately 9.5 g, 30% yield) was buffer exchanged with buffer at pH 6.0 by TFF using Pellicon 3 Ultracel 30 kD membranes then diluted with formulation components for storage.

The resulting product (A280 6.4 mg/mL) was shown to have a DAR of about 2 as measured from a commercially available HIC column and to constitute the following characteristics and DAR species: SEC 99.6% monomer, 0.2% HMW, 0.2% LMW, Endotoxin < 0.06 EU/mg, and DAR E2 was about 99.0 area%, with run-to-run variability between 94 to 99% DAR E2.

In the examples described herein, this **E**nriched DAR **2** ("E2") product is referred to as "anti-CD71 TF02.13-2011-vcMMAE E2".

### EXAMPLE 27: Assessment of Biophysical Characteristics of E2 Versus Higher DAR Species in Preclinical Murine Models

The non-HIC purified, conjugated product obtained from the method described in Example 25 was run over a HIC column using standard buffers and procedures as outlined herein. As shown in Figure 18A, a number of species having a drug load between 0 to 8 were identified.

The purified, E2 product obtained from Example 26 was subjected to the same HIC chromatography. As shown in Figure 18B, only a single species containing a drug load of 2 was observed.

The non-purified and E2 products were radiolabeled and injected into mice to assess overall clearance and toxicity of each product. As shown in Figure 18C, the purified E2 product was shown to have significantly lower clearance relative to the non-purified, high drug-loaded product.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following embodiments:
1. A conjugated activatable antibody comprising the structure of Formula (I) or a salt thereof: 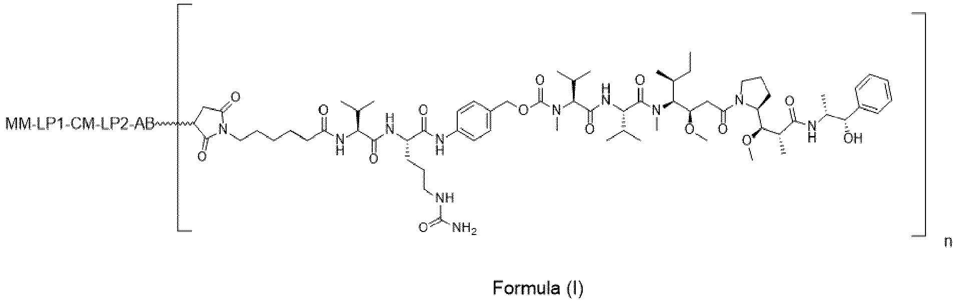
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
2. The conjugated activatable antibody of embodiment 1, wherein the AB comprises an IgG1 isotype.
3. The conjugated activatable antibody of embodiment 1 or 2, wherein the AB is an antibody having a heavy chain constant region, and wherein the C-terminal residue of the heavy chain constant region is not a lysine.
4. A conjugated activatable antibody comprising the structure of Formula (I) or a salt thereof: 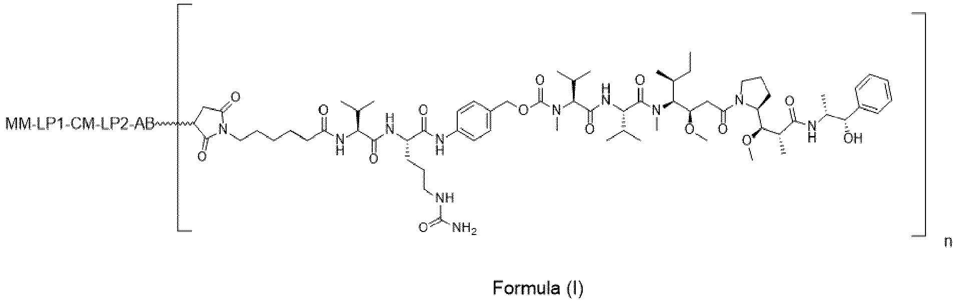
   (a) wherein
      (i) AB is an antibody that specifically binds to human CD71 and comprises a heavy chain variable region comprising a sequence of SEQ ID NO: 5 and a light chain variable region comprising a sequence of SEQ ID NO: 7;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
5. The conjugated activatable antibody of embodiment 4, wherein the AB comprises an IgG1 isotype.
6. The conjugated activatable antibody of embodiment 4 or 5, wherein the AB is an antibody having a heavy chain constant region, and wherein the C-terminal residue of the heavy chain constant region is not a lysine.
7. A conjugated activatable antibody comprising the structure of Formula (I) or a salt thereof: 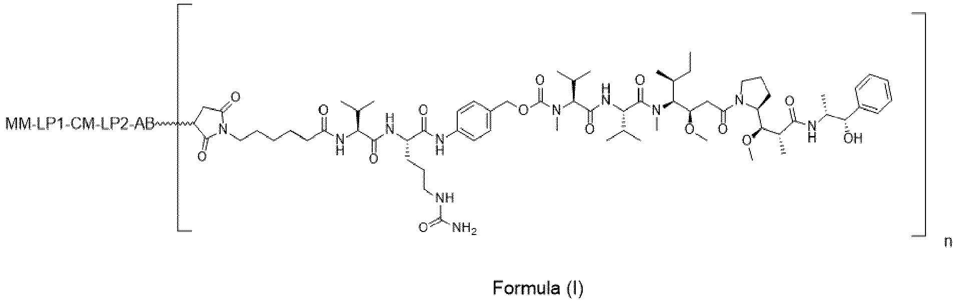
   (a) wherein
      (i) AB is an antibody that specifically binds to human CD71 and comprises a heavy chain comprising a sequence of SEQ ID NO: 167 and a light chain comprising a sequence of SEQ ID NO: 19;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38; and
   (b) wherein "n" is 2.
8. The conjugated activatable antibody of embodiment 7, wherein the AB comprises an IgG1 isotype.
9. The conjugated activatable antibody of embodiment 7 or 8, wherein the AB is an antibody having a heavy chain constant region, and wherein the C-terminal residue of the heavy chain constant region is not a lysine.
10. A conjugated activatable antibody comprising the structure of Formula (I) or a salt thereof: 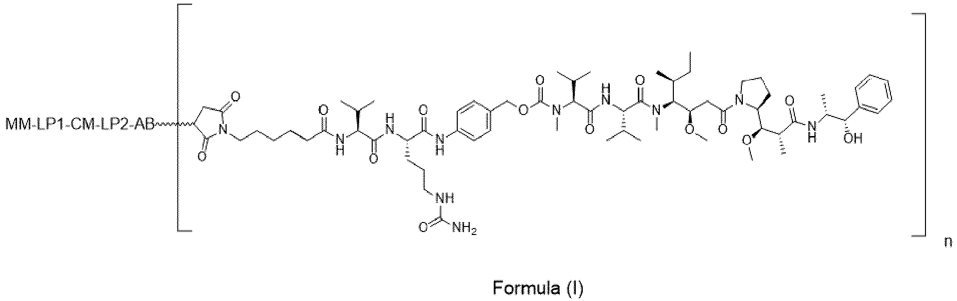
   wherein MM-LP1-CM-LP2-AB is an activatable antibody, wherein the AB is an antibody that specifically binds to human CD71,
   wherein the activatable antibody comprises a heavy chain comprising a sequence of SEQ ID NO: 167 and a light chain comprising a sequence of SEQ ID NO: 170, and
   wherein "n" is 2.
11. The conjugated activatable antibody of embodiment 10, wherein the AB comprises an IgG1 isotype.
12. The conjugated activatable antibody of embodiment 10 or 11, wherein the AB is an antibody having a heavy chain constant region, and wherein the C-terminal residue of the heavy chain constant region is not a lysine.
13. A conjugated activatable antibody comprising the structure of Formula (II) or a salt thereof: 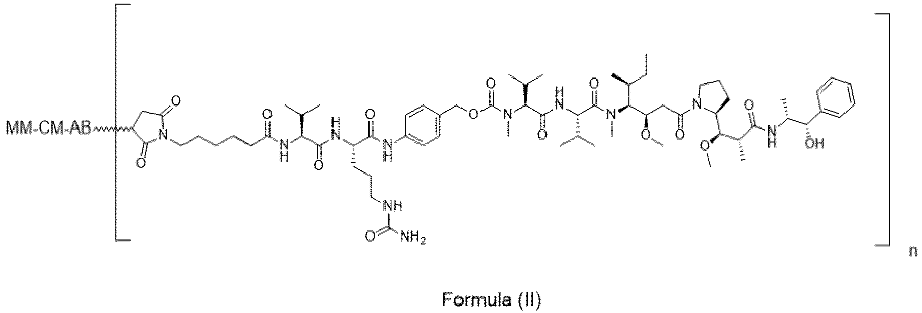
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and (b) wherein "n" is 2.
14. The conjugated activatable antibody of embodiment 13, wherein the AB comprises an IgG1 isotype.
15. The conjugated activatable antibody of embodiment 13 or 14, wherein the AB is an antibody having a heavy chain constant region, and wherein the C-terminal residue of the heavy chain constant region is not a lysine.
16. A conjugated activatable antibody comprising the structure of Formula (II) or a salt thereof: 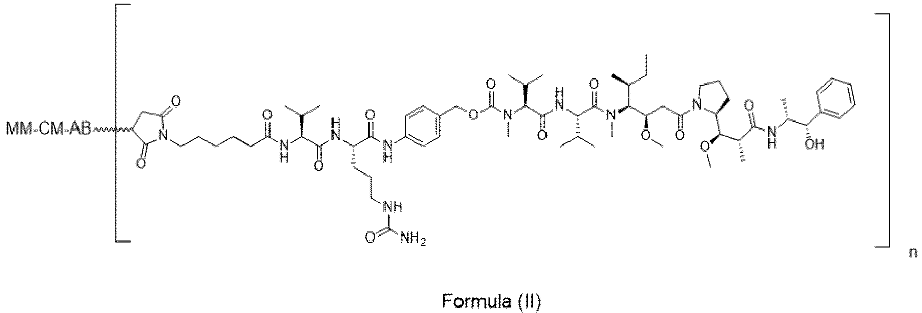
   (a) wherein
      (i) AB is an antibody that specifically binds to human CD71 and comprises a heavy chain variable region comprising a sequence of SEQ ID NO: 5 and a light chain variable region comprising a sequence of SEQ ID NO: 7;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and
   (b) wherein "n" is 2.
17. The conjugated activatable antibody of embodiment 16, wherein the AB comprises an IgG1 isotype.
18. The conjugated activatable antibody of embodiment 16 or 17, wherein the AB is an antibody having a heavy chain constant region, and wherein the C-terminal residue of the heavy chain constant region is not a lysine.
19. A conjugated activatable antibody comprising the structure of Formula (II) or a salt thereof: 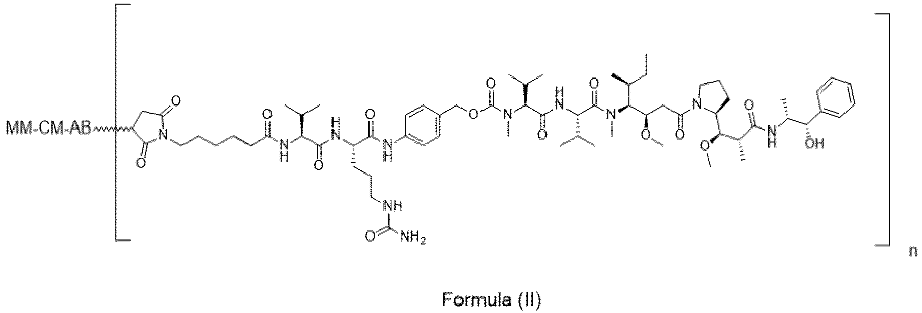
   (a) wherein
      (i) AB is an antibody that specifically binds to human CD71 and comprises a heavy chain comprising a sequence of SEQ ID NO: 167 and a light chain comprising a sequence of SEQ ID NO: 19;
      (ii) MM is a masking moiety comprising the amino acid sequence of SEQ ID NO: 18, wherein the MM inhibits the binding of the AB to human CD71 when the conjugated activatable antibody is in an uncleaved state; and
      (iii) CM is a cleavable moiety comprising the sequence of SEQ ID NO: 156, wherein the CM is a polypeptide that functions as a substrate for a protease; and
   (b) wherein "n" is 2.
20. The conjugated activatable antibody of embodiment 19, wherein the AB comprises an IgG1 isotype.
21. The conjugated activatable antibody of embodiment 19 or 20, wherein the AB is an antibody having a heavy chain constant region, and wherein the C-terminal residue of the heavy chain constant region is not a lysine.
22. A conjugated activatable antibody comprising the structure of Formula (II) or a salt thereof: 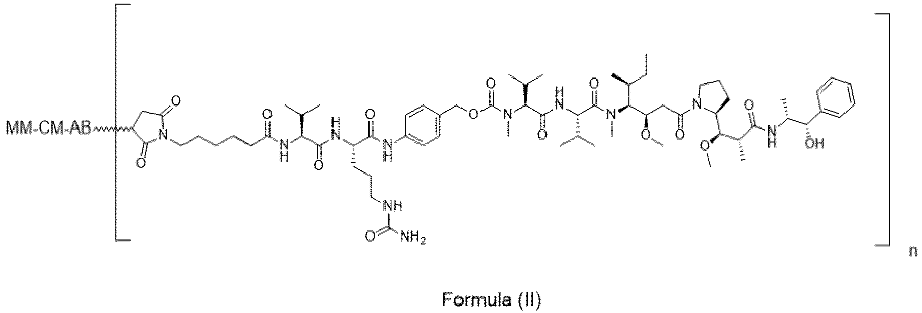
   wherein MM-CM-AB is an activatable antibody, wherein the AB is an antibody that specifically binds to human CD71,
   wherein the activatable antibody comprises a heavy chain comprising a sequence of SEQ ID NO: 167 and a light chain comprising a sequence of SEQ ID NO: 169, and
   wherein "n" is 2.
23. The conjugated activatable antibody of embodiment 22, wherein the AB comprises an IgG1 isotype.
24. The conjugated activatable antibody of embodiment 22 or 23, wherein the AB is an antibody having a heavy chain constant region, and wherein the C-terminal residue of the heavy chain constant region is not a lysine.
25. A method of manufacturing a conjugated activatable antibody comprising the structure of Formula (I) or a salt thereof: 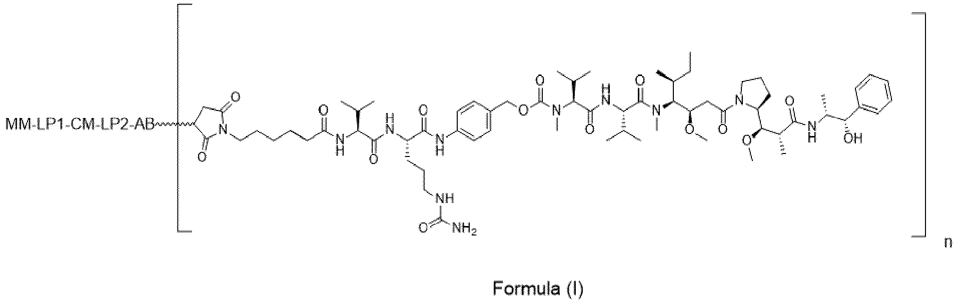
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (iii) LP1 is a first linking moiety comprising the amino acid sequence of SEQ ID NO: 207;
      (iv) CM is a cleavable moiety comprising the sequence of SEQ ID NO: 156, wherein the CM is a polypeptide that functions as a substrate for a protease; and
      (v) LP2 is a second linking moiety comprising the amino acid sequence of SEQ ID NO: 38;
         and wherein "n" is 2;
   (b) the method comprising
      (i) reducing an activatable antibody comprising MM-LP1-CM-LP2-AB with a reducing agent; and
      (ii) conjugating one or more vcMMAE to the reduced activatable antibody.
26. A method of manufacturing a conjugated activatable antibody comprising the structure of Formula (II) or a salt thereof: 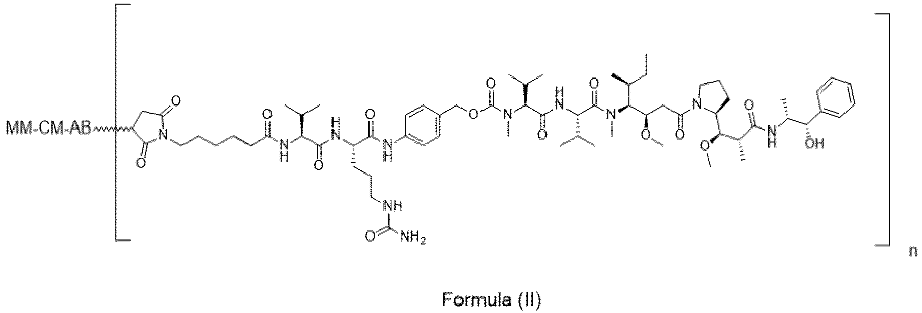
   (a) wherein
      (i) AB is an antibody that specifically binds to human CD71 and comprises
         i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
         ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
      (ii) MM is a masking moiety comprising the amino acid sequence of SEQ ID NO: 18, wherein the MM inhibits the binding of the AB to human CD71 when the conjugated activatable antibody is in an uncleaved state;
      (ii) CM is a cleavable moiety comprising the sequence of SEQ ID NO: 156, wherein the CM is a polypeptide that functions as a substrate for a protease; and
         and wherein "n" is 2;
   (b) the method comprising
      (i) reducing an activatable antibody comprising MM-CM-AB with a reducing agent; and
      (ii) conjugating one or more vcMMAE to the reduced activatable antibody.
27. A pharmaceutical composition comprising:
   the conjugated activatable antibody of any one of embodiments 1 to 24; and
   optionally a pharmaceutically acceptable carrier.
28. A method of treating, alleviating a symptom of, or delaying the progression of a cancer in a subject, the method comprising administering a therapeutically effective amount of the conjugated activatable antibody of any one of embodiments 1 to 24 or the pharmaceutical composition of embodiment 27 to a subject in need thereof for a cancer selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.
29. A conjugated activatable antibody of any one of embodiments 1 to 24 or a pharmaceutical composition of embodiment 27, for use as a medicament.
30. A conjugated activatable antibody of any one of embodiments 1 to 24 or a pharmaceutical composition of embodiment 27, for use in the treatment of cancer, optionally wherein the cancer is selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.

## Claims

1. A conjugated activatable antibody comprising the structure of Formula (I) or a salt thereof: 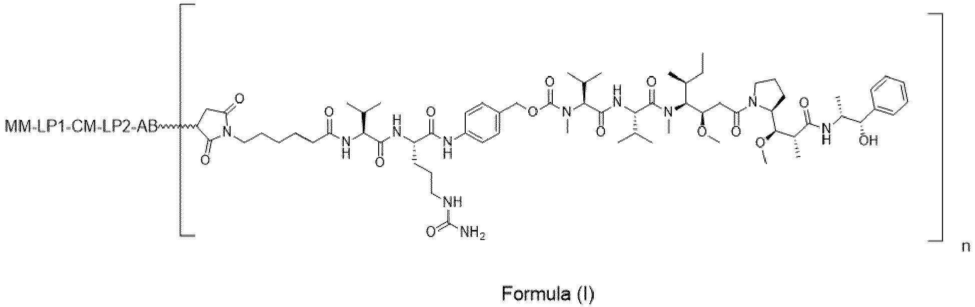
(a) wherein
(i) AB is an antibody that specifically binds to human CD71 and comprises
i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
(ii) MM is a masking moiety comprising the amino acid sequence of SEQ ID NO: 18, wherein the MM inhibits the binding of the AB to human CD71 when the conjugated activatable antibody is in an uncleaved state;
(iii) LP1 is a first linking moiety comprising the amino acid sequence of SEQ ID NO: 207;
(iv) CM is a cleavable moiety comprising the sequence of SEQ ID NO: 156, wherein the CM is a polypeptide that functions as a substrate for a protease; and
(v) LP2 is a second linking moiety comprising the amino acid sequence of SEQ ID NO: 38; and
(b) wherein "n" is 2.

2. The conjugated activatable antibody of claim 1, wherein the AB comprises an IgG1 isotype.

3. The conjugated activatable antibody of claim 1 or 2, wherein the AB is an antibody having a heavy chain constant region, and wherein the C-terminal residue of the heavy chain constant region is not a lysine.

4. The conjugated activatable antibody of any of claims 1-3, wherein the heavy chain variable region comprises a sequence of SEQ ID NO: 5 and the light chain variable region comprising a sequence of SEQ ID NO: 7.

5. The conjugated activatable antibody of any of claims 1-4, comprising a heavy chain comprising a sequence of SEQ ID NO: 167 and a light chain comprising a sequence of SEQ ID NO: 19.

6. A conjugated activatable antibody comprising the structure of Formula (II) or a salt thereof: 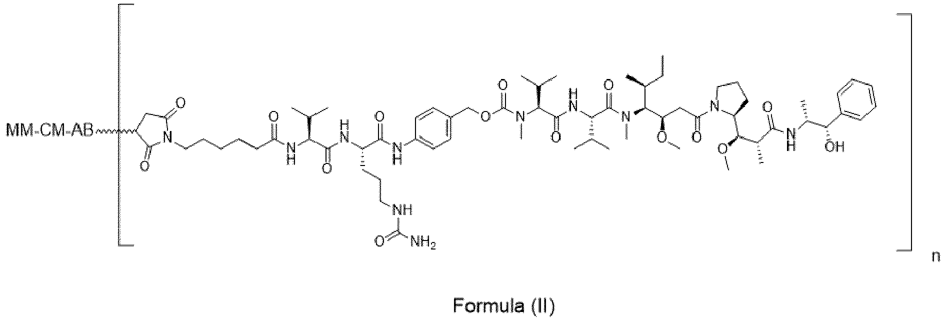
(a) wherein
(i) AB is an antibody that specifically binds to human CD71 and comprises
i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
(ii) MM is a masking moiety comprising the amino acid sequence of SEQ ID NO: 18, wherein the MM inhibits the binding of the AB to human CD71 when the conjugated activatable antibody is in an uncleaved state; and
(iii) CM is a cleavable moiety comprising the sequence of SEQ ID NO: 156, wherein the CM is a polypeptide that functions as a substrate for a protease; and
(b) wherein "n" is 2.

7. The conjugated activatable antibody of claim 6, wherein the AB comprises an IgG1 isotype.

8. The conjugated activatable antibody of claim 6 or 7, wherein the AB is an antibody having a heavy chain constant region, and wherein the C-terminal residue of the heavy chain constant region is not a lysine.

9. The conjugated activatable antibody of any of claims 6-8, wherein the heavy chain variable region comprises a sequence of SEQ ID NO: 5 and the light chain variable region comprises a sequence of SEQ ID NO: 7.

10. The conjugated activatable antibody of any of claims 6-9, comprising a heavy chain comprising a sequence of SEQ ID NO: 167 and a light chain comprising a sequence of SEQ ID NO: 19.

11. A method of manufacturing a conjugated activatable antibody comprising the structure of Formula (I) or a salt thereof: 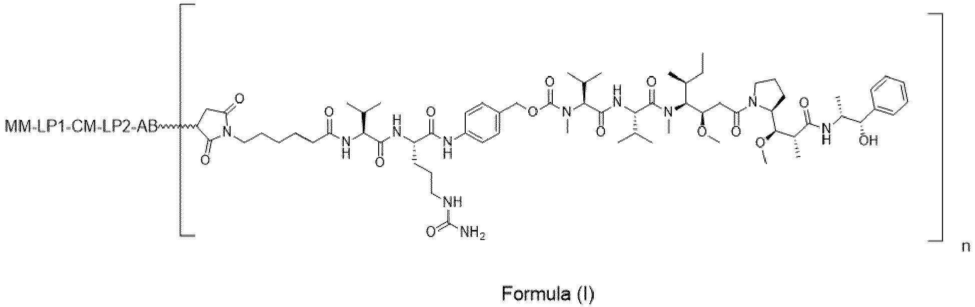
(a) wherein
(i) AB is an antibody that specifically binds to human CD71 and comprises
i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
(ii) MM is a masking moiety comprising the amino acid sequence of SEQ ID NO: 18, wherein the MM inhibits the binding of the AB to human CD71 when the conjugated activatable antibody is in an uncleaved state;
(iii) LP1 is a first linking moiety comprising the amino acid sequence of SEQ ID NO: 207;
(iv) CM is a cleavable moiety comprising the sequence of SEQ ID NO: 156, wherein the CM is a polypeptide that functions as a substrate for a protease; and
(v) LP2 is a second linking moiety comprising the amino acid sequence of SEQ ID NO: 38;
and wherein "n" is 2;
(b) the method comprising
(i) reducing an activatable antibody comprising MM-LP1-CM-LP2-AB with a reducing agent; and
(ii) conjugating one or more vcMMAE to the reduced activatable antibody.

12. A method of manufacturing a conjugated activatable antibody comprising the structure of Formula (II) or a salt thereof: 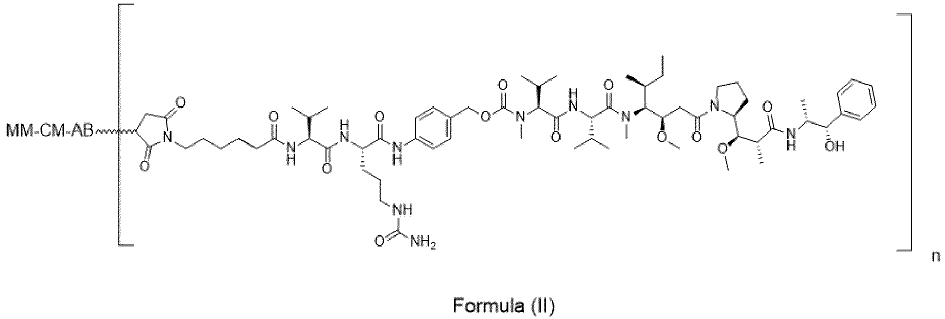
(a) wherein
(i) AB is an antibody that specifically binds to human CD71 and comprises
i. a heavy chain variable region comprising a CDRH1 sequence comprising SEQ ID NO: 9, a CDRH2 sequence comprising SEQ ID NO: 10, and a CDRH3 sequence comprising SEQ ID NO: 11; and
ii. a light chain variable region comprising a CDRL1 sequence comprising SEQ ID NO: 12 or SEQ ID NO:13, a CDRL2 sequence comprising SEQ ID NO: 14, and a CDRL3 sequence comprising SEQ ID NO: 15;
(ii) MM is a masking moiety comprising the amino acid sequence of SEQ ID NO: 18, wherein the MM inhibits the binding of the AB to human CD71 when the conjugated activatable antibody is in an uncleaved state;
(ii) CM is a cleavable moiety comprising the sequence of SEQ ID NO: 156, wherein the CM is a polypeptide that functions as a substrate for a protease; and
and wherein "n" is 2;
(b) the method comprising
(i) reducing an activatable antibody comprising MM-CM-AB with a reducing agent; and
(ii) conjugating one or more vcMMAE to the reduced activatable antibody.

13. A pharmaceutical composition comprising:
the conjugated activatable antibody of any one of claims 1 to 10; and
optionally a pharmaceutically acceptable carrier.

14. The conjugated activatable antibody of any one of claims 1 to 10 or the pharmaceutical composition of claim 13, for use as a medicament.

15. The conjugated activatable antibody of any one of claims 1 to 10 or the pharmaceutical composition of claim 13, for use in the treatment of cancer, optionally wherein the cancer is selected from the group consisting of: gastric cancer, ovarian cancer, esophageal cancer, non-small cell lung cancer, ER+ breast cancer, triple-negative breast cancer, colorectal cancer, melanoma, prostate cancer, multiple myeloma, diffuse large B-cell lymphoma, head and neck small cell carcinoma, pancreatic cancer, mesothelioma, non-Hodgkin's lymphoma, hepatocellular carcinoma, and glioblastoma.
